(19) Europäisches Patentamt
European Patent Office
Office européen des brevets

(11) EP 3 505 171 A1

(12) EUROPEAN PATENT APPLICATION
published in accordance with Art. 153(4) EPC

(43) Date of publication:
**03.07.2019 Bulletin 2019/27**

(21) Application number: **17843741.4**

(22) Date of filing: **25.08.2017**

(51) Int Cl.:
***A61K 31/437*** *(2006.01)* ***A61K 31/4985*** *(2006.01)*
***A61K 31/519*** *(2006.01)* ***A61K 31/52*** *(2006.01)*
***A61K 31/53*** *(2006.01)* ***A61K 31/55*** *(2006.01)*

(86) International application number:
**PCT/JP2017/030609**

(87) International publication number:
**WO 2018/038265 (01.03.2018 Gazette 2018/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **26.08.2016 JP 2016166277**

(71) Applicants:
- **Mitsubishi Tanabe Pharma Corporation**
**Osaka 541-8505 (JP)**
- **UBE Industries, Ltd.**
**Ube-shi, Yamaguchi 755-8633 (JP)**

(72) Inventors:
- **NAKAJIMA, Tatsuo**
**Osaka-shi**
**Osaka 541-8505 (JP)**
- **HAYASHI, Norimitsu**
**Osaka-shi**
**Osaka 541-8505 (JP)**
- **ISHIZAWA, Kouhei**
**Osaka-shi**
**Osaka 541-8505 (JP)**
- **TSUZAKI, Yasunori**
**Ube-shi**
**Yamaguchi 755-8633 (JP)**
- **IWAMURA, Ryo**
**Ube-shi**
**Yamaguchi 755-8633 (JP)**
- **TSUBOIKE, Kazunari**
**Ube-shi**
**Yamaguchi 755-8633 (JP)**

(74) Representative: **Hoffmann Eitle**
**Patent- und Rechtsanwälte PartmbB**
**Arabellastraße 30**
**81925 München (DE)**

# (54) BICYCLIC NITROGENATED HETEROCYCLIC COMPOUND

(57) The present invention provides: a novel use of a specific bicyclic nitrogen-containing heterocyclic compound as a PDE7 inhibitor; a novel bicyclic nitrogen-containing heterocyclic compound having a PDE7 inhibitory effect, a method for producing the compound, a use of the compound, and a pharmaceutical composition containing the PDE7 inhibitor or the compound; and others. More specifically, the present invention provides a PDE7 inhibitor containing the compound represented by the formula (I):

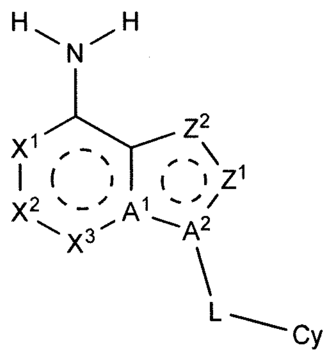

(I)

[wherein the symbols have the same meanings as those described in the description] or a pharmaceutically acceptable salt thereof as an active ingredient.

EP 3 505 171 A1

## Description

TECHNICAL FIELD

The present invention relates to novel PDE7 inhibitors having excellent PDE7 inhibitory effects and novel bicyclic nitrogen-containing heterocyclic compounds which could be used as active ingredients of the inhibitors.

BACKGROUND ART

Dependence is a condition characterized in that a person is dependent on certain things, and cannot keep physical and/or mental normality without said things. Dependence on a substance such as alcohol dependence and nicotine dependence, dependence on an action such as gambling dependence and internet dependence, and the others are known. It is believed that a dopamine nervous system projecting from a ventral tegmental area to a nucleus accumbens in a brain called reward system is involved in the formation of dependence. The reward system is involved not only in alcohol dependence, but also in the development of a wide range of dependence, for example dependence on an addictive drug such as cocaine and morphine.

In the treatment of alcohol dependence, a control of the amount of alcohol intake by the reduction of the amount of alcohol intake or the abstinence from alcohol is required. While social supports to a patient by the family, societies for the abstinence from alcohol, and the like are used in order to maintain the treatment, a drug therapy to reduce the amount of alcohol intake may also be used in combination. Examples of the drug to be used in the drug therapy include antialcoholic drugs such as disulfiram and cyanamide which inhibit the function of the acetaldehyde-metabolizing enzymes in a liver and cause discomfort when drinking alcohol; a NMDA receptor antagonist, acamprosate which acts on a central nervous system and suppresses the appetite for drinking alcohol; and μ opioid antagonists such as naltrexone and nalmefene. Although these drug therapies achieve a certain degree of effects, they do not sufficiently meet medical needs especially for patients who need to maintain the abstinence from alcohol. It is believed that the patients with alcohol dependence who cannot keep the abstinence would drink alcohol again when they receive stress during the abstinence or stimulus which evokes drinking alcohol. Regarding the therapeutic drugs which are currently clinically used, there is no sufficient evidence for effectiveness against stress stimulus (Nonpatent Document 1), and thus it is believed that a drug which suppresses drinking alcohol again caused by stress stimulus would contribute considerably to the treatment of alcohol dependence. Also, there is no drug therapy so far which achieves a sufficient effect on addictive drugs such as cocaine, and thus the development of such therapeutic drug is desired.

Phosphodiesterase (PDE) is an enzyme which hydrolyzes the cyclic nucleotides, cyclic adenosine monophosphate (cAMP) and cyclic guanosine monophosphate (cGMP) which are intracellular transmitters, and controls the amounts of these molecules which serve as intracellular second messengers (Nonpatent Document 2). There are PDE1 to 11 families, and PDE degrades either cAMP or cGMP, or both of them. Among them, it is known that PDE7 does not act on cGMP, and selectively hydrolyzes cAMP. Also, some PDE families have further subdivided isozymes, and PDE7 has two types of isozymes, PDE7A and PDE7B.

It is reported that PDE7 is expressed in vivo in a brain (especially highly expressed in a putamen, a caudate nucleus, and the like), a heart, a skeletal muscle, a pancreas, an immunological cell, and the like (Nonpatent Document 3). It is believed that PDE7 present in a brain controls the signal transduction by cAMP in the several parts of the brain. For example, it is reported that PDE7 coexists with cells having dopamine receptors in nucleus accumbens at a high rate (Nonpatent Document 4), and thus it is shown that PDE7 may control the function of reward system. Also, Patent Document 1 discloses that a PDE7 inhibitor suppresses the neural activity of reward system caused by nicotine stimulation. Further, it is shown that intake of addictive drugs such as nicotine and cocaine is suppressed in an animal to which a PDE7 inhibitor is preliminarily systemically administered. Accordingly, it is believed that a PDE7 inhibitor which controls the function of reward system is useful for the treatment of dependence.

Examples of other diseases which are expected to be improved by inhibiting PDE7 include glioblastoma. Glioblastoma is one of brain tumors and a malignant disease among the brain tumors, and a treatment method for significantly improving the survival rate of patients with glioblastoma has not been established. A recent article reports that the prognosis of patients with glioblastoma correlates with the expression level of PDE7B. Thus, a PDE7 inhibitor is also expected to have therapeutic and survival benefits on patients with glioblastoma (Nonpatent Document 5). Examples of other diseases which are expected to be prevented or treated by inhibiting PDE7 include gambling dependence, internet dependence, overuse of an electronic device, overuse of a game device, sex dependence, bulimia, and binge eating disorder.

Meanwhile, the enzyme inhibition against PDE1 to 6 and PDE8 to 11 may cause different clinical and pharmacological effects from the enzyme inhibition against PDE7. For example, it is reported that the enzyme inhibition against PDE4 could cause vomiting (Nonpatent Document 6), and the enzyme inhibition against PDE10 could cause dystonia (Nonpatent Document 7). Accordingly, it is desired to develop a PDE7 inhibitor which is PDE7-selective as compared

to PDE1 to 6 and PDE8 to 11 for use in the treatment of the above diseases including dependence.

Patent Document 1 discloses that a compound of the following structure has a PDE7 inhibitory activity, and thus said compound may be used in the treatment of dependence. However, said compound has a different structure from the compound of the present invention.

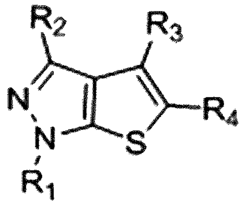

Also, Patent Document 2 discloses that the compound of the following structure has a PDE inhibitory activity, and said compound may be used in the treatment of psoriasis. However, said compound has a different structure from the compound of the present invention in that, for example, X in said structure is a halogen atom or a di(lower alkyl)amino group. Further, Patent Document 2 does not disclose that said compound has a PDE7-selective inhibitory activity.

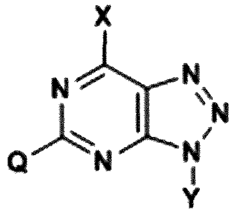

CITATION LIST

PATENT DOCUMENT

Patent Document 1: WO 2013/176877 pamphlet
Patent Document 2: JP S52-122395 A

NONPATENT DOCUMENT

Nonpatent Document 1: Neuropsychopharmacology, 2011, vol. 36, p. 1178-1186
Nonpatent Document 2: Seisan to Gijutu (Manufacturing & Technology), 2014, vol. 66, No. 2, p. 80-83
Nonpatent Document 3: Biochemical and Biophysical Research Communication), 2000, 271, p. 575-583
Nonpatent Document 4: Brain Research, 2010, vol. 1310, p. 37-45
Nonpatent Document 5: PLOS ONE, 2014, vol. 9, ISSUE 9, e0107397
Nonpatent Document 6: British Journal of Pharmacology, 2008, vol. 155, p. 308-315
Nonpatent Document 7: Neuropharmacology, 2014, vol. 77, p. 257-267

SUMMARY OF THE INVENTION

PROBLEMS TO BE SOLVED BY INVENTION

The object of the present invention is to provide novel use of specific bicyclic nitrogen-containing heterocyclic compounds as PDE7 inhibitors, novel bicyclic nitrogen-containing heterocyclic compounds having PDE7 inhibitory effects, methods for producing the compounds, use of the compounds, and pharmaceutical compositions comprising the above PDE7 inhibitors or compounds, or methods for treating or preventing diseases associated with PDE7 using them.

MEANS TO SOLVE PROBLEMS

The present inventors have earnestly studied to solve the above problems, as a result thereof found that specific bicyclic nitrogen-containing heterocyclic compounds may achieve a desired object, and finally completed the present invention.

The present invention relates to a compound of the following formula (I) (hereinafter also referred to as "Compound (I)") or a pharmaceutically acceptable salt thereof, and use thereof. Also, the present invention relates to a method for treating or preventing various diseases (for example, drug dependence) associated with PDE7 comprising administering an effective amount of the following Compound (I) or a pharmaceutically acceptable salt thereof to a patient. Also, the present invention relates to a pharmaceutical composition comprising the following Compound (I) or a pharmaceutically acceptable salt thereof as an active ingredient and use of the Compound (I) or a pharmaceutically acceptable salt thereof in the manufacture of the pharmaceutical composition. Further, the present invention relates to a method for producing the following Compound (I) or a pharmaceutically acceptable salt thereof.

The present invention includes the following specific aspects.

[1] A PDE7 inhibitor comprising a compound represented by the formula (I):

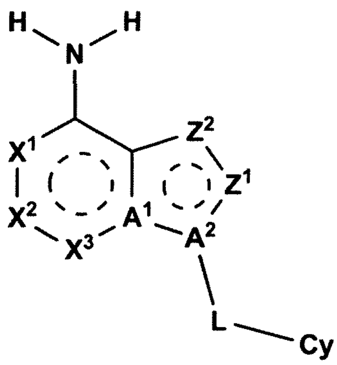

(I)

[wherein:
the partial structure represented by the following formula (I-1):

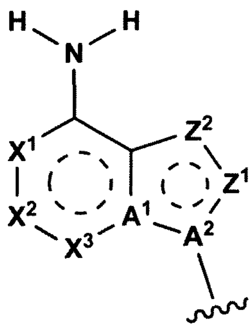

(I-1)

represents a partial structure selected from the group consisting of
the following formula (I-1-A):

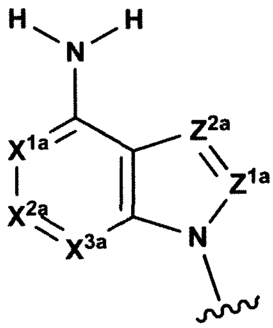

(I-1-A)

(wherein $X^{1a}$ is $CR^{X1a}$ or N; $X^{2a}$ is $CR^{X2a}$ or N; $X^{3a}$ is $CR^{X3a}$ or N; one or two of $X^{1a}$, $X^{2a}$, and $X^{3a}$ is/are N; $Z^{1a}$ is $CR^{Z1a}$ or N; and $Z^{2a}$ is $CR^{Z2a}$ or N);
the following formula (I-1-B):

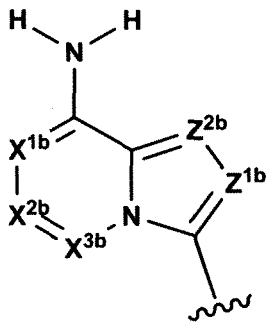

(I-1-B)

(wherein $X^{1b}$ is $CR^{X1b}$ or N; $X^{2b}$ is $CR^{X2b}$ or N; $X^{3b}$ is $CR^{X3b}$ or N; zero, one, or two of $X^{1b}$, $X^{2b}$, and $X^{3b}$ is/are N; $Z^{1b}$ is $CR^{Z1b}$ or N; and $Z^{2b}$ is $CR^{Z2b}$ or N);
the following formula (I-1-C):

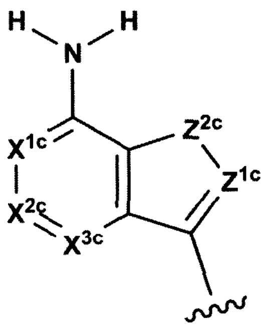

(I-1-C)

(wherein $X^{1c}$ is $CR^{X1c}$ or N; $X^{2c}$ is $CR^{X2c}$ or N; $X^{3c}$ is $CR^{X3c}$ or N; one or two of $X^{1c}$, $X^{2c}$, and $X^{3c}$ is/are N; $Z^{1c}$ is $CR^{Z1c}$ or N; and $Z^{2c}$ is $NR^{Z2c}$ or O); and
the following formula (I-1-D):

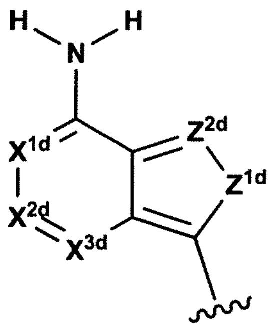

(I-1-D)

(wherein $X^{1d}$ is $CR^{X1d}$ or N; $X^{2d}$ is $CR^{X2d}$ or N; $X^{3d}$ is $CR^{X3a}$ or N; one or two of $X^{1d}$, $X^{2d}$, and $X^{3d}$ is/are N; $Z^{1d}$ is $NR^{Z1d}$ or O; and $Z^{2d}$ is $CR^{Z2d}$ or N);
$R^{X1a}$, $R^{X1b}$, $R^{X1C}$, and $R^{X1d}$ each independently represent a hydrogen atom, an optionally substituted alkyl group, or a halogen atom;
$R^{X2a}$, $R^{X2b}$, $R^{X2c}$, and $R^{X2d}$ each independently represent a hydrogen atom, an optionally substituted alkyl group, an optionally substituted alkoxy group, or an optionally substituted alkylthio group;
$R^{X3a}$, $R^{X3b}$, $R^{X3c}$, and $R^{X3d}$ each independently represent a hydrogen atom, an optionally substituted alkyl group, an optionally substituted cycloalkyl group, a halogen atom, a cyano group, or an optionally substituted aryl group;
$R^{Z1a}$, $R^{Z1b}$, and $R^{Z1c}$ each independently represent a hydrogen atom, a hydroxy group, or an optionally substituted alkyl group;
$R^{Z1d}$ represents a hydrogen atom or an optionally substituted alkyl group;
$R^{Z2a}$, $R^{Z2b}$, and $R^{Z2d}$ each independently represent a hydrogen atom, an optionally substituted alkyl group, an optionally substituted cycloalkyl group, or a halogen atom;
$R^{Z2c}$ represents a hydrogen atom or an optionally substituted alkyl group;
L represents a single bond or $CR^{L1}R^{L2}$;
$R^{L1}$ and $R^{L2}$ each independently represent a hydrogen atom or an optionally substituted alkyl group, or $R^{L1}$ and $R^{L2}$ each independently represent an alkylene group and are combined with each other together with the carbon atom to which they are attached to form an optionally substituted monocyclic saturated hydrocarbon group; and Cy represents

(i) an aryl group optionally substituted with the same or different 1 to 5 substituent(s) selected from

an optionally substituted alkyl group;
an optionally substituted alkoxy group;
a halogen atom; and
an optionally substituted carboxamide group;

(ii) a heteroaryl group optionally substituted with the same or different 1 to 5 substituent(s) selected from an optionally substituted alkyl group and a halogen atom;
(iii) an alicyclic hydrocarbon group optionally substituted with the same or different 1 to 5 substituent(s) selected from
an optionally substituted alkyl group;
an optionally substituted alkenyl group;
an optionally substituted alkylidene group;
an optionally substituted alkoxy group;
a hydroxy group;
a halogen atom;
an oxo group;
an optionally substituted aryl group; and
an optionally substituted heteroaryl group; or
(iv) a nonaromatic heterocyclic group optionally substituted with the same or different 1 to 5 substituent(s) selected from
an optionally substituted alkyl group;
an optionally substituted cycloalkyl group;
an optionally substituted alkoxy group;

a hydroxy group;
a halogen atom;
an oxo group;
an optionally substituted aryl group;
an optionally substituted heteroaryl group;
an optionally substituted alkylcarbonyl group;
a formyl group;
an optionally substituted alkoxycarbonyl group; and
an optionally substituted arylcarbonyl group]

or a pharmaceutically acceptable salt thereof as an active ingredient.
[2] The PDE7 inhibitor according to [1], wherein
$R^{X1a}$, $R^{X1b}$, $R^{X1C}$, and $R^{X1d}$ each independently represent a hydrogen atom, an alkyl group optionally substituted with the same or different 1 to 7 halogen atom(s), or a halogen atom;
$R^{X2a}$, $R^{X2b}$, $R^{X2c}$, and $R^{X2d}$ each independently represent a hydrogen atom, an alkyl group optionally substituted with the same or different 1 to 7 halogen atom(s), an alkoxy group optionally substituted with the same or different 1 to 7 halogen atom(s), or an alkylthio group optionally substituted with the same or different 1 to 7 halogen atom(s);
$R^{X3a}$, $R^{X3b}$, $R^{X3c}$, and $R^{X3d}$ each independently represent a hydrogen atom, an alkyl group optionally substituted with the same or different 1 to 7 halogen atom(s), a cycloalkyl group optionally substituted with the same or different 1 to 5 halogen atom(s), a halogen atom, a cyano group, or an aryl group optionally substituted with the same or different 1 to 5 halogen atom(s);
$R^{Z1a}$, $R^{Z1b}$, and $R^{Z1c}$ each independently represent a hydrogen atom, a hydroxy group, or an alkyl group optionally substituted with the same or different 1 to 7 halogen atom(s);
$R^{Z1d}$ represents a hydrogen atom or an alkyl group optionally substituted with the same or different 1 to 5 halogen atom(s);
$R^{Z2a}$, $R^{Z2b}$, and $R^{Z2d}$ each independently represent a hydrogen atom, an alkyl group optionally substituted with the same or different 1 to 7 halogen atom(s), a cycloalkyl group optionally substituted with the same or different 1 to 5 halogen atom(s), or a halogen atom;
$R^{Z2c}$ represents a hydrogen atom or an alkyl group optionally substituted with the same or different 1 to 5 halogen atom(s);
L represents a single bond or $CR^{L1}R^{L2}$;
$R^{L1}$ and $R^{L2}$ each independently represent a hydrogen atom or an alkyl group optionally substituted with the same or different 1 to 7 halogen atom(s), or $R^{L1}$ and $R^{L2}$ each independently represent a straight alkylene group and are combined with each other together with the carbon atom to which they are attached to form a monocyclic saturated hydrocarbon group optionally substituted with the same or different 1 to 6 halogen atom(s); and
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s);
(ii) a heteroaryl group optionally substituted with the same or different 1 to 5 substituent(s) selected from an alkyl group optionally substituted with the same or different 1 to 7 halogen atom(s) and a halogen atom;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group optionally substituted with the same or different 1, 2, or 3 substituent(s) selected from an alkyl group optionally substituted with the same or different 1 to 7 halogen atom(s) and a halogen atom;
an alkenyl group optionally substituted with the same or different 1 to 5 halogen atom(s);
an alkylidene group optionally substituted with the same or different 1 to 6 halogen atom(s);
an alkoxy group optionally substituted with the same or different 1 to 7 halogen atom(s);
a hydroxy group;
a halogen atom;
an oxo group;

an aryl group optionally substituted with the same or different 1 to 5 halogen atom(s); and
a heteroaryl group optionally substituted with the same or different 1, 2, or 3 substituent(s) selected from an alkyl group optionally substituted with the same or different 1 to 7 halogen atom(s) and a halogen atom; or (iv) a nonaromatic heterocyclic group optionally substituted with the same or different 1 to 5 substituent(s) selected from
an alkyl group optionally substituted with the same or different 1, 2, or 3 substituent(s) selected from an alkoxy group optionally substituted with the same or different 1 to 7 halogen atom(s), a halogen atom, and an aryl group optionally substituted with the same or different 1, 2, or 3 substituent(s) selected from an alkyl group optionally substituted with the same or different 1 to 7 halogen atom(s) and a halogen atom;
a cycloalkyl group optionally substituted with the same or different 1 to 5 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s);
a heteroaryl group optionally substituted with the same or different 1 to 5 halogen atom(s);
an alkylcarbonyl group optionally substituted with the same or different 1, 2, or 3 aryl group(s);
a formyl group;
an alkoxycarbonyl group optionally substituted with the same or different 1 to 7 halogen atom(s); and
an arylcarbonyl group optionally substituted with the same or different 1 to 5 halogen atom(s).

[3] The PDE7 inhibitor according to [1] or [2], wherein
$R^{X1a}$, $R^{X1b}$, $R^{X1C}$, and $R^{X1d}$ each represent a hydrogen atom;
$R^{X2a}$, $R^{X2b}$, $R^{X2c}$, and $R^{X2d}$ each independently represent a hydrogen atom, an alkyl group optionally substituted with the same or different 1 to 7 halogen atom(s), an alkoxy group, or an alkylthio group;
$R^{X3a}$, $R^{X3b}$, $R^{X3c}$, and $R^{X3d}$ each independently represent a hydrogen atom, an alkyl group optionally substituted with the same or different 1 to 7 halogen atom(s), a cycloalkyl group, a halogen atom, a cyano group, or an aryl group;
$R^{Z1a}$, $R^{Z1b}$, and $R^{Z1c}$ each independently represent a hydrogen atom, a hydroxy group, or an alkyl group;
$R^{Z1d}$ represents an alkyl group;
$R^{Z2a}$, $R^{Z2b}$, and $R^{Z2d}$ each independently represent a hydrogen atom, an alkyl group optionally substituted with the same or different 1 to 7 halogen atom(s), a cycloalkyl group, or a halogen atom;
$R^{Z2c}$ represents an alkyl group;
L represents a single bond or $CR^{L1}R^{L2}$;
$R^{L1}$ and $R^{L2}$ each independently represent a hydrogen atom or an alkyl group, or $R^{L1}$ and $R^{L2}$ each independently represent a straight alkylene group and are combined with each other together with the carbon atom to which they are attached to form a monocyclic saturated hydrocarbon group; and
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s);
(ii) a heteroaryl group optionally substituted with the same or different 1 to 5 halogen atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group;
an alkenyl group;
an alkylidene group;
an alkoxy group;
a hydroxy group;
a halogen atom;
an oxo group;
an aryl group; and
a heteroaryl group optionally substituted with the same or different 1, 2, or 3 alkyl group(s); or

(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a cycloalkyl group;
a halogen atom;
an oxo group;
an aryl group;
a heteroaryl group;
an alkylcarbonyl group optionally substituted with the same or different 1, 2, or 3 aryl group(s);
a formyl group; and
an alkoxycarbonyl group.

[4] The PDE7 inhibitor according to any one of [1] to [3], wherein
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a heteroaryl group optionally substituted with the same or different 1 to 5 halogen atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s); or (iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group.

[5] The PDE7 inhibitor according to any one of [1] to [4], wherein
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a 6 to 11 membered monocyclic or bicyclic aromatic hydrocarbon group;
(ii) a heteroaryl group optionally substituted with the same or different 1 to 5 halogen atom(s), wherein said heteroaryl group is a 5 to 11 membered monocyclic or bicyclic aromatic heterocyclic group comprising 1 to 4 heteroatom(s) selected from an oxygen atom, a sulfur atom, and a nitrogen atom other than carbon atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;

an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a $C_3$-$C_8$ cycloalkyl group, a $C_6$-$C_{12}$ bicycloalkyl group, a $C_6$-$C_{12}$ bicycloalkenyl group, a $C_6$-$C_{12}$ spiroalkyl group, or a $C_{10}$-$C_{14}$ tricyclic tricycloalkyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a 4 to 8 membered monocyclic nonaromatic heterocyclic group or a 6 to 12 membered bicyclic nonaromatic heterocyclic group.

[6] The PDE7 inhibitor according to any one of [1] to [5], wherein $X^{1a}$, $X^{1b}$, $X^{1c}$, and $X^{1d}$ each represent N.
[7] The PDE7 inhibitor according to any one of [1] to [6], wherein
$Z^{1a}$, $Z^{1b}$, and $Z^{1c}$ each represent N; and
$Z^{1d}$ represents $NR^{Z1d}$.
[8] The PDE7 inhibitor according to any one of [1] to [7], wherein
$Z^{2a}$, $Z^{2b}$, and $Z^{2d}$ each represent N; and
$Z^{2c}$ represents $NR^{Z2c}$.
[9] The PDE7 inhibitor according to any one of [1] to [8], wherein $X^{3a}$, $X^{3b}$, $X^{3c}$, and $X^{3d}$ each represent N.
[10] The PDE7 inhibitor according to any one of [1] to [9], wherein L represents a single bond.
[11] The PDE7 inhibitor according to any one of [1] to [10], wherein the formula (I-1) is the formula (I-1-A).
[12] The PDE7 inhibitor according to any one of [1] to [10], wherein the formula (I-1) is the formula (I-1-B).
[13] The PDE7 inhibitor according to any one of [1] to [10], wherein the formula (I-1) is the formula (I-1-C).
[14] The PDE7 inhibitor according to any one of [1] to [10], wherein the formula (I-1) is the formula (I-1-D).
[15] A compound represented by the following formula (II):

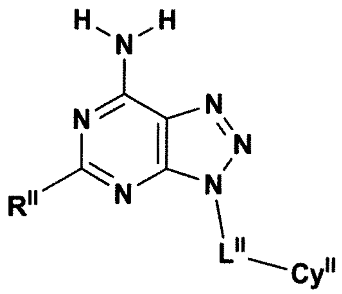

(II)

[wherein:

$R^{II}$ represents a hydrogen atom, an alkyl group optionally substituted with the same or different 1 to 7 halogen atom(s), an alkoxy group optionally substituted with the same or different 1 to 7 halogen atom(s), or an alkylthio group optionally substituted with the same or different 1 to 7 halogen atom(s);
$L^{II}$ represents a single bond or $CR^{LII-1}R^{LII-2}$;
$R^{LII-1}$ and $R^{LII-2}$ each independently represent a hydrogen atom or an alkyl group optionally substituted with the same or different 1 to 7 halogen atom(s), or $R^{LII-1}$ and $R^{LII-2}$ each independently represent an alkylene group and are combined with each other together with the carbon atom to which they are attached to form a monocyclic saturated hydrocarbon group optionally substituted with the same or different 1 to 6 halogen atom(s); and
$Cy^{II}$ represents

(i) an aryl group optionally substituted with the same or different 1 to 5 substituent(s) selected from

an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s)
(provided that said aryl group is not a phenyl group);
(ii) a heteroaryl group optionally substituted with the same or different 1 to 5 substituent(s) selected from an alkyl group optionally substituted with the same or different 1 to 7 halogen atom(s) and a halogen atom (provided that said heteroaryl group is not a furyl group);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group optionally substituted with the same or different 1, 2, or 3 substituent(s) selected from an alkyl group optionally substituted with the same or different 1 to 7 halogen atom(s) and a halogen atom;
an alkenyl group optionally substituted with the same or different 1 to 5 halogen atom(s);
an alkylidene group optionally substituted with the same or different 1 to 6 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s); and
a heteroaryl group optionally substituted with the same or different 1, 2, or 3 substituent(s) selected from an alkyl group optionally substituted with the same or different 1 to 7 halogen atom(s) and a halogen atom
(provided that said alicyclic hydrocarbon group is not a cyclobutyl group, a cyclopentyl group, a cyclopentenyl group, or a 2-cyclohexenyl group); or
(iv) a nonaromatic heterocyclic group optionally substituted with the same or different 1 to 5 substituent(s) selected from
an alkyl group optionally substituted with the same or different 1, 2, or 3 substituent(s) selected from an alkoxy group optionally substituted with the same or different 1 to 7 halogen atom(s), a halogen atom, and an aryl group optionally substituted with the same or different 1, 2, or 3 substituent(s) selected from an alkyl group optionally substituted with the same or different 1 to 7 halogen atom(s) and a halogen atom;
a cycloalkyl group optionally substituted with the same or different 1 to 5 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s);
a heteroaryl group optionally substituted with the same or different 1 to 5 halogen atom(s);
an alkylcarbonyl group optionally substituted with the same or different 1, 2, or 3 aryl group(s);
a formyl group;
an alkoxycarbonyl group optionally substituted with the same or different 1 to 7 halogen atom(s); and
an arylcarbonyl group optionally substituted with the same or different 1 to 5 halogen atom(s)
(provided that said nonaromatic heterocyclic group is not a tetrahydrofuryl group, a dihydrofuran-2-yl group, a tetrahydropyran-2-yl group, a pyrrolidin-3-yl group, a morpholin-2-yl group, or a thiolan-2-yl group)
(provided that

(a) $Cy^{II}$ is not a cyclopropyl group or a 2,2-dimethyl-1,3-dioxolanyl group; and
(b) the above compound is not 3-cyclohexyl-3H-[1,2,3]triazolo[4,5-d]pyrimidin-7-amine, 2-[(7-amino-3H-1,2,3-triazolo[4,5-d]pyrimidin-3-yl)methyl]-1-azabicyclo[2.2.2]octan-3-one, 2-(7-amino-3H-1,2,3-triazolo[4,5-d]pyrimidin-3-yl)cyclohexanemethanol, or 4-(7-amino-3H-1,2,3-triazolo[4,5-d]pyrimidin-3-yl)-2-hydroxybicyclo[3.1.0]hexane-1-methanol)]

or a pharmaceutically acceptable salt thereof.
[16] The compound according to [15] or a pharmaceutically acceptable salt thereof, wherein
$R^{II}$ represents a hydrogen atom, an alkyl group optionally substituted with the same or different 1 to 7 halogen atom(s), an alkoxy group, or an alkylthio group;

$L^{II}$ represents a single bond or $CR^{LII-1}R^{LII-2}$;
$R^{LII-1}$ and $R^{LII-2}$ each independently represent a hydrogen atom or an alkyl group, or $R^{LII-1}$ and $R^{LII-2}$ each independently represent a straight alkylene group and are combined with each other together with the carbon atom to which they are attached to form a monocyclic saturated hydrocarbon group; and
$Cy^{II}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a 6 to 11 membered monocyclic or bicyclic aromatic hydrocarbon group;
(ii) a heteroaryl group optionally substituted with the same or different 1 to 5 halogen atom(s), wherein said heteroaryl group is a 5 to 11 membered monocyclic or bicyclic aromatic heterocyclic group comprising 1 to 4 heteroatom(s) selected from an oxygen atom, a sulfur atom, and a nitrogen atom other than carbon atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a $C_3$-$C_8$ cycloalkyl group, a $C_6$-$C_{12}$ bicycloalkyl group, a $C_6$-$C_{12}$ bicycloalkenyl group, a $C_6$-$C_{12}$ spiroalkyl group, or a $C_{10}$-$C_{14}$ tricyclic tricycloalkyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a 4 to 8 membered monocyclic nonaromatic heterocyclic group or a 6 to 12 membered bicyclic nonaromatic heterocyclic group.

[17] The compound according to [15] or [16] or a pharmaceutically acceptable salt thereof, wherein
$L^{II}$ represents a single bond; and
$Cy^{II}$ represents

(i) a naphthyl group or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;

a hydroxy group;
a halogen atom; and
a heteroaryl group optionally substituted with the same or different 1, 2, or 3 alkyl group(s),
wherein said alicyclic hydrocarbon group is a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a piperidinyl group, a piperidino group, a perhydroazepinyl group, a perhydroazocinyl group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

[18] A compound represented by the following formula (III):

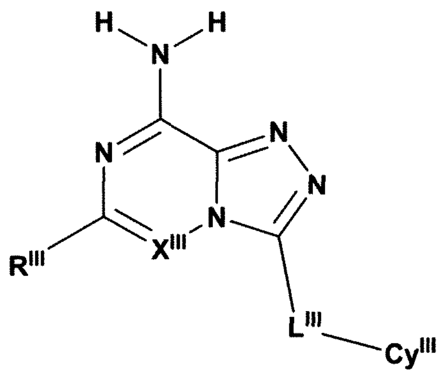

(III)

[wherein:

$X^{III}$ is $CR^{XIII}$ or N;
$R^{III}$ represents a hydrogen atom, an alkyl group optionally substituted with the same or different 1 to 7 halogen atom(s), an alkoxy group optionally substituted with the same or different 1 to 7 halogen atom(s), or an alkylthio group optionally substituted with the same or different 1 to 7 halogen atom(s);
$R^{XIII}$ represents a hydrogen atom, an alkyl group optionally substituted with the same or different 1 to 7 halogen atom(s), a cycloalkyl group optionally substituted with the same or different 1 to 5 halogen atom(s), a halogen atom, a cyano group, or an aryl group optionally substituted with the same or different 1 to 5 halogen atom(s);
$L^{III}$ represents a single bond or $CR^{LIII-1}R^{LIII-2}$;
$R^{LIII-1}$ and $R^{LIII-2}$ each independently represent a hydrogen atom or an alkyl group optionally substituted with the same or different 1 to 7 halogen atom(s), or $R^{LIII-1}$ and $R^{LIII-2}$ each independently represent an alkylene group and are combined with each other together with the carbon atom to which they are attached to form a monocyclic saturated hydrocarbon group optionally substituted with the same or different 1 to 6 halogen atom(s); and
$Cy^{III}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s);

(ii) a heteroaryl group optionally substituted with the same or different 1 to 5 substituent(s) selected from an alkyl group optionally substituted with the same or different 1 to 7 halogen atom(s) and a halogen atom;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group optionally substituted with the same or different 1, 2, or 3 substituent(s) selected from an alkyl group optionally substituted with the same or different 1 to 7 halogen atom(s) and a halogen atom;
an alkenyl group optionally substituted with the same or different 1 to 5 halogen atom(s);
an alkylidene group optionally substituted with the same or different 1 to 6 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s); and
a heteroaryl group optionally substituted with the same or different 1, 2, or 3 substituent(s) selected from an alkyl group optionally substituted with the same or different 1 to 7 halogen atom(s) and a halogen atom;
or (iv) a nonaromatic heterocyclic group optionally substituted with the same or different 1 to 5 substituent(s) selected from
an alkyl group optionally substituted with the same or different 1, 2, or 3 substituent(s) selected from an alkoxy group optionally substituted with the same or different 1 to 7 halogen atom(s), a halogen atom, and an aryl group optionally substituted with the same or different 1, 2, or 3 substituent(s) selected from an alkyl group optionally substituted with the same or different 1 to 7 halogen atom(s) and a halogen atom;
a cycloalkyl group optionally substituted with the same or different 1 to 5 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s);
a heteroaryl group optionally substituted with the same or different 1 to 5 halogen atom(s);
an alkylcarbonyl group optionally substituted with the same or different 1, 2, or 3 aryl group(s);
a formyl group;
an alkoxycarbonyl group optionally substituted with the same or different 1 to 7 halogen atom(s); and
an arylcarbonyl group optionally substituted with the same or different 1 to 5 halogen atom(s)
(provided that said nonaromatic heterocyclic group is not a tetrahydrofuryl group)
(provided that

(a) when $X^{III}$ is CH and $Cy^{III}$ is a phenyl group optionally substituted with the same or different 1 or 2 halogen atom(s), then $R^{III}$ is not a hydrogen atom; and
(b) the above compound is not 3-cyclopropyl[1,2,4]triazolo[4,3-a]pyrazin-8-amine)]

or a pharmaceutically acceptable salt thereof.
[19] The compound according to [18] or a pharmaceutically acceptable salt thereof, wherein
$R^{III}$ represents a hydrogen atom, an alkyl group optionally substituted with the same or different 1 to 7 halogen atom(s), an alkoxy group, or an alkylthio group;
$R^{XIII}$ represents a hydrogen atom, an alkyl group optionally substituted with the same or different 1 to 7 halogen atom(s), a cycloalkyl group, a halogen atom, a cyano group, or an aryl group;
$L^{III}$ represents a single bond or $CR^{LIII-1}R^{LIII-2}$;
$R^{LIII-1}$ and $R^{LIII-2}$ each independently represent a hydrogen atom or an alkyl group, or $R^{LIII-1}$ and $R^{LIII-2}$ each independently represent a straight alkylene group and are combined with each other together with the carbon atom to which they are attached to form a monocyclic saturated hydrocarbon group; and
$Cy^{III}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted

with the same or different 1, 2, or 3 aryl group(s),
wherein said aryl group is a 6 to 11 membered monocyclic or bicyclic aromatic hydrocarbon group;
(ii) a heteroaryl group optionally substituted with the same or different 1 to 5 halogen atom(s), wherein said heteroaryl group is a 5 to 11 membered monocyclic or bicyclic aromatic heterocyclic group comprising 1 to 4 heteroatom(s) selected from an oxygen atom, a sulfur atom, and a nitrogen atom other than carbon atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a $C_3$-$C_8$ cycloalkyl group, a $C_6$-$C_{12}$ bicycloalkyl group, a $C_6$-$C_{12}$ bicycloalkenyl group, a $C_6$-$C_{12}$ spiroalkyl group, or a $C_{10}$-$C_{14}$ tricyclic tricycloalkyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a 4 to 8 membered monocyclic nonaromatic heterocyclic group or a 6 to 12 membered bicyclic nonaromatic heterocyclic group.

[20] The compound according to [18] or [19] or a pharmaceutically acceptable salt thereof, wherein
$L^{III}$ represents a single bond; and
$Cy^{III}$ represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;

an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

[21] The compound according to any one of [18] to [20] or a pharmaceutically acceptable salt thereof, wherein $X^{III}$ represents $CR^{XIII}$.
[22] The compound according to any one of [18] to [20] or a pharmaceutically acceptable salt thereof, wherein $X^{III}$ represents N.
[23] A compound selected from
3-(cis-2-methylcyclohexyl)-3H-[1,2,3]triazolo[4,5-d]pyrimidin-7-amine (Example 95 (racemate), Example 190 (Enantiomer 1), or Example 191 (Enantiomer 2));
3-(trans-2-methylcyclohexyl)-3H-[1,2,3]triazolo[4,5-d]pyrimidin-7-amine (Example 1 (racemate), Example 192 (Enantiomer 1), or Example 193 (Enantiomer 2));
3-(cis-2-fluorocyclohexyl)-3H-[1,2,3]triazolo[4,5-d]pyrimidin-7-amine (Example 97 (racemate), Example 194 (Enantiomer 1), or Example 195 (Enantiomer 2));
3-(2,2-difluorocyclohexyl)-3H-[1,2,3]triazolo[4,5-d]pyrimidin-7-amine (Example 113 (racemate), Example 114 (Enantiomer 1), or Example 115 (Enantiomer 2));
3-(cis-3-methylcyclohexyl)-3H-[1,2,3]triazolo[4,5-d]pyrimidin-7-amine (Example 98 (racemate), Example 196 (Enantiomer 1), or Example 197 (Enantiomer 2));
3-(trans-3-methylcyclohexyl)-3H-[1,2,3]triazolo[4,5-d]pyrimidin-7-amine (Example 94 (racemate), Example 198 (Enantiomer 1), or Example 199 (Enantiomer 2));
3-(3,3-dimethylcyclohexyl)-3H-[1,2,3]triazolo[4,5-d]pyrimidin-7-amine (Example 128 (racemate));
3-[cis-3-(trifluoromethyl)cyclohexyl]-3H-[1,2,3]triazolo[4,5-d]pyrimidin-7-amine (Example 87 (racemate), Example 202 (Enantiomer 1), or Example 203 (Enantiomer 2));
3-(cis-4-methylcyclohexyl)-3H-[1,2,3]triazolo[4,5-d]pyrimidin-7-amine (Example 99);
3-(trans-4-methylcyclohexyl)-3H-[1,2,3]triazolo[4,5-d]pyrimidin-7-amine (Example 8);
3-(4,4-dimethylcyclohexyl)-3H-[1,2,3]triazolo[4,5-d]pyrimidin-7-amine (Example 101);
3-(trans-3,3,5-trimethylcyclohexyl)-3H-[1,2,3]triazolo[4,5-d]pyrimidin-7-amine (Example 109 (racemate));
3-cycloheptyl-3H-[1,2,3]triazolo[4,5-d]pyrimidin-7-amine (Example 110);
3-cyclohexyl[1,2,4]triazolo[4,3-a]pyrazin-8-amine (Example 141);
3-(1-fluorocyclohexyl)[1,2,4]triazolo[4,3-a]pyrazin-8-amine (Example 142);
3-(cis-3-methylcyclohexyl)[1,2,4]triazolo[4,3-a]pyrazin-8-amine (Example 140 (racemate), Example 204 (Enantiomer 1), or Example 205 (Enantiomer 2));
3-(trans-3-methylcyclohexyl)[1,2,4]triazolo[4,3-a]pyrazin-8-amine (Example 143 (racemate));
3-(3,3-dimethylcyclohexyl)[1,2,4]triazolo[4,3-a]pyrazin-8-amine (Example 144 (racemate), Example 206 (Enantiomer 1), or Example 207 (Enantiomer 2));
3-(spiro[2,5]oct-5-yl)[1,2,4]triazolo[4,3-a]pyrazin-8-amine (Example 145 (racemate), Example 208 (Enantiomer 1), or Example 209 (Enantiomer 2));
3-[cis-3-(trifluoromethyl)cyclohexyl][1,2,4]triazolo[4,3-a]pyrazin-8-amine (Example 146 (racemate), Example 210 (Enantiomer 1), or Example 211 (Enantiomer 2));
3-(3,3-difluorocyclohexyl)[1,2,4]triazolo[4,3-a]pyrazin-8-amine (Example 148 (racemate), Example 212 (Enantiomer 1), or Example 213 (Enantiomer 2));
3-(trans-4-methylcyclohexyl)[1,2,4]triazolo[4,3-a]pyrazin-8-amine (Example 149);
3-[2-methyl-5-(trifluoromethyl)cyclohexyl][1,2,4]triazolo[4,3-a]pyrazin-8-amine (Example 151 (racemate), Example 173 (Enantiomer 1), or Example 174 (Enantiomer 2));
3-(cis-5,5-difluoro-2-methylcyclohexyl)[1,2,4]triazolo[4,3-a]pyrazin-8-amine (Example 152 (racemate), Example 175 (Enantiomer 1), or Example 176 (Enantiomer 2));
3-(trans-3,3-difluoro-5-methylcyclohexyl[1,2,4]triazolo[4,3-a]pyrazin-8-amine (Example 155 (racemate), Example 216 (Enantiomer 1), or Example 217 (Enantiomer 2));
3-(3,3-difluoro-5,5-dimethylcyclohexyl)[1,2,4]triazolo[4,3-a]pyrazin-8-amine (Example 156 (racemate), Example 177 (Enantiomer 1), or Example 178 (Enantiomer 2));
3-[cis-2,2-difluoro-5-(trifluoromethyl)cyclohexyl][1,2,4]triazolo[4,3-a]pyrazin-8-amine (Example 172 (racemate));
3-(bicyclo[4.1.0]hept-3-yl)[1,2,4]triazolo[4,3-a]pyrazin-8-amine (Example 158 (mixture of four types of isomers), Example 218 (Enantiomer 1 having relative configuration (1R*,3S*,6R*)), Example 219 (Enantiomer 2 having relative

configuration (1R*,3S*,6R*)), Example 220 (Enantiomer 1 having relative configuration (1S*,3S*,6S*)), or Example 221 (Enantiomer 2 having relative configuration (1S*,3S*,6S*)));
3-[(1R,6S,7r)-bicyclo[4.1.0]hept-7-yl][1,2,4]triazolo[4,3-a]pyrazin-8-amine (Example 164);
3-(2-methylpiperidin-1-yl)[1,2,4]triazolo[4,3-a]pyrazin-8-amine (Example 21 (S-enantiomer) or Example 22 (R-enantiomer));
3-(2-ethylpiperidin-1-yl)[1,2,4]triazolo[4,3-a]pyrazin-8-amine (Example 23 (racemate), Example 222 (Enantiomer 1), or Example 223 (Enantiomer 2));
3-(3,3-dimethylpiperidin-1-yl)[1,2,4]triazolo[4,3-a]pyrazin-8-amine (Example 26);
3-(3,3-dimethylpiperidin-1-yl)-5-methyl[1,2,4]triazolo[4,3-a]pyrazin-8-amine (Example 80);
3-(3,3-dimethylpiperidin-1-yl)-5-ethyl[1,2,4]triazolo[4,3-a]pyrazin-8-amine (Example 42);
5-cyclopropyl-3-(3,3-dimethylpiperidin-1-yl)[1,2,4]triazolo[4,3-a]pyrazin-8-amine (Example 43);
3-(3,3-dimethylpiperidin-1-yl)-5-(trifluoromethyl)[1,2,4]triazolo[4,3-a]pyrazin-8-amine (Example 252);
5-chloro-3-(3,3-dimethylpiperidin-1-yl)[1,2,4]triazolo[4,3-a]pyrazin-8-amine (Example 248);
8-amino-3-(3,3-dimethylpiperidin-1-yl)[1,2,4]triazolo[4,3-a]pyrazine-5-carbonitrile (Example 2);
3-[trans-3,5-dimethylpiperidin-1-yl][1,2,4]triazolo[4,3-a]pyrazin-8-amine (Example 53 (racemate), Example 226 (Enantiomer 1), or Example 227 (Enantiomer 2));
8-amino-3-(3,5-dimethylpiperidin-1-yl)[1,2,4]triazolo[4,3-a]pyrazine-5-carbonitrile (Example 54 (trans, racemate));
3-(3,4-dimethylpiperidin-1-yl)[1,2,4]triazolo[4,3-a]pyrazin-8-amine (Example 55 (mixture of four types of isomers), Example 228 (Enantiomer 1), Example 229 (Enantiomer 2), Example 230 (Enantiomer 3), or Example 231 (Enantiomer 4));
3-(2,3-dimethylpiperidin-1-yl)[1,2,4]triazolo[4,3-a]pyrazin-8-amine (Example 56 (Diastereomer 1, racemate) or Example 57 (Diastereomer 2, racemate));
3-(2,5-dimethylpiperidin-1-yl)[1,2,4]triazolo[4,3-a]pyrazin-8-amine (Example 58 (cis, racemate), Example 232 (cis, Enantiomer 1), Example 233 (cis, Enantiomer 2), or Example 59 (trans, racemate));
8-amino-3-(2,5-dimethylpiperidin-1-yl)[1,2,4]triazolo[4,3-a]pyrazine-5-carbonitrile (Example 60 (cis, racemate), Example 234 (cis, Enantiomer 1), or Example 235 (cis, Enantiomer 2));
3-(2,4-dimethylpiperidin-1-yl)[1,2,4]triazolo[4,3-a]pyrazin-8-amine (Example 61 (trans, racemate) or Example 62 (cis, racemate));
3-(2,5,5-trimethylpiperidin-1-yl)[1,2,4]triazolo[4,3-a]pyrazin-8-amine (Example 65 (racemate), Example 236 (Enantiomer 1), or Example 237 (Enantiomer 2));
3-cyclohexyl[1,2,4]triazolo[3,4-f][1,2,4]triazin-8-amine (Example 68);
3-(cis-2-methylcyclohexyl) [1,2,4]triazolo[3,4-f][1,2,4]triazin-8-amine (Example 69 (Diastereomer 1, racemate), Example 238 (Diastereomer 1, Enantiomer 1), Example 239 (Diastereomer 1, Enantiomer 2), or Example 70 (Diastereomer 2, racemate));
3-(trans-2-methylcyclohexyl)[1,2,4]triazolo[3,4-f][1,2,4]triazin-8-amine (Example 69 (Diastereomer 1, racemate), Example 238 (Diastereomer 1, Enantiomer 1), Example 239 (Diastereomer 1, Enantiomer 2), or Example 70 (Diastereomer 2, racemate));
3-(cis-3-methylcyclohexyl)[1,2,4]triazolo[3,4-f][1,2,4]triazin-8-amine (Example 71 (Diastereomer 1, racemate), Example 240 (Diastereomer 1, Enantiomer 1), Example 241 (Diastereomer 1, Enantiomer 2), or Example 72 (Diastereomer 2, racemate));
3-(trans-3-methylcyclohexyl)[1,2,4]triazolo[3,4-f][1,2,4]triazin-8-amine (Example 71 (Diastereomer 1, racemate), Example 240 (Diastereomer 1, Enantiomer 1), Example 241 (Diastereomer 1, Enantiomer 2), or Example 72 (Diastereomer 2, racemate));
3-(3,3-dimethylcyclohexyl)[1,2,4]triazolo[3,4-f][1,2,4]triazin-8-amine (Example 73 (racemate), Example 242 (Enantiomer 1), or Example 243 (Enantiomer 2));
3-[cis-3-(trifluoromethyl)cyclohexyl][1,2,4]triazolo[3,4-f][1,2,4]triazin-8-amine (Example 74 (Diastereomer 1, racemate), Example 244 (Diastereomer 1, Enantiomer 1), Example 245 (Diastereomer 1, Enantiomer 2), or Example 75 (Diastereomer 2, racemate));
3-[trans-3-(trifluoromethyl)cyclohexyl][1,2,4]triazolo[3,4-f][1,2,4]triazin-8-amine (Example 74 (Diastereomer 1, racemate), Example 244 (Diastereomer 1, Enantiomer 1), Example 245 (Diastereomer 1, Enantiomer 2), or Example 75 (Diastereomer 2, racemate));
3-(3,3-difluorocyclohexyl)[1,2,4]triazolo[3,4-f][1,2,4]triazin-8-amine (Example 82 (racemate));
3-(cis-5,5-difluoro-2-methylcyclohexyl)[1,2,4]triazolo[3,4-f][1,2,4]triazin-8-amine (Example 76 (racemate), Example 246 (Enantiomer 1), or Example 247 (Enantiomer 2)); and
3-[2-methyl-5-(trifluoromethyl)cyclohexyl][1,2,4]triazolo[3,4-f][1,2,4]triazin-8-amine (Example 83 (relative configuration (1R*, 2S*, 5R*), racemate))
or a pharmaceutically acceptable salt thereof.
[24] A compound selected from

3-(cis-2-methylcyclohexyl)-3H-[1,2,3]triazolo[4,5-d]pyrimidin-7-amine (Example 95 (racemate), Example 190 (Enantiomer 1), or Example 191 (Enantiomer 2));
3-(trans-2-methylcyclohexyl)-3H-[1,2,3]triazolo[4,5-d]pyrimidin-7-amine (Example 1 (racemate), Example 192 (Enantiomer 1), or Example 193 (Enantiomer 2));
3-(cis-2-fluorocyclohexyl)-3H-[1,2,3]triazolo[4,5-d]pyrimidin-7-amine (Example 97 (racemate), Example 194 (Enantiomer 1), or Example 195 (Enantiomer 2));
3-(2,2-difluorocyclohexyl)-3H-[1,2,3]triazolo[4,5-d]pyrimidin-7-amine (Example 113 (racemate), Example 114 (Enantiomer 1), or Example 115 (Enantiomer 2));
3-(cis-3-methylcyclohexyl)-3H-[1,2,3]triazolo[4,5-d]pyrimidin-7-amine (Example 98 (racemate), Example 196 (Enantiomer 1), or Example 197 (Enantiomer 2));
3-(trans-3-methylcyclohexyl)-3H-[1,2,3]triazolo[4,5-d]pyrimidin-7-amine (Example 94 (racemate), Example 198 (Enantiomer 1), or Example 199 (Enantiomer 2));
3-(3,3-dimethylcyclohexyl)-3H-[1,2,3]triazolo[4,5-d]pyrimidin-7-amine (Example 128 (racemate));
3-[cis-3-(trifluoromethyl)cyclohexyl]-3H-[1,2,3]triazolo[4,5-d]pyrimidin-7-amine (Example 87 (racemate), Example 202 (Enantiomer 1), or Example 203 (Enantiomer 2));
3-(cis-4-methylcyclohexyl)-3H-[1,2,3]triazolo[4,5-d]pyrimidin-7-amine (Example 99);
3-(trans-4-methylcyclohexyl)-3H-[1,2,3]triazolo[4,5-d]pyrimidin-7-amine (Example 8);
3-(4,4-dimethylcyclohexyl)-3H-[1,2,3]triazolo[4,5-d]pyrimidin-7-amine (Example 101);
3-(trans-3,3,5-trimethylcyclohexyl)-3H-[1,2,3]triazolo[4,5-d]pyrimidin-7-amine (Example 109 (racemate)); and
3-cycloheptyl-3H-[1,2,3]triazolo[4,5-d]pyrimidin-7-amine (Example 110)
or a pharmaceutically acceptable salt thereof.
[25] A compound selected from
3-cyclohexyl[1,2,4]triazolo[4,3-a]pyrazin-8-amine (Example 141);
3-(1-fluorocyclohexyl)[1,2,4]triazolo[4,3-a]pyrazin-8-amine (Example 142);
3-(cis-3-methylcyclohexyl)[1,2,4]triazolo[4,3-a]pyrazin-8-amine (Example 140 (racemate), Example 204 (Enantiomer 1), or Example 205 (Enantiomer 2));
3-(trans-3-methylcyclohexyl)[1,2,4]triazolo[4,3-a]pyrazin-8-amine (Example 143 (racemate));
3-(3,3-dimethylcyclohexyl)[1,2,4]triazolo[4,3-a]pyrazin-8-amine (Example 144 (racemate), Example 206 (Enantiomer 1), or Example 207 (Enantiomer 2));
3-(spiro[2,5]oct-5-yl)[1,2,4]triazolo[4,3-a]pyrazin-8-amine (Example 145 (racemate), Example 208 (Enantiomer 1), or Example 209 (Enantiomer 2));
3-[cis-3-(trifluoromethyl)cyclohexyl][1,2,4]triazolo[4,3-a]pyrazin-8-amine (Example 146 (racemate), Example 210 (Enantiomer 1), or Example 211 (Enantiomer 2));
3-(3,3-difluorocyclohexyl)[1,2,4]triazolo[4,3-a]pyrazin-8-amine (Example 148 (racemate), Example 212 (Enantiomer 1), or Example 213 (Enantiomer 2));
3-(trans-4-methylcyclohexyl)[1,2,4]triazolo[4,3-a]pyrazin-8-amine (Example 149);
3-[2-methyl-5-(trifluoromethyl)cyclohexyl][1,2,4]triazolo[4,3-a]pyrazin-8-amine (Example 151 (racemate), Example 173 (Enantiomer 1), or Example 174 (Enantiomer 2));
3-(cis-5,5-difluoro-2-methylcyclohexyl)[1,2,4]triazolo[4,3-a]pyrazin-8-amine (Example 152 (racemate), Example 175 (Enantiomer 1), or Example 176 (Enantiomer 2));
3-(trans-3,3-difluoro-5-methylcyclohexyl[1,2,4]triazolo[4,3-a]pyrazin-8-amine (Example 155 (racemate), Example 216 (Enantiomer 1), or Example 217 (Enantiomer 2));
3-(3,3-difluoro-5,5-dimethylcyclohexyl)[1,2,4]triazolo[4,3-a]pyrazin-8-amine (Example 156 (racemate), Example 177 (Enantiomer 1), or Example 178 (Enantiomer 2));
3-[cis-2,2-difluoro-5-(trifluoromethyl)cyclohexyl][1,2,4]triazolo[4,3-a]pyrazin-8-amine (Example 172 (racemate));
3-(bicyclo[4.1.0]hept-3-yl)[1,2,4]triazolo[4,3-a]pyrazin-8-amine (Example 158 (mixture of four types of isomers), Example 218 (Enantiomer 1 having relative configuration (1R*, 3S*, 6R*)), Example 219 (Enantiomer 2 having relative configuration (1R*, 3S*, 6R*)), Example 220 (Enantiomer 1 having relative configuration (1S*, 3S*, 6S*)), or Example 221 (Enantiomer 2 having relative configuration (1S*, 3S*, 6S*)));
3-[(1R,6S,7r)-bicyclo[4.1.0]hept-7-yl][1,2,4]triazolo[4,3-a]pyrazin-8-amine (Example 164);
3-(2-methylpiperidin-1-yl)[1,2,4]triazolo[4,3-a]pyrazin-8-amine (Example 21 (S-enantiomer) or Example 22 (R-enantiomer));
3-(2-ethylpiperidin-1-yl)[1,2,4]triazolo[4,3-a]pyrazin-8-amine (Example 23 (racemate), Example 222 (Enantiomer 1), or Example 223 (Enantiomer 2));
3-(3,3-dimethylpiperidin-1-yl)[1,2,4]triazolo[4,3-a]pyrazin-8-amine (Example 26);
3-(3,3-dimethylpiperidin-1-yl)-5-methyl[1,2,4]triazolo[4,3-a]pyrazin-8-amine (Example 80);
3-(3,3-dimethylpiperidin-1-yl)-5-ethyl[1,2,4]triazolo[4,3-a]pyrazin-8-amine (Example 42);
5-cyclopropyl-3-(3,3-dimethylpiperidin-1-yl)[1,2,4]triazolo[4,3-a]pyrazin-8-amine (Example 43);

3-(3,3-dimethylpiperidin-1-yl)-5-(trifluoromethyl)[1,2,4]triazolo[4,3-a]pyrazin-8-amine (Example 252);
5-chloro-3-(3,3-dimethylpiperidin-1-yl)[1,2,4]triazolo[4,3-a]pyrazin-8-amine (Example 248);
8-amino-3-(3,3-dimethylpiperidin-1-yl)[1,2,4]triazolo[4,3-a]pyrazine-5-carbonitrile (Example 2);
3-[trans-3,5-dimethylpiperidin-1-yl][1,2,4]triazolo[4,3-a]pyrazin-8-amine (Example 53 (racemate), Example 226 (Enantiomer 1), or Example 227 (Enantiomer 2));
8-amino-3-(3,5-dimethylpiperidin-1-yl)[1,2,4]triazolo[4,3-a]pyrazine-5-carbonitrile (Example 54 (trans, racemate));
3-(3,4-dimethylpiperidin-1-yl)[1,2,4]triazolo[4,3-a]pyrazin-8-amine (Example 55 (mixture of four types of isomers), Example 228 (Enantiomer 1), Example 229 (Enantiomer 2), Example 230 (Enantiomer 3), or Example 231 (Enantiomer 4));
3-(2,3-dimethylpiperidin-1-yl)[1,2,4]triazolo[4,3-a]pyrazin-8-amine (Example 56 (Diastereomer 1, racemate), or Example 57 (Diastereomer 2, racemate));
3-(2,5-dimethylpiperidin-1-yl)[1,2,4]triazolo[4,3-a]pyrazin-8-amine (Example 58 (cis, racemate), Example 232 (cis, Enantiomer 1), Example 233 (cis, Enantiomer 2), or Example 59 (trans, racemate);
8-amino-3-(2,5-dimethylpiperidin-1-yl)[1,2,4]triazolo[4,3-a]pyrazine-5-carbonitrile (Example 60 (cis, racemate), Example 234 (cis, Enantiomer 1), or Example 235 (cis, Enantiomer 2));
3-(2,4-dimethylpiperidin-1-yl)[1,2,4]triazolo[4,3-a]pyrazin-8-amine (Example 61 (trans, racemate), or Example 62 (cis, racemate));
3-(2,5,5-trimethylpiperidin-1-yl)[1,2,4]triazolo[4,3-a]pyrazin-8-amine (Example 65 (racemate), Example 236 (Enantiomer 1), or Example 237 (Enantiomer 2));
3-cyclohexyl[1,2,4]triazolo[3,4-f][1,2,4]triazin-8-amine (Example 68);
3-(cis-2-methylcyclohexyl)[1,2,4]triazolo[3,4-f][1,2,4]triazin-8-amine (Example 69 (Diastereomer 1, racemate), Example 238 (Diastereomer 1, Enantiomer 1), Example 239 (Diastereomer 1, Enantiomer 2), or Example 70 (Diastereomer 2, racemate));
3-(trans-2-methylcyclohexyl)[1,2,4]triazolo[3,4-f][1,2,4]triazin-8-amine (Example 69 (Diastereomer 1, racemate), Example 238 (Diastereomer 1, Enantiomer 1), Example 239 (Diastereomer 1, Enantiomer 2), or Example 70 (Diastereomer 2, racemate));
3-(cis-3-methylcyclohexyl)[1,2,4]triazolo[3,4-f][1,2,4]triazin-8-amine (Example 71 (Diastereomer 1, racemate), Example 240 (Diastereomer 1, Enantiomer 1), Example 241 (Diastereomer 1, Enantiomer 2), or Example 72 (Diastereomer 2, racemate));
3-(trans-3-methylcyclohexyl)[1,2,4]triazolo[3,4-f][1,2,4]triazin-8-amine (Example 71 (Diastereomer 1, racemate), Example 240 (Diastereomer 1, Enantiomer 1), Example 241 (Diastereomer 1, Enantiomer 2), or Example 72 (Diastereomer 2, racemate));
3-(3,3-dimethylcyclohexyl)[1,2,4]triazolo[3,4-f][1,2,4]triazin-8-amine (Example 73 (racemate), Example 242 (Enantiomer 1), or Example 243 (Enantiomer 2));
3-[cis-3-(trifluoromethyl)cyclohexyl][1,2,4]triazolo[3,4-f][1,2,4]triazin-8-amine (Example 74 (Diastereomer 1, racemate), Example 244 (Diastereomer 1, Enantiomer 1), Example 245 (Diastereomer 1, Enantiomer 2), or Example 75 (Diastereomer 2, racemate));
3-[trans-3-(trifluoromethyl)cyclohexyl][1,2,4]triazolo[3,4-f][1,2,4]triazin-8-amine (Example 74 (Diastereomer 1, racemate), Example 244 (Diastereomer 1, Enantiomer 1), Example 245 (Diastereomer 1, Enantiomer 2), or Example 75 (Diastereomer 2, racemate));
3-(3,3-difluorocyclohexyl)[1,2,4]triazolo[3,4-f][1,2,4]triazin-8-amine (Example 82 (racemate));
3-(cis-5,5-difluoro-2-methylcyclohexyl)[1,2,4]triazolo[3,4-f][1,2,4]triazin-8-amine (Example 76 (racemate), Example 246 (Enantiomer 1), or Example 247 (Enantiomer 2)); and
3-[2-methyl-5-(trifluoromethyl)cyclohexyl][1,2,4]triazolo[3,4-f][1,2,4]triazin-8-amine (Example 83 (relative configuration (1R*, 2S*, 5R*), racemate))
or a pharmaceutically acceptable salt thereof.
[26] A compound selected from
3-cyclohexyl[1,2,4]triazolo[4,3-a]pyrazin-8-amine (Example 141);
3-(1-fluorocyclohexyl)[1,2,4]triazolo[4,3-a]pyrazin-8-amine (Example 142);
3-(cis-3-methylcyclohexyl)[1,2,4]triazolo[4,3-a]pyrazin-8-amine (Example 140 (racemate), Example 204 (Enantiomer 1), or Example 205 (Enantiomer 2));
3-(trans-3-methylcyclohexyl)[1,2,4]triazolo[4,3-a]pyrazin-8-amine (Example 143 (racemate));
3-(3,3-dimethylcyclohexyl)[1,2,4]triazolo[4,3-a]pyrazin-8-amine (Example 144 (racemate), Example 206 (Enantiomer 1), or Example 207 (Enantiomer 2));
3-(spiro[2,5]oct-5-yl)[1,2,4]triazolo[4,3-a]pyrazin-8-amine (Example 145 (racemate), Example 208 (Enantiomer 1), or Example 209 (Enantiomer 2));
3-[cis-3-(trifluoromethyl)cyclohexyl][1,2,4]triazolo[4,3-a]pyrazin-8-amine (Example 146 (racemate), Example 210 (Enantiomer 1), or Example 211 (Enantiomer 2));

3-(3,3-difluorocyclohexyl)[1,2,4]triazolo[4,3-a]pyrazin-8-amine (Example 148 (racemate), Example 212 (Enantiomer 1), or Example 213 (Enantiomer 2));
3-(trans-4-methylcyclohexyl)[1,2,4]triazolo[4,3-a]pyrazin-8-amine (Example 149);
3-[2-methyl-5-(trifluoromethyl)cyclohexyl][1,2,4]triazolo[4,3-a]pyrazin-8-amine (Example 151 (racemate), Example 173 (Enantiomer 1), or Example 174 (Enantiomer 2));
3-(cis-5,5-difluoro-2-methylcyclohexyl)[1,2,4]triazolo[4,3-a]pyrazin-8-amine (Example 152 (racemate), Example 175 (Enantiomer 1), or Example 176 (Enantiomer 2));
3-(trans-3,3-difluoro-5-methylcyclohexyl[1,2,4]triazolo[4,3-a]pyrazin-8-amine (Example 155 (racemate), Example 216 (Enantiomer 1), or Example 217 (Enantiomer 2));
3-(3,3-difluoro-5,5-dimethylcyclohexyl)[1,2,4]triazolo[4,3-a]pyrazin-8-amine (Example 156 (racemate), Example 177 (Enantiomer 1), or Example 178 (Enantiomer 2));
3-[cis-2,2-difluoro-5-(trifluoromethyl)cyclohexyl][1,2,4]triazolo[4,3-a]pyrazin-8-amine (Example 172 (racemate));
3-(bicyclo[4.1.0]hept-3-yl) [1,2,4]triazolo[4,3-a]pyrazin-8-amine (Example 158 (mixture of four types of isomers), Example 218 (Enantiomer 1 having relative configuration (1R*, 3S*, 6R*)), Example 219 (Enantiomer 2 having relative configuration (1R*, 3S*, 6R*)), Example 220 (Enantiomer 1 having relative configuration (1S*, 3S*, 6S*)), or Example 221 (Enantiomer 2 having relative configuration (1S*, 3S*, 6S*)));
3-[(1R,6S,7r)-bicyclo[4.1.0]hept-7-yl][1,2,4]triazolo[4,3-a]pyrazin-8-amine (Example 164);
3-(2-methylpiperidin-1-yl)[1,2,4]triazolo[4,3-a]pyrazin-8-amine (Example 21 (S-enantiomer) or Example 22 (R-enantiomer));
3-(2-ethylpiperidin-1-yl)[1,2,4]triazolo[4,3-a]pyrazin-8-amine (Example 23 (racemate), Example 222 (Enantiomer 1), or Example 223 (Enantiomer 2));
3-(3,3-dimethylpiperidin-1-yl)[1,2,4]triazolo[4,3-a]pyrazin-8-amine (Example 26);
3-(3,3-dimethylpiperidin-1-yl)-5-methyl[1,2,4]triazolo[4,3-a]pyrazin-8-amine (Example 80);
3-(3,3-dimethylpiperidin-1-yl)-5-ethyl[1,2,4]triazolo[4,3-a]pyrazin-8-amine (Example 42);
5-cyclopropyl-3-(3,3-dimethylpiperidin-1-yl)[1,2,4]triazolo[4,3-a]pyrazin-8-amine (Example 43);
3-(3,3-dimethylpiperidin-1-yl)-5-(trifluoromethyl)[1,2,4]triazolo[4,3-a]pyrazin-8-amine (Example 252);
5-chloro-3-(3,3-dimethylpiperidin-1-yl)[1,2,4]triazolo[4,3-a]pyrazin-8-amine (Example 248);
8-amino-3-(3,3-dimethylpiperidin-1-yl)[1,2,4]triazolo[4,3-a]pyrazine-5-carbonitrile (Example 2);
3-[trans-3,5-dimethylpiperidin-1-yl][1,2,4]triazolo[4,3-a]pyrazin-8-amine (Example 53 (racemate), Example 226 (Enantiomer 1), or Example 227 (Enantiomer 2));
8-amino-3-(3,5-dimethylpiperidin-1-yl)[1,2,4]triazolo[4,3-a]pyrazine-5-carbonitrile (Example 54 (trans, racemate));
3-(3,4-dimethylpiperidin-1-yl)[1,2,4]triazolo[4,3-a]pyrazin-8-amine (Example 55 (mixture of four types of isomers), Example 228 (Enantiomer 1), Example 229 (Enantiomer 2), Example 230 (Enantiomer 3), or Example 231 (Enantiomer 4));
3-(2,3-dimethylpiperidin-1-yl)[1,2,4]triazolo[4,3-a]pyrazin-8-amine (Example 56 (Diastereomer 1, racemate) or Example 57 (Diastereomer 2, racemate));
3-(2,5-dimethylpiperidin-1-yl)[1,2,4]triazolo[4,3-a]pyrazin-8-amine (Example 58 (cis, racemate), Example 232 (cis, Enantiomer 1), Example 233 (cis, Enantiomer 2), or Example 59 (trans, racemate);
8-amino-3-(2,5-dimethylpiperidin-1-yl)[1,2,4]triazolo[4,3-a]pyrazine-5-carbonitrile (Example 60 (cis, racemate), Example 234 (cis, Enantiomer 1), or Example 235 (cis, Enantiomer 2));
3-(2,4-dimethylpiperidin-1-yl)[1,2,4]triazolo[4,3-a]pyrazin-8-amine (Example 61 (trans, racemate) or Example 62 (cis, racemate)); and
3-(2,5,5-trimethylpiperidin-1-yl)[1,2,4]triazolo[4,3-a]pyrazin-8-amine (Example 65 (racemate), Example 236 (Enantiomer 1), or Example 237 (Enantiomer 2))
or a pharmaceutically acceptable salt thereof.
[27] A compound selected from
3-cyclohexyl[1,2,4]triazolo[3,4-f][1,2,4]triazin-8-amine (Example 68);
3-(cis-2-methylcyclohexyl)[1,2,4]triazolo[3,4-f][1,2,4]triazin-8-amine (Example 69 (Diastereomer 1, racemate), Example 238 (Diastereomer 1, Enantiomer 1), Example 239 (Diastereomer 1, Enantiomer 2), or Example 70 (Diastereomer 2, racemate));
3-(trans-2-methylcyclohexyl)[1,2,4]triazolo[3,4-f][1,2,4]triazin-8-amine (Example 69 (Diastereomer 1, racemate), Example 238 (Diastereomer 1, Enantiomer 1), Example 239 (Diastereomer 1, Enantiomer 2), or Example 70 (Diastereomer 2, racemate));
3-(cis-3-methylcyclohexyl)[1,2,4]triazolo[3,4-f][1,2,4]triazin-8-amine (Example 71 (Diastereomer 1, racemate), Example 240 (Diastereomer 1, Enantiomer 1), Example 241 (Diastereomer 1, Enantiomer 2), or Example 72 (Diastereomer 2, racemate));
3-(trans-3-methylcyclohexyl)[1,2,4]triazolo[3,4-f][1,2,4]triazin-8-amine (Example 71 (Diastereomer 1, racemate), Example 240 (Diastereomer 1, Enantiomer 1), Example 241 (Diastereomer 1, Enantiomer 2), or Example 72 (Diastereomer 2, racemate));
3-(3,3-dimethylcyclohexyl)[1,2,4]triazolo[3,4-f][1,2,4]triazin-8-amine (Example 73 (racemate), Example 242 (Enantiomer 1), or Example 243 (Enantiomer 2));
3-[cis-3-(trifluoromethyl)cyclohexyl][1,2,4]triazolo[3,4-f][1,2,4]triazin-8-amine (Example 74 (Diastereomer 1, racemate), Example 244 (Diastereomer 1, Enantiomer 1), Example 245 (Diastereomer 1, Enantiomer 2), or Example 75 (Diastereomer 2, racemate));
3-[trans-3-(trifluoromethyl)cyclohexyl][1,2,4]triazolo[3,4-f][1,2,4]triazin-8-amine (Example 74 (Diastereomer 1, racemate), Example 244 (Diastereomer 1, Enantiomer 1), Example 245 (Diastereomer 1, Enantiomer 2), or Example 75 (Diastereomer 2, racemate));
3-(3,3-difluorocyclohexyl)[1,2,4]triazolo[3,4-f][1,2,4]triazin-8-amine (Example 82 (racemate));
3-(cis-5,5-difluoro-2-methylcyclohexyl)[1,2,4]triazolo[3,4-f][1,2,4]triazin-8-amine (Example 76 (racemate), Example 246 (Enantiomer 1), or Example 247 (Enantiomer 2)); and
3-[2-methyl-5-(trifluoromethyl)cyclohexyl][1,2,4]triazolo[3,4-f][1,2,4]triazin-8-amine (Example 83 (relative configuration (1R*, 2S*, 5R*), racemate))
or a pharmaceutically acceptable salt thereof.
[28] A pharmaceutical composition comprising a compound which is an active ingredient of the PDE7 inhibitor according to any one of [1] to [14] or the compound according to any one of [15] to [27], or a pharmaceutically acceptable salt thereof as an active ingredient.
[29] The pharmaceutical composition according to [28] which is a PDE7 inhibitor.
[30] The pharmaceutical composition according to [28] or [29] for the treatment or prevention of a disease which is improved by inhibiting PDE7.
[31] The pharmaceutical composition according to [30], wherein the disease which is improved by inhibiting PDE7 is a disease selected from the group consisting of a psychiatric disorder and a neurological disorder, a movement disorder, cancer and leukemia, pain, an inflammatory disease and an immunological disease, and a cardiovascular disease.
[32] The pharmaceutical composition according to [30], wherein the disease which is improved by inhibiting PDE7 is a disease selected from the group consisting of

(i) dependence on an addictive drug and a specified act (for example, alcohol dependence, drug dependence such as nicotine dependence and cocaine dependence, gambling dependence, internet dependence, overuse of an electronic device, overuse of a game device, shopping dependence, sex dependence, bulimia, binge eating disorder, kleptomania, pyromania, or trichotillomania), obsessive-compulsive disorder, post-traumatic stress disorder (PTSD), anxiety, depression, mood disorder, insomnia, delirium disorder, psychiatric disease, schizophrenia-related disorder, attention deficit hyperactivity disorder (ADHD) in a child with hyperactivity, migraine, stress, a disorder related to a disease caused by psychosomatic disease, panic attack, epilepsy, memory disorder, cognitive disorder, Alzheimer's disease, senile dementia, attention disorder, wakefulness disorder, ischemia, and brain injury-related disorder;
(ii) Parkinson's disease, dopa-responsive dystonia, spinal cord injury, dyskinesia, a disorder related to acute or chronic neurodegenerative disease (including Huntington's chorea), Shy-Drager syndrome, periodic limb movement disorder (PLMD), periodic limb movements in sleep (PLMS), Tourette's syndrome, and restless legs syndrome (RLS);
(iii) glioblastoma and chronic lymphocytic leukemia;
(iv) neuropathic pain and visceral pain;
(v) autoimmune encephalomyelitis, multiple sclerosis, atopic dermatitis, allergic rhinitis, asthma, psoriasis, Crohn's disease, ulcerative colitis, rheumatoid arthritis, post-transplantation rejection, diabetes mellitus, and chronic obstructive pulmonary disease (COPD); and (vi) myocardial infarction.

[33] The pharmaceutical composition according to [30], wherein the disease which is improved by inhibiting PDE7 is a disease selected from the group consisting of alcohol dependence, drug dependence, gambling dependence, internet dependence, overuse of an electronic device, overuse of a game device, sex dependence, bulimia, binge eating disorder, and glioblastoma.
[34] The pharmaceutical composition according to [30], wherein the disease which is improved by inhibiting PDE7 is a disease selected from the group consisting of alcohol dependence, drug dependence, and glioblastoma.
[35] A PDE7 inhibitor comprising the compound according to any one of [15] to [27] or a pharmaceutically acceptable salt thereof as an active ingredient.
[36] The PDE7 inhibitor according to any one of [1] to [14] or [35] for the treatment or prevention of a disease which is improved by inhibiting PDE7.
[37] The PDE7 inhibitor according to [36], wherein the disease which is improved by inhibiting PDE7 is a disease

selected from the group consisting of a psychiatric disorder and a neurological disorder, a movement disorder, cancer and leukemia, pain, an inflammatory disease and an immunological disease, and a cardiovascular disease.
[38] The PDE7 inhibitor according to [36], wherein the disease which is improved by inhibiting PDE7 is a disease selected from the group consisting of

(i) dependence on an addictive drug and a specified act (for example, alcohol dependence, drug dependence such as nicotine dependence and cocaine dependence, gambling dependence, internet dependence, overuse of an electronic device, overuse of a game device, shopping dependence, sex dependence, bulimia, binge eating disorder, kleptomania, pyromania, or trichotillomania), obsessive-compulsive disorder, post-traumatic stress disorder (PTSD), anxiety, depression, mood disorder, insomnia, delirium disorder, psychiatric disease, schizophrenia-related disorder, attention deficit hyperactivity disorder (ADHD) in a child with hyperactivity, migraine, stress, a disorder related to a disease caused by psychosomatic disease, panic attack, epilepsy, memory disorder, cognitive disorder, Alzheimer's disease, senile dementia, attention disorder, wakefulness disorder, ischemia, and brain injury-related disorder;
(ii) Parkinson's disease, dopa-responsive dystonia, spinal cord injury, dyskinesia, a disorder related to acute or chronic neurodegenerative disease (including Huntington's chorea), Shy-Drager syndrome, periodic limb movement disorder (PLMD), periodic limb movements in sleep (PLMS), Tourette's syndrome, and restless legs syndrome (RLS);
(iii) glioblastoma and chronic lymphocytic leukemia;
(iv) neuropathic pain and visceral pain;
(v) autoimmune encephalomyelitis, multiple sclerosis, atopic dermatitis, allergic rhinitis, asthma, psoriasis, Crohn's disease, ulcerative colitis, rheumatoid arthritis, post-transplantation rejection, diabetes mellitus, and chronic obstructive pulmonary disease (COPD); and
(vi) myocardial infarction.

[39] The PDE7 inhibitor according to [36], wherein the disease which is improved by inhibiting PDE7 is a disease selected from the group consisting of alcohol dependence, drug dependence, gambling dependence, internet dependence, overuse of an electronic device, overuse of a game device, sex dependence, bulimia, binge eating disorder, and glioblastoma.
[40] The PDE7 inhibitor according to [36], wherein the disease which is improved by inhibiting PDE7 is a disease selected from the group consisting of alcohol dependence, drug dependence, and glioblastoma.
[41] Use of the PDE7 inhibitor according to any one of [1] to [14] or the compound according to any one of [15] to [27] or a pharmaceutically acceptable salt thereof in the manufacture of a medicament for the treatment or prevention of a disease which is improved by inhibiting PDE7.
[42] The use according to [41], wherein the disease which is improved by inhibiting PDE7 is a disease selected from the group consisting of a psychiatric disorder and a neurological disorder, a movement disorder, cancer and leukemia, pain, an inflammatory disease and an immunological disease, and a cardiovascular disease.
[43] The use according to [41], wherein the disease which is improved by inhibiting PDE7 is a disease selected from the group consisting of

(i) dependence on an addictive drug and a specified act (for example, alcohol dependence, drug dependence such as nicotine dependence and cocaine dependence, gambling dependence, internet dependence, overuse of an electronic device, overuse of a game device, shopping dependence, sex dependence, bulimia, binge eating disorder, kleptomania, pyromania, or trichotillomania), obsessive-compulsive disorder, post-traumatic stress disorder (PTSD), anxiety, depression, mood disorder, insomnia, delirium disorder, psychiatric disease, schizophrenia-related disorder, attention deficit hyperactivity disorder (ADHD) in a child with hyperactivity, migraine, stress, a disorder related to a disease caused by psychosomatic disease, panic attack, epilepsy, memory disorder, cognitive disorder, Alzheimer's disease, senile dementia, attention disorder, wakefulness disorder, ischemia, and brain injury-related disorder;
(ii) Parkinson's disease, dopa-responsive dystonia, spinal cord injury, dyskinesia, a disorder related to acute or chronic neurodegenerative disease (including Huntington's chorea), Shy-Drager syndrome, periodic limb movement disorder (PLMD), periodic limb movements in sleep (PLMS), Tourette's syndrome, and restless legs syndrome (RLS);
(iii) glioblastoma and chronic lymphocytic leukemia;
(iv) neuropathic pain and visceral pain;
(v) autoimmune encephalomyelitis, multiple sclerosis, atopic dermatitis, allergic rhinitis, asthma, psoriasis, Crohn's disease, ulcerative colitis, rheumatoid arthritis, post-transplantation rejection, diabetes mellitus, and chronic obstructive pulmonary disease (COPD); and

(vi) myocardial infarction.

[44] The use according to [41], wherein the disease which is improved by inhibiting PDE7 is a disease selected from the group consisting of alcohol dependence, drug dependence, gambling dependence, internet dependence, overuse of an electronic device, overuse of a game device, sex dependence, bulimia, binge eating disorder, and glioblastoma.
[45] The use according to [41], wherein the disease which is improved by inhibiting PDE7 is a disease selected from the group consisting of alcohol dependence, drug dependence, and glioblastoma.
[46] The compound according to any one of [15] to [27] or a pharmaceutically acceptable salt thereof for the treatment or prevention of a disease which is improved by inhibiting PDE7.
[47] The compound according to [46] or a pharmaceutically acceptable salt thereof, wherein the disease which is improved by inhibiting PDE7 is a disease selected from the group consisting of a psychiatric disorder and a neurological disorder, a movement disorder, cancer and leukemia, pain, an inflammatory disease and an immunological disease, and a cardiovascular disease.
[48] The compound according to [46] or a pharmaceutically acceptable salt thereof, wherein the disease which is improved by inhibiting PDE7 is a disease selected from the group consisting of

(i) dependence on an addictive drug and a specified act (for example, alcohol dependence, drug dependence such as nicotine dependence and cocaine dependence, gambling dependence, internet dependence, overuse of an electronic device, overuse of a game device, shopping dependence, sex dependence, bulimia, binge eating disorder, kleptomania, pyromania, or trichotillomania), obsessive-compulsive disorder, post-traumatic stress disorder (PTSD), anxiety, depression, mood disorder, insomnia, delirium disorder, psychiatric disease, schizophrenia-related disorder, attention deficit hyperactivity disorder (ADHD) in a child with hyperactivity, migraine, stress, a disorder related to a disease caused by psychosomatic disease, panic attack, epilepsy, memory disorder, cognitive disorder, Alzheimer's disease, senile dementia, attention disorder, wakefulness disorder, ischemia, and brain injury-related disorder;
(ii) Parkinson's disease, dopa-responsive dystonia, spinal cord injury, dyskinesia, a disorder related to acute or chronic neurodegenerative disease (including Huntington's chorea), Shy-Drager syndrome, periodic limb movement disorder (PLMD), periodic limb movements in sleep (PLMS), Tourette's syndrome, and restless legs syndrome (RLS);
(iii) glioblastoma and chronic lymphocytic leukemia;
(iv) neuropathic pain and visceral pain;
(v) autoimmune encephalomyelitis, multiple sclerosis, atopic dermatitis, allergic rhinitis, asthma, psoriasis, Crohn's disease, ulcerative colitis, rheumatoid arthritis, post-transplantation rejection, diabetes mellitus, and chronic obstructive pulmonary disease (COPD); and
(vi) myocardial infarction.

[49] The compound according to [46] or a pharmaceutically acceptable salt thereof, wherein the disease which is improved by inhibiting PDE7 is a disease selected from the group consisting of alcohol dependence, drug dependence, gambling dependence, internet dependence, overuse of an electronic device, overuse of a game device, sex dependence, bulimia, binge eating disorder, and glioblastoma.
[50] The compound according to [46] or a pharmaceutically acceptable salt thereof, wherein the disease which is improved by inhibiting PDE7 is a disease selected from the group consisting of alcohol dependence, drug dependence, and glioblastoma.
[51] A method for treating or preventing a disease which is improved by inhibiting PDE7 comprising administering to a patient an effective amount of the PDE7 inhibitor according to any one of [1] to [14] or the compound according to any one of [15] to [27] or a pharmaceutically acceptable salt thereof.
[52] The method for treating or preventing according to [51], wherein the disease which is improved by inhibiting PDE7 is a disease selected from the group consisting of a psychiatric disorder and a neurological disorder, a movement disorder, cancer and leukemia, pain, an inflammatory disease and an immunological disease, and a cardiovascular disease.
[53] The method for treating or preventing according to [51], wherein the disease which is improved by inhibiting PDE7 is a disease selected from the group consisting of

(i) dependence on an addictive drug and a specified act (for example, alcohol dependence, drug dependence such as nicotine dependence and cocaine dependence, gambling dependence, internet dependence, overuse of an electronic device, overuse of a game device, shopping dependence, sex dependence, bulimia, binge eating disorder, kleptomania, pyromania, or trichotillomania), obsessive-compulsive disorder, post-traumatic stress disorder (PTSD), anxiety, depression, mood disorder, insomnia, delirium disorder, psychiatric disease,

schizophrenia-related disorder, attention deficit hyperactivity disorder (ADHD) in a child with hyperactivity, migraine, stress, a disorder related to a disease caused by psychosomatic disease, panic attack, epilepsy, memory disorder, cognitive disorder, Alzheimer's disease, senile dementia, attention disorder, wakefulness disorder, ischemia, and brain injury-related disorder;
(ii) Parkinson's disease, dopa-responsive dystonia, spinal cord injury, dyskinesia, a disorder related to acute or chronic neurodegenerative disease (including Huntington's chorea), Shy-Drager syndrome, periodic limb movement disorder (PLMD), periodic limb movements in sleep (PLMS), Tourette's syndrome, and restless legs syndrome (RLS);
(iii) glioblastoma and chronic lymphocytic leukemia;
(iv) neuropathic pain and visceral pain;
(v) autoimmune encephalomyelitis, multiple sclerosis, atopic dermatitis, allergic rhinitis, asthma, psoriasis, Crohn's disease, ulcerative colitis, rheumatoid arthritis, post-transplantation rejection, diabetes mellitus, and chronic obstructive pulmonary disease (COPD); and
(vi) myocardial infarction.

[54] The method for treating or preventing according to [51], wherein the disease which is improved by inhibiting PDE7 is a disease selected from the group consisting of alcohol dependence, drug dependence, gambling dependence, internet dependence, overuse of an electronic device, overuse of a game device, sex dependence, bulimia, binge eating disorder, and glioblastoma.
[55] The method for treating or preventing according to [51], wherein the disease which is improved by inhibiting PDE7 is a disease selected from the group consisting of alcohol dependence, drug dependence, and glioblastoma.

EFFECT OF INVENTION

The compounds of the present invention or pharmaceutically acceptable salts thereof, and pharmaceutical compositions comprising the same as an active ingredient, and methods of treatment or prevention using the same have excellent PDE7 inhibitory effects. The compounds of the present invention or pharmaceutically acceptable salts thereof, and pharmaceutical compositions comprising the same as an active ingredient, and methods of treatment or prevention using the same have inhibitory effects on cAMP degradation based on the PDE7 inhibitory effects.

MODE FOR CARRYING OUT THE INVENTION

The definition of each term used in the present description is as follows.
The term of "alkyl" refers to a straight or branched saturated hydrocarbon chain having 1 to 6 carbon atom(s) ($C_1$-$C_6$), for example 1 to 4 carbon atom(s) ($C_1$-$C_4$), and examples thereof include methyl, ethyl, propyl, isopropyl, butyl, tert-butyl, and isobutyl groups, and various branched chain isomers thereof.
The term of "alkenyl" refers to a straight or branched unsaturated hydrocarbon chain having one carbon-carbon double bond and having 2 to 6 carbon atoms ($C_2$-$C_6$), for example 2 to 4 carbon atoms ($C_2$-$C_4$), and examples thereof include vinyl, propenyl, isopropenyl, and butenyl groups, and various branched chain isomers thereof.
The term of "alkylene" refers to a straight or branched divalent saturated hydrocarbon chain having 1 to 6 carbon atom(s) ($C_1$-$C_6$), for example 1 to 4 carbon atom(s) ($C_1$-$C_4$), and examples thereof include methylene, ethylene, propylene, trimethylene, butylene, tetramethylene, pentamethylene, and 1,1,2,2-tetramethylethylene groups, and various branched chain isomers thereof.
The term of "straight alkylene" refers to a straight divalent saturated hydrocarbon chain having 1 to 6 carbon atom(s) ($C_1$-$C_6$), for example 1 to 4 carbon atom(s) ($C_1$-$C_4$), and examples thereof include methylene, ethylene, trimethylene, tetramethylene, and pentamethylene groups.
The term of "alkylidene" refers to, for example, a group represented by R'R"C = (wherein R' and R" are each independently selected from a hydrogen atom and an alkyl group), and examples thereof include methylidene, ethylidene, propylidene, propan-2-ylidene, butylidene, and butan-2-ylidene groups.
The term of "cycloalkyl" refers to a monocyclic alicyclic saturated hydrocarbon group having 3 to 8 ring carbon atoms ($C_3$-$C_8$), for example 3 to 6 ring carbon atoms ($C_3$-$C_6$), and examples thereof include cyclopropyl, cyclobutyl, cyclopentyl, cyclohexyl, cycloheptyl, and cyclooctyl groups.
The term of "cycloalkenyl" refers to a monocyclic alicyclic unsaturated hydrocarbon group having one carbon-carbon double bond and having 3 to 8 ring carbon atoms ($C_3$-$C_8$), for example 3 to 6 ring carbon atoms ($C_3$-$C_6$), and examples thereof include cyclopropenyl, cyclobutenyl, cyclopentenyl, cyclohexenyl, cycloheptenyl, and cyclooctenyl groups.
The term of "alicyclic hydrocarbon group" refers to a monocyclic, bicyclic, or tricyclic alicyclic hydrocarbon group having 3 to 14 ring carbon atoms ($C_3$-$C_{14}$), and examples thereof include monocyclic alicyclic hydrocarbon groups such

as cycloalkyl groups having 3 to 8 ring carbon atoms ($C_3$-$C_8$) (for example, a cyclopropyl group, a cyclobutyl group, a cyclopentyl group, a cyclohexyl group, a cycloheptyl group, or a cyclooctyl group), and cycloalkenyl groups having 3 to 8 ring carbon atoms ($C_3$-$C_8$) (for example, a cyclopropenyl group, a cyclobutenyl group, a cyclopentenyl group, a cyclohexenyl group, a cycloheptenyl group, or a cyclooctenyl group); bicyclic alicyclic hydrocarbon groups having 6 to 12 ring carbon atoms such as bicycloalkyl groups having a 6 to 12 ring carbon atoms ($C_6$-$C_{12}$) (for example, a bicyclohexyl group, a bicycloheptyl group, a bicyclooctyl group, a bicyclononyl group, a bicyclodecyl group, a bicycloundecyl group, or a bicyclododecyl group), bicycloalkenyl groups having 6 to 12 ring carbon atoms ($C_6$-$C_{12}$) (for example, a bicyclohexenyl group, a bicycloheptenyl group, a bicyclooctenyl group, a bicyclononenyl group, a bicyclodecenyl group, a bicycloundecenyl group, or a bicyclododecenyl group), and spiroalkyl groups having 6 to 12 ring carbon atoms ($C_6$-$C_{12}$) (for example, a spirohexyl group, a spiroheptyl group, a spirooctyl group, a spirononyl group, a spirodecyl group, a spiroundecyl group, or a spirododecyl group); and tricyclic alicyclic hydrocarbon groups such as tricycloalkyl groups having 10 to 14 ring carbon atoms ($C_{10}$-$C_{14}$) such as adamantyl.

The term of "monocyclic saturated hydrocarbon group" refers to a ring structure formed by, for example, a group represented by $>CR^{L1}R^{L2}$, $>CR^{LII-1}R^{LII-2}$, or $>CR^{LIII-1}R^{LIII-2}$ (wherein $R^{L1}$, $R^{L2}$, $R^{LII-1}$, $R^{LII-2}$, $R^{LIII-1}$, and $R^{LIII-2}$ have the same meanings as those described above) wherein $R^{L1}$ and $R^{L2}$, $R^{LII-1}$ and $R^{LII-2}$, or $R^{LIII-1}$ and $R^{LIII-2}$ are combined with each other together with the carbon atom to which they are attached to form said ring. The number of ring carbon atoms is 3 to 8 ($C_3$-$C_8$), for example 3 to 6 ($C_3$-$C_6$).

The term of "halogen atom" refers to a fluorine atom, a chlorine atom, a bromine atom, or an iodine atom.

The term of "alkoxy" refers to a group in which an oxygen atom is attached to said straight or branched alkyl, and examples thereof include methoxy, ethoxy, propoxy, isopropoxy, butoxy, tert-butoxy, and isobutoxy groups, and various branched chain isomers thereof.

The term of "alkylthio" refers to a group in which a sulfur atom is attached to said straight or branched alkyl, and examples thereof include methylthio, ethylthio, propylthio, isopropylthio, butylthio, tert-butylthio, and isobutylthio groups, and various branched chain isomers thereof.

The term of "alkylcarbonyl" refers to a group in which a carbonyl group is attached to said straight or branched alkyl, and examples thereof include methylcarbonyl (i.e., acetyl), ethylcarbonyl (i.e., propionyl), propylcarbonyl (i.e., butyryl), and butylcarbonyl (i.e., pentanoyl) groups, and various branched chain isomers thereof.

The term of "alkoxycarbonyl" refers to a group in which a carbonyl group is attached to said straight or branched alkoxy, and examples thereof include methoxycarbonyl, ethoxycarbonyl, propoxycarbonyl, isopropoxycarbonyl, butoxycarbonyl, tert-butoxycarbonyl, and isobutoxycarbonyl groups, and various branched chain isomers thereof.

The term of "aryl" refers to a monocyclic or bicyclic aromatic hydrocarbon group having 6 to 11 ring carbon atoms ($C_6$-$C_{11}$), and examples thereof include monocyclic aryl groups such as a phenyl group; and optionally partially saturated bicyclic aryl groups having 9 to 11 ring carbon atoms ($C_9$-$C_{11}$) such as naphthyl, tetrahydronaphthyl, indenyl, and indanyl groups.

The term of "heteroaryl" refers to a 5 to 11 membered monocyclic or bicyclic aromatic heterocyclic group comprising 1 to 4 heteroatom(s) selected from an oxygen atom, a sulfur atom, and a nitrogen atom other than carbon atom(s), and examples thereof include 5 to 6 membered monocyclic heteroaryl groups comprising 1 to 4 heteroatom(s) selected from an oxygen atom, a sulfur atom, and a nitrogen atom other than carbon atom(s) such as pyrrolyl, furyl, thienyl, pyrazolyl, imidazolyl, oxazolyl, isoxazolyl, thiazolyl, isothiazolyl, thiadiazolyl, pyridyl, pyrazinyl, pyrimidinyl, pyridazinyl, and triazinyl groups; and 8 to 11 membered bicyclic heteroaryl groups comprising 1 to 4 heteroatom(s) selected from an oxygen atom, a sulfur atom, and a nitrogen atom other than carbon atom(s) such as indolyl, indolinyl, isoindolinyl, indazolyl, tetrahydroindazolyl, benzofuranyl, dihydrobenzofuranyl, dihydroisobenzofuranyl, benzothiophenyl, dihydrobenzothiophenyl, dihydroisobenzothiophenyl, benzoxazolyl, dihydrobenzoxazolyl, benzothiazolyl, dihydrobenzothiazolyl, quinolyl, tetrahydroquinolyl, isoquinolyl, tetrahydroisoquinolyl, naphthyridinyl, tetrahydronaphthyridinyl, quinoxalinyl, tetrahydroquinoxalinyl, and quinazolinyl groups.

The term of "nonaromatic heterocyclic group" refers to a 4 to 8 membered monocyclic nonaromatic heterocyclic group or a 6 to 12 membered bicyclic nonaromatic heterocyclic group comprising 1 to 4 heteroatom(s) selected from an oxygen atom, a sulfur atom, and a nitrogen atom other than carbon atom(s), and examples thereof include azetidinyl, oxetanyl, thietanyl, pyrrolidinyl, piperidinyl, piperidino, tetrahydrofuryl, tetrahydropyranyl, tetrahydrothienyl (i.e., thiolanyl), piperazinyl, morpholinyl, morpholino, perhydroazepinyl, perhydroazocinyl, 6 to 12 membered azabicycloalkyl (for example, azabicyclohexyl, azabicycloheptyl, azabicyclooctyl, azabicyclononyl, azabicyclodecyl, azabicycloundecyl, and azabicyclododecyl), 6 to 12 membered azabicycloalkenyl (for example, azabicyclohexenyl, azabicycloheptenyl, azabicyclooctenyl, azabicyclononenyl, azabicyclodecenyl, azabicycloundecenyl, and azabicyclododecenyl), and 6 to 12 membered azaspiroalkyl (for example, azaspirohexyl, azaspiroheptyl, azaspirooctyl, azaspirononyl, azaspirodecyl, azaspiroundecyl, and azaspirododecyl) groups.

The term of "aryloxy" refers to a group in which an oxygen atom is attached to said aryl, and examples thereof include phenoxy and naphthyloxy groups.

The term of "arylalkyloxy" refers to a group in which said alkoxy is attached to said aryl, and examples thereof

include a benzyloxy group.

The term of "arylcarbonyl" refers to a group in which a carbonyl group is attached to said aryl, and examples thereof include a phenylcarbonyl (i.e., benzoyl) group.

Examples of the term of "optionally substituted alkyl group" include an alkyl group optionally substituted with the same or different 1 to 7 (for example, 1 to 5 or 1 to 3) group(s) selected from a cyano group, a hydroxy group, a nitro group, an amino group, an oxo group, an optionally substituted cycloalkyl group, an optionally substituted nonaromatic heterocyclic group, an optionally substituted alkoxy group, an optionally substituted aryl group, an optionally substituted heteroaryl group, an aryloxy group, an arylalkyloxy group, and a halogen atom. Preferably, "optionally substituted alkyl group" is an alkyl group optionally substituted with the same or different 1 to 7 (for example, 1 to 5 or 1 to 3) substituent(s) selected from a halogen atom, a hydroxy group, an aryloxy group, an arylalkyloxy group, and an optionally substituted aryl group.

In one aspect, the substituent(s) of "optionally substituted alkyl group" in $R^{X1a}$, $R^{X1b}$, $R^{X1c}$, and $R^{X1d}$ is/are the same or different 1 to 7 (for example, 1 to 5 or 1 to 3) halogen atom(s).

In one aspect, the substituent(s) of "optionally substituted alkyl group" in $R^{X2a}$, $R^{X2b}$, $R^{X2c}$, and $R^{X2d}$ is/are the same or different 1 to 7 (for example, 1 to 5 or 1 to 3) halogen atom(s).

In one aspect, the substituent(s) of "optionally substituted alkyl group" in $R^{X3a}$, $R^{X3b}$, $R^{X3c}$, and $R^{X3d}$ is/are the same or different 1 to 7 (for example, 1 to 5 or 1 to 3) halogen atom(s).

In one aspect, the substituent(s) of "optionally substituted alkyl group" in $R^{Z1a}$, $R^{Z1b}$, and $R^{Z1c}$ is/are the same or different 1 to 7 (for example, 1 to 5 or 1 to 3) halogen atom(s).

In one aspect, the substituent(s) of "optionally substituted alkyl group" in $R^{Z1d}$ is/are the same or different 1 to 5 (for example, 1 to 3) halogen atom(s).

In one aspect, the substituent(s) of "optionally substituted alkyl group" in $R^{Z2a}$, $R^{Z2b}$, and $R^{Z2d}$ is/are the same or different 1 to 7 (for example, 1 to 5 or 1 to 3) halogen atom(s).

In one aspect, the substituent(s) of "optionally substituted alkyl group" in $R^{Z2c}$ is/are the same or different 1 to 5 (for example, 1 to 3) halogen atom(s).

In one aspect, the substituent(s) of "optionally substituted alkyl group" in $R^{L1}$ and $R^{L2}$ is/are the same or different 1 to 7 (for example, 1 to 5 or 1 to 3) halogen atom(s).

In one aspect, the substituent(s) of "optionally substituted alkyl group" which is a substituent of (i) an optionally substituted aryl group in Cy is/are the same or different 1 to 7 (for example, 1 to 5 or 1 to 3) halogen atom(s).

In one aspect, the substituent(s) of "optionally substituted alkyl group" which is a substituent of (ii) an optionally substituted heteroaryl group in Cy is/are the same or different 1 to 7 (for example, 1 to 5 or 1 to 3) halogen atom(s).

In one aspect, the substituent(s) of "optionally substituted alkyl group" which is a substituent of (iii) an optionally substituted alicyclic hydrocarbon group in Cy is/are the same or different 1, 2, or 3 substituent(s) selected from a halogen atom, a hydroxy group, an aryloxy group, an arylalkyloxy group, and an aryl group optionally substituted with the same or different 1, 2, or 3 substituent(s) selected from an alkyl group optionally substituted with the same or different 1 to 7 halogen atom(s) and a halogen atom.

In one aspect, the substituent(s) of "optionally substituted alkyl group" which is a substituent of (iv) an optionally substituted nonaromatic heterocyclic group in Cy is/are the same or different 1, 2, or 3 substituent(s) selected from an alkoxy group optionally substituted with the same or different 1 to 7 halogen atom(s), a halogen atom, and an aryl group optionally substituted with the same or different 1, 2, or 3 substituent(s) selected from an alkyl group optionally substituted with the same or different 1 to 7 halogen atom(s) and a halogen atom.

Examples of the term of "optionally substituted alkylthio group" include an alkylthio group optionally substituted with the same or different 1 to 7 (for example, 1 to 5 or 1 to 3) group(s) selected from a cyano group, a hydroxy group, a nitro group, an amino group, an oxo group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted alkylthio group" is an alkylthio group optionally substituted with the same or different 1 to 7 (for example, 1 to 5 or 1 to 3) halogen atom(s).

In one aspect, the substituent(s) of "optionally substituted alkylthio group" in $R^{X2a}$, $R^{X2b}$, $R^{X2c}$, and $R^{X2d}$ is/are the same or different 1 to 7 (for example, 1 to 5 or 1 to 3) halogen atom(s).

Examples of the term of "optionally substituted alkoxy group" include an alkoxy group optionally substituted with the same or different 1 to 7 (for example, 1 to 5 or 1 to 3) group(s) selected from a cyano group, a hydroxy group, a nitro group, an amino group, an oxo group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted alkoxy group" is an alkoxy group optionally substituted with the same or different 1 to 7 (for example, 1 to 5 or 1 to 3) substituent(s) selected from a halogen atom and an aryl group.

In one aspect, the substituent(s) of "optionally substituted alkoxy group" in $R^{X2a}$, $R^{X2b}$, $R^{X2c}$, and $R^{X2d}$ is/are the same or different 1 to 7 (for example, 1 to 5 or 1 to 3) halogen atom(s).

In one aspect, the substituent(s) of "optionally substituted alkoxy group" which is a substituent of (i) an optionally substituted aryl group in Cy is/are the same or different 1, 2, or 3 substituent(s) selected from a halogen atom and an aryl group.

In one aspect, the substituent(s) of "optionally substituted alkoxy group" which is a substituent of (iii) an optionally substituted alicyclic hydrocarbon group in Cy is/are the same or different 1 to 7 (for example, 1 to 5 or 1 to 3) halogen atom(s).

In one aspect, the substituent(s) of "optionally substituted alkoxy group" which is a substituent of (iv) an optionally substituted nonaromatic heterocyclic group in Cy is/are the same or different 1 to 7 (for example, 1 to 5 or 1 to 3) halogen atom(s).

Examples of the term of "optionally substituted cycloalkyl group" include a cycloalkyl group optionally substituted with the same or different 1 to 5 (for example, 1 to 3) group(s) selected from a cyano group, a hydroxy group, a nitro group, an amino group, an oxo group, an optionally substituted alkyl group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted cycloalkyl group" is a cycloalkyl group optionally substituted with the same or different 1 to 5 halogen atom(s).

In one aspect, the substituent(s) of "optionally substituted cycloalkyl group" in $R^{X3a}$, $R^{X3b}$, $R^{X3c}$, and $R^{X3d}$ is/are the same or different 1 to 5 (for example, 1 to 3) halogen atom(s).

In one aspect, the substituent(s) of "optionally substituted cycloalkyl group" in $R^{Z2a}$, $R^{Z2b}$, and $R^{Z2d}$ is/are the same or different 1 to 5 (for example, 1 to 3) halogen atom(s).

In one aspect, the substituent(s) of "optionally substituted cycloalkyl group" which is a substituent of (iv) an optionally substituted nonaromatic heterocyclic group in Cy is/are the same or different 1 to 5 (for example, 1 to 3) halogen atom(s).

Examples of the term of "optionally substituted aryl group" include an aryl group optionally substituted with the same or different 1 to 5 (for example, 1 to 3) group(s) selected from a cyano group, a hydroxy group, a nitro group, an amino group, an oxo group, an optionally substituted alkyl group, an optionally substituted cycloalkyl group, an optionally substituted nonaromatic heterocyclic group, an optionally substituted alkoxy group, an optionally substituted aryl group, an optionally substituted heteroaryl group, an optionally substituted carboxamide group, and a halogen atom. Preferably, "optionally substituted aryl group" is an aryl group optionally substituted with the same or different 1 to 5 (for example, 1 to 3) substituent(s) selected from an optionally substituted alkyl group, an optionally substituted alkoxy group, a halogen atom, and an optionally substituted carboxamide group.

In one aspect, the substituent(s) of "optionally substituted aryl group" in $R^{X3a}$, $R^{X3b}$, $R^{X3c}$, and $R^{X3d}$ is/are the same or different 1 to 5 (for example, 1 to 3) halogen atom(s).

In one aspect, the substituent(s) of "optionally substituted aryl group" which is a substituent of (iii) an optionally substituted alicyclic hydrocarbon group in Cy is/are the same or different 1 to 5 (for example, 1 to 3) halogen atom(s).

In one aspect, the substituent(s) of "optionally substituted aryl group" which is a substituent of (iv) an optionally substituted nonaromatic heterocyclic group in Cy is/are the same or different 1 to 5 (for example, 1 to 3) halogen atom(s).

Examples of the term of "optionally substituted heteroaryl group" include a heteroaryl group optionally substituted with the same or different 1 to 5 (for example, 1 to 3) group(s) selected from a cyano group, a hydroxy group, a nitro group, an amino group, an oxo group, an optionally substituted alkyl group, an optionally substituted cycloalkyl group, an optionally substituted nonaromatic heterocyclic group, an optionally substituted alkoxy group, an optionally substituted aryl group, an optionally substituted heteroaryl group, an optionally substituted carboxamide group, and a halogen atom. Preferably, "optionally substituted heteroaryl group" is a heteroaryl group optionally substituted with the same or different 1 to 5 (for example, 1 to 3) substituent(s) selected from an optionally substituted alkyl group and a halogen atom.

In one aspect, the substituent(s) of "optionally substituted heteroaryl group" which is a substituent of (iii) an optionally substituted alicyclic hydrocarbon group in Cy is/are the same or different 1, 2, or 3 substituent(s) selected from an alkyl group optionally substituted with the same or different 1 to 7 halogen atom(s) and a halogen atom.

In one aspect, the substituent(s) of "optionally substituted heteroaryl group" which is a substituent of (iv) an optionally substituted nonaromatic heterocyclic group in Cy is/are the same or different 1 to 5 (for example, 1 to 3) halogen atom(s).

Examples of the term of "optionally substituted alicyclic hydrocarbon group" include an alicyclic hydrocarbon group optionally substituted with the same or different 1 to 5 (for example, 1 to 3) group(s) selected from a cyano group, a hydroxy group, a nitro group, an amino group, an oxo group, an optionally substituted alkyl group, an optionally substituted alkenyl group, an optionally substituted alkylidene group, an optionally substituted cycloalkyl group, an optionally substituted nonaromatic heterocyclic group, an optionally substituted alkoxy group, an optionally substituted aryl group, an optionally substituted heteroaryl group, an optionally substituted alicyclic hydrocarbon group, and a halogen atom. Preferably, "optionally substituted alicyclic hydrocarbon group" is an alicyclic hydrocarbon group optionally substituted with the same or different 1 to 5 (for example, 1 to 3) substituent(s) selected from an optionally substituted alkyl group, an optionally substituted alkenyl group, an optionally substituted alkylidene group, an optionally substituted alkoxy

group, a hydroxy group, a halogen atom, an oxo group, an optionally substituted aryl group, and an optionally substituted heteroaryl group.

Examples of the term of "optionally substituted nonaromatic heterocyclic group" include a nonaromatic heterocyclic group optionally substituted with the same or different 1 to 5 (for example, 1 to 3) group(s) selected from a cyano group, a hydroxy group, a nitro group, an amino group, an oxo group, an optionally substituted alkyl group, an optionally substituted cycloalkyl group, an optionally substituted nonaromatic heterocyclic group, an optionally substituted alkoxy group, an optionally substituted aryl group, an optionally substituted heteroaryl group, an optionally substituted alkylcarbonyl group, a formyl group, an optionally substituted alkoxycarbonyl group, an optionally substituted arylcarbonyl group, and a halogen atom. Preferably, "optionally substituted nonaromatic heterocyclic group" is a nonaromatic heterocyclic group optionally substituted with the same or different 1 to 5 (for example, 1 to 3) substituent(s) selected from an optionally substituted alkyl group, an optionally substituted cycloalkyl group, an optionally substituted alkoxy group, a hydroxy group, a halogen atom, an oxo group, an optionally substituted aryl group, an optionally substituted heteroaryl group, an optionally substituted alkylcarbonyl group, a formyl group, an optionally substituted alkoxycarbonyl group, and an optionally substituted arylcarbonyl group.

Examples of the term of "optionally substituted monocyclic saturated hydrocarbon group" include an monocyclic saturated hydrocarbon group optionally substituted with the same or different 1 to 6 (for example, 1 to 4) group(s) selected from a cyano group, a hydroxy group, a nitro group, an amino group, an oxo group, an optionally substituted alkyl group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted monocyclic saturated hydrocarbon group" is a monocyclic saturated hydrocarbon group optionally substituted with the same or different 1 to 6 (for example, 1 to 4) halogen atom(s).

In one aspect, the substituent(s) of "optionally substituted monocyclic saturated hydrocarbon group" formed by combining $R^{L1}$ and $R^{L2}$ with each other together with the carbon atom to which they are attached is/are the same or different 1 to 6 (for example, 1 to 4) halogen atom(s).

Examples of the term of "optionally substituted carboxamide group" include a carboxamide group optionally substituted with the same or different 1 to 2 group(s) selected from an optionally substituted alkyl group, an optionally substituted cycloalkyl group, an optionally substituted nonaromatic heterocyclic group, an optionally substituted aryl group, and an optionally substituted heteroaryl group. Preferably, "optionally substituted carboxamide group" is a carboxamide group optionally substituted with the same or different 1 to 2 optionally substituted alkyl group(s).

In one aspect, the substituent(s) of "optionally substituted carboxamide group" which is a substituent of (i) an optionally substituted aryl group in Cy is/are the same or different 1 or 2 alkyl group(s) optionally substituted with the same or different 1, 2, or 3 aryl group(s).

Examples of the term of "optionally substituted alkenyl group" include an alkenyl group optionally substituted with the same or different 1 to 5 (for example, 1 to 3) group(s) selected from a cyano group, a hydroxy group, a nitro group, an amino group, an oxo group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted alkenyl group" is an alkenyl group optionally substituted with the same or different 1 to 5 (for example, 1 to 3) halogen atom(s).

In one aspect, the substituent(s) of "optionally substituted alkenyl group" which is a substituent of (iii) an optionally substituted alicyclic hydrocarbon group in Cy is/are the same or different 1 to 5 (for example, 1 to 3) halogen atom(s).

Examples of the term of "optionally substituted alkylidene group" include an alkylidene group optionally substituted with the same or different 1 to 6 (for example, 1 to 4) group(s) selected from a cyano group, a hydroxy group, a nitro group, an amino group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted alkylidene group" is an alkylidene group optionally substituted with the same or different 1 to 6 (for example, 1 to 4) halogen atom(s).

In one aspect, the substituent(s) of "optionally substituted alkylidene group" which is a substituent of (iii) an optionally substituted alicyclic hydrocarbon group in Cy is/are the same or different 1 to 6 (for example, 1 to 4) halogen atom(s).

Examples of the term of "optionally substituted alkylcarbonyl group" include an alkylcarbonyl group optionally substituted with the same or different 1 to 5 (for example, 1 to 3) group(s) selected from a cyano group, a hydroxy group, a nitro group, an amino group, an oxo group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted alkylcarbonyl group" is an alkylcarbonyl group optionally substituted with the same or different 1 to 5 (for example, 1 to 3) optionally substituted aryl group(s).

In one aspect, the substituent(s) of "optionally substituted alkylcarbonyl group" which is a substituent of (iv) an optionally substituted nonaromatic heterocyclic group in Cy is/are the same or different 1, 2, or 3 aryl group(s).

Examples of the term of "optionally substituted alkoxycarbonyl group" include an alkoxycarbonyl group optionally substituted with the same or different 1 to 7 (for example, 1 to 5 or 1 to 3) group(s) selected from a cyano group, a hydroxy group, a nitro group, an amino group, an oxo group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted alkoxycarbonyl group" is an alkoxycarbonyl group optionally substituted with the same or different 1 to 7 (for example, 1 to 5 or 1 to 3) halogen atom(s).

In one aspect, the substituent(s) of "optionally substituted alkoxycarbonyl group" which is a substituent of (iv) an optionally substituted nonaromatic heterocyclic group in Cy is/are the same or different 1 to 7 (for example, 1 to 5 or 1 to 3) halogen atom(s).

Examples of the term of "optionally substituted arylcarbonyl group" include an arylcarbonyl group optionally substituted with the same or different 1 to 5 (for example, 1 to 3) group(s) selected from a cyano group, a hydroxy group, a nitro group, an amino group, an oxo group, an optionally substituted alkyl group, an optionally substituted cycloalkyl group, an optionally substituted nonaromatic heterocyclic group, an optionally substituted alkoxy group, an optionally substituted aryl group, an optionally substituted heteroaryl group, and a halogen atom. Preferably, "optionally substituted arylcarbonyl group" is an arylcarbonyl group optionally substituted with the same or different 1 to 5 (for example, 1 to 3) halogen atom(s).

In one aspect, the substituent(s) of "optionally substituted arylcarbonyl group" which is a substituent of (iv) an optionally substituted nonaromatic heterocyclic group in Cy is/are the same or different 1 to 5 (for example, 1 to 3) halogen atom(s).

Hereinafter, the embodiments of the present invention, of which the specific aspects are described in the above [1] to [55], are described in detail.

(PDE7 inhibitor)

The present invention provides a PDE7 inhibitor comprising a compound represented by the formula (I):

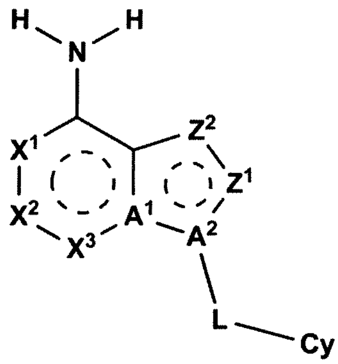

(I)

[wherein:
the partial structure represented by the following formula (I-1):

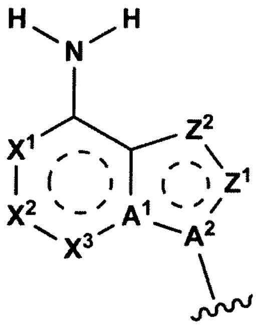

(I-1)

represents a partial structure selected from the group consisting of the following formula (I-1-A):

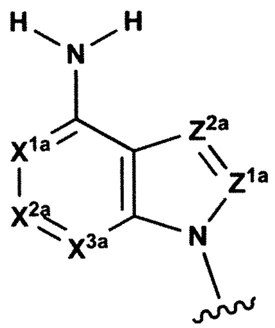

(I-1-A)

(wherein $X^{1a}$, $X^{2a}$, $X^{3a}$, $Z^{1a}$, and $Z^{2a}$ have the same meanings as those described above); the following formula (I-1-B) :

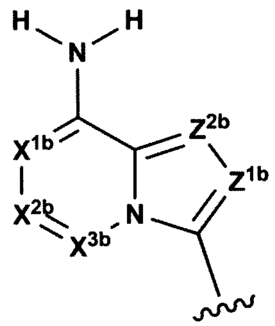

(I-1-B)

(wherein $X^{1b}$, $X^{2b}$, $X^{3b}$, $Z^{1b}$, and $Z^{2b}$ have the same meanings as those described above); the following formula (I-1-C):

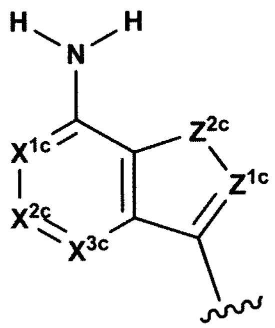

(I-1-C)

(wherein $X^{1c}$, $X^{2c}$, $X^{3c}$, $Z^{1c}$, and $Z^{2c}$ have the same meanings as those described above); and the following formula (I-1-D) :

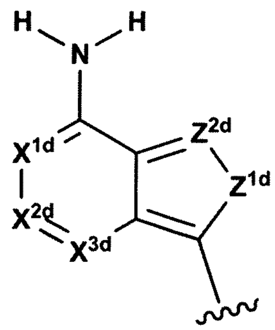

(I-1-D)

(wherein $X^{1d}$, $X^{2d}$, $X^{3d}$, $Z^{1d}$, and $Z^{2d}$ have the same meanings as those described above); and

L and Cy each have the same meaning as those described above]

or a pharmaceutically acceptable salt thereof as an active ingredient.

Unless otherwise specified, the wavy line: 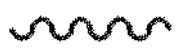 in the formulas described in the present description represents the point of attachment to the rest of the molecule.

In one embodiment, in each compound represented by the formula (I-1-A), (I-1-B), (I-1-C), and (I-1-D), $X^{1a}$, $X^{1c}$, and $X^{1d}$ each are N, and $X^{1b}$ is $CR^{X1b}$. In another embodiment, in each compound represented by the formula (I-1-A), (I-1-B), (I-1-C), and (I-1-D), $X^{1a}$, $X^{1b}$, $X^{1c}$, and $X^{1d}$ each are N.

In one embodiment, in each compound represented by the formula (I-1-A), (I-1-B), (I-1-C) and (I-1-D), $X^{2a}$ is $CR^{X2a}$, $X^{2b}$ is $CR^{X2b}$, $X^{2c}$ is $CR^{X2c}$, and $X^{2d}$ is $CR^{X2d}$. In another embodiment, in each compound represented by the formula (I-1-A), (I-1-B), (I-1-C), and (I-1-D), $X^{2a}$ is $CR^{X2a}$, $X^{2b}$ is N, $X^{2c}$ is $CR^{X2c}$, and $X^{2d}$ is $CR^{X2d}$.

In one embodiment, in each compound represented by the formula (I-1-A), (I-1-B), (I-1-C), and (I-1-D), $X^{3a}$ is $CR^{X3a}$, $X^{3b}$ is $CR^{X3b}$, $X^{3c}$ is N, and $X^{3d}$ is N. In another embodiment, in each compound represented by the formula (I-1-A), (I-1-B), (I-1-C), and (I-1-D), $X^{3a}$, $X^{3b}$, $X^{3c}$, and $X^{3d}$ each are N.

In one embodiment, in each compound represented by the formula (I-1-A), (I-1-B), (I-1-C), and (I-1-D), $Z^{1a}$ is $CR^{Z1a}$, $Z^{1b}$ is N, $Z^{1c}$ is N, and $Z^{1d}$ is O. In another embodiment, in each compound represented by the formula (I-1-A), (I-1-B), (I-1-C), and (I-1-D), $Z^{1a}$, $Z^{1b}$, and $Z^{1c}$ each are N, and $Z^{1d}$ is O. In still another embodiment, $Z^{1a}$, $Z^{1b}$, and $Z^{1c}$ each are N, and $Z^{1d}$ is $NR^{Z1d}$.

In one embodiment, in each compound represented by the formula (I-1-A), (I-1-B), (I-1-C), and (I-1-D), $Z^{2a}$ is $CR^{Z2a}$, $Z^{2b}$ is $CR^{Z2b}$, $Z^{2c}$ is $NR^{Z2c}$, and $Z^{2d}$ is N. In another embodiment, in each compound represented by the formula (I-1-A), (I-1-B), (I-1-C), and (I-1-D), $Z^{2a}$, $Z^{2b}$, and $Z^{2d}$ each are N, and $Z^{2c}$ is O. In still another embodiment, $Z^{2a}$, $Z^{2b}$, and $Z^{2d}$ each are N, and $Z^{2c}$ is $NR^{Z2c}$.

In one embodiment, the partial structure represented by the formula (I-1-A) has a structure represented by the following formula (I-1-a1), (I-1-a2), (I-1-a3), (I-1-a4), or (I-1-a5) :

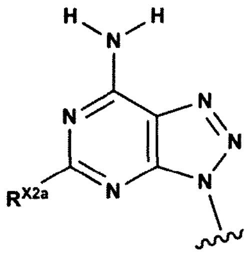

**(I-1-a1)**

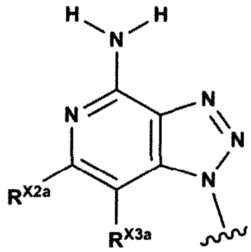

**(I-1-a2)**

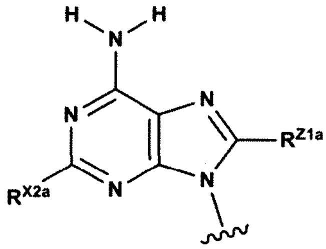

**(I-1-a3)**

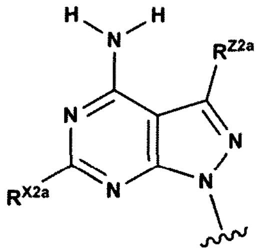

**(I-1-a4)**

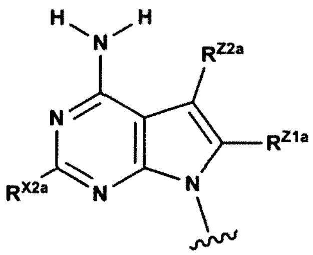

**(I-1-a5)**

(wherein the symbols have the same meanings as those described above). In another embodiment, the partial structure represented by the formula (I-1-A) has a structure represented by the following formula (I-1-a6), (I-1-a7), (I-1-a8), (I-1-a9), (I-1-a10), (I-1-a11), (I-1-a12), (I-1-a13), (I-1-a14), (I-1-a15), (I-1-a16), (I-1-a17), (I-1-a18), (I-1-a19), (I-1-a20), (I-1-a21), (I-1-a22), (I-1-a23), or (I-1-a24) :

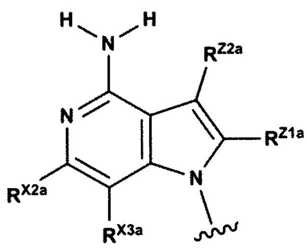
(I-1-a6)

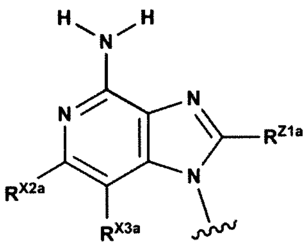
(I-1-a7)

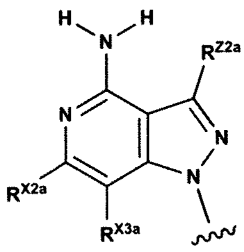
(I-1-a8)

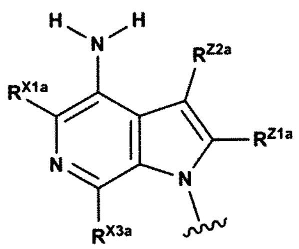
(I-1-a9)

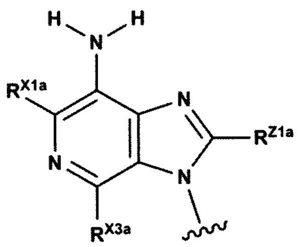
(I-1-a10)

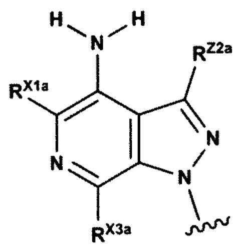
(I-1-a11)

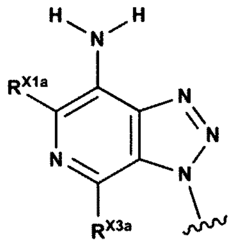
(I-1-a12)

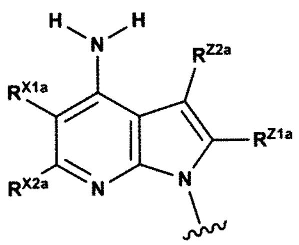
(I-1-a13)

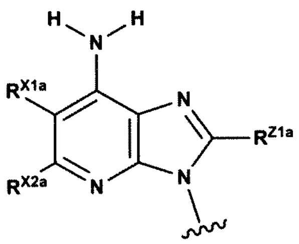
(I-1-a14)

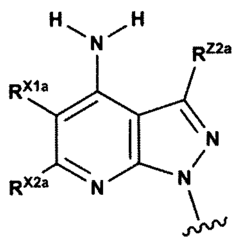
(I-1-a15)

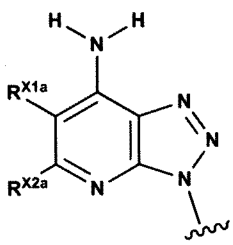
(I-1-a16)

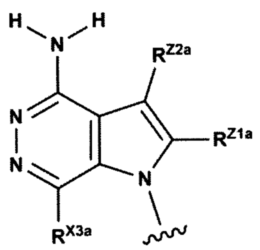
(I-1-a17)

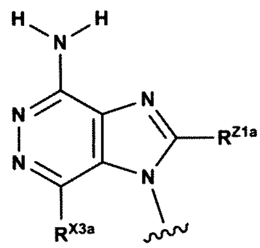
(I-1-a18)

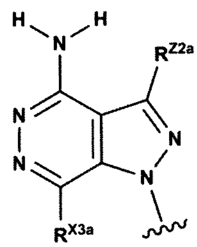
(I-1-a19)

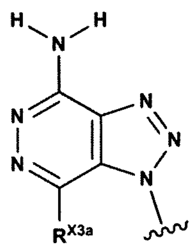
(I-1-a20)

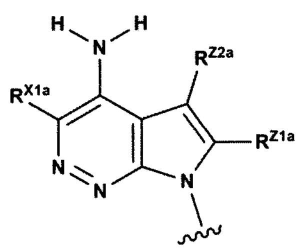
(I-1-a21)

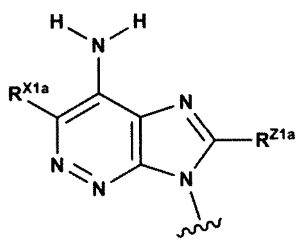
(I-1-a22)

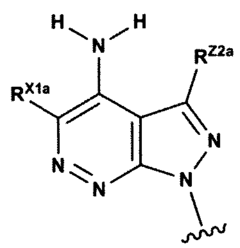
(I-1-a23)

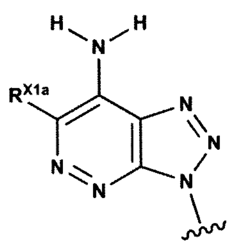
(I-1-a24)

(wherein the symbols have the same meanings as those described above). In a preferable embodiment, the formula (I-1-A) has a structure represented by the formula (I-1-a1).

In one embodiment, the partial structure represented by the formula (I-1-B) has a structure represented by the following formula (I-1-b1), (I-1-b2), (I-1-b3), (I-1-b4), or (I-1-b5):

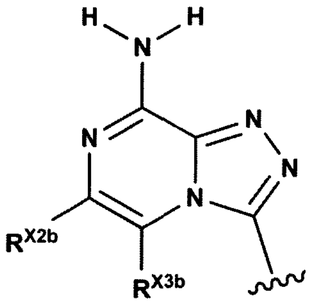

**(I-1-b1)**

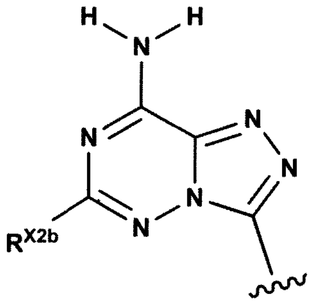

**(I-1-b2)**

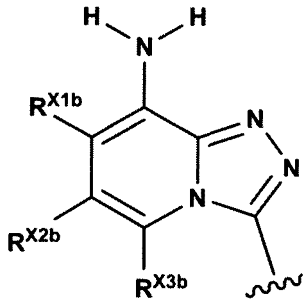

**(I-1-b3)**

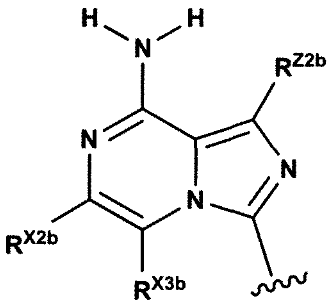

**(I-1-b4)**

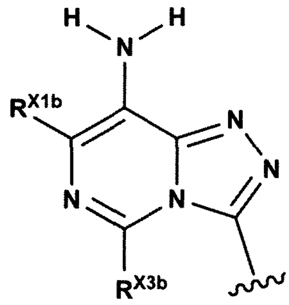

**(I-1-b5)**

(wherein the symbols have the same meanings as those described above). In another embodiment, the partial structure represented by the formula (I-1-B) has a structure represented by the following formula (I-1-b6), (I-1-b7), (I-1-b8), (I-1-b9), (I-1-b10), (I-1-b11), (I-1-b12), (I-1-b13), (I-1-b14), (I-1-b15), (I-1-b16), (I-1-b17), (I-1-b18), (I-1-b19), (I-1-b20), (I-1-b21), (I-1-b22), (I-1-b23), (I-1-b24), (I-1-b25), (I-1-b26), (I-1-b27), or (I-1-b28) :

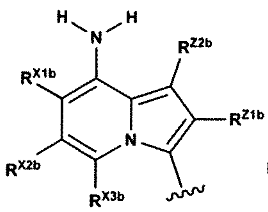

**(I-1-b6)**

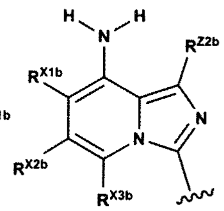

**(I-1-b7)**

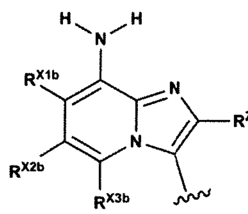

**(I-1-b8)**

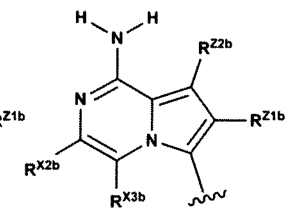

**(I-1-b9)**

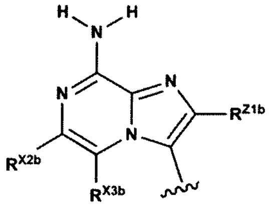

**(I-1-b10)**

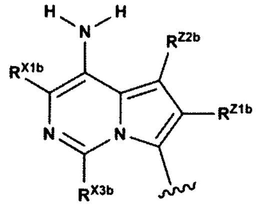

**(I-1-b11)**

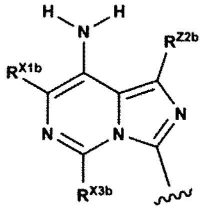

**(I-1-b12)**

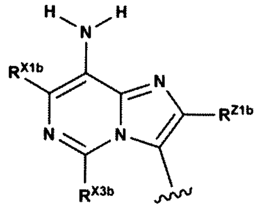

**(I-1-b13)**

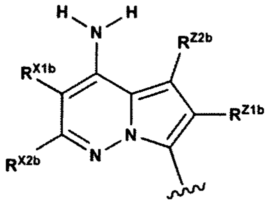
(I-1-b14)

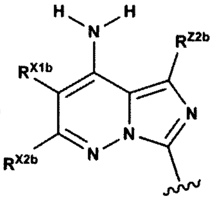
(I-1-b15)

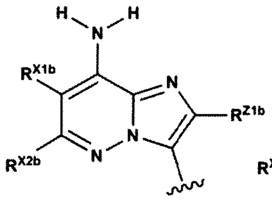
(I-1-b16)

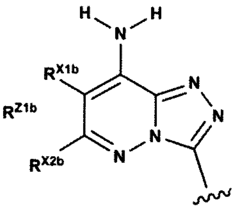
(I-1-b17)

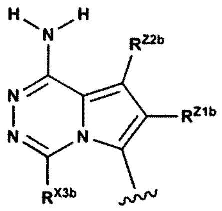
(I-1-b18)

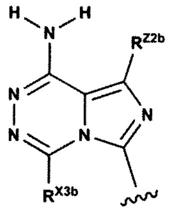
(I-1-b19)

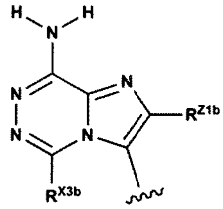
(I-1-b20)

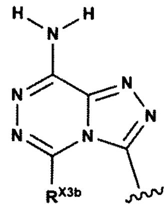
(I-1-b21)

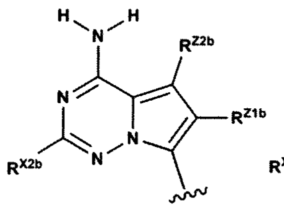
(I-1-b22)

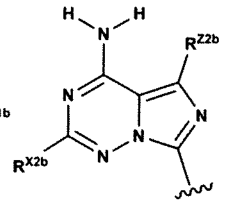
(I-1-b23)

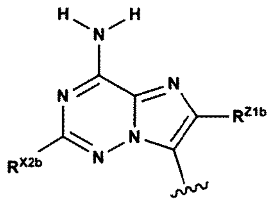
(I-1-b24)

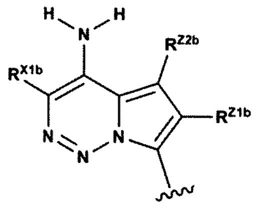
(I-1-b25)

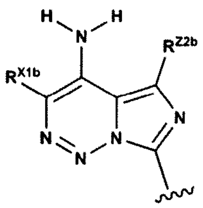
(I-1-b26)

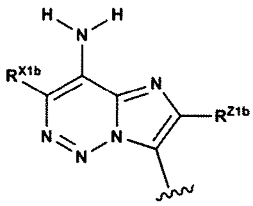
(I-1-b27)

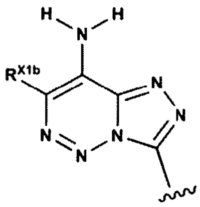
(I-1-b28)

(wherein the symbols have the same meanings as those described above). In a preferable embodiment, the formula (I-1-B) has a structure represented by the formula (I-1-b1) or (I-1-b2).

In one embodiment, the partial structure represented by the formula (I-1-C) has a structure represented by the following formula (I-1-c1) or (I-1-c2):

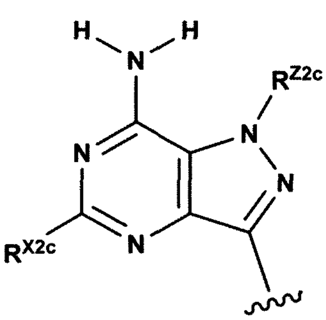
(I-1-c1)

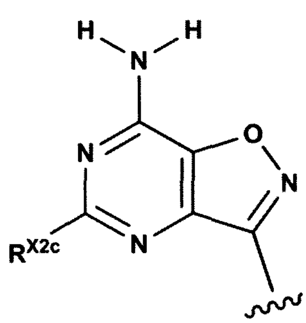
(I-1-c2)

(wherein the symbols have the same meanings as those described above). In another embodiment, the partial structure represented by the formula (I-1-C) has a structure represented by the following formula (I-1-c3), (I-1-c4), (I-1-c5), (I-1-c6), (I-1-c7), (I-1-c8), (I-1-c9), (I-1-c10), (I-1-c11), (I-1-c12), (I-1-c13), (I-1-c14), (I-1-c15), (I-1-c16), (I-1-c17), (I-1-c18), (I-1-c19), (I-1-c20), (I-1-c21), (I-1-c22), (I-1-c23), or (I-1-c24):

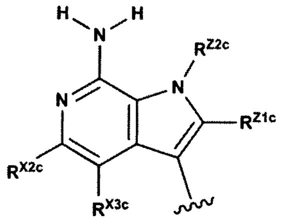
(I-1-c3)

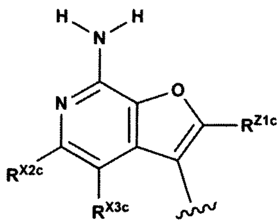
(I-1-c4)

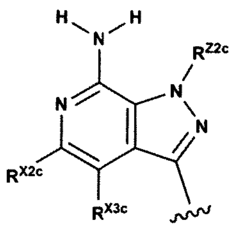
(I-1-c5)

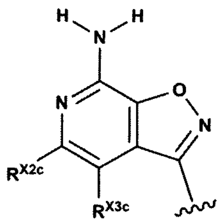
(I-1-c6)

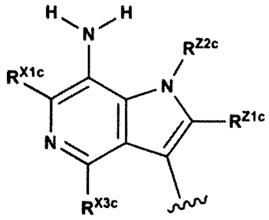
(I-1-c7)

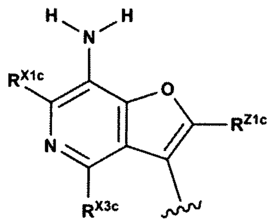
(I-1-c8)

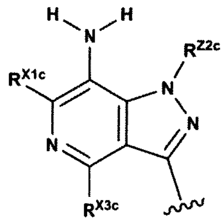
(I-1-c9)

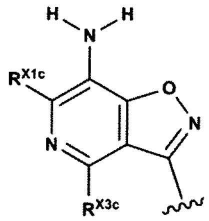
(I-1-c10)

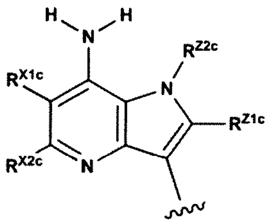
(I-1-c11)

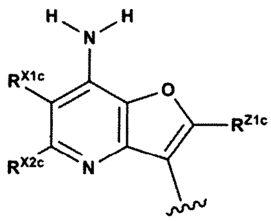
(I-1-c12)

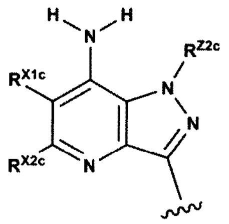
(I-1-c13)

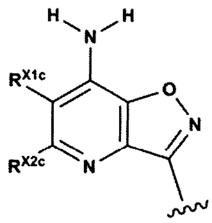
(I-1-c14)

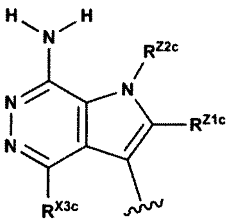
(I-1-c15)

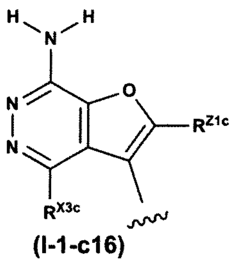
(I-1-c16)

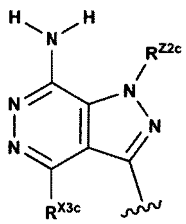
(I-1-c17)

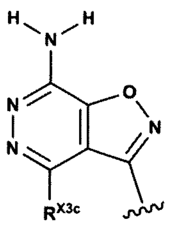
(I-1-c18)

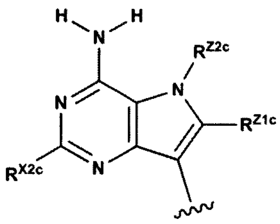
(I-1-c19)

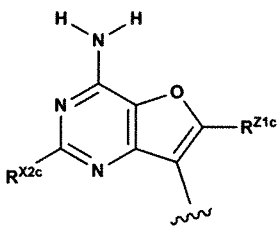
(I-1-c20)

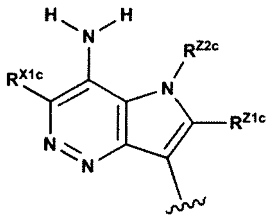

(I-1-c21)

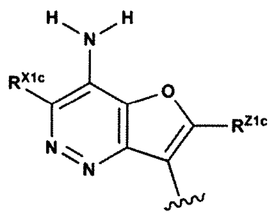

(I-1-c22)

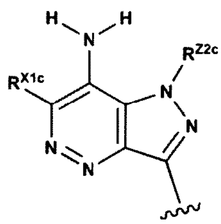

(I-1-c23)

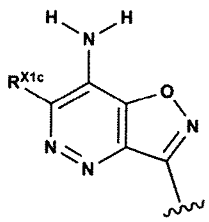

(I-1-c24)

(wherein the symbols have the same meanings as those described above).

In one embodiment, the partial structure represented by the formula (I-1-D) has a structure represented by the following formula (I-1-d1):

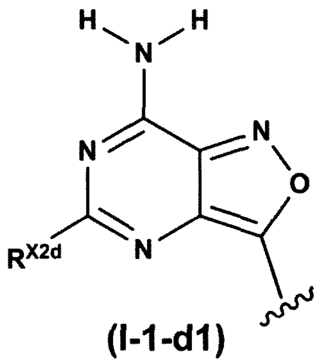

(I-1-d1)

(wherein the symbol has the same meaning as that described above). In another embodiment, the partial structure represented by the formula (I-1-D) has a structure represented by the following formula (I-1-d2), (I-1-d3), (I-1-d4), (I-1-d5), (I-1-d6), (I-1-d7), (I-1-d8), (1-1-d9), (I-1-d10), (I-1-d11), (I-1-d12), (I-1-d13), (I-1-d14), (I-1-d15), (I-1-d16), (I-1-d17), (I-1-d18), (I-1-d19), (I-1-d20), (I-1-d21), (I-1-d22), (I-1-d23), or (I-1-d24):

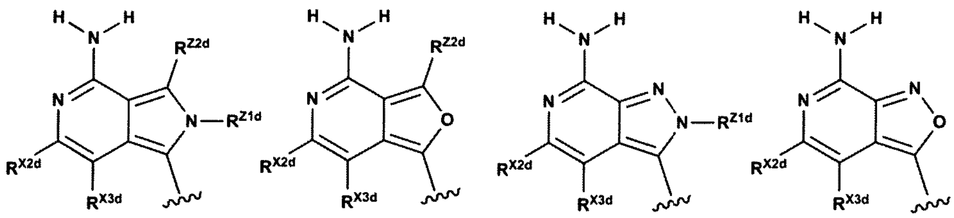

(I-1-d2) (I-1-d3) (I-1-d4) (I-1-d5)

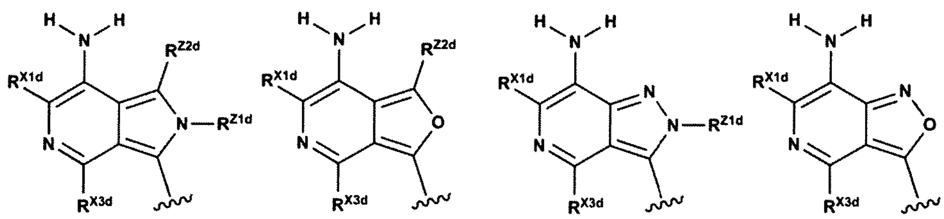

(I-1-d6) (I-1-d7) (I-1-d8) (I-1-d9)

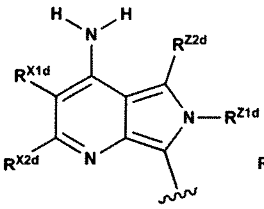

(I-1-d10)

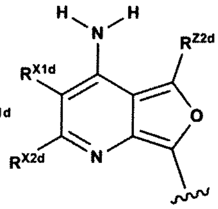

(I-1-d11)

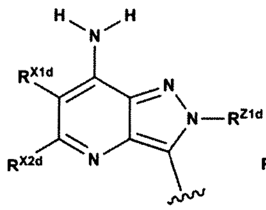

(I-1-d12)

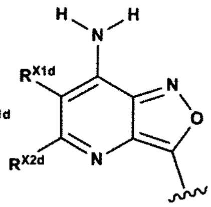

(I-1-d13)

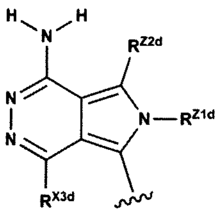

(I-1-d14)

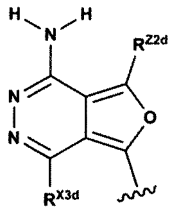

(I-1-d15)

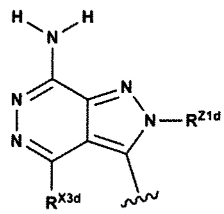

(I-1-d16)

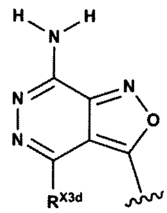

(I-1-d17)

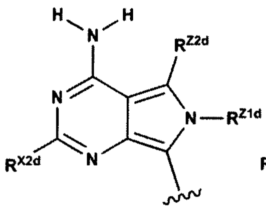

(I-1-d18)

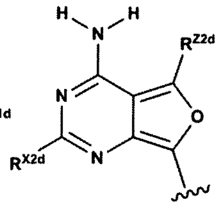

(I-1-d19)

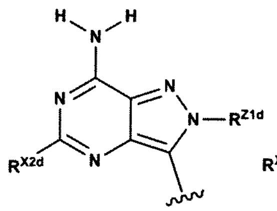

(I-1-d20)

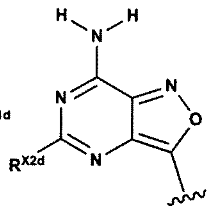

(I-1-d21)

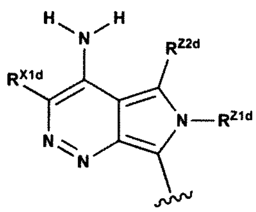

(I-1-d22)

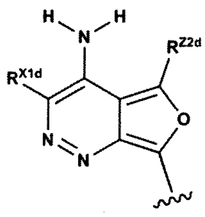

(I-1-d23)

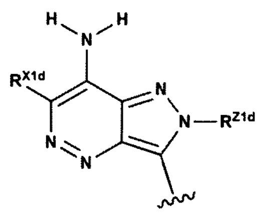

(I-1-d24)

(wherein the symbols have the same meanings as those described above).

In one preferable embodiment, the partial structure represented by the formula (I-1):

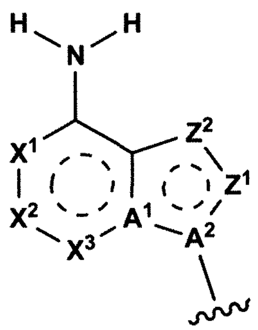

(I-1)

has a structure represented by the formula (I-1-a1), (I-1-a2), (I-1-a3), (I-1-a4), (I-1-a5), (I-1-b1), (I-1-b2), (I-1-b3), (I-1-b4), (I-1-b5), (I-1-c1), (I-1-c2), or (I-1-d1), and more preferably has a structure represented by the formula (I-1-a1), (I-1-b1), or (I-1-b2).

In one embodiment, $R^{X1a}$, $R^{X1b}$, $R^{X1c}$, and $R^{X1d}$ each independently represent a hydrogen atom, an alkyl group, or a halogen atom. In one preferable embodiment, $R^{X1a}$, $R^{X1b}$, $R^{X1c}$, and $R^{X1d}$ each are a hydrogen atom.

In one embodiment, $R^{X2a}$, $R^{X2b}$, $R^{X2c}$, and $R^{X2d}$ each independently represent a hydrogen atom, an alkyl group optionally substituted with the same or different 1 to 7 halogen atom(s), an alkoxy group, or an alkylthio group. In one preferable embodiment, $R^{X2a}$, $R^{X2b}$, $R^{X2c}$, and $R^{X2d}$ each are a hydrogen atom.

In one embodiment, $R^{X3a}$, $R^{X3b}$, $R^{X3c}$, and $R^{X3d}$ each independently represent a hydrogen atom, an alkyl group optionally substituted with the same or different 1 to 7 halogen atom(s), a cycloalkyl group, a halogen atom, a cyano group, or an aryl group. In one preferable embodiment, $R^{X3a}$, $R^{X3b}$, $R^{X3c}$, and $R^{X3d}$ each independently represent a hydrogen atom, an alkyl group optionally substituted with 1 to 7 fluorine atom(s), a cyclopropyl group, a chlorine atom, a cyano group, or a phenyl group. In more preferable one embodiment, $R^{X3a}$, $R^{X3c}$, and $R^{X3d}$ each are a hydrogen atom, and $R^{X3b}$ is a hydrogen atom, a methyl group, an ethyl group, a trifluoromethyl group, a cyclopropyl group, a chlorine atom, a cyano group, or a phenyl group. In further preferable one embodiment, $R^{X3a}$, $R^{X3c}$, and $R^{X3d}$ each are a hydrogen atom, and $R^{X3b}$ is a hydrogen atom, a methyl group, an ethyl group, a trifluoromethyl group, or a cyano group.

In one embodiment, $R^{Z1a}$, $R^{Z1b}$, and $R^{Z1c}$ each independently represent a hydrogen atom, a hydroxy group, or an alkyl group. In one preferable embodiment, $R^{Z1a}$, $R^{Z1b}$, and $R^{Z1c}$ each are a hydrogen atom.

In one embodiment, $R^{Z1d}$ represents an alkyl group.

In one embodiment, $R^{Z2a}$, $R^{Z2b}$, and $R^{Z2d}$ each independently represent a hydrogen atom, an alkyl group optionally substituted with the same or different 1 to 7 halogen atom(s), a cycloalkyl group, or a halogen atom. In one preferable embodiment, $R^{Z2a}$, $R^{Z2b}$, and $R^{Z2d}$ each independently are a hydrogen atom or an alkyl group optionally substituted with 1 to 7 fluorine atom(s).

In one embodiment, $R^{Z2c}$ represents an alkyl group.

In one embodiment, in the formula (I), L represents a single bond or $CR^{L1}R^{L2}$, and $R^{L1}$ and $R^{L2}$ each independently are a hydrogen atom or an alkyl group optionally substituted with the same or different 1 to 7 halogen atom(s). In one preferable embodiment, L represents a single bond or $CR^{L1}R^{L2}$, and $R^{L1}$ and $R^{L2}$ each independently represent a hydrogen atom or an alkyl group, and in a more preferable embodiment, L represents a single bond.

In one embodiment, in the formula (I), Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a phenyl group, a naphthyl group, a tetrahydronaphthyl group, an indenyl group, or an indanyl group;
(ii) a pyrrolyl group, a furyl group, a thienyl group, a pyrazolyl group, an imidazolyl group, an oxazolyl group, an isoxazolyl group, a thiazolyl group, an isothiazolyl group, a thiadiazolyl group, a pyridyl group, a pyrazinyl group, a pyrimidinyl group, a pyridazinyl group, a triazinyl group, an indolyl group, an indolinyl group, an isoindolinyl group, an indazolyl group, a tetrahydroindazolyl group, a benzofuranyl group, a dihydrobenzofuranyl group, a dihydroisobenzofuranyl group, a benzothiophenyl group, a dihydrobenzothiophenyl group, a dihydroisobenzothiophenyl group, a benzoxazolyl group, a dihydrobenzoxazolyl group, a benzothiazolyl group, a dihydrobenzothiazolyl group, a quinolyl group, a tetrahydroquinolyl group, an isoquinolyl group, a tetrahydroisoquinolyl group, a naphthyridinyl group, a tetrahydronaphthyridinyl group, a quinoxalinyl group, a tetrahydroquinoxalinyl group, or a quinazolinyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclopropyl group, a cyclobutyl group, a cyclopentyl group, a cyclohexyl group, a cycloheptyl group, a cyclooctyl group, a cyclopropenyl group, a cyclobutenyl group, a cyclopentenyl group, a cyclohexenyl group, a cycloheptenyl group, a cyclooctenyl group, a bicyclohexyl group, a bicycloheptyl group, a bicyclooctyl group, a bicyclononyl group, a bicyclodecyl group, a bicycloundecyl group, a bicyclododecyl group, a bicyclohexenyl group, a bicycloheptenyl group, a bicyclooctenyl group, a bicyclononenyl group, a bicyclodecenyl group, a bicycloundecenyl group, a bicyclododecenyl group, a spirohexyl group, a spiroheptyl group, a spirooctyl

group, a spirononyl group, a spirodecyl group, a spiroundecyl group, a spirododecyl group, or an adamantyl group; or (iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is an azetidinyl group, an oxetanyl group, a thietanyl group, a pyrrolidinyl group, a piperidinyl group, a piperidino group, a tetrahydrofuryl group, a tetrahydropyranyl group, a tetrahydrothienyl group, a piperazinyl group, a morpholinyl group, a morpholino group, a perhydroazepinyl group, a perhydroazocinyl group, an azabicyclohexyl group, an azabicycloheptyl group, an azabicyclooctyl group, an azabicyclononyl group, an azabicyclodecyl group, an azabicycloundecyl group, an azabicyclododecyl group, an azabicyclohexenyl group, an azabicycloheptenyl group, an azabicyclooctenyl group, an azabicyclononenyl group, an azabicyclodecenyl group, an azabicycloundecenyl group, an azabicyclododecenyl group, an azaspirohexyl group, an azaspiroheptyl group, an azaspirooctyl group, an azaspirononyl group, an azaspirodecyl group, an azaspiroundecyl group, or an azaspirododecyl group.

In one embodiment, in the formula (I), Cy represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or (iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In one preferable embodiment, in the formula (I), Cy represents
an alicyclic hydrocarbon group optionally substituted with the same or different 1 to 5 substituent(s) selected from
an alkyl group optionally substituted with 1, 2, or 3 halogen atom(s); and
a halogen atom,
wherein said alicyclic hydrocarbon group is a cyclohexyl group, a cycloheptyl group, a bicyclo[4.1.0]heptyl group, or a

spiro[2.5]octyl group; or
a nonaromatic heterocyclic group optionally substituted with the same or different 1 to 5 substituent(s) selected from
an alkyl group optionally substituted with 1, 2, or 3 halogen atom(s); and
a halogen atom,
wherein said nonaromatic heterocyclic group is a piperidinyl group or a piperidino group.

In one preferable embodiment, in the formula (I), Cy represents
an alicyclic hydrocarbon group optionally substituted with the same or different 1 to 5 substituent(s) selected from
an alkyl group optionally substituted with 1, 2, or 3 halogen atom(s); and
a halogen atom,
wherein said alicyclic hydrocarbon group is a cyclohexyl group or a spiro[2.5]octyl group; or
a nonaromatic heterocyclic group optionally substituted with the same or different 1 to 5 substituent(s) selected from
an alkyl group optionally substituted with 1, 2, or 3 halogen atom(s); and
a halogen atom,
wherein said nonaromatic heterocyclic group is a piperidinyl group or a piperidino group.

In one preferable embodiment, the partial structure represented by the formula (I-1):

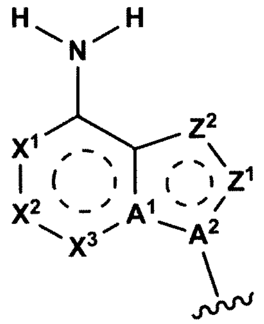

(I-1)

has a structure represented by the formula (I-1-a1), (I-1-a2), (I-1-a3), (I-1-a4), (I-1-a5), (I-1-b1), (I-1-b2), (I-1-b3), (I-1-b4), (I-1-b5), (I-1-c1), (I-1-c2), or (I-1-d1);

$R^{X1b}$ represents a hydrogen atom;
$R^{X2a}$, $R^{X2b}$, $R^{X2c}$, and $R^{X2d}$ each independently represent a hydrogen atom, an alkyl group optionally substituted with the same or different 1 to 7 halogen atom(s), an alkoxy group, or an alkylthio group;
$R^{X3a}$ and $R^{X3b}$ each independently represent a hydrogen atom, an alkyl group optionally substituted with the same or different 1 to 7 halogen atom(s), a cycloalkyl group, a halogen atom, a cyano group, or an aryl group;
$R^{Z1a}$ represents a hydrogen atom, a hydroxy group, or an alkyl group;
$R^{Z2a}$ and $R^{Z2b}$ each independently represent a hydrogen atom, an alkyl group optionally substituted with the same or different 1 to 7 halogen atom(s), a cycloalkyl group, or a halogen atom;
$R^{Z2c}$ represents an alkyl group;
L represents a single bond or $CR^{L1}R^{L2}$, $R^{L1}$ and $R^{L2}$ each independently represent a hydrogen atom or an alkyl group optionally substituted with the same or different 1 to 7 halogen atom(s); and
Cy represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;

an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or (iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In another preferable embodiment, the partial structure represented by the formula (I-1):

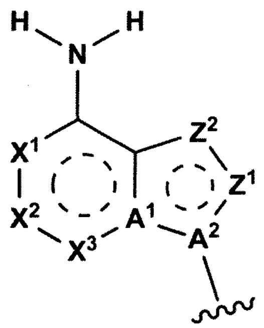

**(I-1)**

has a structure represented by the formula (I-1-a1), (I-1-b1), or (I-1-b2);

$R^{X2a}$ and $R^{X2b}$ each are a hydrogen atom;
$R^{X3b}$ is a hydrogen atom, an alkyl group optionally substituted with 1 to 7 fluorine atom(s), or a cyano group;
L represents a single bond or $CR^{L1}R^{L2}$, $R^{L1}$ and $R^{L2}$ each independently represent a hydrogen atom or an alkyl group; and
Cy represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;

a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

The terms "heteroaryl group" and "nonaromatic heterocyclic group" in the present description refer to a heterocyclic group comprising heteroatom(s) selected from an oxygen atom, a sulfur atom, and a nitrogen atom other than carbon atom(s) as ring atoms, and thus a compound wherein Cy comprises another heteroatom as a ring atom is not included in the compound of formula (I). For example, the compound of the formula (I) does not include a compound comprising a phosphorus atom as a ring atom such as cyclic adenosine 3',5'-monophosphate represented by the following formula:

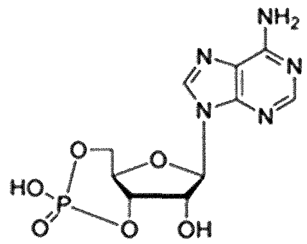

(Bicyclic nitrogen-containing heterocyclic compound)

The present invention also provides the compound represented by the following formula (II):

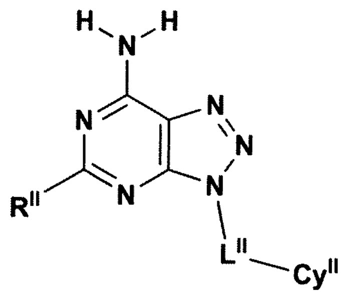

(II)

[wherein $R^{II}$, $L^{II}$, and $Cy^{II}$ each have the same meaning as that described above]
(provided that the above compound is not 3-cyclohexyl-3H-[1,2,3]triazolo[4,5-d]pyrimidin-7-amine, 2-[(7-amino-3H-1,2,3-triazolo[4,5-d]pyrimidin-3-yl)methyl]-1-azabicyclo[2.2.2]octan-3-one, 2-(7-amino-3H-1,2,3-triazolo[4,5-d]pyrimidin-3-yl)cyclohexanemethanol, or 4-(7-amino-3H-1,2,3-triazolo[4,5-d]pyrimidin-3-yl)-2-hydroxybicyclo[3.1.0]hexane-1-methanol)
or a pharmaceutically acceptable salt thereof.

In one embodiment, in the compound represented by the formula (II) (hereinafter also referred to as "Compound (II)"), $R^{II}$ represents a hydrogen atom, an alkyl group optionally substituted with the same or different 1 to 7 halogen

atom(s), an alkoxy group, or an alkylthio group. In one preferable embodiment, $R^{II}$ is a hydrogen atom.

In one embodiment, in the formula (II), $L^{II}$ represents a single bond or $CR^{LII-1}R^{LII-2}$, and $R^{LII-1}$ and $R^{LII-2}$ each independently represent a hydrogen atom or an alkyl group. In one preferable embodiment, $L^{II}$ represents a single bond.

In one embodiment, in the formula (II),
$Cy^{II}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a naphthyl group, a tetrahydronaphthyl group, an indenyl group, or an indanyl group;
(ii) a pyrrolyl group, a thienyl group, a pyrazolyl group, an imidazolyl group, an oxazolyl group, an isoxazolyl group, a thiazolyl group, an isothiazolyl group, a thiadiazolyl group, a pyridyl group, a pyrazinyl group, a pyrimidinyl group, a pyridazinyl group, a triazinyl group, an indolyl group, an indolinyl group, an isoindolinyl group, an indazolyl group, a tetrahydroindazolyl group, a benzofuranyl group, a dihydrobenzofuranyl group, a dihydroisobenzofuranyl group, a benzothiophenyl group, a dihydrobenzothiophenyl group, a dihydroisobenzothiophenyl group, a benzoxazolyl group, a dihydrobenzoxazolyl group, a benzothiazolyl group, a dihydrobenzothiazolyl group, a quinolyl group, a tetrahydroquinolyl group, an isoquinolyl group, a tetrahydroisoquinolyl group, a naphthyridinyl group, a tetrahydronaphthyridinyl group, a quinoxalinyl group, a tetrahydroquinoxalinyl group, or a quinazolinyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclopropyl group, a cyclohexyl group, a cycloheptyl group, a cyclooctyl group, a cyclopropenyl group, a cyclobutenyl group, a cycloheptenyl group, a cyclooctenyl group, a bicyclohexyl group, a bicycloheptyl group, a bicyclooctyl group, a bicyclononyl group, a bicyclodecyl group, a bicycloundecyl group, a bicyclododecyl group, a bicyclohexenyl group, a bicycloheptenyl group, a bicyclooctenyl group, a bicyclononenyl group, a bicyclodecenyl group, a bicycloundecenyl group, a bicyclododecenyl group, a spirohexyl group, a spiroheptyl group, a spirooctyl group, a spirononyl group, a spirodecyl group, a spiroundecyl group, a spirododecyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is an azetidinyl group, an oxetanyl group, a thietanyl group, a piperidinyl group, a piperidino group, a piperazinyl group, a morpholino group, a perhydroazepinyl group, a perhydroazocinyl group, an azabicyclohexyl group, an azabicycloheptyl group, an azabicyclooctyl group, an azabicyclononyl group, an azabicyclodecyl group, an azabicycloundecyl group, an azabicyclododecyl group, an azabicyclohexenyl group, an azabicycloheptenyl group, an azabicyclooctenyl group, an azabicyclononenyl group, an azabicyclodecenyl group, an azabicycloundecenyl group, an azabicyclododecenyl group, an azaspirohexyl group, an azaspiroheptyl group, an azaspirooctyl group, an azaspirononyl group, an azaspirodecyl group, an azaspiroundecyl group, or an azaspirododecyl group.

In one embodiment, in the formula (II),
$Cy^{II}$ represents

(i) a naphthyl group or a tetrahydronaphthyl group, each of which is optionally substituted with the same or different

1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a piperidinyl group, a piperidino group, a perhydroazepinyl group, a perhydroazocinyl group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In one preferable embodiment, in the formula (II),
$R^{II}$ represents a hydrogen atom, an alkyl group optionally substituted with the same or different 1 to 7 halogen atom(s), an alkoxy group, or an alkylthio group;
$L^{II}$ represents a single bond or $CR^{LII-1}R^{LII-2}$ $R^{LII-1}$ and $R^{LII-2}$ each independently represent a hydrogen atom or an alkyl group; and
$Cy^{II}$ represents

(i) a naphthyl group or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a

spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a piperidinyl group, a piperidino group, a perhydroazepinyl group, a perhydroazocinyl group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In another preferable embodiment, in the formula (II),
$R^{II}$ is a hydrogen atom;
$L^{II}$ is a single bond; and
$Cy^{II}$ represents

(i) a naphthyl group or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a piperidinyl group, a piperidino group, a perhydroazepinyl group, a perhydroazocinyl group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In one preferable embodiment, in the formula (II), $Cy^{II}$ is
an alicyclic hydrocarbon group optionally substituted with the same or different 1 to 5 substituent(s) selected from
an alkyl group optionally substituted with 1, 2, or 3 halogen atom(s); and
a halogen atom,
wherein said alicyclic hydrocarbon group is a cyclohexyl group, a cycloheptyl group, a bicyclo[4.1.0]heptyl group, or a spiro[2.5]octyl group; or

a nonaromatic heterocyclic group optionally substituted with the same or different 1 to 5 substituent(s) selected from an alkyl group optionally substituted with 1, 2, or 3 halogen atom(s); and
a halogen atom,
wherein said nonaromatic heterocyclic group is a piperidinyl group or a piperidino group.

In one preferable embodiment, in the formula (II), $Cy^{II}$ is
an alicyclic hydrocarbon group optionally substituted with the same or different 1 to 5 substituent(s) selected from an alkyl group optionally substituted with 1, 2, or 3 halogen atom(s); and
a halogen atom,
wherein said alicyclic hydrocarbon group is a cyclohexyl group or a spiro[2.5]octyl group; or
a nonaromatic heterocyclic group optionally substituted with the same or different 1 to 5 substituent(s) selected from an alkyl group optionally substituted with 1, 2, or 3 halogen atom(s); and
a halogen atom,
wherein said nonaromatic heterocyclic group is a piperidinyl group or a piperidino group.

The present invention also provides the compound represented by the following formula (III):

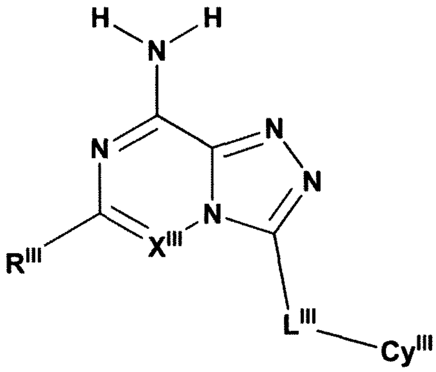

(III)

[wherein $X^{III}$, $R^{III}$, $L^{III}$, and $Cy^{III}$ each have the same meaning as that described above]
(provided that the above compound is not 3-cyclopropyl[1,2,4]triazolo[4,3-a]pyrazin-8-amine)
or a pharmaceutically acceptable salt thereof.

In one embodiment, in the compound represented by the formula (III) (hereinafter also referred to as "Compound (III)"), $X^{III}$ is $CR^{XIII}$. In another embodiment, $X^{III}$ is N. In another embodiment, $X^{III}$ is CH or N.

In one embodiment, in the formula (III), $R^{III}$ represents a hydrogen atom, an alkyl group optionally substituted with the same or different 1 to 7 halogen atom(s), an alkoxy group, or an alkylthio group. In one preferable embodiment, $R^{III}$ is a hydrogen atom.

In one embodiment, in the formula (III), $R^{XIII}$ represents a hydrogen atom, an alkyl group optionally substituted with the same or different 1 to 7 halogen atom(s), a cycloalkyl group, a halogen atom, a cyano group, or an aryl group. In one preferable embodiment, $R^{XIII}$ is a hydrogen atom, an alkyl group optionally substituted with 1 to 7 fluorine atom(s), a cyclopropyl group, a chlorine atom, a cyano group, or a phenyl group, and in a more preferable embodiment, a hydrogen atom.

In one embodiment, in the formula (III), $L^{III}$ represents a single bond or $CR^{LIII-1}R^{LIII-2}$, and $R^{LIII-1}$ and $R^{LIII-2}$ each independently represent a hydrogen atom or an alkyl group. In one preferable embodiment, $L^{III}$ represents a single bond.

In one embodiment, in the formula (III), $Cy^{III}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a naphthyl group, a tetrahydronaphthyl group, an indenyl group, or an indanyl group;
(ii) a pyrrolyl group, a furyl group, a thienyl group, a pyrazolyl group, an imidazolyl group, an oxazolyl group, an isoxazolyl group, a thiazolyl group, an isothiazolyl group, a thiadiazolyl group, a pyridyl group, a pyrazinyl group, a pyrimidinyl group, a pyridazinyl group, a triazinyl group, an indolyl group, an indolinyl group, an isoindolinyl group, an indazolyl group, a tetrahydroindazolyl group, a benzofuranyl group, a dihydrobenzofuranyl group, a dihydroisobenzofuranyl group, a benzothiophenyl group, a dihydrobenzothiophenyl group, a dihydroisobenzothiophenyl group, a benzoxazolyl group, a dihydrobenzoxazolyl group, a benzothiazolyl group, a dihydrobenzothiazolyl group, a quinolyl

group, a tetrahydroquinolyl group, an isoquinolyl group, a tetrahydroisoquinolyl group, a naphthyridinyl group, a tetrahydronaphthyridinyl group, a quinoxalinyl group, a tetrahydroquinoxalinyl group, or a quinazolinyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a cyclooctyl group, a cyclopropenyl group, a cyclobutenyl group, a cyclopentenyl group, a cyclohexenyl group, a cycloheptenyl group, a cyclooctenyl group, a bicyclohexyl group, a bicycloheptyl group, a bicyclooctyl group, a bicyclononyl group, a bicyclodecyl group, a bicycloundecyl group, a bicyclododecyl group, a bicyclohexenyl group, a bicycloheptenyl group, a bicyclooctenyl group, a bicyclononenyl group, a bicyclodecenyl group, a bicycloundecenyl group, a bicyclododecenyl group, a spirohexyl group, a spiroheptyl group, a spirooctyl group, a spirononyl group, a spirodecyl group, a spiroundecyl group, a spirododecyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is an azetidinyl group, an oxetanyl group, a thietanyl group, a pyrrolidinyl group, a piperidinyl group, a piperidino group, a tetrahydropyranyl group, a tetrahydrothienyl group, a piperazinyl group, a morpholinyl group, a morpholino group, a perhydroazepinyl group, a perhydroazocinyl group, an azabicyclohexyl group, an azabicycloheptyl group, an azabicyclooctyl group, an azabicyclononyl group, an azabicyclodecyl group, an azabicycloundecyl group, an azabicyclododecyl group, an azabicyclohexenyl group, an azabicycloheptenyl group, an azabicyclooctenyl group, an azabicyclononenyl group, an azabicyclodecenyl group, an azabicycloundecenyl group, an azabicyclododecenyl group, an azaspirohexyl group, an azaspiroheptyl group, an azaspirooctyl group, an azaspirononyl group, an azaspirodecyl group, an azaspiroundecyl group, or an azaspirododecyl group.

In one embodiment, in the formula (III), $Cy^{III}$ represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected

from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In one preferable embodiment, in the formula (III), $Cy^{III}$ is
an alicyclic hydrocarbon group optionally substituted with the same or different 1 to 5 substituent(s) selected from
an alkyl group optionally substituted with 1, 2, or 3 halogen atom(s); and
a halogen atom,
wherein said alicyclic hydrocarbon group is a cyclohexyl group, a cycloheptyl group, a bicyclo[4.1.0]heptyl group, or a spiro[2.5]octyl group; or
a nonaromatic heterocyclic group optionally substituted with the same or different 1 to 5 substituent(s) selected from
an alkyl group optionally substituted with 1, 2, or 3 halogen atom(s); and
a halogen atom,
wherein said nonaromatic heterocyclic group is a piperidinyl group or a piperidino group.
In one preferable embodiment, in the formula (III), $Cy^{III}$ is
an alicyclic hydrocarbon group optionally substituted with the same or different 1 to 5 substituent(s) selected from
an alkyl group optionally substituted with 1, 2, or 3 halogen atom(s); and
a halogen atom,
wherein said alicyclic hydrocarbon group is a cyclohexyl group or a spiro[2.5]octyl group; or
a nonaromatic heterocyclic group optionally substituted with the same or different 1 to 5 substituent(s) selected from
an alkyl group optionally substituted with 1, 2, or 3 halogen atom(s); and
a halogen atom,
wherein said nonaromatic heterocyclic group is a piperidinyl group or a piperidino group.
In one preferable embodiment, in the formula (III),
$X^{III}$ is $CR^{XIII}$ ;
$R^{III}$ represents a hydrogen atom, an alkyl group optionally substituted with the same or different 1 to 7 halogen atom(s), an alkoxy group, or an alkylthio group;
$R^{XIII}$ represents a hydrogen atom, an alkyl group optionally substituted with the same or different 1 to 7 halogen atom(s), a cycloalkyl group, a halogen atom, a cyano group, or an aryl group;
$L^{III}$ represents a single bond or $CR^{LIII-1}R^{LIII-2}$, $R^{LIII-1}$ and $R^{LIII-2}$ each independently represent a hydrogen atom or an alkyl group; and
$Cy^{III}$ represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),

wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or (iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a perhydroazepinyl group, a perhydroazocinyl group, a morpholinyl group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In another preferable embodiment, in the formula (III),

$X^{III}$ is $CR^{XIII}$;
$R^{III}$ is a hydrogen atom;
$R^{XIII}$ is a hydrogen atom, an alkyl group optionally substituted with 1 to 7 fluorine atom(s), or a cyano group;
$L^{III}$ represents a single bond;
$Cy^{III}$ represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In one preferable embodiment, in the formula (III),

$X^{III}$ is N;
$R^{III}$ represents a hydrogen atom, an alkyl group optionally substituted with the same or different 1 to 7 halogen atom(s), an alkoxy group, or an alkylthio group;
$R^{XIII}$ represents a hydrogen atom, an alkyl group optionally substituted with the same or different 1 to 7 halogen atom(s), a cycloalkyl group, a halogen atom, a cyano group, or an aryl group;
$L^{III}$ represents a single bond or $CR^{LIIII-1}R^{LIII-2}$, $R^{LIII-1}$ and $R^{LIII-2}$ each independently represent a hydrogen atom or an alkyl group; and
$Cy^{III}$ represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or (iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

In another preferable embodiment, in the formula (III),

$X^{III}$ is N;
$R^{III}$ is a hydrogen atom;
$R^{XIII}$ is a hydrogen atom, an alkyl group optionally substituted with 1 to 7 fluorine atom(s), or a cyano group;
$L^{III}$ represents a single bond;
$Cy^{III}$ represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected

from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or (iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

Compound (I), Compound (II), or Compound (III) of the present invention may exist in the form of a tautomer or a mixture thereof. Compound (I), Compound (II), or Compound (III) of the present invention may exist in the form of a stereoisomer such as an enantiomer and a diastereomer or a mixture thereof. Compound (I), Compound (II), or Compound (III) of the present invention encompasses a mixture of tautomers or stereoisomers or each pure or substantially pure isomer. The symbol "*" in a carbon atom in a chemical formula of the present description means that said carbon atom is an asymmetric carbon. Also, the symbols "(R)" and "(S)" in an asymmetric carbon in a chemical formula of the present description have the normal meanings in this technical field, i.e. mean that the configuration in each asymmetric carbon is specified as "(R)" configuration and "(S)" configuration respectively.
When Compound (I), Compound (II), or Compound (III) is obtained in the form of a diastereomer or an enantiomer, it may be isolated by a known conventional method in this technical field such as chromatography and fractional crystallization method.
Compound (I), Compound (II), or Compound (III) of the present invention encompasses compounds labeled with an isotope (for example, $^{2}H$, $^{3}H$, $^{13}C$, $^{14}C$, $^{15}N$, $^{18}F$, $^{32}P$ $^{35}S$, and $^{125}I$) and the like, and deuterated products.
Examples of the pharmaceutically acceptable salt of Compound (I), Compound (II), or Compound (III) include alkali metal salts such as lithium, sodium, and potassium salts; alkaline earth metal salts such as magnesium and calcium salts; salts with aluminum or zinc; salts with an amine such as ammonia, choline, diethanolamine, lysine, ethylenediamine, tert-butylamine, tert-octylamine, tris(hydroxymethyl)aminomethane, N-methyl-glucosamine, triethanolamine, and dehydroabietylamine; salts with an inorganic acid such as hydrogen chloride, hydrogen bromide, hydrogen iodide, sulfuric acid, nitric acid, and phosphoric acid; salts with an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, and benzenesulfonic acid; and salts with an acidic amino acid such as aspartic acid and glutamic acid.
Further, the pharmaceutically acceptable salt of Compound (I), Compound (II), or Compound (III) encompasses inner salts, hydrates, and solvates thereof.
The "pharmaceutically acceptable" ingredients in the present description generally mean that they are not harmful to a subject of administration and are compatible with each other in the preparation of a pharmaceutical composition, and include useful ingredients for use as human medicaments as well as useful ingredients for veterinary use.

(Use)

Compound (I), Compound (II), or Compound (III) or a pharmaceutically acceptable salt thereof of the present invention may be orally or parenterally administered alone or as a pharmaceutical composition comprising it and a pharmaceutically acceptable carrier. The pharmaceutically acceptable carrier may be any conventional carrier in this

technical field, and examples thereof include diluents, binders (for example, syrup, gum arabic, gelatin, sorbitol, tragacanth, and polyvinylpyrrolidone), excipients (for example, lactose, sucrose, cornstarch, potassium phosphate, sorbitol, and glycine), lubricants (for example, magnesium stearate, talc, polyethylene glycol, and silica), disintegrants (for example, potato starch), and humectants (for example, sodium lauryl sulfate). Also, the dosage form of the pharmaceutical composition is not limited to a specific one, and the pharmaceutical composition may be used as a conventional pharmaceutical formulation such as a tablet, a granule, a capsule, a powder, an injection, an inhalant, and a suppository.

The dose (i.e., effective amount) of Compound (I), Compound (II), or Compound (III) or a pharmaceutically acceptable salt thereof of the present invention varies depending on administration method, age, body weight, and condition of patient, and the like, and normally 0.001 to 500 mg/kg/day, in particular 0.01 to 10 mg/kg/day is preferable and administered at one time or two to four divided doses.

The compounds of the present invention have PDE7 inhibitory effects, and are effective in the treatment or prevention of diseases associated with PDE7. The compounds of the present invention have inhibitory effects on cAMP degradation on the basis of their PDE7 inhibitory effects, and thus are effective in the treatment or prevention of diseases affected by the amount of cAMP.

Accordingly, Compound (I), Compound (II), or Compound (III), or PDE7 inhibitor of the present invention is useful for the prevention or treatment of diseases which are expected to be improved by inhibiting PDE7 such as a psychiatric disorder and a neurological disorder [for example, dependence on an addictive drug and a specified act (for example, alcohol dependence, drug dependence such as nicotine dependence and cocaine dependence, gambling dependence, internet dependence, overuse of an electronic device, overuse of a game device, shopping dependence, sex dependence, bulimia, binge eating disorder, kleptomania, pyromania, and trichotillomania), obsessive-compulsive disorder, post-traumatic stress disorder (PTSD), anxiety, depression, mood disorder, insomnia, delirium disorder, psychiatric disease, schizophrenia-related disorder, attention deficit hyperactivity disorder (ADHD) in a child with hyperactivity, migraine, stress, a disorder related to a disease caused by psychosomatic disease, panic attack, epilepsy, memory disorder, cognitive disorder, Alzheimer's disease, senile dementia, attention disorder, wakefulness disorder, ischemia, and brain injury-related disorder], a movement disorder [for example, Parkinson's disease, dopa-responsive dystonia, spinal cord injury, dyskinesia, a disorder related to acute or chronic neurodegenerative disease (including Huntington's chorea), Shy-Drager syndrome, periodic limb movement disorder (PLMD), periodic limb movements in sleep (PLMS), Tourette's syndrome, and restless legs syndrome (RLS)], cancer and leukemia [for example, glioblastoma and chronic lymphocytic leukemia], pain [for example, neuropathic pain and visceral pain], an inflammatory disease and an immunological disease [for example, autoimmune encephalomyelitis, multiple sclerosis, atopic dermatitis, allergic rhinitis, asthma, psoriasis, Crohn's disease, ulcerative colitis, rheumatoid arthritis, post-transplantation rejection, diabetes mellitus, and chronic obstructive pulmonary disease (COPD)], a cardiovascular disease [for example, myocardial infarction], and the others. The compounds or PDE7 inhibitors of the present invention are preferably useful for the prevention or treatment of alcohol dependence, drug dependence, gambling dependence, internet dependence, overuse of an electronic device, overuse of a game device, sex dependence, bulimia, binge eating disorder, and glioblastoma, more preferably useful for the prevention or treatment of alcohol dependence, drug dependence, and glioblastoma, and particularly preferably useful for the prevention or treatment of alcohol dependence and drug dependence.

The compounds of the present invention have PDE7 inhibitory effects, and have selective inhibitory effects on PDE7 as compared to, for example, other PDE isozymes (i.e., PDE1 to 6 and PDE8 to 11). Preferably, selective PDE7 inhibitory effect means that $IC_{50}$ of a compound in relation to the inhibition against any activity of PDE1 to 6 and PDE8 to 11 is 5 times (for example, at least 10 times, at least 50 times, at least 100 times, or at least 200 times) larger than the smaller one of $IC_{50}$ in relation to the inhibition of PDE7A activity and $IC_{50}$ in relation to the inhibition of PDE7B activity. More preferably, selective PDE7 inhibitory effect means that $IC_{50}$ of a compound in relation to the inhibition against any activity of PDE4, 8 and 10 is 5 times (for example, at least 10 times, at least 50 times, at least 100 times, or at least 200 times) larger than the smaller one of $IC_{50}$ in relation to the inhibition of PDE7A activity and $IC_{50}$ in relation to the inhibition of PDE7B activity. Especially preferably, selective PDE7 inhibitory effect means that the smallest value in $IC_{50}$ of a compound in relation to the inhibition against all of PDE4, 8, and 10 activities is 5 times (for example, at least 10 times, at least 50 times, at least 100 times, or at least 200 times) larger than the smaller one of $IC_{50}$ in relation to the inhibition of PDE7A activity and $IC_{50}$ in relation to the inhibition of PDE7B activity. Most preferably, selective PDE7 inhibitory effect means that the smallest value in $IC_{50}$ of a compound in relation to the inhibition against all of PDE1 to 6 and PDE8 to 11 activities is 5 times (for example, at least 10 times, at least 50 times, at least 100 times, or at least 200 times) larger than the smaller one of $IC_{50}$ in relation to the inhibition of PDE7A activity and $IC_{50}$ in relation to the inhibition of PDE7B activity. The selectivity of the above selective PDE7 inhibitory effect may be determined on the basis of the corresponding ratio of Ki instead of ratio of $IC_{50}$.

A selective PDE7 inhibitor may be identified by, for example, comparing the ability of a drug to inhibit the PDE7 (PDE7A, PDE7B, or PDE7A and PDE7B) enzyme activity with the ability of said drug to inhibit a PDE enzyme in the other PDE family. For example, the ability of a drug to inhibit the PDE7 activity, and the ability of said drug to inhibit the PDE1, PDE2, PDE3, PDE4, PDE5, PDE6, PDE8, PDE9, PDE10, and PDE11 activities may be assayed. The ratio of

$IC_{50}$ of the other PDE isozymes (PDE1 to 6 and PDE8 to 11) as compared to $IC_{50}$ of PDE7 (for example, smaller one of $IC_{50}$ in relation to the inhibition of PDE7A activity and $IC_{50}$ in relation to the inhibition of PDE7B activity) may be measured by a standard *in vitro, in vivo,* or *ex vivo* assay including the method described in the present description. The identification of the above selective PDE7 inhibitor may be carried out on the basis of the corresponding ratio of Ki instead of the ratio of $IC_{50}$.

The method for treating or preventing diseases comprising administering an effective amount of Compound (I), Compound (II), or Compound (III), or a pharmaceutically acceptable salt thereof of the present invention to a patient (i.e., target individual of the treatment or prevention, preferably human) is also applied to the above object, and encompassed within the present invention.

Also, use of Compound (I), Compound (II), or Compound (III), or a pharmaceutically acceptable salt thereof of the present invention in the manufacture of a medicament having a PDE7 inhibitory effect is also applied to the above object, and encompassed within the present invention.

According to the present invention, Compound (I), Compound (II), or Compound (III), or a pharmaceutically acceptable salt thereof may be prepared according to, but is not limited to, the following methods.

When a functional group in a compound needs to be protected in each preparation process of Compound (I), Compound (II), or Compound (III) described below, the protection may be appropriately carried out by a conventional method. General descriptions of protecting groups and use thereof are described in T. W. Greene et al., "Protective Groups in Organic Synthesis", John Wiley & Sons, New York, 2006. A protecting group may be removed in a subsequent step by using a conventional method.

Production method 1

Among the compound represented by the formula (I), a compound wherein the partial structure represented by the formula (I-1) has the structure represented by the formula (I-1-A) may be prepared according to, for example, the following Scheme 1.

Scheme 1

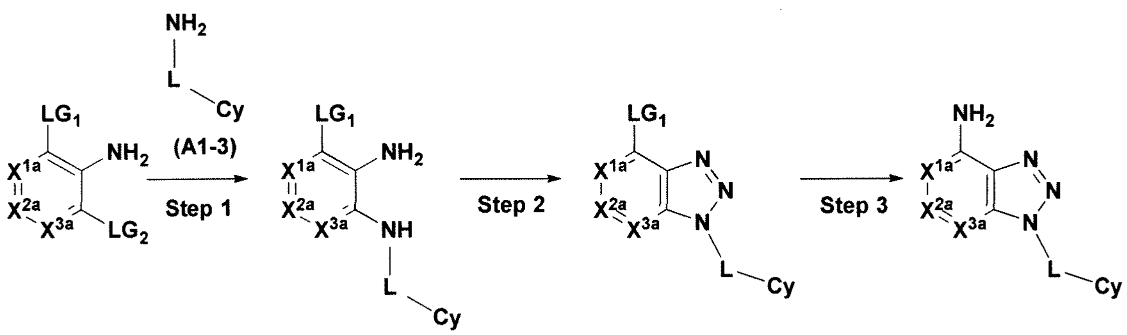


(A1-4) (A1-2) (A1-1) (A1)

[wherein $LG_1$ and $LG_2$ each independently represent a leaving group such as a halogen atom; and the other symbols have the same meanings as those described above.]

Examples of the embodiment include the following scheme.

One embodiment of Scheme 1

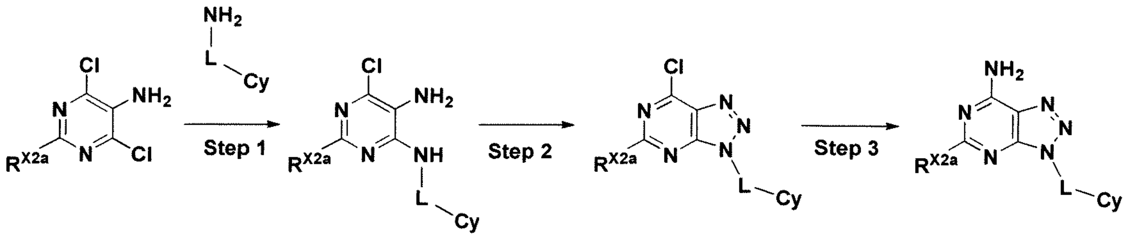

[wherein the symbols have the same meanings as those described above.]

Step 1

The Compound (A1-2) may be prepared by reacting the Compound (A1-3) with the Compound (A1-4) in a solvent, in the presence of a base, and in the presence or absence of microwave radiation. The Compound (A1-3) may be in the free form or a salt form, for example hydrochloride.

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.

Examples of the base include alkali metal carbonates such as cesium carbonate, potassium carbonate, sodium carbonate, and sodium hydrogen carbonate; alkali metal phosphates such as potassium phosphate tribasic, sodium phosphate, and sodium hydrogen phosphate; amines such as triethylamine and N,N-diisopropylethylamine; and alkali metal fluorides such as cesium fluoride and potassium fluoride.

The amount of the Compound (A1-3) to be used may be 0.6 to 5.0 equivalent(s), preferably 0.8 to 3.0 equivalent(s), relative to the Compound (A1-4) in molar ratio.

The amount of the base to be used may be 1.0 to 5.0 equivalent(s), preferably 2.0 to 3.0 equivalents, relative to the Compound (A1-4) in molar ratio.

The reaction may be carried out at room temperature to under heating, for example at room temperature to 200°C, preferably at room temperature to 180°C.

Step 2

The Compound (A1-2) prepared in the Step 1 may be reacted with sodium nitrite in a solvent to prepare the Compound (A1-1).

The solvent may be any which does not affect the reaction, and examples thereof include amides such as N,N-dimethylformamide, halogenated aliphatic hydrocarbons such as chloroform and dichloromethane, aromatic hydrocarbons such as toluene, nitriles such as acetonitrile, carboxylic acids such as acetic acid, water, and mixtures thereof.

The amount of sodium nitrite to be used may be 1.0 to 2.0 equivalent(s), preferably 1.0 to 1.5 equivalent(s), relative to the Compound (A1-2) in molar ratio.

The reaction may be carried out under ice-cooling to under heating, for example under ice-cooling to at room temperature, preferably at room temperature.

Step 3

The Compound (A1-1) prepared in the Step 2 may be reacted with ammonia in a solvent, and in the presence or absence of microwave radiation to prepare the Compound (A1).

The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.

The amount of the ammonia to be used may be 20 to 60 equivalents, preferably 30 to 50 equivalents, relative to the Compound (A1-1) in molar ratio.

The reaction may be carried out at room temperature to under heating, for example at room temperature to 150°C, preferably at room temperature to 120°C.

The Compound (A1-2) may also be prepared by the following scheme.

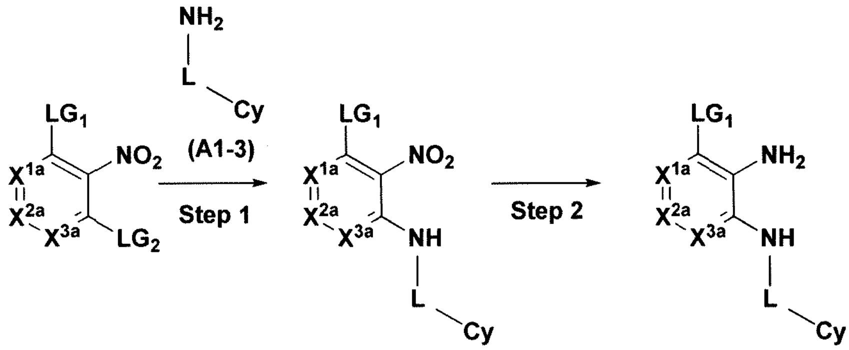


(A1-4') (A1-2') (A1-2)

[wherein the symbols have the same meanings as those described above.]

Examples of the embodiment include the following scheme.

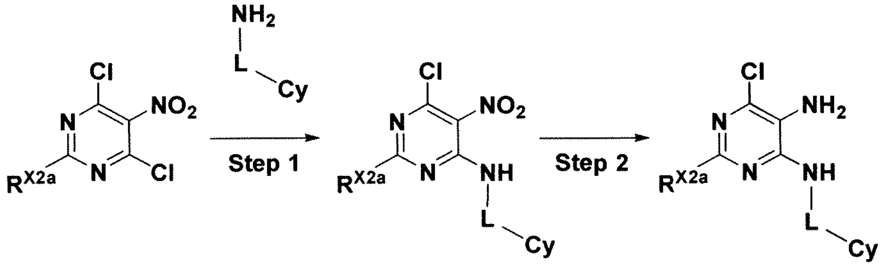


[wherein the symbols have the same meanings as those described above.]

Step 1

The Compound (A1-4') and the Compound (A1-3) may be reacted in a similar manner to the Step 1 in the above Scheme 1 to prepare the Compound (A1-2').

Step 2

The Compound (A1-2') may be reacted in a solvent, and in the presence of a reducing agent to prepare the Compound (A1-2).

The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.

Examples of the reducing agent include tin(II) chloride.

The amount of the reducing agent to be used may be 2.0 to 10.0 equivalents, preferably 3.0 to 5.0 equivalents, relative to the Compound (A1-2') in molar ratio.

The reaction may be carried out under heating, for example at 50 to 200°C, preferably at 100°C to 150°C.

The Compound (A1-3) may also be synthesized by the following Scheme.

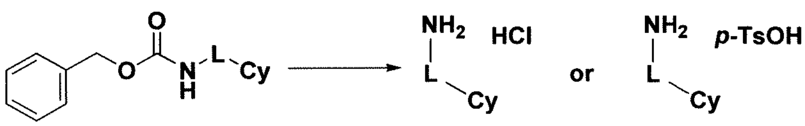


(A1-5) (A1-3)

[wherein the symbols have the same meanings as those described above.]

The Compound (A1-5) may be reacted with hydrogen chloride (for example, a solution of hydrogen chloride in dioxane) in a solvent, and in the presence of a catalyst to prepare hydrochloride of the Compound (A1-3). Alternatively, the Compound (A1-5) may be reacted in a solvent, and in the presence of a catalyst, and reacted with p-toluenesulfonic

acid to prepare p-toluenesulfonate of the Compound (A1-3).

The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.

Examples of the catalyst include palladium carbon.

The amount of hydrogen chloride to be used may be 1.0 to 5.0 equivalent(s), preferably 1.0 to 2.0 equivalent(s), relative to the Compound (A1-5) in molar ratio.

The amount of the catalyst to be used may be 0.05 to 2.0 equivalent(s), preferably 0.1 to 0.5 equivalent(s), relative to the Compound (A1-5) in molar ratio.

The reaction may be carried out at room temperature to under heating, preferably at room temperature.

The compound wherein $R^{X2a}$ is a methylsulfanyl group may be converted into the compound wherein $R^{X2a}$ is a methoxy group according to the following scheme.

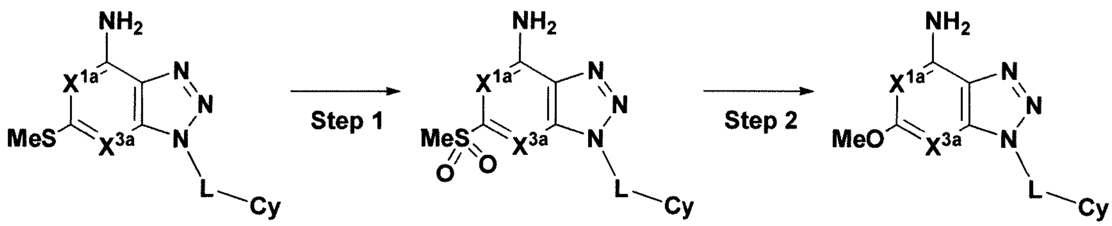


(A1'-2) (A1'-1) (A1')

[wherein the symbols have the same meanings as those described above.]

Examples of the embodiment include the following scheme.

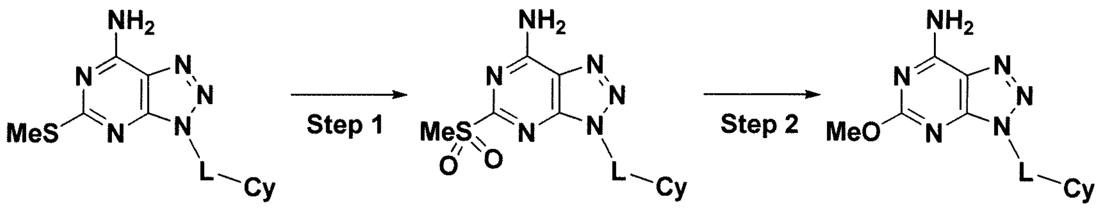


[wherein the symbols have the same meanings as those described above.]

Step 1

The Compound (A1'-2) may be reacted in a solvent, and in the presence of an oxidizing agent to prepare the Compound (A1'-1).

The solvent may be any which does not affect the reaction, and examples thereof include amides such as N,N-dimethylformamide, halogenated aliphatic hydrocarbons such as chloroform and dichloromethane, aromatic hydrocarbons such as toluene, nitriles such as acetonitrile, carboxylic acids such as acetic acid, water, and mixtures thereof.

Examples of the oxidizing agent include m-chloroperbenzoic acid.

The amount of the oxidizing agent to be used may be 2.0 to 5.0 equivalents, preferably 2.0 to 2.5 equivalents, relative to the Compound (A1'-2) in molar ratio.

The reaction may be carried out under ice-cooling to under heating, under ice-cooling to at room temperature, preferably under ice-cooling.

Step 2

The Compound (A1'-1) may be reacted with a metal methoxide in a solvent to prepare the Compound (A1').

The solvent may be any which does not affect the reaction, and examples thereof include ethers such as tetrahydrofuran and 1,4-dioxane, methanol, aromatic hydrocarbons such as toluene, nitriles such as acetonitrile, and mixtures thereof.

Examples of the metal methoxide include sodium methoxide.

The amount of the metal methoxide to be used may be 1.0 to 5.0 equivalent(s), preferably 2.0 to 3.0 equivalents,

relative to the Compound (A1") in molar ratio. The reaction may be carried out at room temperature to under heating, for example at room temperature to 50°C, preferably at room temperature.

Also, a methylsulfanyl group in $R^{X2a}$ may be converted into an ethoxy group according to the following scheme.

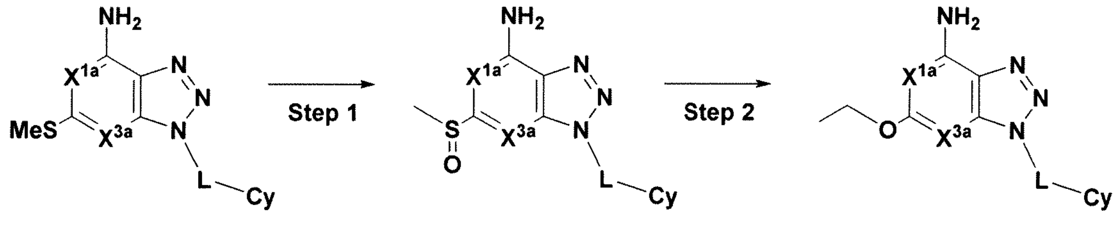


(A1"-2) (A1"-1) (A1")

[wherein the symbols have the same meanings as those described above.]

Examples of the embodiment include the following scheme.

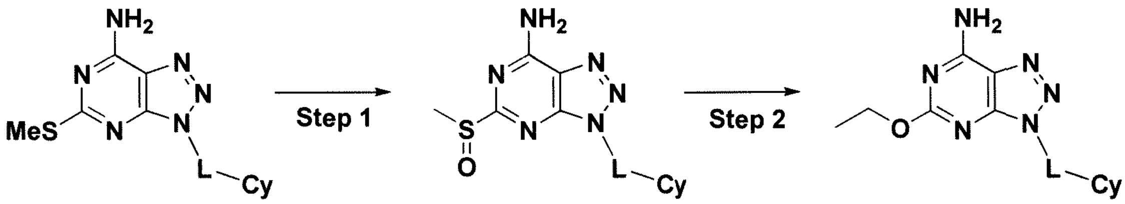


[wherein the symbols have the same meanings as those described above.]

Step 1

The Compound (A1"-2) may be reacted in a solvent, and in the presence of an oxidizing agent to prepare the Compound (A1"-1).

The solvent may be any which does not affect the reaction, and examples thereof include amides such as N,N-dimethylformamide, halogenated aliphatic hydrocarbons such as chloroform and dichloromethane, aromatic hydrocarbons such as toluene, nitriles such as acetonitrile, carboxylic acids such as acetic acid, water, and mixtures thereof.

Examples of the oxidizing agent include m-chloroperbenzoic acid.

The amount of the oxidizing agent to be used may be 1.0 to 2.0 equivalent(s), preferably 1.0 to 1.5 equivalent(s), relative to the Compound (A1"-2) in molar ratio. The reaction may be carried out under ice-cooling to under heating, under ice-cooling to at room temperature, preferably under ice-cooling.

Step 2

The Compound (A1"-1) may be reacted with a metal ethoxide in a solvent to prepare the Compound (A1").

The solvent may be any which does not affect the reaction, and examples thereof include ethers such as tetrahydrofuran and 1,4-dioxane, ethanol, aromatic hydrocarbons such as toluene, nitriles such as acetonitrile, and mixtures thereof.

Examples of the metal ethoxide include sodium ethoxide.

The amount of the metal ethoxide to be used may be 1.0 to 5.0 equivalent(s), preferably 1.5 to 3.0 equivalents, relative to the Compound (A1"-1) in molar ratio.

The reaction may be carried out at room temperature to under heating, for example at room temperature to 100°C, preferably at room temperature.

Production method 2

Among the compound represented by the formula (I), a compound wherein the partial structure represented by the formula (I-1) has the structure represented by the formula (I-1-A) may also be prepared according to, for example, the following Scheme 2.

Scheme 2

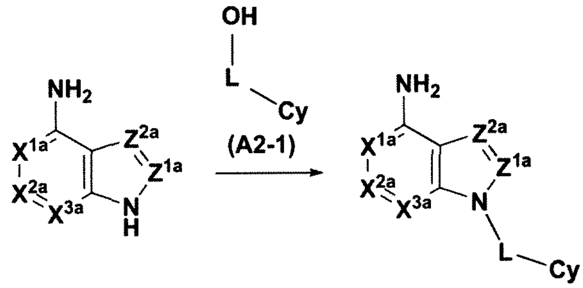

(A2-2) (A2)

[wherein the symbols have the same meanings as those described above.]

Examples of the embodiment include the following scheme.

One embodiment of Scheme 2

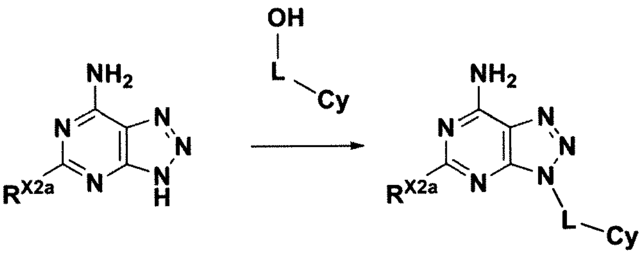

[wherein the symbols have the same meanings as those described above.]

The Compound (A2-2) may be reacted with the Compound (A2-1) in a solvent, and in the presence of an azodicarboxylic acid derivative and a phosphine derivative, or in the presence of a (cyanomethylene)trialkylphosphorane to prepare the Compound (A2).

The solvent may be any which does not affect the reaction, and examples thereof include ethers such as tetrahydrofuran and 1,4-dioxane, aromatic hydrocarbons such as toluene, nitriles such as acetonitrile, and mixtures thereof.

Examples of the azodicarboxylic acid derivative include dialkyl azodicarboxylates such as diethyl azodicarboxylate and diisopropyl azodicarboxylate; and azodicarboxamides such as N,N,N',N'-tetramethylazodicarboxamide.

Examples of the phosphine derivative include triarylphosphines such as triphenylphosphine and trialkylphosphines such as tributylphosphine.

Examples of the (cyanomethylene)trialkylphosphorane include (cyanomethylene)trimethylphosphorane and (cyanomethylene)tributylphosphorane, preferably (cyanomethylene)trimethylphosphorane.

The amount of the Compound (A2-1) to be used may be 1.0 to 5.0 equivalent(s), preferably 2.0 to 3.0 equivalents, relative to the Compound (A2-2) in molar ratio.

The amount of the azodicarboxylic acid derivative to be used may be 1.0 to 5.0 equivalent(s), preferably 2.0 to 3.0 equivalents, relative to the Compound (A2-2) in molar ratio.

The amount of the phosphine derivative to be used may be 1.0 to 5.0 equivalent(s), preferably 2.0 to 3.0 equivalents, relative to the Compound (A2-2) in molar ratio.

The amount of (cyanomethylene)trialkylphosphorane to be used may be 1.0 to 5.0 equivalent(s), preferably 2.0 to 3.0 equivalents, relative to the Compound (A2-2) in molar ratio.

The reaction may be carried out under heating, for example at 80°C to 150°C, preferably at 100°C to 120°C.

Production method 3

Among the compound represented by the formula (I), a compound wherein the partial structure represented

by the formula (I-1) has the structure represented by the formula (I-1-A) may also be prepared according to, for example, the following Scheme 3.

Scheme 3

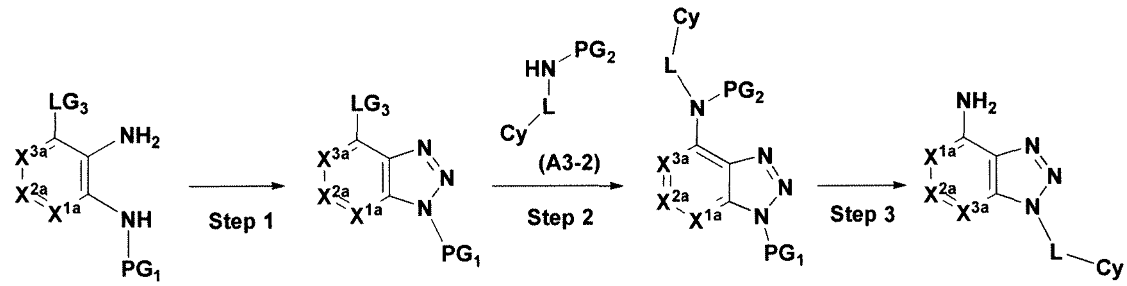


(A3-4) (A3-3) (A3-1) (A3)

[wherein $LG_3$ represents a leaving group such as a halogen atom; $PG_1$ represents a protecting group of amino group; $PG_2$ represents a protecting group of amino group or a hydrogen atom; and the other symbols have the same meanings as those described above.]

Examples of the embodiment include the following scheme.

One embodiment of Scheme 3

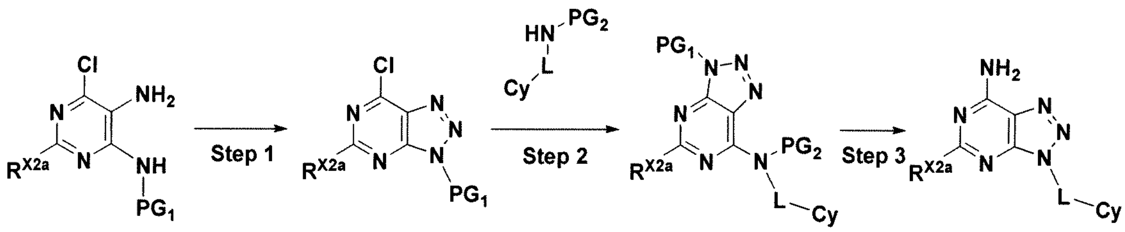


[wherein the symbols have the same meanings as those described above.]

Step 1

The Compound (A3-4) may be reacted in a similar manner to the Step 2 in the Scheme 1 to prepare the Compound (A3-3).

Step 2

The Compound (A3-3) prepared in the Step 1 may be reacted with the Compound (A3-2) in a solvent, in the presence or absence of a base, and in the presence or absence of hydrogen chloride to prepare the Compound (A3-1).

The solvent may be any which does not affect the reaction, and examples thereof include ethers such as tetrahydrofuran and 1,4-dioxane; aromatic hydrocarbons such as toluene; nitriles such as acetonitrile; water; and mixtures thereof.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

The amount of the Compound (A3-2) to be used may be 1.0 to 5.0 equivalent(s), preferably 2.0 to 3.0 equivalents, relative to the Compound (A3-3) in molar ratio.

The amount of the base to be used may be 1.0 to 5.0 equivalent(s), preferably 2.0 to 3.0 equivalents, relative to the Compound (A3-3) in molar ratio.

The reaction may be carried out at room temperature to under heating, for example at room temperature to

100°C, preferably at room temperature to 80°C.

Step 3

The Compound (A3-1) prepared in the Step 2 may be reacted in the presence of an acid, and in the presence or absence of a reducing agent to prepare the Compound (A3).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; dimethyl sulfoxide; water; and mixtures thereof.

Examples of the acid include hydrochloric acid and trifluoroacetic acid.

Examples of the reducing agent include trialkylsilane such as triethylsilane.

The amount of the acid to be used may be 30 to 100 equivalents, preferably 50 to 70 equivalents, relative to the Compound (A3-1) in molar ratio.

The amount of the reducing agent to be used may be 3.0 to 20 equivalents, preferably 5.0 to 10 equivalents, relative to the Compound (A3-1) in molar ratio.

The reaction may be carried out under heating, for example at 50°C to 100°C, preferably at 60°C to 90°C.

Among the Compound (A3-2), the compound represented by

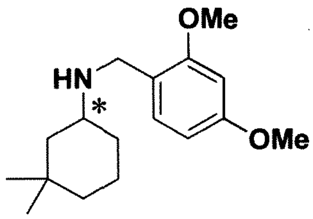

racemate

racemate

may be prepared by reacting 3,3-dimethylcyclohexanone with 2,4-dimethoxybenzylamine in a solvent such as 1,2-dichloroethane, in the presence of an acid such as acetic acid, and in the presence of a reducing agent such as sodium triacetoxyborohydride.

In structural formulas described in the present description, a description of bond line may mean that a methyl group present in one end is omitted. As one example, the above formula means the same structure as the formula:

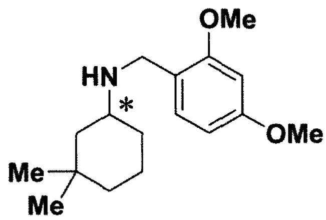

.

Production method 4

Among the compound represented by the formula (I), a compound wherein the partial structure represented by the formula (I-1) has the structure represented by the formula (I-1-A) may also be prepared according to, for example, the following Scheme 4.

Scheme 4

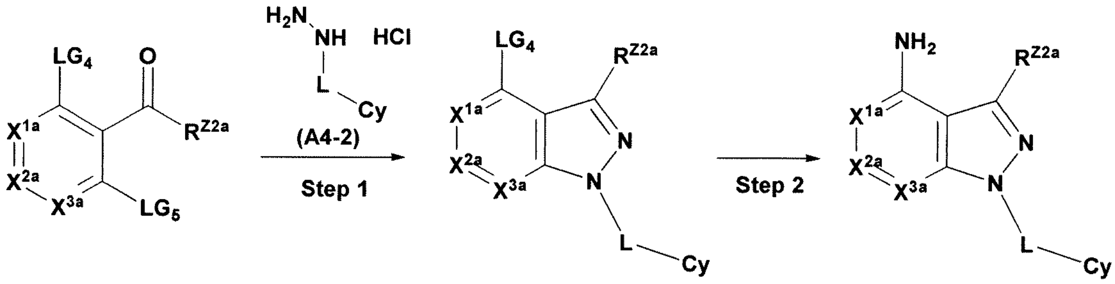


(A4-3) (A4-1) (A4)

[wherein $LG_4$ and $LG_5$ each independently represent a leaving group such as a halogen atom; and the other symbols have the same meanings as those described above.]

Examples of the embodiment include the following scheme.

One embodiment of Scheme 4

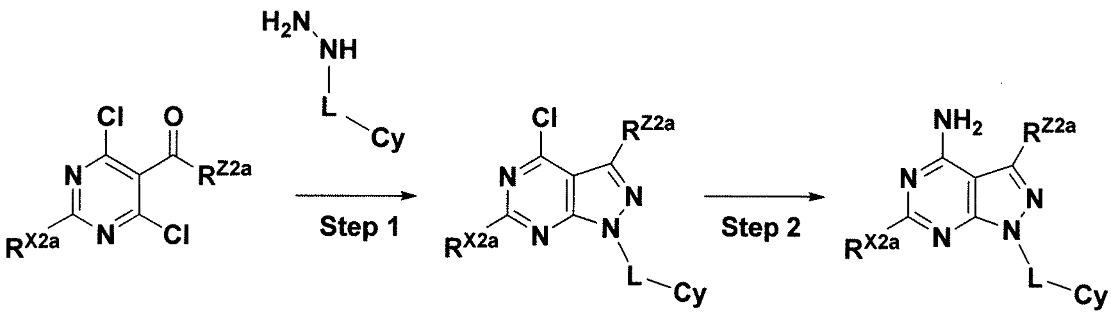


[wherein the symbols have the same meanings as those described above.]

Step 1

The Compound (A4-3) may be reacted with the Compound (A4-2) in a solvent, and in the presence of a base to prepare the Compound (A4-1).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

The amount of the Compound (A4-2) to be used may be 1.0 to 5.0 equivalent(s), preferably 2.0 to 3.0 equivalents, relative to the Compound (A4-3) in molar ratio.

The amount of the base to be used may be 1.0 to 5.0 equivalent(s), preferably 2.0 to 3.0 equivalents, relative to the Compound (A4-3) in molar ratio.

The reaction may be carried out at room temperature to under heating, for example at room temperature to 50°C, preferably at room temperature.

Step 2

The Compound (A4-1) prepared in the Step 1 may be reacted with ammonia in the presence or absence of microwave radiation to prepare the Compound (A4).

The solvent may be any which does not affect the reaction, and examples thereof include ethers such as

tetrahydrofuran and 1,4-dioxane, alcohols such as methanol, ethanol, and isopropanol, aromatic hydrocarbons such as toluene, nitriles such as acetonitrile, and mixtures thereof.

The amount of the ammonia to be used may be 20 to 60 equivalents, preferably 30 to 50 equivalents, relative to the Compound (A4-1) in molar ratio.

The reaction may be carried out at room temperature to under heating, for example at room temperature to 200°C, preferably at 130°C to 180°C.

Among the Compound (A4), a compound wherein $R^{Z2a}$ is a trifluoromethyl group may also be prepared by the following reaction.

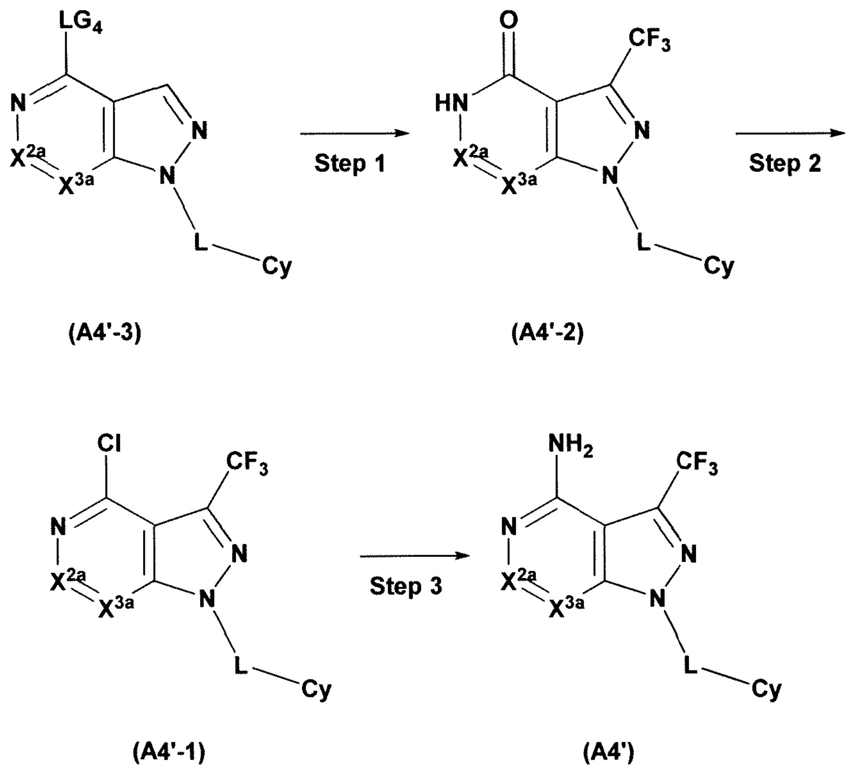


(A4'-3) (A4'-2) (A4'-1) (A4')

[wherein the symbols have the same meanings as those described above.]

Step 1

The Compound (A4'-3) wherein $R^{Z2a}$ is a hydrogen atom prepared in the Step 1 of the Scheme 4 may be reacted with a trifluoromethylating agent in a solvent, and in the presence of an activating agent to prepare the Compound (A4'-2).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; dimethyl sulfoxide; water; and mixtures thereof.

Examples of the activating agent include tert-butyl peroxide, 1-hydroxy-7-azabenzotriazole (HOAt), 1-hydroxybenzotriazole (HOBt), and 4-dimethylaminopyridine.

Examples of the trifluoromethylating agent include sodium trifluoromethanesulfinate.

The amount of the activating agent to be used may be 3.0 to 20 equivalents, preferably 5.0 to 10 equivalents, relative to the Compound (A4'-3) in molar ratio.

The amount of the trifluoromethylating agent to be used may be 3.0 to 20 equivalents, preferably 5.0 to 10 equivalents, relative to the Compound (A4'-3) in molar ratio.

The reaction may be carried out at room temperature to under heating, for example at room temperature to 50°C, preferably at room temperature.

Step 2

The Compound (A4'-2) prepared in the Step 1 may be reacted with a chlorinating agent in a solvent and in the presence of a base to prepare the Compound (A4'-1).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; and mixtures thereof.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

Examples of the chlorinating agent include thionyl chloride.

The amount of the base to be used may be 1.0 to 5.0 equivalent(s), preferably 2.0 to 3.0 equivalents, relative to the Compound (B4'-2) in molar ratio.

The amount of the chlorinating agent to be used may be 30 to 60 equivalents, preferably 40 to 50 equivalents, relative to the Compound (B4'-2) in molar ratio.

The reaction may be carried out at room temperature to under heating, for example at room temperature to 100°C, preferably at room temperature to 80°C.

Step 3

The Compound (A4'-1) prepared in the Step 1 may be reacted in a similar manner to the Step 2 of Scheme 4 to prepare the Compound (A4').

Production method 5

Among the compound represented by the formula (I), a compound wherein the partial structure represented by the formula (I-1) has the structure represented by the formula (I-1-A) may also be prepared according to, for example the following Scheme 5.

Scheme 5

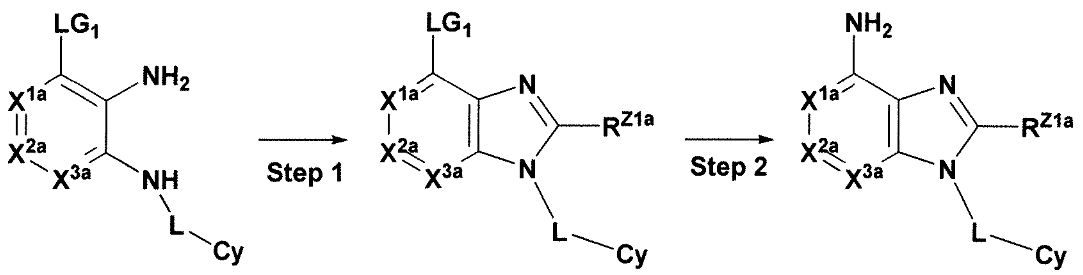


(A1-2) (A5-1) (A5)

[wherein the symbols have the same meanings as those described above.]

Examples of the embodiment include the following scheme.

One embodiment of Scheme 5

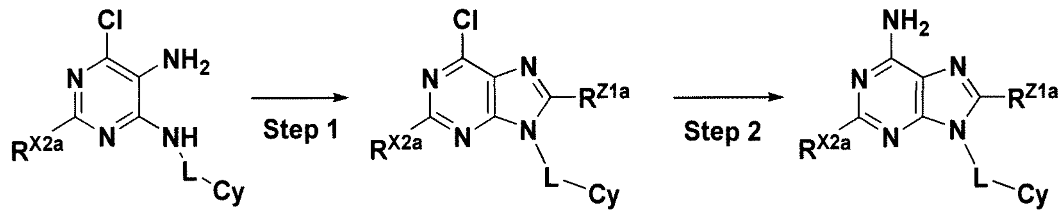


[wherein the symbols have the same meanings as those described above.]

Step 1

The Compound (A1-2) prepared in the Step 1 of the Scheme 1 may be reacted with an ester in the presence of an acid to prepare the Compound (A5-1).
Examples of the acid include p-toluenesulfonic acid.
Examples of the ester include formates such as triethyl orthoformate.
The amount of the acid to be used may be 0.1 to 3.0 equivalent(s), preferably 0.1 to 1.0 equivalent(s), relative to the Compound (A1-2) in molar ratio.
The amount of the ester to be used may be 1.0 to 5.0 equivalent(s), preferably 1.0 to 3.0 equivalent(s), relative to the Compound (A1-2) in molar ratio.
The reaction may be carried out under heating, for example at 70°C to 150°C, preferably at 90°C to 120°C.

Step 2

The Compound (A5-1) prepared in the Step 1 may be reacted with ammonia under microwave radiation to prepare the Compound (A5).
The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.
The amount of the ammonia to be used may be 20 to 60 equivalents, preferably 30 to 50 equivalents, relative to the Compound (A5-1) in molar ratio.
The reaction may be carried out under heating, for example at 100°C to 200°C, preferably at 130°C to 180°C.

Production method 6

Among the compound represented by the formula (I), a compound wherein the partial structure represented by the formula (I-1) has the structure represented by the formula (I-1-A) may also be prepared according to, for example the following Scheme 6.

Scheme 6

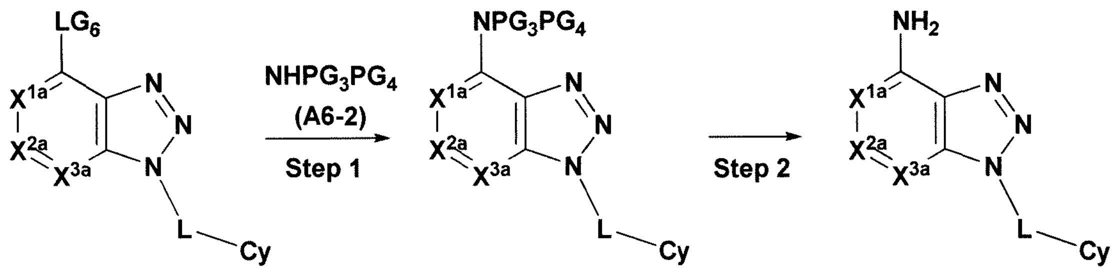


(A6-3) (A6-1) (A6)

[wherein $LG_6$ represents a leaving group such as a halogen atom; $PG_3$ and $PG_4$ each independently represent a protecting group of amino group; and the other symbols have the same meanings as those described above.]
Examples of the embodiment include the following scheme.

One embodiment of Scheme 6

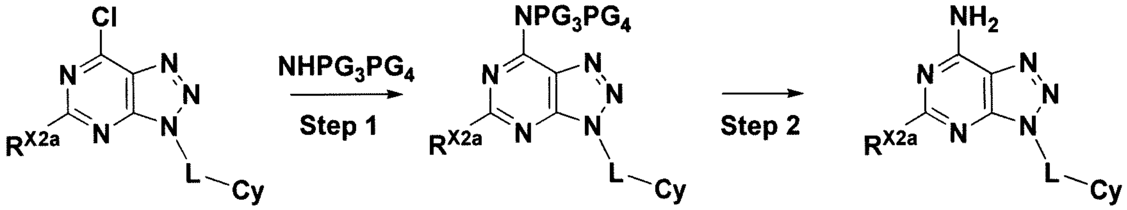


[wherein the symbols have the same meanings as those described above.]

Step 1

The Compound (A6-3) prepared in the Step 2 of the Scheme 1 may be reacted with the Compound (A6-2) in a solvent and in the presence of a base to prepare the Compound (A6-1).

The solvent may be any which does not affect the reaction, and examples thereof include ethers such as tetrahydrofuran and 1,4-dioxane; aromatic hydrocarbons such as toluene; nitriles such as acetonitrile; water; and mixtures thereof.

Examples of the Compound (A6-2) include bis(2,4-dimethoxybenzyl)amine.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

The amount of the Compound (A6-2) to be used may be 1.0 to 5.0 equivalent(s), preferably 1.5 to 3.0 equivalents, relative to the Compound (A6-3) in molar ratio.

The amount of the base to be used may be 1.0 to 5.0 equivalent(s), preferably 2.0 to 3.0 equivalents, relative to the Compound (A6-3) in molar ratio.

The reaction may be carried out at room temperature to under heating, for example at room temperature to 50°C, preferably at room temperature.

Step 2

The Compound (A6-1) prepared in the Step 1 may be reacted in a solvent, in the presence of an acid, and in the presence or absence of a reducing agent to prepare the Compound (A6).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; dimethyl sulfoxide; water; and mixtures thereof.

Examples of the acid include hydrochloric acid and trifluoroacetic acid.

Examples of the reducing agent include trialkylsilane such as triethylsilane.

The amount of the acid to be used may be 30 to 100 equivalents, preferably 50 to 70 equivalents, relative to the Compound (A6-1) in molar ratio.

The amount of the reducing agent to be used may be 3.0 to 20 equivalents, preferably 5.0 to 10 equivalents, relative to the Compound (A6-1) in molar ratio.

The reaction may be carried out at room temperature to under heating, for example at room temperature to 100°C, preferably at room temperature to 70°C.

Production method 7

Among the compound represented by the formula (I), a compound wherein the partial structure represented by the formula (I-1) has the structure represented by the formula (I-1-B) may be prepared according to, for example the following Scheme 7.

Scheme 7

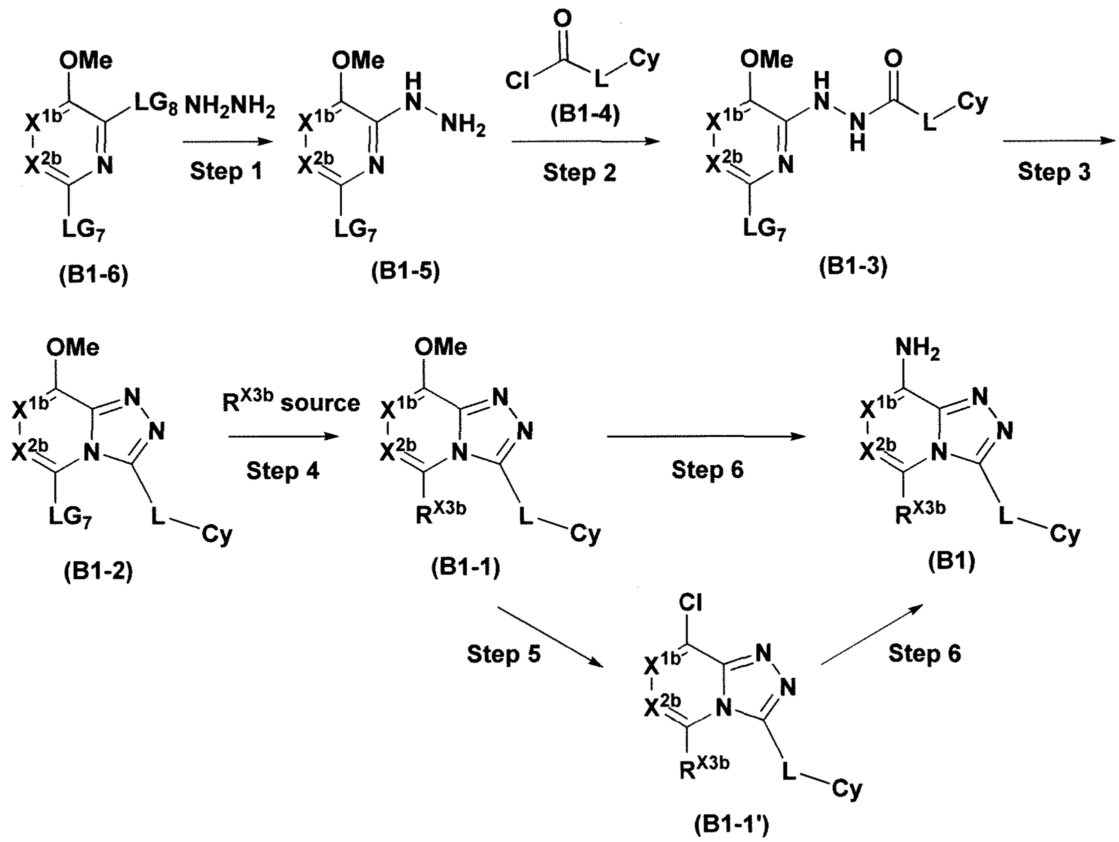


(B1-6) (B1-5) (B1-4) (B1-3) (B1-2) (B1-1) (B1) (B1-1')

[wherein $LG_7$ and $LG_8$ each independently represent a leaving group such as a halogen atom; and the other symbols have the same meanings as those described above.]

Examples of the embodiment include the following scheme.

One embodiment of Scheme 7

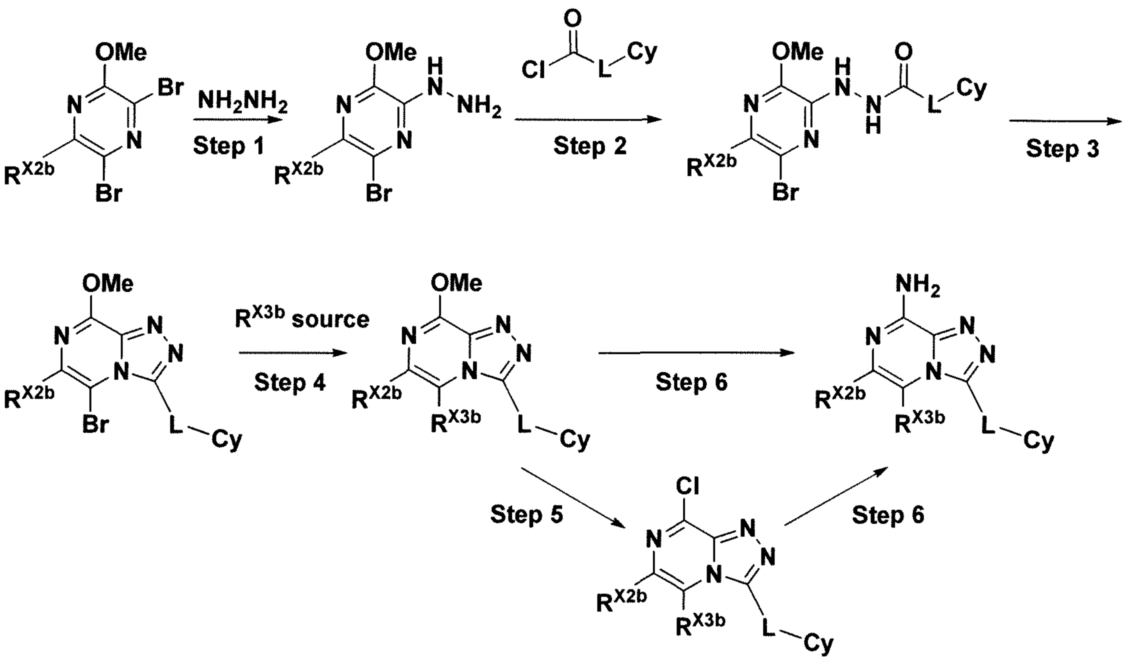

[wherein the symbols have the same meanings as those described above.]

Step 1

The Compound (B1-6) may be reacted with hydrazine in a solvent to prepare the Compound (B1-5).
The solvent may be any which does not affect the reaction, and examples thereof include ethers such as tetrahydrofuran and 1,4-dioxane; alcohols such as methanol, ethanol, and isopropanol; aromatic hydrocarbons such as toluene; nitriles such as acetonitrile; water; and mixtures thereof.
The amount of the hydrazine to be used may be 1.0 to 2.0 equivalent(s), preferably 1.0 to 1.5 equivalent(s), relative to the Compound (B1-6) in molar ratio. The reaction may be carried out under heating, for example at 50°C to 150°C, preferably at 70°C to 100°C.

Step 2

The Compound (B1-5) prepared in the Step 1 may be reacted with the Compound (B1-4) in a solvent and in the presence of a base to prepare the Compound (B1-3).
The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.
Examples of the base include alkali metal carbonates such as cesium carbonate, potassium carbonate, sodium carbonate, and sodium hydrogen carbonate; alkali metal phosphates such as potassium phosphate tribasic, sodium phosphate, and sodium hydrogen phosphate; amine such as N,N-diisopropylethylamine; alkali metal fluorides such as cesium fluoride and potassium fluoride; and alkali metal alkoxides such as sodium t-butoxide and potassium t-butoxide.
The amount of the Compound (B1-4) to be used may be 1.0 to 5.0 equivalent(s), preferably 2.0 to 3.0 equivalents, relative to the Compound (B1-5) in molar ratio.
The amount of the base to be used may be 1.0 to 5.0 equivalent(s), preferably 2.0 to 3.0 equivalents, relative to the Compound (B1-5) in molar ratio.
The reaction may be carried out under heating, for example at 50°C to 150°C, preferably at 70°C to 100°C.

Step 3

The Compound (B1-3) prepared in the Step 2 may be reacted in a solvent, in the presence of a phosphine derivative, in the presence of a base, and in the presence of a perhalogenated aliphatic hydrocarbon to prepare the Compound (B1-2).
The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.
Examples of the phosphine derivative include triphenylphosphine.
Examples of the perhalogenated aliphatic hydrocarbons include carbon tetrachloride and hexachloroethane, preferably hexachloroethane.
Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.
The amount of the phosphine derivative to be used may be 1.0 to 3.0 equivalent(s), preferably 1.5 to 2.5 equivalents, relative to the Compound (B1-3) in molar ratio.
The amount of the base to be used may be 3.0 to 5.0 equivalents, preferably 3.5 to 4.5 equivalents, relative to the Compound (B1-3) in molar ratio.
The amount of the perhalogenated aliphatic hydrocarbons to be used may be 1.0 to 3.0 equivalent(s), preferably 1.5 to 2.5 equivalents, relative to the Compound (B1-3) in molar ratio.
The reaction may be carried out at 0°C to under heating, for example at 0°C to 60°C, preferably at 0°C to room temperature.

Step 4

The Compound (B1-2) prepared in the Step 3 may be reacted with a $R^{X3b}$ source in a solvent, in the presence of a catalyst, in the presence or absence of a ligand, in the presence or absence of a base, in the presence or absence of an additive, and in the presence or absence of microwave radiation to prepare the Compound (B1-1).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; water; and mixtures thereof.

Examples of the catalyst include palladium catalysts such as palladium(II) acetate, [1,1'-bis(diphenylphosphino)ferrocene]palladium(II) dichloride ($PdCl_2$(dppf)), $PdCl_2$(dppf) dichloromethane adduct, tris(dibenzylideneacetone)dipalladium(O) ($Pd_2(dba)_3$), tetrakistriphenylphosphinepalladium, and bis(triphenylphosphine)palladium dichloride; copper(I) iodide; and iron(III) acetylacetonate.

Examples of the ligand include 1,1'-bis(diphenylphosphino)ferrocene (dppf), tricyclohexylphosphine, and phenanthroline.

Examples of the base include alkali metal amides such as lithium diisopropylamide, sodium amide, and lithium bistrimethylsilylamide; alkali metal carbonates such as sodium carbonate, potassium carbonate, sodium hydrogen carbonate, and potassium hydrogen carbonate; alkali metal phosphates such as sodium phosphate and potassium phosphate; amines such as triethylamine, diisopropylethylamine, pyridine, and N-methylmorpholine.

Examples of the additive include alkali metal halides such as potassium fluoride.

Examples of the $R^{X3b}$ source include trimethylboroxine; alkylating agents, for example, Grignard reagents such as ethylmagnesium bromide; cyanating agents such as zinc dicyanide; arylating agents such as phenylboronic acid; trifluoromethylating agents such as (trifluoromethyl)trimethylsilane; and cycloalkylating agents such as cyclopropylboronic acid.

The amount of the $R^{X3b}$ source to be used may be 1.0 to 3.0 equivalent(s), preferably 1.1 to 2.0 equivalents, relative to the Compound (B1-2) in molar ratio.

The amount of the catalyst to be used may be 0.01 to 1.0 equivalent(s), preferably 0.05 to 0.70 equivalent(s), relative to the Compound (B1-2) in molar ratio.

The amount of the ligand to be used may be 0.05 to 1.0 equivalent(s), preferably 0.10 to 0.40 equivalent(s), relative to the Compound (B1-2) in molar ratio.

The amount of the base to be used may be 1.0 to 5.0 equivalent(s), preferably 2.0 to 4.5 equivalents, relative to the Compound (B1-2) in molar ratio.

The amount of the additive to be used may be 1.0 to 5.0 equivalent(s), preferably 1.5 to 2.5 equivalents, relative to the Compound (B1-2) in molar ratio.

The reaction may be carried out at -78°C to under heating, for example at -78°C to 200°C, preferably at -78°C to 120°C.

Step 5

The Compound (B1-1) prepared in the Step 4 may be reacted with a chlorinating agent to prepare the Compound (B1-1').

Examples of the chlorinating agent include phosphoryl chloride.

The amount of the chlorinating agent to be used may be 30 to 60 equivalents, preferably 40 to 50 equivalents, relative to the Compound (B1-1) in molar ratio.

The reaction may be carried out under heating, for example at 80°C to 200°C, preferably at 100°C to 150°C.

Step 6

The Compound (B1-1) prepared in the Step 4 or the Compound (B1-1') prepared in the Step 5 may be reacted with ammonia or ammonium hydroxide in a solvent, and in the presence or absence of microwave radiation to prepare the Compound (B1).

The solvent may be any which does not affect the reaction, and examples thereof include alcohols such as methanol, ethanol, and isopropanol.

The amount of the ammonia or ammonium hydroxide to be used may be 20 to 60 equivalents, preferably 30 to 50 equivalents, relative to the Compound (B1-1) in molar ratio.

The reaction may be carried out under heating, for example at 50°C to 150°C, preferably at 80°C to 120°C.

Among the Compound (B1-4), a compound having the following structure:

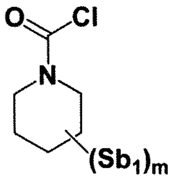

may also be prepared by the following reaction.

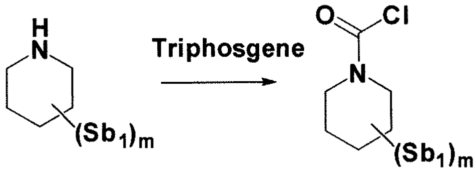


(B1-9) (B1-4')

[wherein $Sb_1$ represents a substituent of the above nonaromatic heterocyclic group or a precursor thereof; and m represents an integer of 0 to 5] or

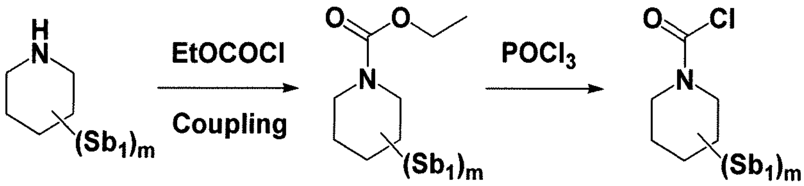


(B1-9) (B1-7) (B1-4')

[wherein the symbols have the same meanings as those described above.]

Also, a Compound (B1-8) may exist as a precursor of the Compound (B1-7) as follows.

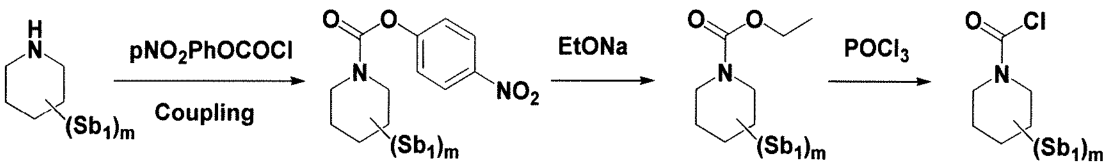


(B1-9) (B1-8) (B1-7) (B1-4')

[wherein the symbols have the same meanings as those described above.]

Production method 8

Among the compound represented by the formula (I), a compound wherein the partial structure represented by the formula (I-1) has the structure represented by the formula (I-1-B) may also be prepared according to, for example, the following Scheme 8.

Scheme 8

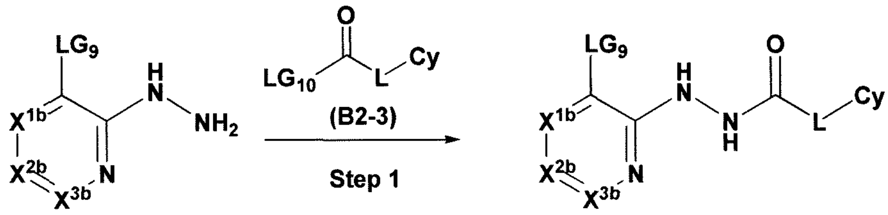


(B2-4) (B2-2)

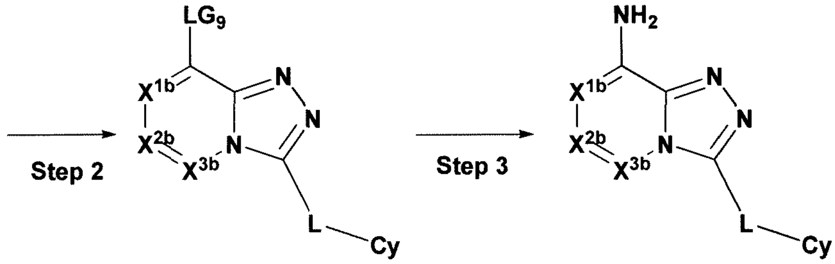


(B2-1) (B2)

[wherein $LG_9$ represents a leaving group such as a halogen atom; $LG_{10}$ represents a chlorine atom or a hydroxy group; and the other symbols have the same meanings as those described above.]

Examples of the embodiment include the following scheme.

One embodiment of Scheme 8

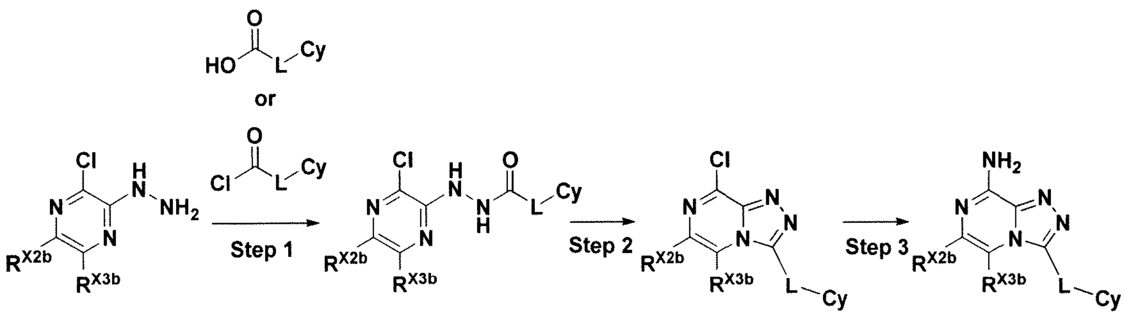


[wherein the symbols have the same meanings as those described above.]

Step 1

The Compound (B2-4) may be reacted with the Compound (B2-3) in a solvent, in the presence of a base, in the presence or absence of a condensing agent, and in the presence or absence of an activating agent to prepare the Compound (B2-2).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; and mixtures thereof.

Examples of the base include inorganic bases, for example, alkali metal hydrogen carbonates such as sodium hydrogen carbonate, alkali metal carbonates such as potassium carbonate, and alkali metal hydroxides such as sodium hydroxide; and organic bases for example, alkylamines such as triethylamine and diisopropylethylamine, pyridines such as pyridine and dimethylaminopyridine, and diisopropylpiperidine.

Examples of the condensing agent include O-(7-azabenzotriazol-1-yl)-N,N,N',N'-tetramethyluronium hexafluorophosphate (HATU), 1-ethyl-3-(3-dimethylaminopropyl)carbodiimide hydrochloride, and 1-(3-dimethylaminopropyl)-3-ethylcarbodiimide hydrochloride.

Examples of the activating agent include 1-hydroxy-7-azabenzotriazole (HOAt), 1-hydroxybenzotriazole (HOBt), and 4-dimethylaminopyridine.

The amount of the Compound (B2-3) to be used may be 1.0 to 5.0 equivalent(s), preferably 2.0 to 3.0 equivalents, relative to the Compound (B2-4) in molar ratio.

The amount of the base to be used may be 1.0 to 5.0 equivalent(s), preferably 2.0 to 3.0 equivalents, relative to the Compound (B2-4) in molar ratio.

The amount of the condensing agent to be used may be 1.0 to 5.0 equivalent(s), preferably 1.5 to 3.0 equivalents, relative to the Compound (B2-4) in molar ratio.

The amount of the activating agent to be used may be 1.0 to 5.0 equivalent(s), preferably 1.5 to 3.0 equivalents, relative to the Compound (B2-4) in molar ratio.

The reaction may be carried out at room temperature to under heating, for example at room temperature to 100°C, preferably at room temperature to 80°C.

Step 2

The Compound (B2-2) prepared in the Step 1 may be reacted with methyl-N-(triethylammoniumsulphonyl)carbamate (also referred to as Burgess reagent) in a solvent to prepare the Compound (B2-1).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.

The amount of the Burgess reagent to be used may be 1.0 to 3.0 equivalent(s), preferably 1.5 to 2.5 equivalents, relative to the Compound (B2-2) in molar ratio. The reaction may be carried out under heating, for example at 50°C to 150°C, preferably at 80°C to 100°C.

Alternatively, the Compound (B2-2) prepared in the Step 1 may be reacted in a solvent, in the presence of a phosphine derivative, in the presence or absence of a base, and in the presence of a perhalogenated aliphatic hydrocarbon to prepare the Compound (B2-1).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.

Examples of the phosphine derivative include triphenylphosphine.

Examples of the perhalogenated aliphatic hydrocarbon include carbon tetrachloride and hexachloroethane, preferably hexachloroethane.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

The amount of the phosphine derivative to be used may be 1.0 to 3.0 equivalent(s), preferably 1.5 to 2.5 equivalents, relative to the Compound (B2-2) in molar ratio.

The amount of the base to be used may be 3.0 to 5.0 equivalents, preferably 3.5 to 4.5 equivalents, relative to the Compound (B2-2) in molar ratio.

The amount of the perhalogenated aliphatic hydrocarbon to be used may be 1.0 to 3.0 equivalent(s), preferably 1.5 to 2.5 equivalents, relative to the Compound (B2-2) in molar ratio.

The reaction may be carried out at room temperature to under heating, preferably at room temperature.

Step 3

The Compound (B2-1) prepared in the Step 2 may be reacted with ammonia in a solvent and in the presence or absence of microwave radiation to prepare the Compound (B2).

The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, water, and mixtures thereof.

The amount of the ammonia to be used may be 20 to 60 equivalents, preferably 30 to 50 equivalents, relative to the Compound (B2-1) in molar ratio.

The reaction may be carried out under heating, for example at 50°C to 150°C, preferably at 80°C to 120°C.

Among the Compound (B2), a compound wherein $X^{3b}$ is $CR^{X3b}$ and $R^{X3b}$ is a chlorine atom may also be prepared by reacting the corresponding starting compound wherein $X^{3b}$ is CH with a chlorinating agent in a solvent.

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; and mixtures thereof.

Examples of the chlorinating agent include N-chlorosuccinimide.

The amount of the chlorinating agent to be used may be 1.0 to 3.0 equivalent(s), preferably 1.1 to 1.5 equivalents, relative to the corresponding starting Compound (B2) wherein $X^{3b}$ is CH in molar ratio.

The reaction may be carried out at room temperature to under heating, for example at room temperature to 50°C, preferably at room temperature.

Among the Compound (B2-3), the compound having the structure represented by

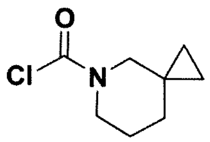

may also be prepared by the following reaction.

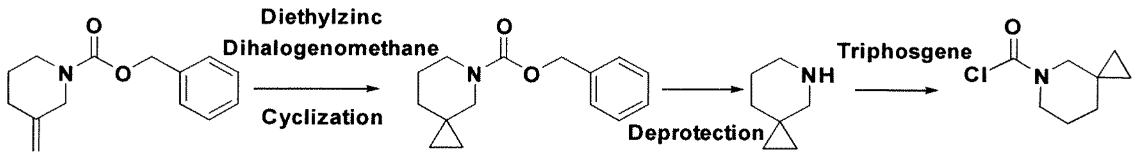


Among the Compound (B2-3), the compound having the structure represented by

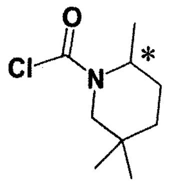

racemate

may also be prepared by the following reaction.

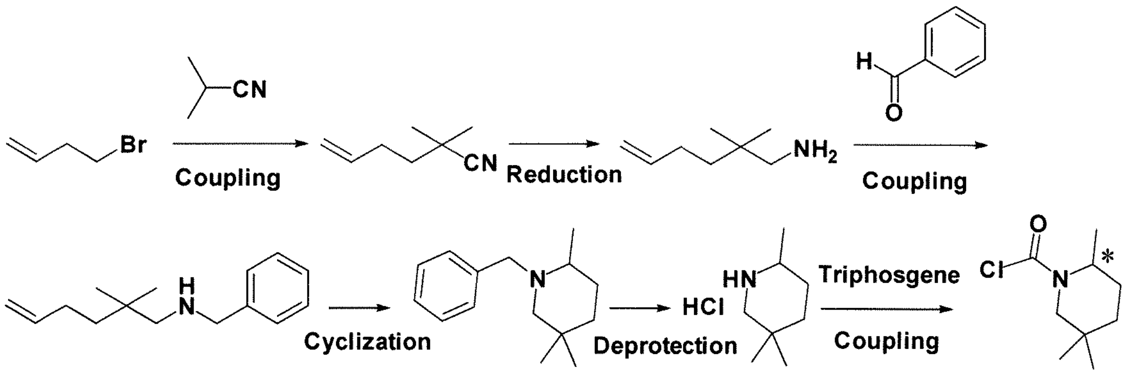


Among the Compound (B2-3), the compound represented by the following formula

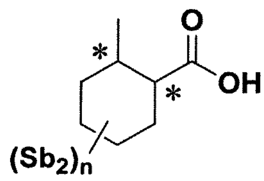

mixture of stereoisomers
may also be prepared by the following reaction.

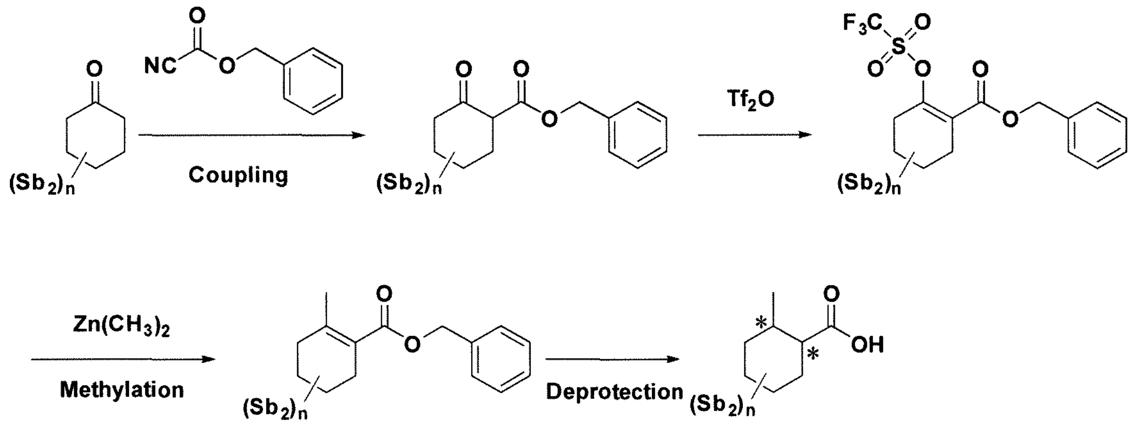


[wherein $Sb_2$ represents a substituent of the above alicyclic hydrocarbon group or a precursor thereof; and n represents an integer of 0 to 4.]

Among the Compound (B2-3), the compound represented by the following formula

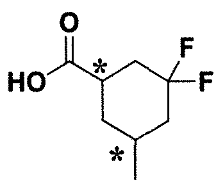

mixture of stereoisomers
may also be prepared by the following reaction.

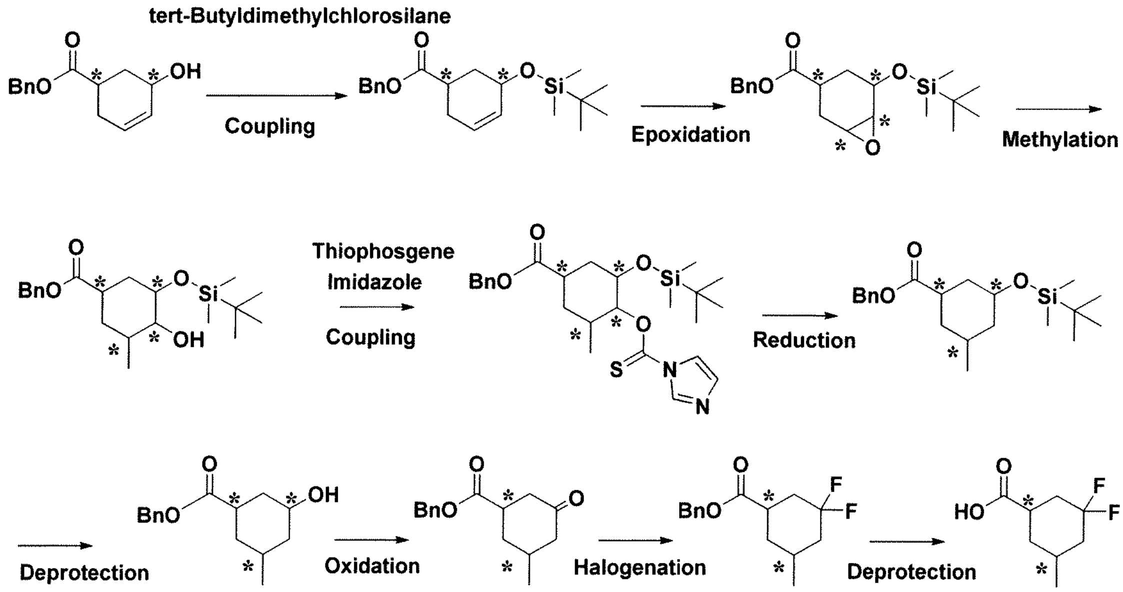


Production method 9

Among the compound represented by the formula (I), a compound wherein the partial structure represented by the formula (I-1) has the structure represented by the formula (I-1-B) may also be prepared according to, for example the following Scheme 9.

Scheme 9

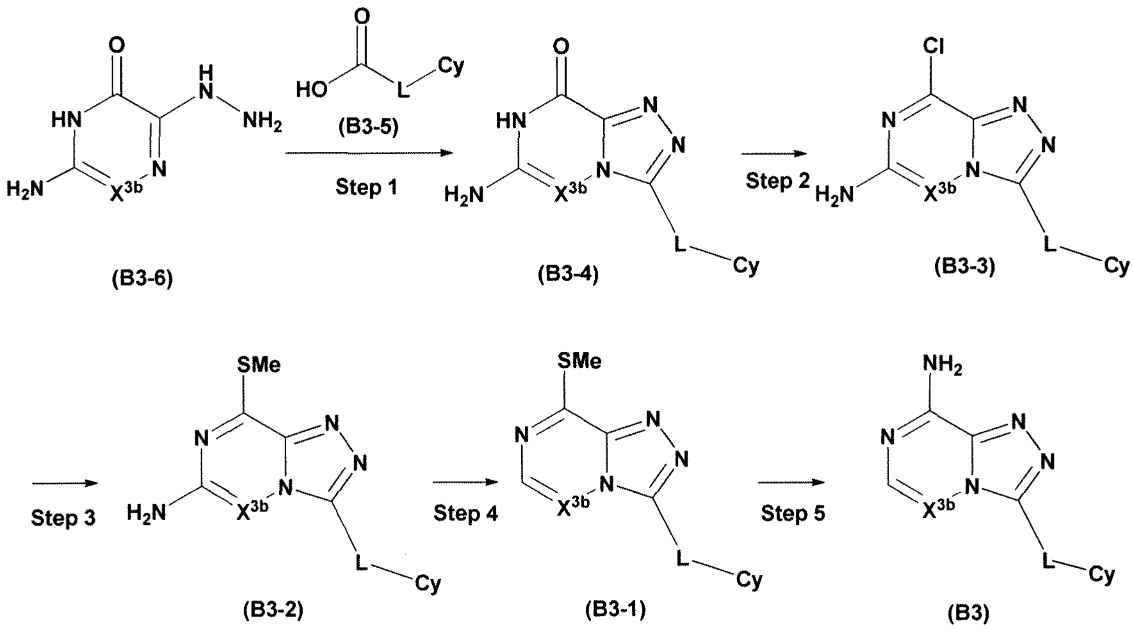


(B3-6) (B3-4) (B3-3) (B3-2) (B3-1) (B3)

[wherein the symbols have the same meanings as those described above.]

Examples of the embodiment include the following scheme.

One embodiment of Scheme 9

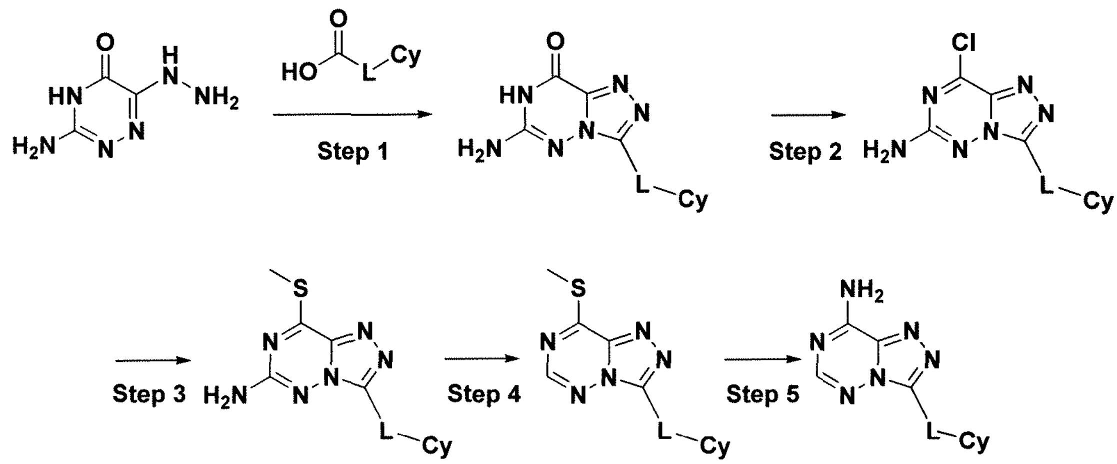


[wherein the symbols have the same meanings as those described above.]

Step 1

The Compound (B3-6) may be reacted with the Compound (B3-5) in a solvent, in the presence of a base, in the presence of a condensing agent, and in the presence of an activating agent, under ice-cooling to under heating (for example, under ice-cooling to at 50°C) to prepare an intermediate compound. The resulting intermediate compound may be heated (for example, at 180°C to 200°C) in a solvent, and allowed to cool to prepare the Compound (B3-4).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; alcohols such as ethylene glycol; and mixtures thereof.

Examples of the base include inorganic bases, for example, alkali metal hydrogen carbonates such as sodium hydrogen carbonate, alkali metal carbonates such as potassium carbonate, and alkali metal hydroxides such as sodium hydroxide; and organic bases, for example, alkylamines such as triethylamine and diisopropylethylamine, and pyridines such as pyridine and dimethylaminopyridine.

Examples of the condensing agent include O-(7-azabenzotriazol-1-yl)-N,N,N',N'-tetramethyluronium hexafluorophosphate (HATU), 1-ethyl-3-(3-dimethylaminopropyl)carbodiimide hydrochloride, and 1-ethyl-3-(3-dimethylaminopropyl)carbodiimide hydrochloride.

Examples of the activating agent include 1-hydroxy-7-azabenzotriazole (HOAt), 1-hydroxybenzotriazole (HOBt), and 4-dimethylaminopyridine.

The amount of the Compound (B3-5) to be used may be 0.90 to 5.0 equivalent(s), preferably 0.95 to 2.0 equivalent(s), relative to the Compound (B3-6) in molar ratio.

The amount of the base to be used may be 1.0 to 5.0 equivalent(s), preferably 1.5 to 3.0 equivalents, relative to the Compound (B3-6) in molar ratio.

The amount of the condensing agent to be used may be 1.0 to 5.0 equivalent(s), preferably 1.1 to 2.0 equivalents, relative to the Compound (B3-6) in molar ratio.

The amount of the activating agent to be used may be 0.20 to 2.0 equivalent(s), preferably 0.40 to 1.0 equivalent(s), relative to the Compound (B3-6) in molar ratio.

Step 2

The Compound (B3-4) prepared in the Step 1 may be reacted with a chlorinating agent in a solvent to prepare the Compound (B3-3).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; N,N-dimethylaniline; and mixtures thereof.

Examples of the chlorinating agent include phosphoryl chloride.

The amount of the chlorinating agent to be used may be 30 to 60 equivalents, preferably 40 to 50 equivalents, relative to the Compound (B3-4) in molar ratio.

The reaction may be carried out under heating, for example at 60°C to 150°C, preferably at 80°C to 120°C.

Step 3

The Compound (B3-3) prepared in the Step 2 may be reacted with an alkylthiolating agent in a solvent to prepare the Compound (B3-2).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.

Examples of the alkylthiolating agent include alkali metal alkyl mercaptides such as sodium methyl mercaptide and sodium ethyl mercaptide.

The amount of the alkylthiolating agent to be used may be 1.0 to 10.0 equivalent(s), preferably 2.0 to 5.0 equivalents, relative to the Compound (B3-3) in molar ratio.

The reaction may be carried out at room temperature to under heating, for example at room temperature to 100°C, preferably at room temperature to 50°C.

Step 4

The Compound (B3-2) prepared in the Step 3 may be reacted with nitrite in a solvent to prepare the Compound (B3-1).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.

Examples of the nitrite include isoamyl nitrite.

The amount of the nitrite to be used may be 1.0 to 20.0 equivalent(s), preferably 2.0 to 10.0 equivalents, relative to the Compound (B3-2) in molar ratio.

The reaction may be carried out under heating, for example at 50 to 100°C, preferably at 60 to 80°C.

Step 5

The Compound (B3-1) prepared in the Step 4 may be treated with an ammonia solution or ammonium hydroxide in a solvent and in the presence or absence of microwave radiation to prepare the Compound (B3).

The solvent may be any which does not affect the reaction, and examples thereof include alcohols such as methanol, ethanol, and isopropanol.

The amount of the ammonia or ammonium hydroxide to be used may be 20 to 60 equivalents, preferably 30 to 50 equivalents, relative to the Compound (A1-1) in molar ratio.

The reaction may be carried out under heating, for example at 50°C to 150°C, preferably at 80°C to 120°C.

Among the Compound (B3-5), the compound represented by the following structure

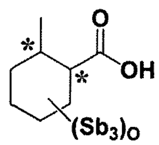

mixture of stereoisomers

may also be prepared by the following reaction.

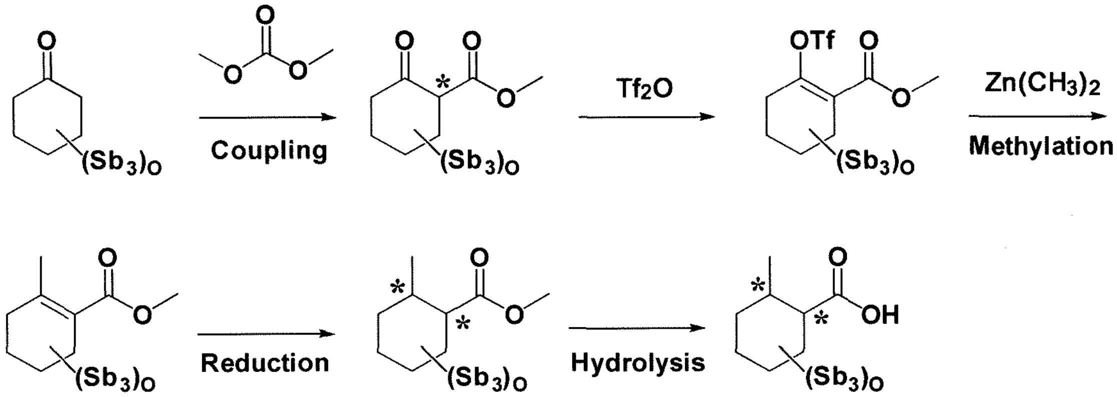


[wherein $Sb_3$ represents a substituent of the above alicyclic hydrocarbon group or a precursor thereof; and o represents an integer of 0 to 4.]

Production method 10

Among the compound represented by the formula (I), a compound wherein the partial structure represented by the formula (I-1) has the structure represented by the formula (I-1-B) may also be prepared according to, for example, the following Scheme 10.

Scheme 10

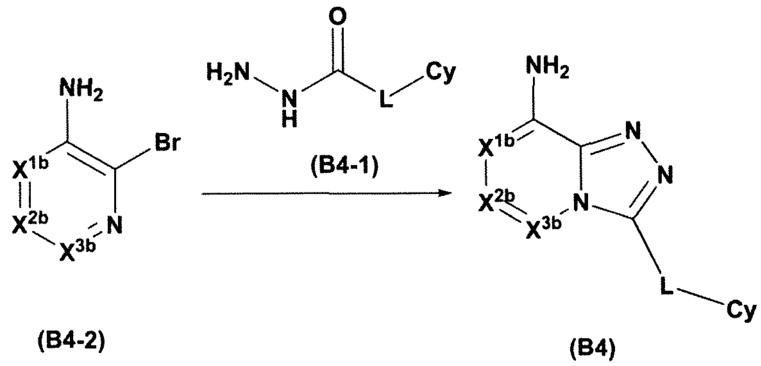

(B4-1)

(B4-2)

(B4)

[wherein the symbols have the same meanings as those described above.]

Examples of the embodiment include the following scheme.

One embodiment of Scheme 10

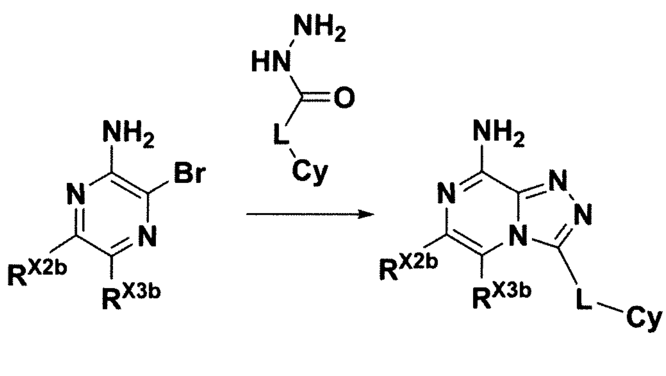

[wherein the symbols have the same meanings as those described above.]

The Compound (B4-2) may be reacted with the Compound (B4-1) in a solvent, in the presence of a base, and under microwave radiation to prepare the Compound (B4).

The solvent may be any which does not affect the reaction, and examples thereof include amides such as N-methylpyrrolidone and N,N-dimethylformamide; ethers such as tetrahydrofuran; nitriles such as acetonitrile; dimethyl sulfoxide; and mixtures thereof.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

The amount of the Compound (A4-1) to be used may be 1.0 to 5.0 equivalent(s), preferably 2.0 to 3.0 equivalents, relative to the Compound (A4-2) in molar ratio.

The amount of the base to be used may be 1.0 to 5.0 equivalent(s), preferably 2.0 to 3.0 equivalents, relative to the Compound (A4-2) in molar ratio.

The reaction may be carried out at room temperature to under heating, for example at 150°C to 300°C, preferably at 200°C to 250°C.

Production method 11

Among the compound represented by the formula (I), a compound wherein the partial structure represented by the formula (I-1) has the structure represented by the formula (I-1-B) may also be prepared according to, for example, the following Scheme 11.

Scheme 11

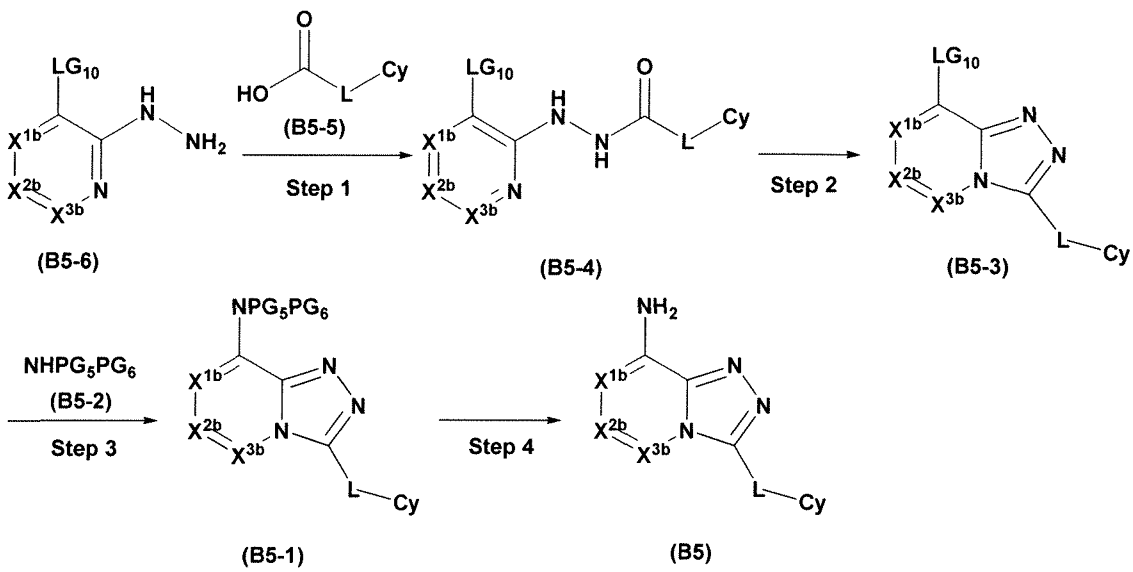


[wherein $LG_{10}$ represents a leaving group such as a halogen atom; $PG_5$ represents a protecting group of amino group; $PG_6$ represents a protecting group of amino group or a hydrogen atom; and the other symbols have the same meanings as those described above.]

Examples of the embodiment include the following scheme.

One embodiment of Scheme 11

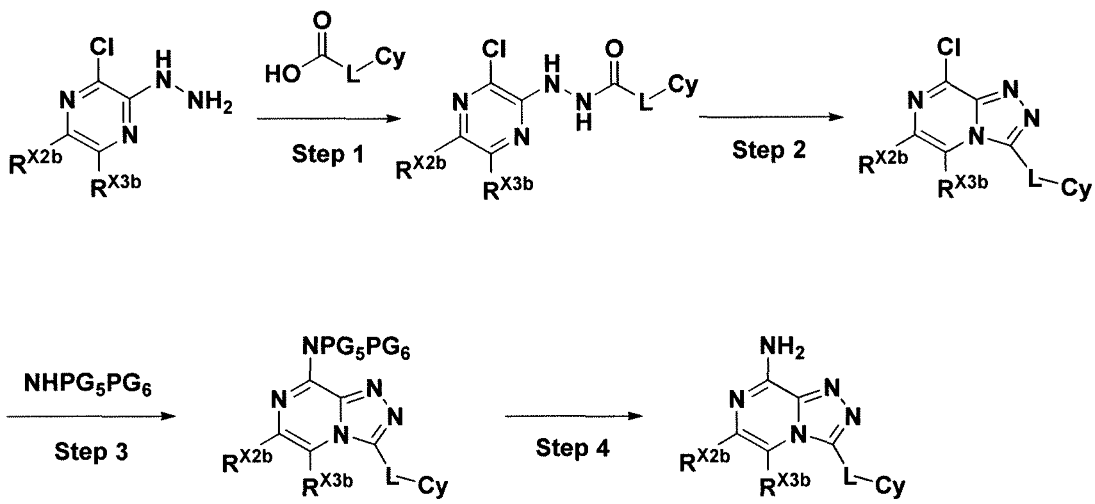


[wherein the symbols have the same meanings as those described above.]

Step 1

The Compound (B5-6) and the Compound (B5-5) may be reacted in a similar manner to the Step 1 of the Scheme 8 to prepare the Compound (B5-4).

Step 2

The Compound (B5-4) may be reacted in a similar manner to the Step 2 of the Scheme 8 to prepare the Compound (B5-3).

Step 3

The Compound (B5-3) prepared in the Step 2 may be reacted with the Compound (B5-2) in a solvent, in the presence of a base, and under microwave radiation to prepare the Compound (B5-1).

Examples of the Compound (B5-2) include bis(2,4-dimethoxybenzyl)amine.

The solvent may be any which does not affect the reaction, and examples thereof include ethers such as tetrahydrofuran and 1,4-dioxane; alcohols such as methanol, ethanol, isopropanol, and tert-butyl alcohol; aromatic hydrocarbons such as toluene; nitriles such as acetonitrile; water; and mixtures thereof.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

The amount of the Compound (B5-2) to be used may be 1.0 to 5.0 equivalent(s), preferably 2.0 to 3.0 equivalents, relative to the Compound (B5-3) in molar ratio.

The amount of the base to be used may be 1.0 to 5.0 equivalent(s), preferably 2.0 to 3.0 equivalents, relative to the Compound (B5-3) in molar ratio.

The reaction may be carried out under heating, for example at 100°C to 200°C, preferably at 130 to 180°C.

Step 4

The Compound (B5-1) prepared in the Step 3 may be reacted in the presence of an acid and in the presence or absence of a reducing agent to prepare the Compound (B5).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; dimethyl sulfoxide; water; and mixtures thereof.

Examples of the acid include hydrochloric acid and trifluoroacetic acid.

Examples of the reducing agent include trialkylsilanes such as triethylsilane.

The amount of the acid to be used may be 30 to 100 equivalents, preferably 50 to 70 equivalents, relative to the Compound (B5-1) in molar ratio.

The amount of the reducing agent to be used may be 3.0 to 20 equivalents, preferably 5.0 to 10 equivalents, relative to the Compound (B5-1) in molar ratio.

The reaction may be carried out under heating, for example at 50°C to 100°C, preferably at 60°C to 90°C.

Production method 12

Among the compound represented by the formula (I), a compound wherein the partial structure represented by the formula (I-1) has the structure represented by the formula (I-1-B) may also be prepared according to, for example the following Scheme 12.

Scheme 12

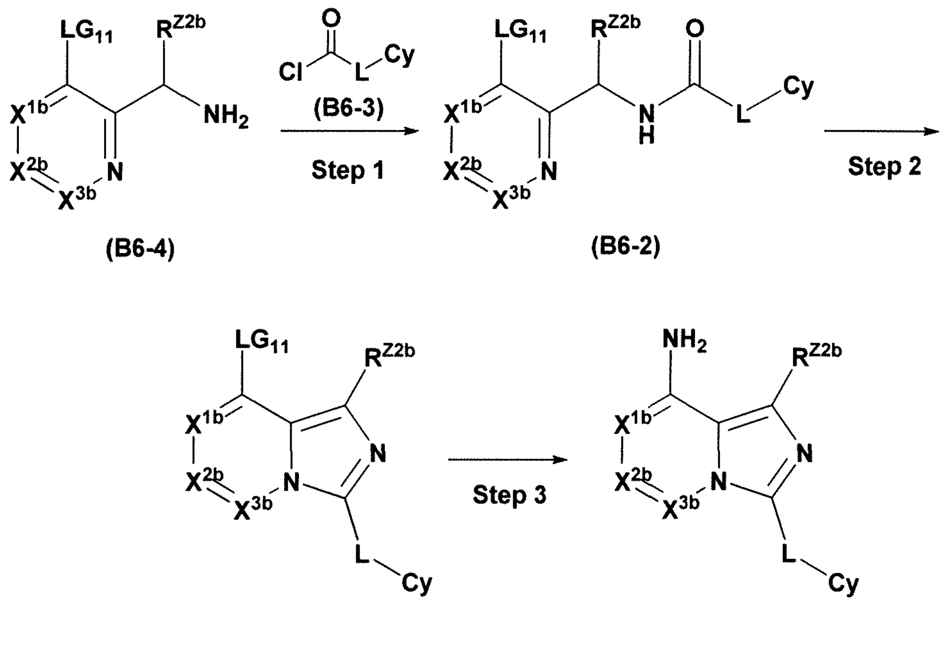


(B6-4) (B6-2)

(B6-1) (B6)

[wherein $LG_{11}$ represents a leaving group such as a halogen atom; and the other symbols have the same meanings as those described above.]

Examples of the embodiment include the following scheme.

One embodiment of Scheme 12

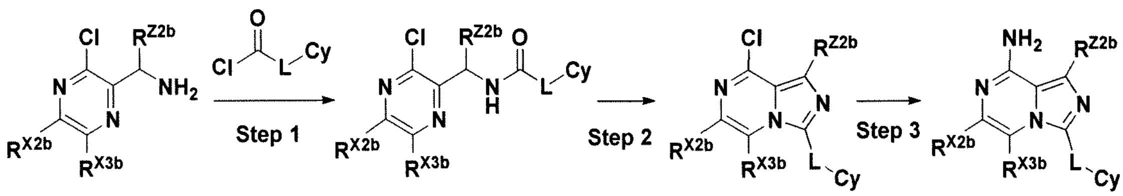


[wherein the symbols have the same meanings as those described above.]

Step 1

The Compound (B6-4) and Compound (B6-3) may be reacted in a similar manner to the Step 1 of the Scheme 8 to prepare the Compound (B6-2).

Step 2

The Compound (B6-2) prepared in the Step 1 may be reacted in a similar manner to the Step 2 of the Scheme 8 to prepare the Compound (B6-1).

Step 3

The Compound (B6-1) prepared in the Step 2 may be reacted with ammonia in a solvent and under microwave radiation to prepare the Compound (B6).
The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.
The amount of the ammonia to be used may be 20 to 60 equivalents, preferably 30 to 50 equivalents, relative to the Compound (B6-1) in molar ratio.
The reaction may be carried out under heating, for example at 100°C to 200°C, preferably at 130°C to 180°C.

Production method 13

Among the compound represented by the formula (I), a compound wherein the partial structure represented by the formula (I-1) has the structure represented by the formula (I-1-B) may also be prepared according to, for example, the following Scheme 13.

Scheme 13

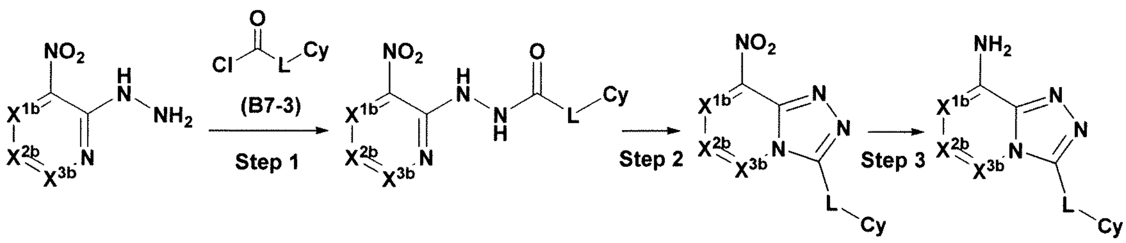


(B7-4) (B7-2) (B7-1) (B7)

[wherein the symbols have the same meanings as those described above.]
Examples of the embodiment include the following scheme.

One embodiment of Scheme 13

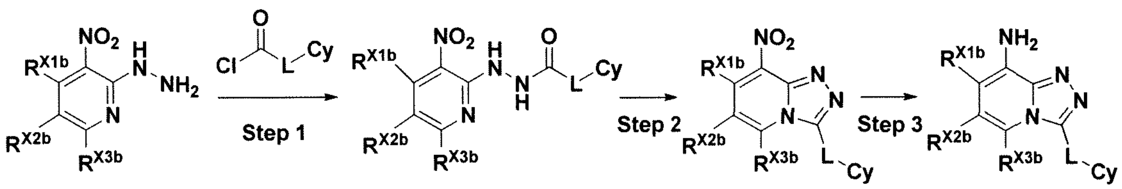


[wherein the symbols have the same meanings as those described above.]

Step 1

The Compound (B7-4) and the Compound (B7-3) may be reacted in a similar manner to the Step 1 of the Scheme 8 to prepare the Compound (B7-2).

Step 2

The Compound (B7-2) prepared in the Step 1 may be reacted in a similar manner to the Step 2 of the Scheme 8 to prepare the Compound (B7-1).

Step 3

The Compound (B7-1) prepared in the Step 2 may be treated with a catalyst in a solvent and under hydrogen atmosphere to prepare the Compound (B7).
The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.
Examples of the catalyst include palladium carbon.
The amount of the catalyst to be used may be 0.01 to 0.1 equivalent(s), preferably 0.03 to 0.05 equivalents, relative to the Compound (B7-1) in molar ratio.
The reaction may be carried out at 0°C to under heating, for example at 0 to 50°C, preferably at room temperature.

Production method 14

Among the compound represented by the formula (I), a compound wherein the partial structure represented by the formula (I-1) has the structure represented by the formula (I-1-B) may also be prepared according to, for example, the following Scheme 14.

Scheme 14

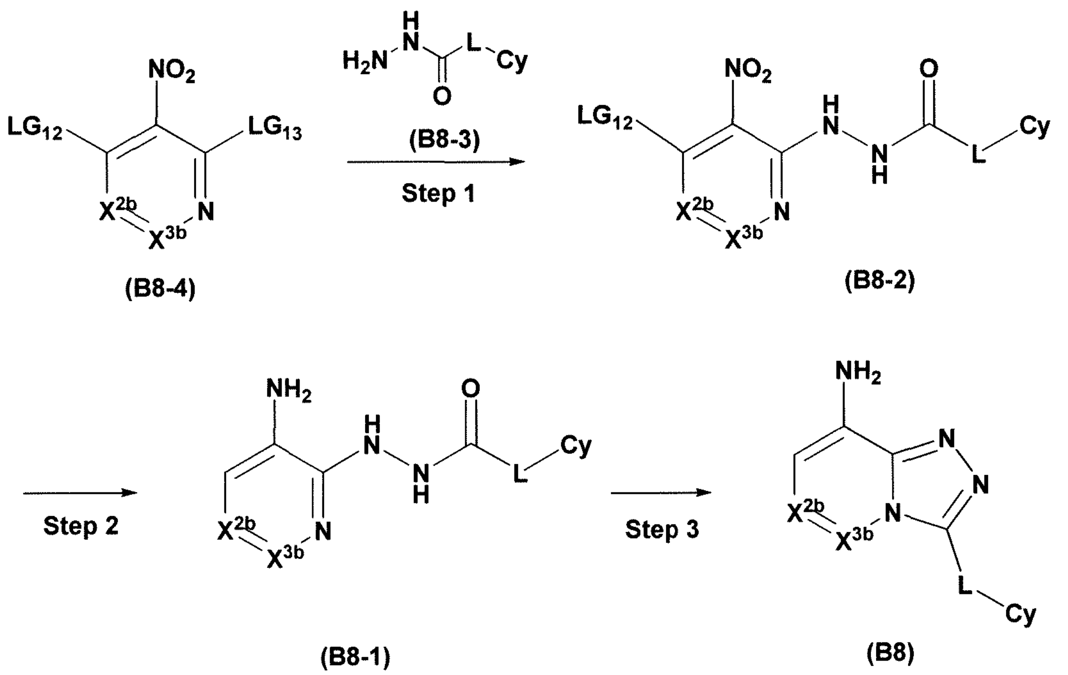


[wherein $LG_{12}$ and $LG_{13}$ each represent a leaving group such as a halogen atom; and the other symbols have the same meanings as those described above.]
Examples of the embodiment include the following scheme.

One embodiment of Scheme 14

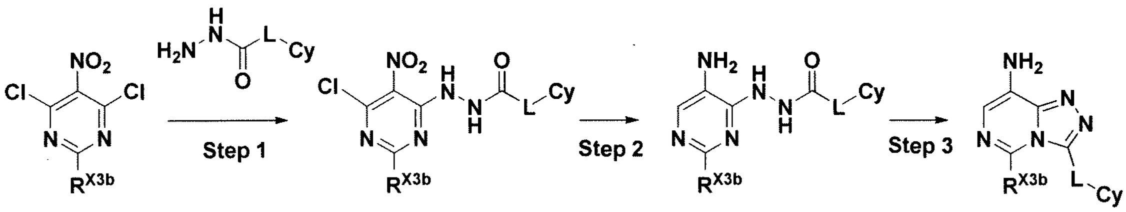


[wherein the symbols have the same meanings as those described above.]

Step 1

The Compound (B8-4) may be reacted with the Compound (B8-3) in a solvent and in the presence of a base to prepare the Compound (B8-2).

The solvent may be any which does not affect the reaction, and examples thereof include amides such as N,N-dimethylformamide and N-methylpyrrolidone; ethers such as tetrahydrofuran and 1,4-dioxane; halogenated aliphatic hydrocarbons such as chloroform and dichloromethane; aromatic hydrocarbons such as toluene; nitriles such as acetonitrile; and mixtures thereof.

Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as triethylamine and diisopropylethylamine; and pyridines such as pyridine and dimethylaminopyridine.

The amount of the Compound (B8-3) to be used may be 1.0 to 5.0 equivalent(s), preferably 2.0 to 3.0 equivalents, relative to the Compound (B8-4) in molar ratio.

The amount of the base to be used may be 1.0 to 5.0 equivalent(s), preferably 2.0 to 3.0 equivalents, relative to the Compound (B8-4) in molar ratio.

The reaction may be carried out at room temperature to under heating, for example at room temperature to 50°C, preferably at room temperature.

Step 2

The Compound (B8-2) prepared in the Step 1 may be treated with a catalyst in a solvent and under hydrogen atmosphere to prepare the Compound (B8-1).

The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.

Examples of the catalyst include palladium carbon.

The amount of the catalyst to be used may be 0.01 to 0.1 equivalent(s), preferably 0.03 to 0.05 equivalents, relative to the Compound (B8-2) in molar ratio.

The reaction may be carried out at 0°C to under heating, for example at 0 to 50°C, preferably at room temperature.

Step 3

The Compound (B8-1) prepared in the Step 2 may be reacted in a similar manner to the Step 2 of the Scheme 8 to prepare the Compound (B8).

Production method 15

Among the compound represented by the formula (I), a compound wherein the partial structure represented by the formula (I-1) has the structure represented by the formula (I-1-C) may also be prepared according to, for example, the following Scheme 15.

Scheme 15

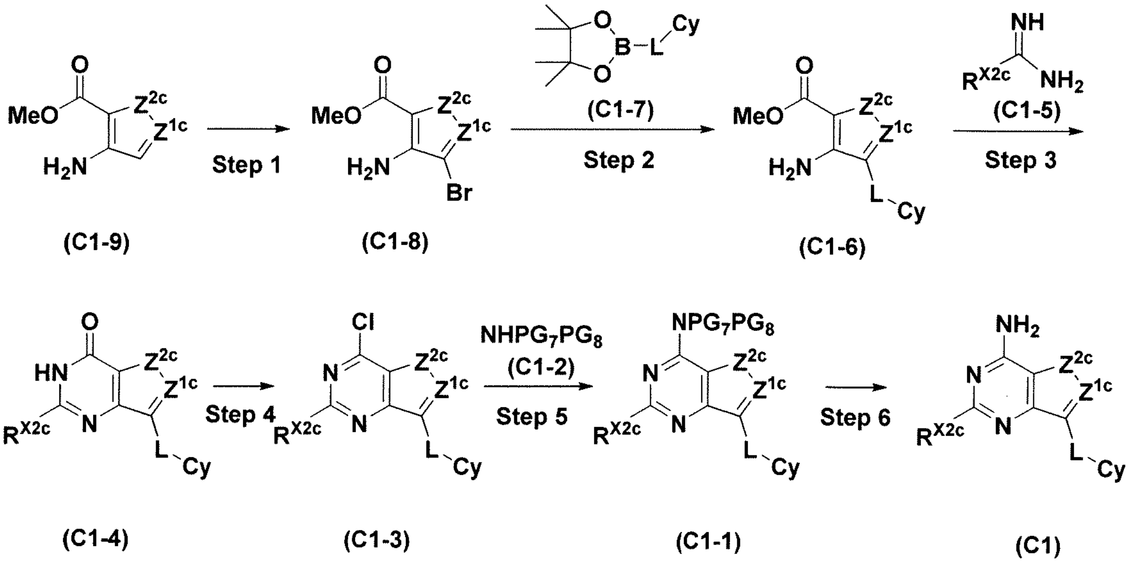


[wherein $PG_7$ and $PG_8$ each independently represent a protecting group of amino group; and the other symbols have the same meanings as those described above.]

Examples of the embodiment include the following scheme.

One embodiment of Scheme 15

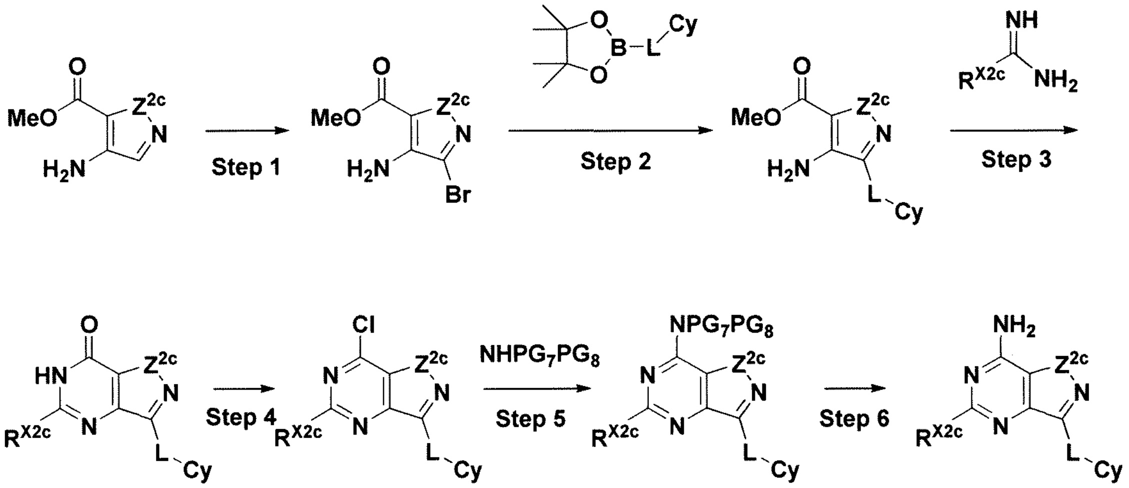


[wherein the symbols have the same meanings as those described above.]

Step 1

The Compound (C1-9) may be reacted with a brominating agent in a solvent to prepare the Compound (C1-8).
The solvent may be any which does not affect the reaction, and examples thereof include amides such as N,N-dimethylformamide, halogenated aliphatic hydrocarbons such as chloroform and dichloromethane, aromatic hydrocarbons such as toluene, nitriles such as acetonitrile, carboxylic acids such as acetic acid, water, and mixtures thereof.
Examples of the brominating agent include N-bromosuccinimide.
The amount of the brominating agent to be used may be 1.0 to 5.0 equivalent(s), preferably 1.0 to 2.0 equivalent(s), relative to the Compound (Cl-9) in molar ratio.

The reaction may be carried out under ice-cooling to under heating, for example under ice-cooling to at room temperature, preferably under ice-cooling.

Step 2

The Compound (C1-8) prepared in the Step 1 may be reacted with the Compound (C1-7) in a solvent, in the presence of a palladium catalyst, and in the presence of a base to prepare the Compound (C1-6).

The solvent may be any which does not affect the reaction, and examples thereof include ethers such as tetrahydrofuran and 1,4-dioxane; alcohols such as methanol, ethanol, and isopropanol; aromatic hydrocarbons such as toluene; nitriles such as acetonitrile; water; and mixtures thereof.

Examples of the palladium catalyst include palladium(II) acetate, [1,1'-bis(diphenylphosphino)ferrocene]palladium(II) dichloride ($PdCl_2$(dppf)), $PdCl_2$(dppf) dichloromethane adduct, tris(dibenzylideneacetone)dipalladium(0) ($Pd_2(dba)_3$), tetrakistriphenylphosphinepalladium, bis(triphenylphosphine)palladium dichloride, and bis(di-tert-butyl(4-dimethylaminophenyl)phosphine)dichloropalladium(II).

Examples of the base include alkali metal carbonates such as cesium carbonate, potassium carbonate, sodium carbonate, and sodium hydrogen carbonate; alkali metal phosphates such as potassium phosphate tribasic, sodium phosphate, and sodium hydrogen phosphate; amines such as triethylamine and N,N-diisopropylethylamine; alkali metal fluorides such as cesium fluoride and potassium fluoride; and alkali metal alkoxides such as sodium t-butoxide and potassium t-butoxide.

The amount of the Compound (C1-7) to be used may be 1.0 to 5.0 equivalent(s), preferably 2.0 to 3.0 equivalents, relative to the Compound (C1-8) in molar ratio.

The amount of the palladium catalyst to be used may be 0.01 to 2.0 equivalent(s), preferably 0.01 to 0.5 equivalent(s), relative to the Compound (C1-8) in molar ratio.

The amount of the base to be used may be 1.0 to 5.0 equivalent(s), preferably 2.0 to 3.0 equivalents, relative to the Compound (C1-8) in molar ratio.

The reaction may be carried out under heating, for example at 50°C to 200°C, preferably at 80 to 120°C.

Step 3

The Compound (C1-6) prepared in the Step 2 may be reacted with the Compound (C1-5) in a solvent and in the presence of a base to prepare the Compound (C1-4).

The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.

Examples of the base include alkali metal carbonates such as cesium carbonate, potassium carbonate, sodium carbonate, and sodium hydrogen carbonate; alkali metal phosphates such as potassium phosphate tribasic, sodium phosphate, and sodium hydrogen phosphate; amines such as triethylamine and N,N-diisopropylethylamine; alkali metal fluorides such as cesium fluoride and potassium fluoride; and alkali metal alkoxides such as sodium t-butoxide and potassium t-butoxide.

The amount of the Compound (C1-5) to be used may be 1.0 to 5.0 equivalent(s), preferably 2.0 to 3.0 equivalents, relative to the Compound (C1-6) in molar ratio.

The amount of the base to be used may be 1.0 to 5.0 equivalent(s), preferably 2.0 to 3.0 equivalents, relative to the Compound (C1-6) in molar ratio.

The reaction may be carried out under heating, for example at 50°C to 200°C, preferably at 80 to 120°C.

Step 4

The Compound (C1-4) prepared in the Step 3 may be reacted with a chlorinating agent in a solvent to prepare the Compound (C1-3).

The solvent may be any which does not affect the reaction, and examples thereof include amides such as N,N-dimethylformamide, halogenated aliphatic hydrocarbons such as chloroform and dichloromethane, aromatic hydrocarbons such as toluene, nitriles such as acetonitrile, carboxylic acids such as acetic acid, water, and mixtures thereof.

Examples of the chlorinating agent include oxalyl chloride.

The amount of the chlorinating agent to be used may be 30 to 60 equivalents, preferably 40 to 50 equivalents, relative to the Compound (C1-4) in molar ratio.

The reaction may be carried out under heating, for example at 50°C to 100°C, preferably at 60 to 90°C.

Step 5

The Compound (C1-3) prepared in the Step 4 and the Compound (Cl-2) may be reacted in a similar manner to the Step 1 of the Scheme 6 to prepare the Compound (C1-1).

Step 6

The Compound (C1-1) prepared in the Step 5 may be reacted in a solvent, in the presence of an acid, and in the presence or absence of a reducing agent to prepare the Compound (C1).

The solvent may be any which does not affect the reaction, and examples thereof include amides such as N,N-dimethylformamide, halogenated aliphatic hydrocarbons such as chloroform and dichloromethane, aromatic hydrocarbons such as toluene, nitriles such as acetonitrile, carboxylic acids such as acetic acid, water, and mixtures thereof.

Examples of the acid include hydrochloric acid and trifluoroacetic acid.

Examples of the reducing agent include trialkylsilanes such as triethylsilane.

The amount of the acid to be used may be 30 to 100 equivalents, preferably 50 to 70 equivalents, relative to the Compound (C1-1) in molar ratio.

The amount of the reducing agent to be used may be 3.0 to 20 equivalents, preferably 5.0 to 10 equivalents, relative to the Compound (C1-1) in molar ratio.

The reaction may be carried out under heating, for example at 50°C to 100°C, preferably at 50°C to 70°C.

Production method 16

Among the compound represented by the formula (I), a compound wherein the partial structure represented by the formula (I-1) has the structure represented by the formula (I-1-C) may also be prepared according to, for example, the following Scheme 16.

Scheme 16

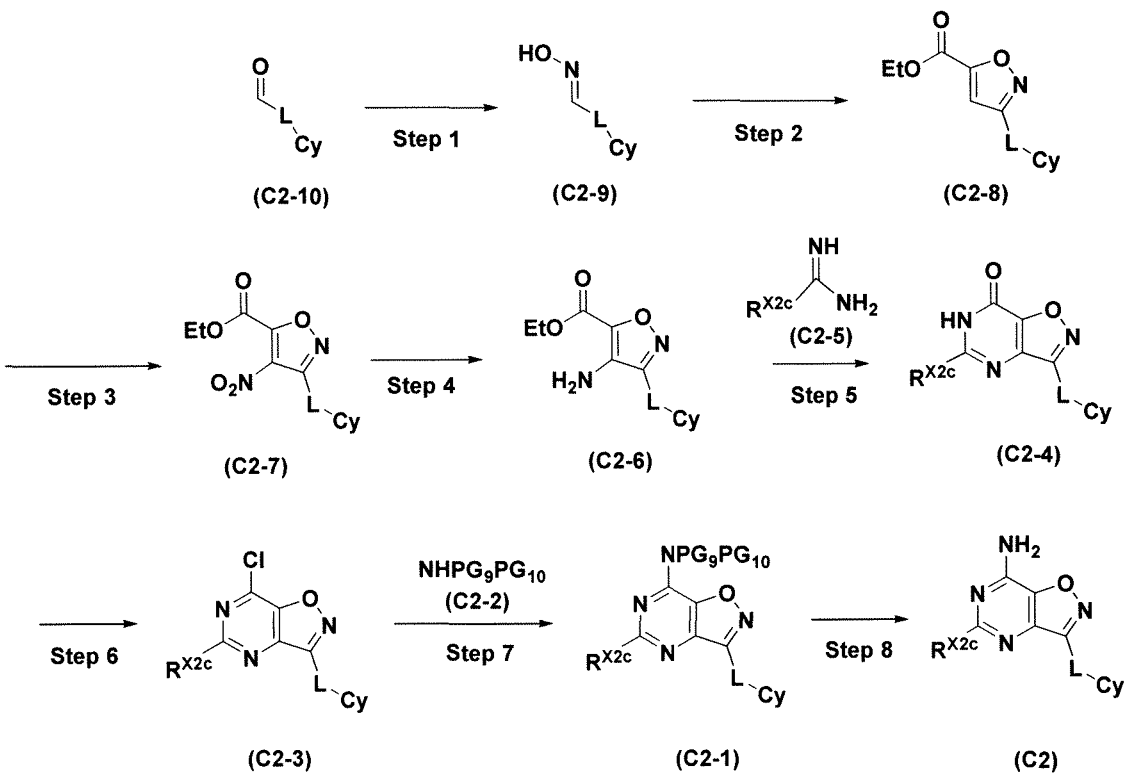


(C2-10) (C2-9) (C2-8) (C2-7) (C2-6) (C2-4) (C2-3) (C2-1) (C2)

[wherein $PG_9$ represents a protecting group of amino group; $PG_{10}$ represents a protecting group of amino group or a hydrogen atom; and the other symbols have the same meanings as those described above.]

Examples of the embodiment include the following scheme.

One embodiment of Scheme 16

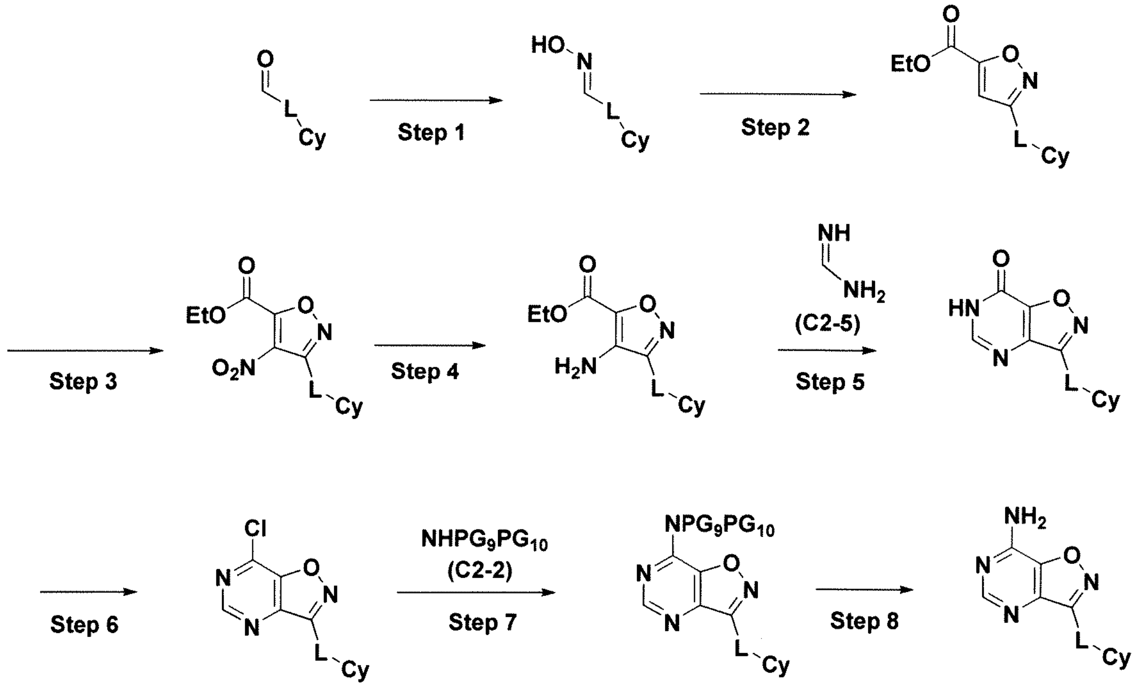


[wherein the symbols have the same meanings as those described above.]

Step 1

The Compound (C2-10) may be reacted with hydroxylamine hydrochloride to prepare the Compound (C2-9).
The amount of the hydroxylamine hydrochloride to be used may be 1.0 to 5.0 equivalent(s), preferably 1.0 to 3.0 equivalent(s), relative to the Compound (C2-10) in molar ratio.
The reaction may be carried out at room temperature to under heating, for example at room temperature to 180°C, preferably at room temperature to 150°C.

Step 2

The Compound (C2-9) prepared in the Step 1 may be reacted with a propiolic acid ester in the presence of an oxidizing agent to prepare the Compound (C2-8).
Examples of the oxidizing agent include sodium hypochlorite.
Examples of the propiolic acid ester include ethyl propiolate.
The amount of the oxidizing agent to be used may be 1.0 to 5.0 equivalent(s), preferably 1.0 to 3.0 equivalent(s), relative to the Compound (C2-9) in molar ratio.
The amount of the propiolic acid ester to be used may be 1.0 to 5.0 equivalent(s), preferably 1.0 to 3.0 equivalent(s), relative to the Compound (C2-9) in molar ratio.
The reaction may be carried out under ice-cooling to under heating, for example under ice-cooling to 100°C, preferably under ice-cooling to at room temperature.

Step 3

The Compound (C2-8) prepared in the Step 2 may be reacted with a nitrating agent in the presence of an activating agent to prepare the Compound (C2-7).
Examples of the nitrating agent include tetramethylammonium nitrate and potassium nitrate.
Examples of the activating agent include trifluoromethanesulfonic anhydride.
The amount of the nitrating agent to be used may be 1.0 to 5.0 equivalent(s), preferably 1.0 to 3.0 equivalent(s), relative to the Compound (C2-8) in molar ratio.

The amount of the activating agent to be used may be 1.0 to 5.0 equivalent(s), preferably 1.0 to 3.0 equivalent(s), relative to the Compound (C2-8) in molar ratio.
The reaction may be carried out at room temperature to under heating, for example at room temperature to 100°C, preferably at room temperature to 70°C.

Step 4

The Compound (C2-7) prepared in the Step 3 may be reacted in a solvent and in the presence of a reducing agent to prepare the Compound (C2-6).
The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.
Examples of the reducing agent include tin(II) chloride and zinc powder.
The amount of the reducing agent to be used may be 2.0 to 10.0 equivalents, preferably 3.0 to 6.0 equivalents, relative to the Compound (C2-7) in molar ratio.
The reaction may be carried out at room temperature to under heating, for example at room temperature to 200°C, preferably at room temperature to 150°C.

Step 5

The Compound (C2-6) prepared in the Step 4 and the Compound (C2-5) may be reacted in a similar manner to the Step 3 of the Scheme 15 to prepare the Compound (C2-4).

Step 6

The Compound (C2-4) prepared in the Step 5 may be reacted in a similar manner to the Step 4 of the Scheme 15 to prepare the Compound (C2-3).

Step 7

The Compound (C2-3) prepared in the Step 6 and the Compound (C2-2) may be reacted in a similar manner to the Step 1 of the Scheme 6 to prepare the Compound (C2-1).

Step 8

The Compound (C2-1) prepared in the Step 7 may be reacted in a similar manner to the Step 4 of the Scheme 11 to prepare the Compound (C2).

Production method 17

Among the compound represented by the formula (I), a compound wherein the partial structure represented by the formula (I-1) has the structure represented by the formula (I-1-D) may be prepared according to, for example, the following Scheme 17.

Scheme 17

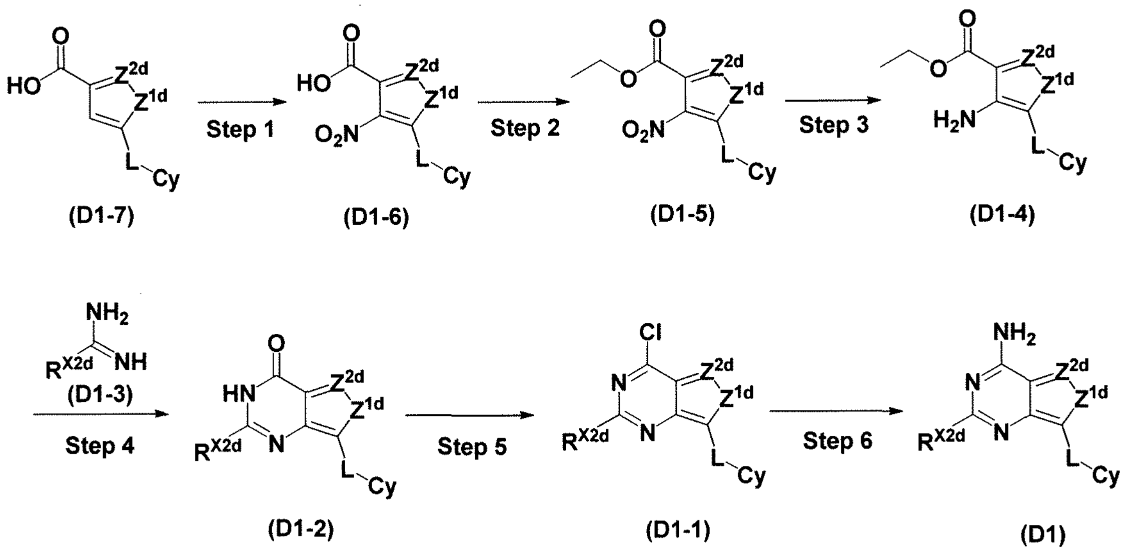


(D1-7) (D1-6) (D1-5) (D1-4) (D1-2) (D1-1) (D1)

Examples of the embodiment include the following scheme.

One embodiment of Scheme 17

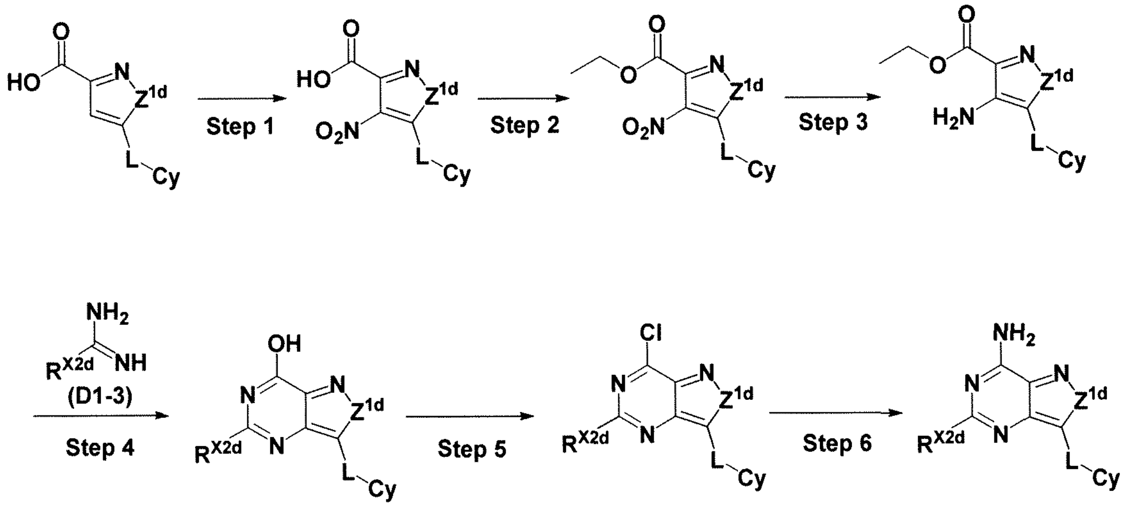


Step 1

The Compound (D1-7) may be reacted with a nitrating agent in the presence of an acid to prepare the Compound (D1-6).

Examples of the acid include sulfuric acid.

Examples of the nitrating agent include potassium nitrate.

While the amount of the acid to be used may be 1.0 to 10.0 equivalent(s) relative to the Compound (D1-7) in molar ratio, a large excess of the acid may be used as a solvent.

The amount of the nitrating agent to be used may be 1.0 to 3.0 equivalent(s), preferably 1.2 to 2.0 equivalents, relative to the Compound (D1-7) in molar ratio.

The reaction may be carried out at room temperature to under heating, for example at room temperature to 100°C, preferably at room temperature to 70°C.

Step 2

The Compound (D1-6) prepared in the Step 1 may be reacted with ethanol in the presence of an acid to prepare the Compound (D1-5).
Examples of the acid include sulfuric acid.
The amount of the acid to be used may be 0.01 to 10.0 equivalent(s), preferably 0.5 to 5.0 equivalent(s), relative to the Compound (D1-6) in molar ratio.
While the amount of the ethanol to be used may be 1.0 to 10.0 equivalent(s) relative to the Compound (D1-6) in molar ratio, a large excess of the ethanol may be used as a solvent.
The reaction may be carried out under heating, for example at 50°C to 200°C, preferably at 60°C to 100°C.

Step 3

The Compound (D1-5) prepared in the Step 2 may be reacted with zinc powder in a solvent and in the presence of an acid to prepare the Compound (D1-4).
The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.
Examples of the acid include acetic acid.
The amount of the acid to be used may be 1.0 to 30.0 equivalent(s), preferably 2.0 to 15.0 equivalents, relative to the Compound (D1-5) in molar ratio.
The amount of the zinc powder to be used may be 1.0 to 30.0 equivalent(s), preferably 2.0 to 15.0 equivalents, relative to the Compound (D1-5) in molar ratio.
The reaction may be carried out at 0°C to under heating, for example at 0°C to 100°C, preferably at room temperature to 60°C.

Step 4

The Compound (D1-4) prepared in the Step 3 and the Compound (D1-3) may be reacted in a solvent and in the presence of a base to prepare the Compound (D1-2).
The solvent may be any which does not affect the reaction, and examples thereof include ethers such as tetrahydrofuran and 1,4-dioxane, alcohols such as methanol, ethanol, and isopropanol, aromatic hydrocarbons such as toluene, nitriles such as acetonitrile, and mixtures thereof.
Examples of the base include inorganic bases, for example, alkali metal hydrogen carbonates such as sodium hydrogen carbonate; alkali metal carbonates such as potassium carbonate; and alkali metal hydroxides such as sodium hydroxide; and organic bases, for example, alkylamines such as isopropylamine, triethylamine, and diisopropylethylamine; and pyridines such as pyridine and dimethylaminopyridine.
The amount of the Compound (D1-3) to be used may be 1.0 to 10.0 equivalent(s), preferably 2.0 to 5.0 equivalents, relative to the Compound (D1-4) in molar ratio.
The amount of the base to be used may be 1.0 to 10.0 equivalent(s), preferably 2.0 to 5.0 equivalents, relative to the Compound (D1-4) in molar ratio.
The reaction may be carried out under heating, for example at 50°C to 200°C, preferably at 80°C to 120°C.

Step 5

The Compound (D1-2) prepared in the Step 4 may be reacted with a chlorinating agent to prepare the Compound (D1-1).
Examples of the chlorinating agent include phosphoryl chloride.
While the amount of the chlorinating agent to be used may be 1.0 to 10.0 equivalent(s) relative to the Compound (D1-2) in molar ratio, a large excess of the chlorinating agent may be used as a solvent.
The reaction may be carried out under heating, for example at 50°C to 200°C, preferably at 80°C to 120°C.

Step 6

The Compound (D1-1) prepared in the Step 5 may be reacted with ammonia in a solvent to prepare the Compound (D1).
The solvent may be any which does not affect the reaction, and examples thereof include ethers such as tetrahydrofuran and 1,4-dioxane, alcohols such as methanol, ethanol, and isopropanol, aromatic hydrocarbons such as

toluene, nitriles such as acetonitrile, water, and mixtures thereof.

While the amount of the ammonia to be used may be 1.0 to 10.0 equivalent(s) relative to the Compound (D1-1) in molar ratio, a large excess of the ammonia may be used in order to neutralize the chlorinating agent used in the Step 5 or as a solvent.

The reaction may be carried out at 0°C to under heating, for example at 0°C to 50°C, preferably at room temperature.

The compounds of the present invention and the intermediate compounds thereof may be prepared by the above production methods, and may also be prepared according to the methods described below in Examples and Reference Examples. Further, the compounds of the present invention and the intermediate compounds thereof may be converted into other target compounds or intermediate compounds according to the above production methods, methods described below in Examples and Reference Examples, and/or known methods, or combinations thereof. Examples of such methods include the methods described in the following (1) to (44):

(1) Conversion of a formyl group into an alkenyl group

A formyl group may be reacted with, for example, a Horner-Emmons reagent or a Wittig reagent to be converted into a corresponding alkenyl group. For example, a corresponding starting compound having a formyl group may be reacted with a Wittig reagent (for example, methyltriphenylphosphonium bromide) in a solvent (for example, toluene or tetrahydrofuran) and in the presence of a base (for example, potassium tert-butoxide) to prepare a compound having a corresponding alkenyl group. The reaction may be carried out at room temperature to under heating, preferably at room temperature.

(2) Conversion of a hydroxymethyl group into a formyl group

A hydroxymethyl group may be reacted with an oxidizing agent to be converted into a corresponding formyl group. For example, a corresponding starting compound having a hydroxymethyl group may be reacted with an oxidizing agent (for example, 2,2,6,6-tetramethylpiperidin-1-oxyl free radical) in a solvent (for example, acetonitrile), in the presence of a metal complex (for example, tetrakis(acetonitrile)copper(I) hexafluorophosphate), in the presence of a chelating agent (for example, 2,2'-bipyridine), and in the presence of a base (for example, 1-methylimidazole) to prepare a compound having a corresponding formyl group. The reaction may be carried out at room temperature to under heating, preferably at room temperature.

(3) Conversion of a methoxycarbonyl group into a hydroxyalkyl group

A methoxycarbonyl group may be reacted with a reducing agent to be converted into a corresponding hydroxyalkyl group. For example, a corresponding starting compound having a methoxycarbonyl group may be reacted with a reducing agent (for example, diisobutylaluminium hydride) in a solvent (for example, dichloromethane) to prepare a compound having a corresponding hydroxyalkyl group. The reaction may be carried out under ice-cooling to at room temperature, preferably under ice-cooling.

(4) Conversion of a methanesulfonyloxy group into a halogen atom

A methanesulfonyloxy group may be reacted with a halogenating agent to be converted into a corresponding halogen atom. For example, a corresponding starting compound having a methanesulfonyloxy group may be reacted with a halogenating agent (for example, fluorinating agents such as cesium fluoride) in a solvent (for example, acetonitrile and water) and in the presence of a reagent such as 1-butyl-3-methylimidazolium tetrafluoroborate to prepare a compound having a corresponding halogen atom. The reaction may be carried out at room temperature to under heating, preferably at room temperature.

(5) Conversion of a hydroxy group into a methanesulfonyloxy group

A hydroxy group may be reacted with, for example methanesulfonyl chloride to be converted into a corresponding methanesulfonyloxy group. For example, a corresponding starting compound having a hydroxy group may be reacted with methanesulfonyl chloride in a solvent (for example, ethyl acetate) and in the presence of a base (for example, triethylamine) to prepare a compound having a corresponding methanesulfonyloxy group. The reaction may be carried out under ice-cooling to at room temperature, preferably at 0°C.

(6) Conversion of a formyl group into an alkyl dihalide group

A formyl group may be reacted with, for example, a halogenating agent to be converted into a corresponding alkyl dihalide group. For example, a corresponding starting compound having a formyl group may be reacted with a halogenating agent (for example, fluorinating agents such as bis(2-methoxyethyl)aminosulfur trifluoride) in a solvent (for example, dichloromethane and ethanol) to prepare a compound having a corresponding alkyl dihalide group. The reaction may be carried out under ice-cooling to at room temperature, preferably at room temperature.

(7) Elimination of a thiocarbonate group

A thiocarbonate group may be treated with a reducing agent to eliminate the thiocarbonate group. For example, a corresponding starting compound having a thiocarbonate group may be treated with a reducing agent (for example, tributyltin hydride) in a solvent (for example, toluene) and in the presence of a radical initiator (for example, 2,2'-azobis(isobutyronitrile)) to eliminate the thiocarbonate group. The reaction may be carried out at room temperature to under heating, preferably at room temperature to 100°C.

(8) Conversion of a hydroxy group into a thiocarbonate group

A hydroxy group may be reacted with a halogenated thionoester to be converted into a corresponding thiocarbonate group. For example, a corresponding starting compound having a hydroxy group may be reacted with a halogenated thionoester (for example, phenyl chlorothionoformate) in the presence of an activating agent (for example, 4-dimethylaminopyridine) to prepare a compound having a corresponding thiocarbonate group. The reaction may be carried out at room temperature to under heating, preferably at room temperature.

(9) Conversion of a formyl group into a 1-hydroxy-2,2,2-trifluoroethyl group

A formyl group may be reacted with a trifluoromethylating agent to be converted into a 1-hydroxy-2,2,2-trifluoroethyl group. For example, a corresponding starting compound having a formyl group may be reacted with a trifluoromethylating agent (for example, (trifluoromethyl)trimethylsilane) in a solvent (for example, tetrahydrofuran) and in the presence of a base (for example, cesium fluoride) to prepare a compound having a corresponding 1-hydroxy-2,2,2-trifluoroethyl group. The reaction may be carried out at room temperature to under heating, preferably at room temperature.

(10) Conversion of a hydroxy group into an alkoxy group

A hydroxy group may be reacted with an alkylating agent to be converted into a corresponding alkoxy group. For example, a corresponding starting compound having a hydroxy group may be reacted with an alkylating agent (for example, methylating agents such as methyl iodide) in a solvent (for example, dichloromethane) and in the presence of a base (for example, sodium hydride) to prepare a compound having a corresponding alkoxy group. The reaction may be carried out under ice-cooling to at room temperature, preferably at room temperature.

(11) Conversion of a hydroxy group into a phenoxy group

A hydroxy group may be reacted with phenol to be converted into a corresponding phenoxy group. For example, a corresponding starting compound having a hydroxy group may be reacted with phenol in a solvent (for example, tetrahydrofuran), and in the presence of a phosphine derivative (for example, triphenylphosphine) and an activating agent (for example, diisopropyl azodicarboxylate) to prepare a compound having a corresponding phenoxy group. The reaction may be carried out at room temperature to under heating, preferably at room temperature.

(12) Conversion of a hydroxymethyl group into a benzyloxymethyl group

A hydroxymethyl group may be reacted with a benzyl halide to be converted into a corresponding benzyloxymethyl group. For example, a corresponding starting compound having a hydroxymethyl group may be reacted with a benzyl halide (for example, benzyl bromide) in a solvent (for example, N,N-dimethylformamide), in the presence of a base (for example, sodium hydride), and in the presence of a reaction adjuvant (for example, sodium iodide) to prepare a compound having a corresponding benzyloxymethyl group. The reaction may be carried out at room temperature to under heating, preferably at room temperature to 50°C.

(13) Conversion of a hydroxy group into a benzyloxy group

A hydroxy group may be reacted with benzylalcohol to be converted into a corresponding benzyloxy group. For example, a corresponding starting compound having a hydroxy group may be reacted with benzylalcohol in a solvent (for example, tetrahydrofuran), in the presence of a phosphine derivative (for example, triphenylphosphine), and in the presence of an activating agent (for example, diisopropyl azodicarboxylate) to prepare a compound having a corresponding benzyloxy group. The reaction may be carried out at room temperature to under heating, preferably at room temperature to 50°C.

(14) Conversion of an N-(2-oxopropyl)carboxamide group into a 5-methyl-1,3-thiazolyl group

An N-(2-oxopropyl)carboxamide group may be reacted with a sulfurating agent to be converted into a corresponding 5-methyl-1,3-thiazole group. For example, a corresponding starting compound having an N-(2-oxopropyl)carboxamide group may be reacted with a sulfurating agent (for example, Lawesson's reagent (2,4-bis(4-methoxyphenyl)-1,3-dithia-2,4-diphosphetane 2,4-disulfide)) in a solvent (for example, tetrahydrofuran) to prepare a compound having a corresponding 5-methyl-1,3-thiazole group. The reaction may be carried out at room temperature to under heating, preferably at room temperature to 80°C.

(15) Conversion of a carboxyl group into a carboxamide group

A carboxyl group may be reacted with an amine to be converted into a corresponding carboxamide group. For example, a corresponding starting compound having a carboxyl group may be reacted with an amine (for example, aminoacetone hydrochloride and benzylamine) in a solvent (for example, chloroform), in the presence of an activating agent (for example, 1-hydroxybenzotriazole), and in the presence of a condensing agent (for example, 1-ethyl-3-(3-dimethylaminopropyl)carbodiimide hydrochloride), and treated with a base (for example, triethylamine) to be converted into a compound having a corresponding carboxamide group (N-(2-oxopropyl)carboxamide and benzamide). The reaction may be carried out at room temperature to under heating, preferably at room temperature.

(16) Conversion of a methoxycarbonyl group into a carboxyl group

A methoxycarbonyl group may be treated with a base or an acid to be converted into a corresponding carboxyl group. For example, a corresponding starting compound having a methoxycarbonyl group may be treated with a base (for example, sodium hydroxide and lithium hydroxide) and treated with an acid (for example, citric acid and hydrochloric acid) in a solvent (for example, tetrahydrofuran and methanol) to prepare a compound having a corresponding carboxyl group. The reaction may be carried out at room temperature to under heating, preferably at room temperature.

(17) Conversion of an ethoxycarbonyl group into a carboxyl group

An ethoxycarbonyl group may be treated with a base or an acid to be converted into a corresponding carboxyl group. For example, a corresponding starting compound having an ethoxycarbonyl group may be treated with a base (for example, sodium hydroxide) and treated with an acid (for example, citric acid and hydrochloric acid) in a solvent (for example, tetrahydrofuran and ethanol) to prepare a compound having a corresponding carboxyl group. The reaction may be carried out at room temperature to under heating, preferably at room temperature to 60°C.

(18) Conversion of an alkenyl group into an alkyl group

An alkenyl group may be reduced with hydrogen using a catalyst such as palladium carbon to be converted into a corresponding alkyl group. For example, a corresponding starting compound having an alkenyl group may be treated with a palladium carbon and treated with an acid (for example, citric acid and hydrochloric acid) in a solvent (for example, ethanol and tetrahydrofuran) and under hydrogen atmosphere to be converted into a carboxyl group. The reaction may be carried out at room temperature to under heating, preferably at room temperature.

(19) Conversion of an alkylidene group into an alkyl group

An alkylidene group may be reduced with hydrogen using a catalyst such as a palladium carbon to be converted into a corresponding alkyl group. For example, a corresponding starting compound having an alkylidene group may be treated with a palladium carbon (for example, BNA-Type (trade name) manufactured by NE CHEMCAT Corporation) in a solvent (for example, ethanol and acetic acid) and under hydrogen atmosphere to prepare a compound having a

corresponding alkyl group. The reaction may be carried out under heating, preferably at 50°C to 100°C.

(20) Deprotection of an amino group

A protecting group of amino group may be treated with an acid to be deprotected. For example, a protecting group of amino group (for example, tert-butoxycarbonyl group and benzyloxycarbonyl group) may be treated with a solution of hydrogen chloride (for example, a solution in ethyl acetate or dioxane) and concentrated hydrochloric acid if appropriate in a solvent (for example, ethyl acetate) if appropriate to deprotect the amino group. The reaction may be carried out at room temperature to under heating, preferably at room temperature.

Alternatively, an amino group may also be deprotected by using a catalytic reduction reaction. For example, a protecting group of amino group (for example, benzyloxycarbonyl group) may be treated with a transition metal (for example, palladium, rhodium, and platinum) catalyst and a hydrogen source by a conventional method to deprotect the amino group.

(21) N-methylation of a nonaromatic nitrogen-containing ring (for example, piperidine ring)

A nitrogen-containing ring may be reacted with a methylating agent to be N-methylated. For example, a nitrogen-containing ring may be reacted with formaldehyde in a solvent (for example, dichloromethane), in the presence of a base (for example, N,N-diisopropylethylamine), and in the presence of a reducing agent (for example, sodium triacetoxyborohydride) to be N-methylated. The reaction may be carried out at room temperature to under heating, preferably at room temperature. Alternatively, a hydrochloride of a nitrogen-containing ring may be reacted with methyl iodide in a solvent (for example, acetonitrile) and in the presence of a base (for example, potassium carbonate) to be N-methylated. The reaction may be carried out at room temperature to under heating, preferably at 40 to 60°C.

(22) N-phenylation of a nonaromatic nitrogen-containing ring

A nonaromatic nitrogen-containing ring may be reacted with phenylboronic acid to be N-phenylated. For example, a nonaromatic nitrogen-containing ring (for example, piperidine ring) may be reacted with phenylboronic acid in a solvent (for example, dichloromethane), in the presence of a base (for example, N,N-diisopropylethylamine), and in the presence of a copper catalyst (for example, copper(II) acetate) to be N-methylated. The reaction may be carried out at room temperature to under heating, preferably at room temperature.

(23) N-benzylation of a nonaromatic nitrogen-containing ring

A nonaromatic nitrogen-containing ring may be reacted with benzaldehyde to be N-benzylated. For example, a nonaromatic nitrogen-containing ring (for example, piperidine ring) may be reacted with benzaldehyde in a solvent (for example, dichloromethane), in the presence of a base (for example, N,N-diisopropylethylamine), and in the presence of a reducing agent (for example, sodium triacetoxyborohydride) to be N-benzylated. The reaction may be carried out at room temperature to under heating, preferably at room temperature.

(24) Conversion of a dioxaspiro[4.5]decanyl group into an oxocyclohexyl group

A dioxaspiro[4.5]decanyl group may be treated with an acid to be converted into an oxocyclohexyl group. For example, a corresponding starting compound having a dioxaspiro[4.5]decanyl group may be treated with an acid (for example, hydrochloric acid) in a solvent (for example, tetrahydrofuran) to prepare a compound having a corresponding oxocyclohexyl group. The reaction may be carried out under heating, preferably at 40°C to 60°C.

(25) Conversion of a benzyloxycarbonyl group into a carboxyl group

A benzyloxycarbonyl group may be treated with hydrogen using a palladium catalyst to be converted into a carbonyl group. For example, a corresponding starting compound having a benzyloxycarbonyl group may be treated with a palladium catalyst (for example, a palladium carbon) in a solvent (for example, tetrahydrofuran and ethanol) and under hydrogen pressure to prepare a compound having a corresponding carboxyl group. The reaction may be carried out at room temperature to under heating, preferably at 40°C to 60°C.

(26) Conversion of an oxocyclohexyl group into a dioxaspiro[4.5]decanyl group

An oxocyclohexyl group may be treated with ethylene glycol and an acid to be converted into a dioxaspiro[4.5]decanyl group. For example, a corresponding starting compound havingan oxocyclohexyl group may be treated with an acid (for example, p-toluenesulfonic acid) in a solvent (for example, toluene) to prepare a compound having a corresponding dioxaspiro[4.5]decanyl group. The reaction may be carried out at room temperature to under heating, preferably at 40°C to 60°C.

(27) Conversion of a bicycloalkenyl group into a bicycloalkyl group

A bicycloalkenyl group may be treated with hydrogen using a catalyst such as a palladium carbon to be converted into a corresponding bicycloalkyl group. For example, a corresponding starting compound having a bicycloalkenyl group (for example, bicyclo[3.1.0]hexenyl group) may be treated with a palladium carbon in a solvent (for example, ethanol), under hydrogen atmosphere to be converted into a bicycloalkyl group (for example, bicyclo[3.1.0]hexyl group). The reaction may be carried out at room temperature to under heating, preferably at room temperature.

(28) Conversion of a cycloalkenyl group into a cycloalkyl group

A cycloalkenyl group may be treated with hydrogen using a catalyst such as a palladium carbon to be converted into a corresponding cycloalkyl group. For example, a corresponding starting compound having a cycloalkenyl group (for example, cyclohexenyl group) may be treated with a palladium carbon in a solvent (for example, ethanol) and under hydrogen atmosphere to be converted into a cycloalkyl group (for example, cyclohexyl group). The reaction may be carried out at room temperature to under heating, preferably at room temperature.

(29) Formation of an N-pyrimidyl on a nonaromatic nitrogen-containing ring

A nonaromatic nitrogen-containing ring may be reacted with a halogenated pyrimidine to form an N-pyrimidyl. For example, a nonaromatic nitrogen-containing ring (for example, piperidine ring) may be reacted in a solvent (for example, dimethyl sulfoxide), in the presence of a base (for example, potassium carbonate), and at room temperature, and then reacted with a halogenated pyridine (for example, 2-chloropyridine) in the presence of a base (for example, diisopropylethylamine) and under heating (for example, 140°C) to form an N-methyl.

(30) N-alkoxycarbonylation of a nonaromatic nitrogen-containing ring

A nonaromatic nitrogen-containing ring may be reacted with an alkyl formate halide to be N-alkoxycarbonylated. For example, a nonaromatic nitrogen-containing ring (for example, piperidine ring) may be reacted with an alkyl formate halide (for example, methyl chloroformate and ethyl chloroformate) in a solvent (for example, dichloromethane) and in the presence of a base (for example, triethylamine and dimethylaminopyridine) to be N-alkoxycarbonylated. The reaction may be carried out at room temperature to under heating, preferably at room temperature.

(31) Conversion of an oxocycloalkyl group into a cyanocycloalkyl group

An oxocycloalkyl group may be reacted with a cyanating agent (for example, p-toluenesulfonylmethyl isocyanide) to be converted into a corresponding cyanocycloalkyl group. For example, a corresponding starting compound having an oxocycloalkyl group may be reacted with a cyanating agent (for example, p-toluenesulfonylmethyl isocyanide) in a solvent (for example, 1,2-dimethoxyethane) and in the presence of a base (for example, potassium tert-butoxide) to prepare a compound having a corresponding cyanocycloalkyl group. The reaction may be carried out under ice-cooling to at room temperature, preferably at room temperature.

(32) Conversion of a cyano group into a carboxyl group

A cyano group may be treated with concentrated hydrochloric acid to be converted into a carboxyl group. The reaction may be carried out under heating, preferably at 80°C to 120°C.

(33) Conversion of a cyclopentenyl group into a difluorobicyclo[3.1.0]hexyl group

A cyclopentenyl group may be reacted with sodium chlorodifluoroacetate to be converted into a difluorobicyclo[3.1.0]hexyl group. For example, a corresponding starting compound having a cyclopentenyl group may be reacted with sodium chlorodifluoroacetate in a solvent (for example, diethylene glycol dimethyl ether) to prepare a compound having a corresponding difluorobicyclo[3.1.0]hexyl group. The reaction may be carried out under heating, preferably at 150°C to 200°C.

(34) Conversion of a cyclohexenyl group into a bicyclo[4.1.0]heptyl group

A cyclohexenyl group may be reacted with a dihalogenomethane to be converted into a bicyclo[4.1.0]heptyl group. For example, a corresponding starting compound having a cyclohexenyl group may be treated with a dihalogenomethane (for example, diiodomethane and chloroiodomethane) in a solvent (for example, dichloromethane) and in the presence of diethylzinc to prepare a compound having a corresponding bicyclo[4.1.0]heptyl group. The reaction may be carried out under ice-cooling to at room temperature, preferably at room temperature.

(35) Conversion of a carboxyl group into a benzyloxycarbonyl group

A carboxyl group may be reacted with benzylalcohol or a benzyl halide to be converted into a benzyloxycarbonyl group. For example, a corresponding starting compound having a carboxyl group may be reacted with benzylalcohol in a solvent (for example, chloroform), in the presence of an activating agent (for example, 4-dimethylaminopyridine), and in the presence of a condensing agent (for example, 1-(3-dimethylaminopropyl)-3-ethylcarbodiimide hydrochloride) to prepare a compound having a corresponding benzyloxycarbonyl group. Alternatively, a corresponding starting compound having a carboxyl group may be reacted with a benzyl halide (for example, benzyl bromide) in a solvent (for example, N,N-dimethylformamide) and in the presence of a base (for example, cesium carbonate) to prepare a compound having a corresponding benzyloxycarbonyl group. These reactions may be carried out at room temperature to under heating, preferably at room temperature to 60°C.

(36) Conversion of a hydroxycycloalkyl group into an oxocycloalkyl group

A hydroxycycloalkyl group may be reacted with an oxidizing agent to be converted into a corresponding oxocycloalkyl group. For example, a corresponding starting compound having a hydroxycycloalkyl group may be reacted with an oxidizing agent (for example, N-methylmorpholine N-oxide and tetrapropylammonium perruthenate) in a solvent (for example, dichloromethane) to prepare a compound having a corresponding oxocycloalkyl group. The reaction may be carried out under ice-cooling to at room temperature, preferably at room temperature.

(37) Conversion of an oxocycloalkyl group into a dihalogenated cycloalkyl group

An oxocycloalkyl group may be reacted with a halogenating agent to be converted into a corresponding dihalogenated cycloalkyl group. For example, a corresponding starting compound having an oxocycloalkyl group may be reacted with a halogenating agent (for example, fluorinating agents such as bis(2-methoxyethyl)aminosulfur trifluoride) in a solvent (for example, dichloromethane and ethanol) to prepare a compound having a corresponding dihalogenated cycloalkyl group. The reaction may be carried out under ice-cooling to at room temperature, preferably at room temperature.

(38) Conversion of a phenyl group into a cyclohexyl group

A phenyl group may be reduced with hydrogen to be converted into a corresponding cyclohexyl group. For example, to a corresponding starting compound having a phenyl group may be applied hydrogen pressure in a solvent (for example, acetic acid) and in the presence of a catalyst (for example, platinum(IV) oxide) to prepare a compound having a corresponding cyclohexyl group. The reaction may be carried out under heating, preferably at 40°C to 80°C.

(39) 1,4-Addition to an $\alpha$,$\beta$-unsaturated carbonyl group

An $\alpha$,$\beta$-unsaturated carbonyl group may be subjected to a 1,4-addition reaction to be alkylated. For example, a corresponding starting compound may be reacted with an alkylating agent (for example, methylating agents such as methyllithium) in a solvent (for example, tetrahydrofuran) and in the presence of a catalyst (for example, copper(I) iodide) to be alkylated. The reaction may be carried out at -78°C to at room temperature, preferably at -78°C to 25°C.

(40) Conversion of a methylenecycloalkyl group into a spiroalkyl group

A methylenecycloalkyl group may be reacted in the presence of diethylzinc and a dihalogenomethane to be converted into a corresponding spiroalkyl group. For example, a corresponding starting compound having a methylenecycloalkyl group may be reacted in the presence of diethylzinc and a dihalogenomethane (for example, chloroiodomethane) in a solvent (for example, dichloromethane) to prepare a compound having a corresponding spiroalkyl group. The reaction may be carried out at 0°C to at room temperature, preferably at room temperature.

(41) Conversion of an alkoxycarbonyl group into a chlorocarbonyl group

An alkoxycarbonyl group may be reacted with a chlorinating agent to be converted into a corresponding chlorocarbonyl group. For example, a corresponding starting compound having an alkoxycarbonyl group may be reacted with a chlorinating agent (for example, phosphoryl chloride) in a solvent (for example, acetonitrile) to prepare a compound having a corresponding chlorocarbonyl group. The reaction may be carried out under heating, preferably at 80°C to 120°C.

(42) N-chlorocarbonylation of a nonaromatic nitrogen-containing ring

A nonaromatic nitrogen-containing ring may be reacted with triphosgene to be N-chlorocarbonylated. For example, a nonaromatic nitrogen-containing ring (for example, piperidine ring) may be reacted with triphosgene in a solvent (for example, dichloromethane) and in the presence of a base (for example, pyridine and diisopropylethylamine) to be N-chlorocarbonylated. The reaction may be carried out at 0°C to at room temperature, preferably at room temperature.

(43) Conversion of a hydroxy group into a halogen atom

A hydroxy group may be reacted with a halogenating agent to be converted into a halogen atom. For example, a corresponding starting compound having a hydroxy group may be reacted with a halogenating agent (for example, fluorinating agents such as (diethylamino)sulfur trifluoride) in a solvent (for example, dichloromethane) to prepare a compound having a corresponding halogen atom. The reaction may be carried out at 0°C to at room temperature, preferably at room temperature.

EXAMPLES

Hereinafter, the present invention is more specifically illustrated by way of Examples and Reference Examples, but is not limited to them.

(Examples)

Example 1

Preparation of 3-(trans-2-methylcyclohexyl)-3H-[1,2,3]triazolo[4,5-d]pyrimidin-7-amine

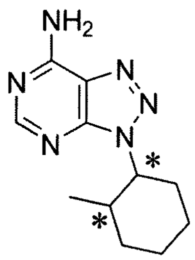

trans, racemate

A mixture of 7-chloro-3-(trans-2-methylcyclohexyl)-3H-[1,2,3]triazolo[4,5-d]pyrimidine (130 mg) prepared in the Reference Example 1-1 and a 7 mol/L ammonia-methanol solution (3 mL) was stirred at 50°C for 6 hours. To the reaction mixture was additionally added a 7 mol/L ammonia-methanol solution (3 mL), and the resulting mixture was stirred at 50°C for 2 hours. The reaction mixture was allowed to cool to room temperature, and water was added thereto. The resulting precipitates were collected by filtration, washed with water and a small amount of ethanol, and dried under reduced pressure to give the title compound (75 mg) (yield 62%) as a colorless powder.
MS(APCI) m/z: 233 $[M+H]^+$

Example 2

Preparation of 8-amino-3-(3,3-dimethylpiperidin-1-yl)-[1,2,4]triazolo[4,3-a]pyrazine-5-carbonitrile

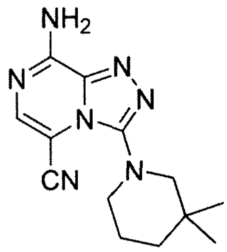

A mixed solution of 3-(3,3-dimethylpiperidin-1-yl)-8-methoxy-[1,2,4]triazolo[4,3-a]pyrazine-5-carbonitrile (240 mg) prepared in the Reference Example 2-1 and a 2 mol/L ammonia-methanol solution (10 mL) was stirred under microwave radiation at 100°C for 30 minutes. The reaction mixture was allowed to cool to room temperature, water was added thereto, and the resulting mixture was concentrated under reduced pressure. The resulting precipitates were collected by filtration, washed with water and ethyl acetate, and dried under reduced pressure to give the title compound (185 mg) (yield 81%) as a white solid. MS(CI) m/z: 272 $[M+H]^+$

Examples 3 to 16:

A corresponding starting compound was treated in a similar manner to the Example 1 to give each compound described in the following Table 1.

Table 1

| Example | Structural formula | Physical property etc. |
|---|---|---|
| 3 | 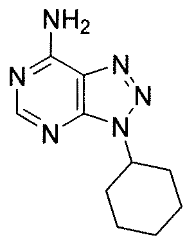 | MS(APCI) m/z; 219 $[M+H]^+$ |
| 4 | 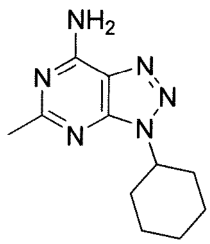 | MS(APCI) m/z; 233 $[M+H]^+$ |
| 5 | 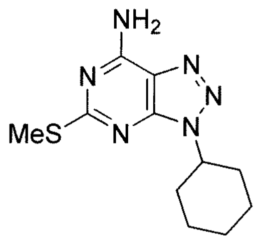 | MS(APCI) m/z; 265 $[M+H]^+$ |

(continued)

| Example | Structural formula | Physical property etc. |
|---|---|---|
| 6 | 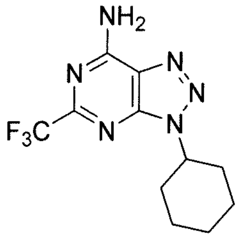 | MS(APCI) m/z; 287 [M+H]$^+$ |
| 7 | 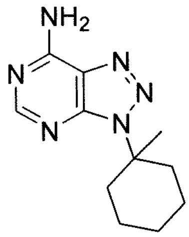 | MS(APCI) m/z; 233 [M+H]$^+$ |
| 8 | 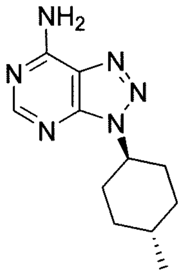 trans | MS(ESI) m/z; 233 [M+H]$^+$ |
| 9 | 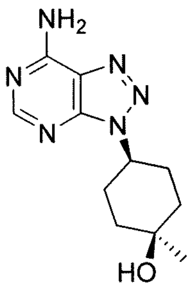 cis | MS(ESI) m/z; 249 [M+H]$^+$ |
| 10 | 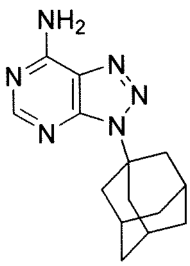 | MS(APCI) m/z; 271 [M+H]$^+$ |

(continued)

| Example | Structural formula | Physical property etc. |
|---|---|---|
| 11 | 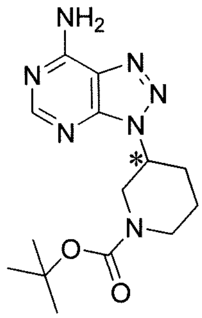 racemate | MS(APCI) m/z; 320 [M+H]+ |
| 12 | 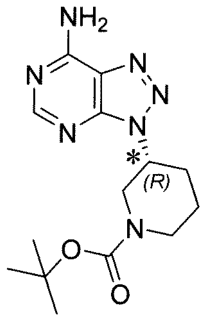 | MS(ESI) m/z; 320 [M+H]+ |
| 13 | 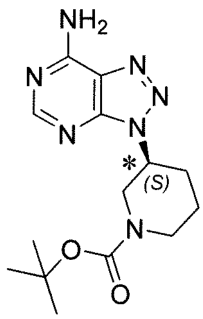 | MS(APCI) m/z; 320 [M+H]+ |
| 14 | 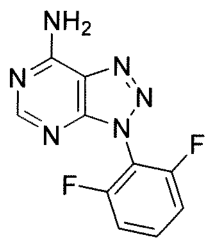 | MS(APCI) m/z; 249 [M+H]+ |
| 15 | 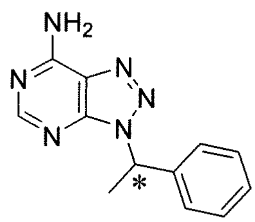 racemate | MS(ESI) m/z; 241 [M+H]+ |

(continued)

| Example | Structural formula | Physical property etc. |
|---|---|---|
| 16 | 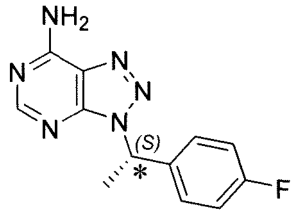 | MS(ESI) m/z; 259 [M+H]$^+$ |

Example 17

Preparation of 3-(1,2,3,4-tetrahydronaphthalen-1-yl)-[1,2,4]triazolo[4,3-a]pyrazin-8-amine

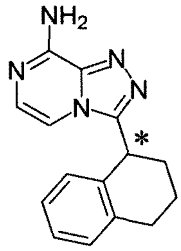

racemate

To a 2 to 5 mL flask for microwave were added 8-chloro-3-(1,2,3,4-tetrahydronaphthalen-1-yl)-[1,2,4]triazolo[4,3-a]pyrazine (180 mg) prepared in the Reference Example 17-1 and a 2 mol/L ammonia-isopropanol solution (2 mL), and the resulting mixture was stirred under microwave radiation at 100°C for 3 hours. The reaction mixture was allowed to cool to room temperature, and water was added thereto. The resulting precipitates were collected by filtration, washed sequentially with water and ethyl acetate, and dried under reduced pressure to give the title compound (132 mg) (yield 79%) as a white powder.
MS(CI) m/z: 266 [M+H]$^+$

Examples 18 to 35:

A corresponding starting compound was treated in a similar manner to the Example 17 to give each compound described in the following Table 2.

Table 2

| Example | Structural formula | Physical property etc. |
|---|---|---|
| 18 | 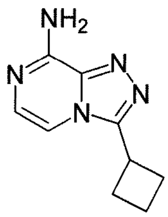 | MS(CI) m/z; 190 [M+H]$^+$ |

(continued)

| Example | Structural formula | Physical property etc. |
|---|---|---|
| 19 | 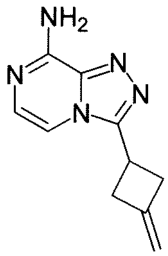 | MS(CI) m/z; 202 [M+H]+ |
| 20 | 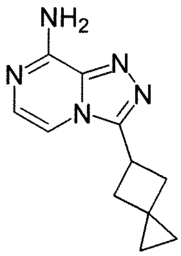 | MS(CI) m/z; 216 [M+H]+ |
| 21 | 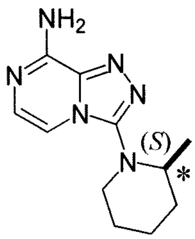 | MS(CI) m/z; 233 [M+H]+ |
| 22 | 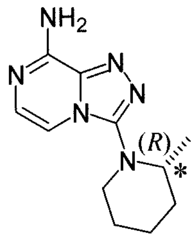 | MS(CI) m/z; 233 [M+H]+ |
| 23 | 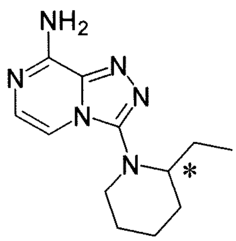 racemate | MS(CI) m/z; 247 [M+H]+ |
| 24 | 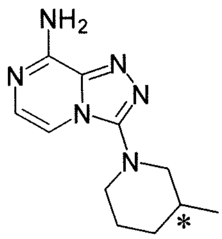 racemate | MS(CI) m/z; 233 [M+H]+ |

(continued)

| Example | Structural formula | Physical property etc. |
|---|---|---|
| 25 | 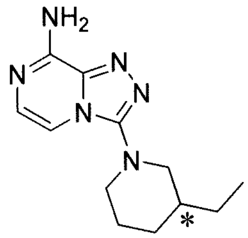 racemate | MS(CI) m/z; 247 $[M+H]^+$ |
| 26 | 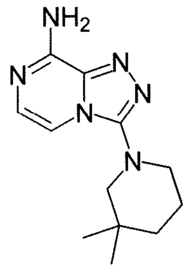 | MS(CI) m/z; 247 $[M+H]^+$ |
| 27 | 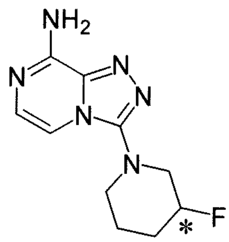 racemate | MS(CI) m/z; 237 $[M+H]^+$ |
| 28 | 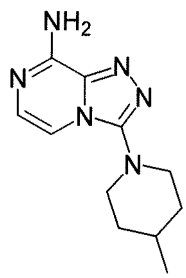 | MS(CI) m/z; 233 $[M+H]^+$ |
| 29 | 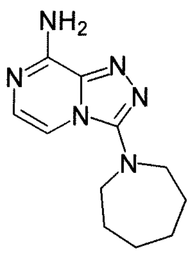 | MS(CI) m/z; 233 $[M+H]^+$ |

(continued)

| Example | Structural formula | Physical property etc. |
|---|---|---|
| 30 | 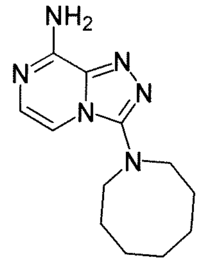 | MS(CI) m/z; 247 [M+H]$^+$ |
| 31 | 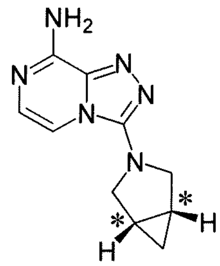 | MS(CI) m/z; 217 [M+H]$^+$ |
| 32 | 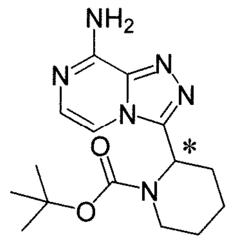 racemate | MS(CI) m/z; 319 [M+H]$^+$ |
| 33 | 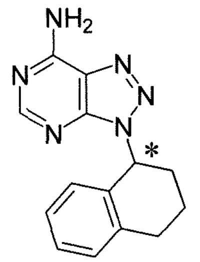 racemate | MS(CI) m/z; 267 [M+H]$^+$ |
| 34 | 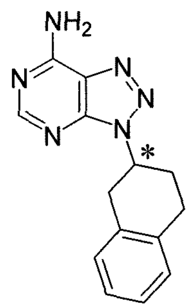 racemate | MS(CI) m/z; 267 [M+H]$^+$ |

(continued)

| Example | Structural formula | Physical property etc. |
|---|---|---|
| 35 | 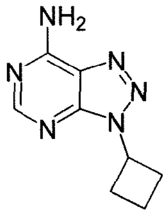 | MS(CI) m/z; 191 [M+H]$^+$ |

Example 36

Preparation of 3-(2,2-dimethylpiperidin-1-yl)-[1,2,4]triazolo[4,3-a]pyrazin-8-amine

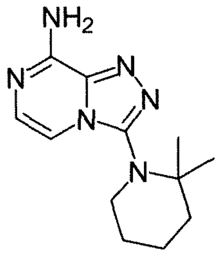

To a 0.5 to 2 mL flask for microwave were added 8-chloro-3-(2,2-dimethylpiperidin-1-yl)-[1,2,4]triazolo[4,3-a]pyrazine (19.6 mg) prepared in the Reference Example 36-1, isopropanol (1 mL), and a 28% aqueous ammonia (0.5 mL) (7.40 mmol), and the resulting mixture was stirred under microwave radiation at 100°C for 1 hour. The reaction mixture was allowed to cool to room temperature, water was added thereto, and the resulting mixture was concentrated under reduced pressure. The resulting precipitates were collected by filtration, and washed with water to give the title compound (13.2 mg) (yield 73%) as a pale brown powder. MS(CI) m/z: 247 [M+H]$^+$

Examples 37 to 76:

A corresponding starting compound was treated in a similar manner to the Example 36 to give each compound described in the following Table 3.

Table 3

| Example | Structural formula | Physical property etc. |
|---|---|---|
| 37 | 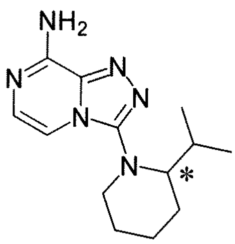 racemate | MS(CI) m/z; 261 [M+H]$^+$ |

(continued)

| Example | Structural formula | Physical property etc. |
|---|---|---|
| 38 | 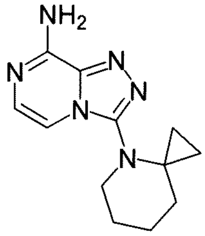 | MS(CI) m/z; 245 [M+H]+ |
| 39 | 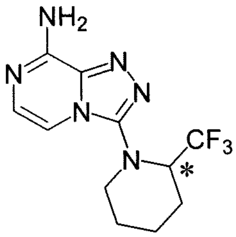 racemate | MS(CI) m/z; 287 [M+H]+ |
| 40 | 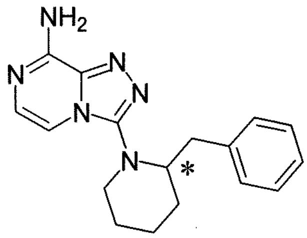 racemate | MS(CI) m/z; 309 [M+H]+ |
| 41 | 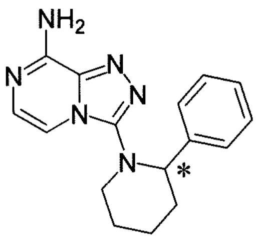 racemate | MS(CI) m/z; 295 [M+H]+ |
| 42 | 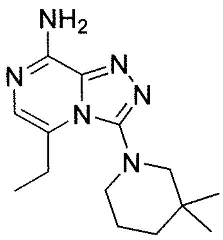 | MS(CI) m/z; 275 [M+H]+ |
| 43 | 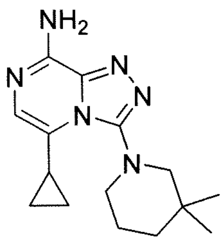 | MS(CI) m/z; 287 [M+H]+ |

(continued)

| Example | Structural formula | Physical property etc. |
|---|---|---|
| 44 | 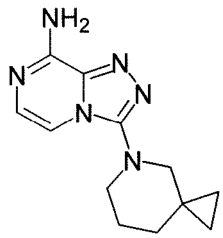 | MS(CI) m/z; 245 $[M+H]^+$ |
| 45 | 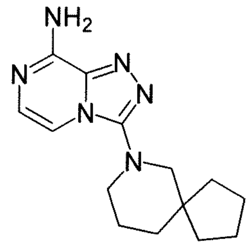 | MS(CI) m/z; 273 $[M+H]^+$ |
| 46 | 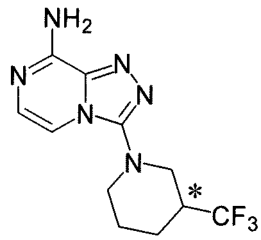 racemate | MS(CI) m/z; 287 $[M+H]^+$ |
| 47 | 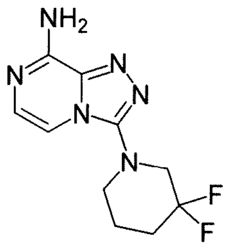 | MS(CI) m/z; 255 $[M+H]^+$ |
| 48 | 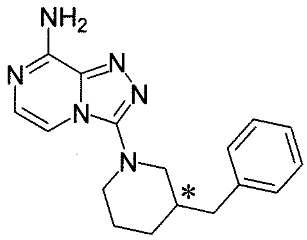 racemate | MS(CI) m/z; 309 $[M+H]^+$ |

(continued)

| Example | Structural formula | Physical property etc. |
|---|---|---|
| 49 | 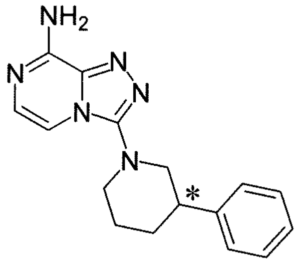 racemate | MS(CI) m/z; 295 [M+H]+ |
| 50 | 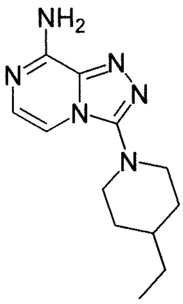 | MS(CI) m/z; 247 [M+H]+ |
| 51 | 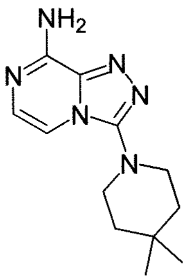 | MS(CI) m/z; 247 [M+H]+ |
| 52 | 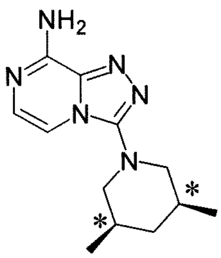 cis | MS(CI) m/z; 247 [M+H]+ |
| 53 | 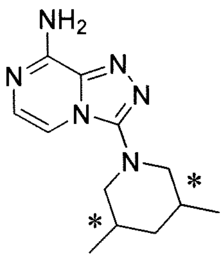 trans, racemate | MS(CI) m/z; 247 [M+H]+ |

(continued)

| Example | Structural formula | Physical property etc. |
|---|---|---|
| 54 | 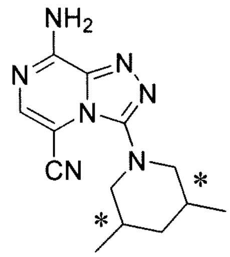<br>trans, racemate | MS(CI) m/z; 272 [M+H]+ |
| 55 | 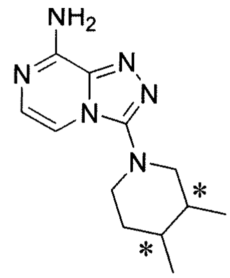<br>mixture of four types of stereoisomers | MS(CI) m/z; 247 [M+H]+ |
| 56 | 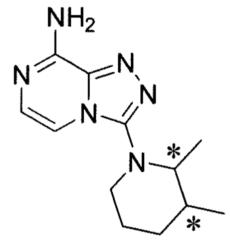<br>unknown relative configuration single diastereomer derived from Reference Example 56-1<br>racemate | MS(CI) m/z; 247 [M+H]+ |
| 57 | 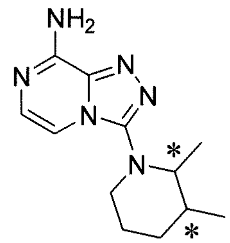<br>unknown relative configuration single diastereomer derived from Reference Example 57-1<br>racemate | MS(CI) m/z; 247 [M+H]+ |
| 58 | 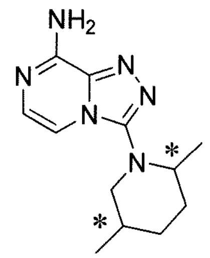<br>cis, racemate | MS(CI) m/z; 247 [M+H]+ |

(continued)

| Example | Structural formula | Physical property etc. |
|---|---|---|
| 59 | 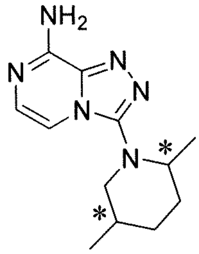<br>trans, racemate | MS(CI) m/z; 247 $[M+H]^+$ |
| 60 | 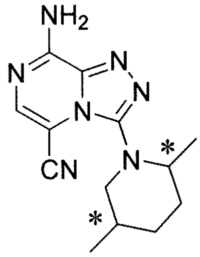<br>cis, racemate | MS(CI) m/z; 272 $[M+H]^+$ |
| 61 | 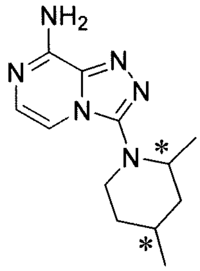<br>trans, racemate | MS(CI) m/z; 247 $[M+H]^+$ |
| 62 | 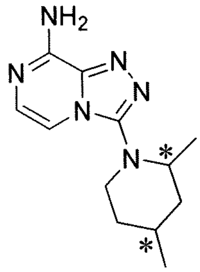<br>cis, racemate | MS(CI) m/z; 247 $[M+H]^+$ |
| 63 | 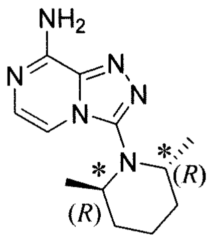 | MS(CI) m/z; 247 $[M+H]^+$ |

(continued)

| Example | Structural formula | Physical property etc. |
|---|---|---|
| 64 | 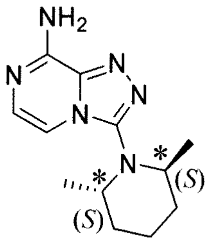 | MS(CI) m/z; 247 [M+H]+ |
| 65 | 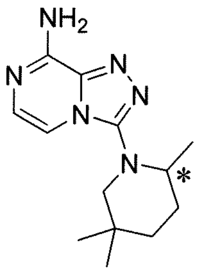 racemate | MS(CI) m/z; 261 [M+H]+ |
| 66 | 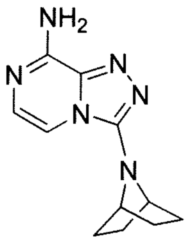 | MS(CI) m/z; 231 [M+H]+ |
| 67 | 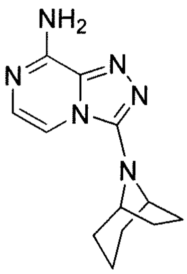 | MS(CI) m/z; 245 [M+H]+ |
| 68 | 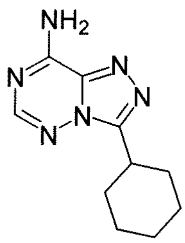 | MS(CI) m/z; 219 [M+H]+ |

(continued)

| Example | Structural formula | Physical property etc. |
|---|---|---|
| 69 | 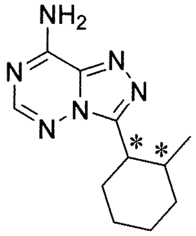 unknown relative configuration single diastereomer derived from Reference Example 69-1 racemate | MS(CI) m/z; 233 [M+H]+ |
| 70 | 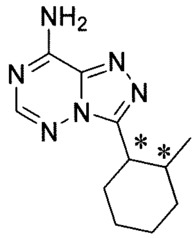 unknown relative configuration single diastereomer different from Example 69 racemate | MS(CI) m/z; 233 [M+H]+ |
| 71 | 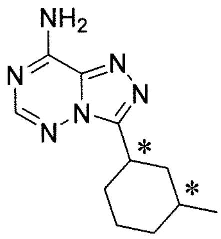 unknown relative configuration single diastereomer derived from Reference Example 71-1 racemate | MS(CI) m/z; 233 [M+H]+ |
| 72 | 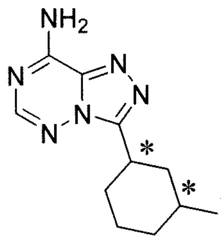 unknown relative configuration single diastereomer different from Example 71 racemate | MS(CI) m/z; 233 [M+H]+ |
| 73 | 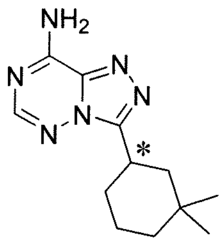 racemate | MS(CI) m/z; 247 [M+H]+ |

(continued)

| Example | Structural formula | Physical property etc. |
|---|---|---|
| 74 | 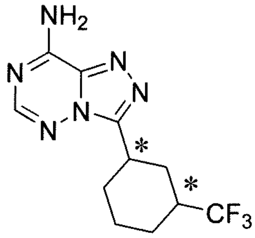 unknown relative configuration single diastereomer derived from Reference Example 74-1<br>racemate | MS(CI) m/z; 287 [M+H]+ |
| 75 | 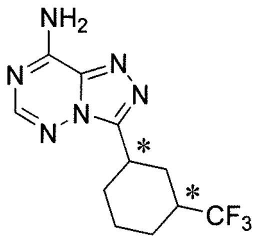 unknown relative configuration single diastereomer different from Example 74<br>racemate | MS(CI) m/z; 287 [M+H]+ |
| 76 | 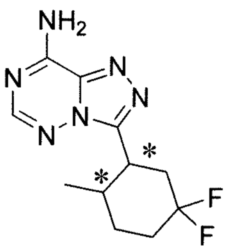 cis, racemate | MS(CI) m/z; 269 [M+H]+ |

Example 77

Preparation of 3-(1,2,3,4-tetrahydronaphthalen-2-yl)-[1,2,4]triazolo[4,3-a]pyrazin-8-amine

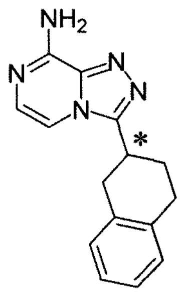

racemate

To a 2 to 5 mL reaction container were added 8-chloro-3-(1,2,3,4-tetrahydronaphthalen-2-yl)-[1,2,4]triazolo[4,3-a]pyrazine (250 mg) prepared in the Reference Example 77-1 and a 2 mol/L ammonia-isopropanol solution (4.4 mL), and the resulting mixture was stirred at 100°C for 5 hours. The reaction mixture was poured into water, filtered, and the filtered residues were washed with ethanol. The resulting pale yellow solid (180 mg) was subjected to preparative HPLC (eluent: 0.1 vol% trifluoroacetic acid/acetonitrile = 8/2) using Xbridge (C18.5 μm, OBD, 19 × 150 mm). The fractions comprising the target compound were collected, concentrated under reduced pressure, to the resulting residues was added a saturated aqueous solution of sodium hydrogen carbonate, the precipitated solid was collected by filtration, washed with water and ethanol, and dried under reduced pressure to give the title compound (116 mg) (yield 50%) as a white solid.
MS(CI) m/z: 266 $[M+H]^+$

Example 78:

A corresponding starting compound was treated in a similar manner to the Example 77 to give the compound described in the following Table 4.

Table 4

| Example | Structural formula | Physical property etc. |
|---|---|---|
| 78 | 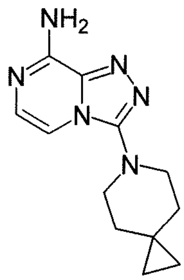 | MS(CI) m/z; 245 $[M+H]^+$ |

Example 79

Preparation of 3-(2-propylpiperidin-1-yl)-[1,2,4]triazolo[4,3-a]pyrazin-8-amine

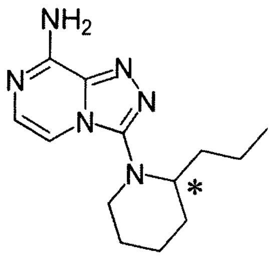

racemate

To a 10 to 20 mL flask for microwave were added 8-chloro-3-(2-propylpiperidin-1-yl)-[1,2,4]triazolo[4,3-a]pyrazine (0.61 g) prepared in the Reference Example 79-1, isopropyl alcohol (10 mL), and a 28% aqueous ammonia (5 mL), and the resulting mixture was stirred under heating under microwave radiation at 100°C for 1 hour. After the reaction was completed, the reaction mixture was allowed to cool to room temperature, then water was added to the reaction solution, the resulting mixture was concentrated under reduced pressure, then the resulting precipitates were filtered, and washed with water. The resulting solid was subjected to silica gel column chromatography (hexane : ethyl acetate = 10 : 90 to 0 : 100) using Moritex medium pressure preparative (Purif-Pack SI size 60 (30 g)), the fractions comprising the target compound were collected, and said fractions were concentrated under reduced pressure to give the title compound (0.38 g) (yield 67%) as a slightly red solid.
MS(CI) m/z: 261 $[M+H]^+$

Examples 80 to 83:

A corresponding starting compound was treated in a similar manner to the Example 79 to give each compound described in the following Table 5.

Table 5

| Example | Structural formula | Physical property etc. |
|---|---|---|
| 80 | 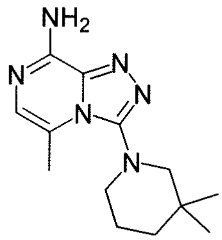 | MS(CI) m/z; 261 $[M+H]^+$ |
| 81 | 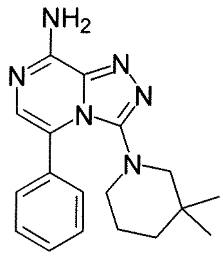 | MS(CI) m/z; 323 $[M+H]^+$ |
| 82 | 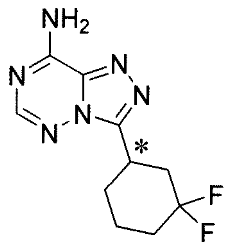 racemate | MS(CI) m/z; 255 $[M+H]^+$ |
| 83 | 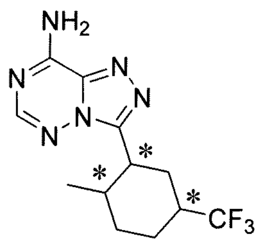 relative configuration (1R*, 2S*, 5R*), racemate | MS(CI) m/z; 301 $[M+H]^+$ |

Example 84

Preparation of 3-(3,3-difluorocyclobutyl)-[1,2,4]triazolo[4,3-a]pyrazin-8-amine

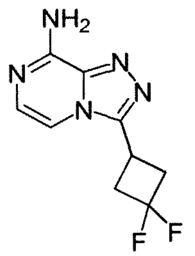

8-Chloro-3-(3,3-difluorocyclobutyl)-[1,2,4]triazolo[4,3-a]pyrazine prepared in the Reference Example 84-1 was reacted in a similar manner to the Example 17 to give a crude product. To the resulting crude product (165 mg) were added ethanol (2 mL) and water (1 mL) at room temperature, and the resulting mixture was stirred for 4 hours and 30 minutes. The mixture was filtered, then the resulting filtered residues were washed with ethanol, and dried under reduced pressure to give the title compound (0.15 g) (yield 91%) as a white solid.
MS(CI) m/z: 226 $[M+H]^+$

Example 85

Preparation of 3-cyclohexyl-3H-[1,2,3]triazolo[4,5-d]pyrimidin-7-amine hydrochloride

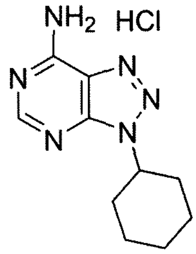

To 3-cyclohexyl-3H-[1,2,3]triazolo[4,5-d]pyrimidin-7-amine (165 mg) prepared in the Example 3 was added a 4 mol/L hydrogen chloride-ethyl acetate solution (5 mL), and the resulting mixture was stirred at room temperature for 1 hour. The resulting precipitates were collected by filtration, washed with ethyl acetate, and dried under reduced pressure to give the title compound (139 mg) (yield 72%) as a colorless powder.
MS(APCI) m/z: 219 $[M+H]^+$

Example 86

Preparation of 3-[trans-3-(trifluoromethyl)cyclohexyl]-3H-[1,2,3]triazolo[4,5-d]pyrimidin-7-amine

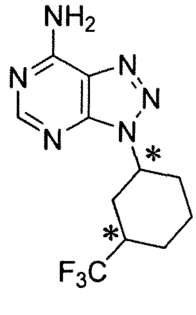

trans, racemate

(1) To a 200 mL eggplant flask were added 6-chloro-$N^4$-[trans-3-(trifluoromethyl)cyclohexyl]pyrimidine-4,5-diamine (116 mg) prepared in the Reference Example 86-2, dichloromethane (1.6 mL), and acetic acid (1.6 mL), and a solution of sodium nitrite (36 mg) in water (315 μL) was added dropwise thereto under ice-cooling. The reaction mixture was stirred under ice-cooling for 30 minutes, then ethyl acetate (28 mL) and iced water (28 mL) were added thereto, and the resulting mixture was separated. The resulting organic layer was washed with a saturated aqueous solution of sodium hydrogen carbonate and a saturated aqueous solution of sodium chloride, and dried over anhydrous sodium sulfate. The insoluble matters were removed by filtration, and the resulting filtrate was concentrated under reduced pressure to give a crude product of 7-chloro-3-[trans-3-(trifluoromethyl)cyclohexyl]-3H-[1,2,3]triazolo[4,5-d]pyrimidine.

(2) To the crude product of 7-chloro-3-[trans-3-(trifluoromethyl)cyclohexyl]-3H-[1,2,3]triazolo[4,5-d]pyrimidine prepared in the above (1) was added a 7 mol/L ammonia-methanol solution (2 mL), and the resulting mixture was stirred at room temperature overnight. To the reaction mixture was added water, the resulting precipitates were collected by filtration, and dried under reduced pressure to give the title compound (37.8 mg) (yield 34% (two steps)) as a colorless powder.

MS(ESI) m/z: 287 [M+H]+

Examples 87 to 91:

A corresponding starting compound was treated in a similar manner to the Example 86 to give each compound described in the following Table 6.

Table 6

| Example | Structural formula | Physical property etc. |
|---|---|---|
| 87 | 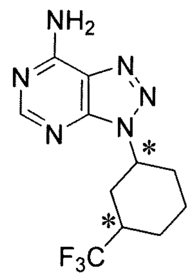 cis, racemate | MS(ESI) m/z; 287 [M+H]+ |
| 88 | 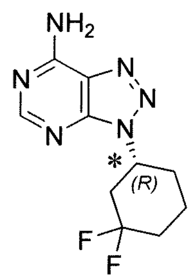 | MS (ESI) m/z; 255 [M+H]+ |
| 89 | 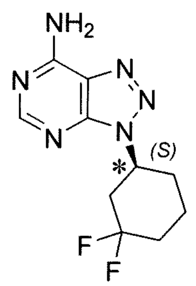 | MS (ESI) m/z; 255 [M+H]+ |

(continued)

| Example | Structural formula | Physical property etc. |
|---|---|---|
| 90 | 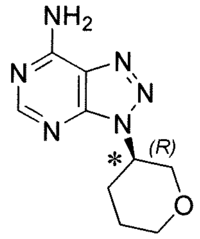 | MS(ESI) m/z; 221 $[M+H]^+$ |
| 91 | 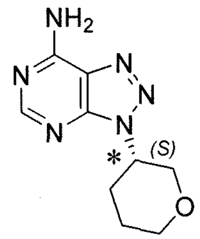 | MS(ESI) m/z; 221 $[M+H]^+$ |

Example 92

Preparation of 3-cyclohexyl-5-methoxy-3H-[1,2,3]triazolo[4,5-d]pyrimidin-7-amine

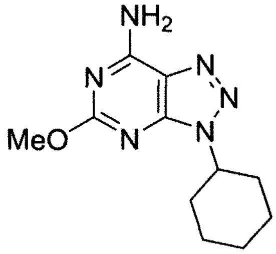

(1) To a solution of 3-cyclohexyl-5-(methylsulfanyl)-3H-[1,2,3]triazolo[4,5-d]pyrimidin-7-amine (199 mg) prepared in the Example 5 in dichloromethane (5 mL) was added m-chloroperbenzoic acid (wetted with ca. 30% water) (451 mg) under ice-cooling, and the resulting mixture was stirred under ice-cooling for 3 hours. To the reaction mixture was added a saturated aqueous solution of sodium hydrogen carbonate, and the resulting mixture was extracted twice with chloroform. The resulting organic layers were combined, washed with saturated brine, dried over anhydrous magnesium sulfate, and the insoluble matters were removed by filtration. The resulting filtrate was concentrated under reduced pressure to give a crude product of 3-cyclohexyl-5-(methylsulfonyl)-3H-[1,2,3]triazolo[4,5-d]pyrimidin-7-amine (270 mg) as a yellow powder.

(2) To a mixture of the crude product of 3-cyclohexyl-5-(methylsulfonyl)-3H-[1,2,3]triazolo[4,5-d]pyrimidin-7-amine (146 mg) prepared in the above (1) and methanol (3 mL) was added a 1 mol/L sodium methoxide / methanol solution (0.74 mL), and the resulting mixture was stirred at room temperature for 2 hours and 30 minutes. The mixture was diluted with methanol, and the resulting precipitates were collected by filtration. The precipitates were washed sequentially with chloroform and ethyl acetate, and dried under reduced pressure to give the title compound (46 mg) (yield 46% (two steps)) as a colorless powder.

MS(APCI) m/z: 249 $[M+H]^+$

Example 93

Preparation of 3-cyclohexyl-5-ethoxy-3H-[1,2,3]triazolo[4,5-d]pyrimidin-7-amine

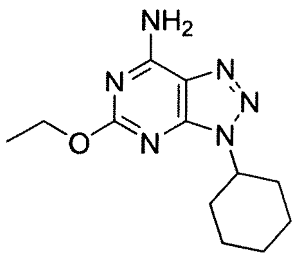

A mixture of 3-cyclohexyl-5-(methylsulfinyl)-3H-[1,2,3]triazolo[4,5-d]pyrimidin-7-amine (120 mg) prepared in the Reference Example 93-1, sodium ethoxide (220 mg), and ethanol (5 mL) was stirred at room temperature for 3 hours. To the reaction mixture was added water, and the resulting precipitates were collected by filtration. The precipitates were washed with water and ethanol, and dried under reduced pressure to give the title compound (93 mg) (yield 83%) as a pale yellow powder.
MS(APCI) m/z: 263 $[M+H]^+$

Example 94

Preparation of 3-[trans-3-methylcyclohexyl]-3H-[1,2,3]triazolo[4,5-d]pyrimidin-7-amine

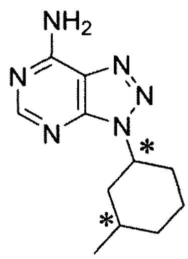

trans, racemate

A mixture of 3H-triazolo[4,5-d]pyrimidin-7-amine (140 mg), cis-3-methylcyclohexanol (352 mg), and 1,4-dioxane (50 mL) was subjected to nitrogen replacement, then to the mixture was added cyanomethylenetrimethylphosphorane (355 mg), and the resulting mixture was stirred at 110°C for 4 hours. The reaction mixture was allowed to cool to room temperature, and concentrated under reduced pressure. The resulting residues were purified by silica gel column chromatography (solvent: hexane/ethyl acetate = 40/60 to 0/100) to give the title compound (122 mg) (yield 51%) as a colorless powder.
MS(APCI) m/z: 233 $[M+H]^+$

Examples 95 to 110:

A corresponding starting compound was treated in a similar manner to the Example 94 to give each compound described in the following Table 7.

Table 7

| Example | Structural formula | Physical property etc. |
|---|---|---|
| 95 | 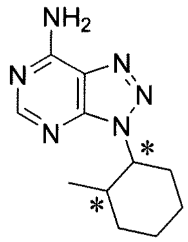 cis, racemate | MS(ESI) m/z; 233 $[M+H]^+$ |
| 96 | 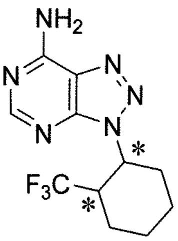 trans, racemate | MS(APCI) m/z; 287 $[M+H]^+$ |
| 97 | 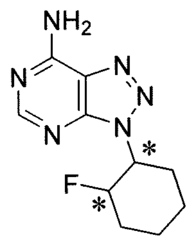 cis, racemate | MS (ESI) m/z; 237 $[M+H]^+$ |
| 98 | 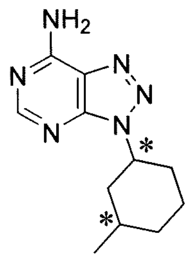 cis, racemate | MS(APCI) m/z; 233 $[M+H]^+$ |
| 99 | 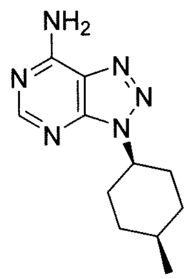 cis | MS(ESI) m/z; 233 $[M+H]^+$ |

(continued)

| Example | Structural formula | Physical property etc. |
|---|---|---|
| 100 | 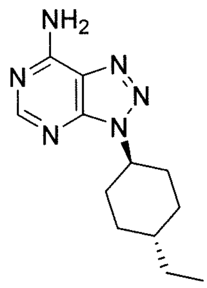 trans | MS(ESI) m/z; 247 $[M+H]^+$ |
| 101 | 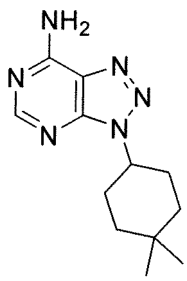 | MS(ESI) m/z; 247 $[M+H]^+$ |
| 102 | 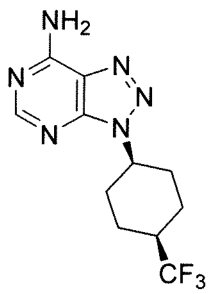 cis | MS(APCI) m/z; 287 $[M+H]^+$ |
| 103 | 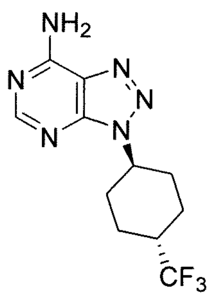 trans | MS(APCI) m/z; 287 $[M+H]^+$ |
| 104 | 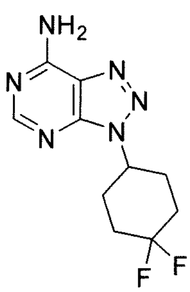 | MS(APCI) m/z; 255 $[M+H]^+$ |

(continued)

| Example | Structural formula | Physical property etc. |
| --- | --- | --- |
| 105 | 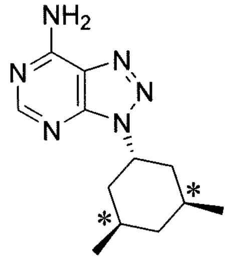 | MS(ESI) m/z; 247 [M+H]+ |
| 106 | 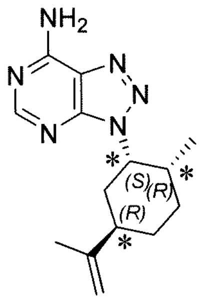 | MS(APCI) m/z; 273 [M+H]+ |
| 107 | 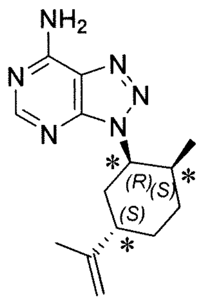 | MS(APCI) m/z; 273 [M+H]+ |
| 108 | 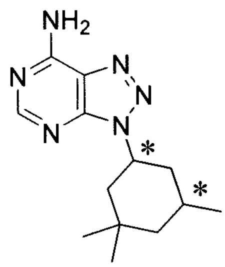 cis, racemate | MS(APCI) m/z; 261 [M+H]+ |
| 109 | 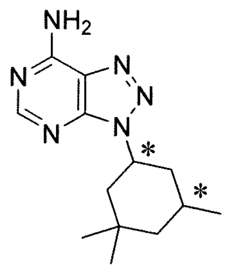 trans, racemate | MS(ESI) m/z; 261 [M+H]+ |

(continued)

| Example | Structural formula | Physical property etc. |
|---|---|---|
| 110 | 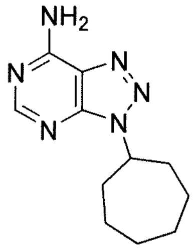 | MS(ESI) m/z; 233 $[M+H]^+$ |

Example 111

Preparation of 3-[cis-2-methoxycyclohexyl]-3H-[1,2,3]triazolo[4,5-d]pyrimidin-7-amine

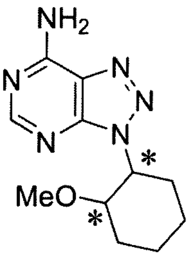

cis, racemate

To a mixture of N,N-bis(2,4-dimethoxybenzyl)-3-(cis-2-methoxycyclohexyl)-3H-[1,2,3]triazolo[4,5-d]pyrimidin-7-amine (231 mg) prepared in the Reference Example 111-1, chloroform (2 mL), and trifluoroacetic acid (2 mL) was added triethylsilane (0.336 mL), and the resulting mixture was stirred at 50°C for 3 days. The reaction mixture was purified by NH-silica gel column chromatography (solvent: hexane/ethyl acetate = 50/50 to 0/100) to give the title compound (49 mg) (yield 47%) as a colorless powder. MS(APCI) m/z: 249 $[M+H]^+$

Examples 112 to 127:

A corresponding starting compound was treated in a similar manner to the Example 111 to give each compound described in the following Table 8.

Table 8

| Example | Structural formula | Physical property etc. |
|---|---|---|
| 112 | 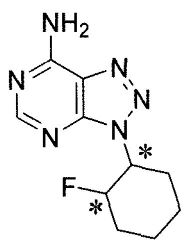 trans, racemate | MS(APCI) m/z; 237 $[M+H]^+$ |

(continued)

| Example | Structural formula | Physical property etc. |
|---|---|---|
| 113 | 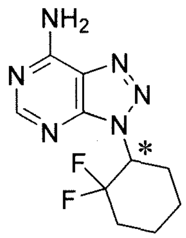 racemate | MS(APCI) m/z; 255 [M+H]+ |
| 114 | 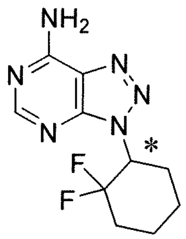 single enantiomer wherein the absolute configuration is derived from Reference Example 114-1 | MS(ESI) m/z; 255 [M+H]+ |
| 115 | 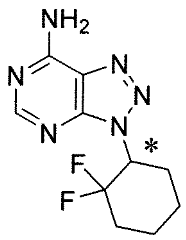 single enantiomer wherein the absolute configuration is derived from Reference Example 115-1 | MS (ESI) m/z; 255 [M+H]+ |
| 116 | 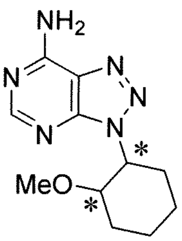 trans, racemate | MS(APCI) m/z; 249 [M+H]+ |
| 117 | 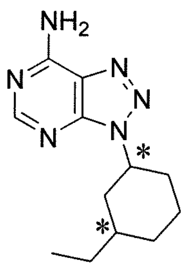 cis, racemate | MS(ESI) m/z; 247 [M+H]+ |

(continued)

| Example | Structural formula | Physical property etc. |
|---|---|---|
| 118 | 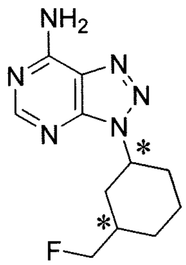 cis, racemate | MS(ESI) m/z; 251 [M+H]+ |
| 119 | 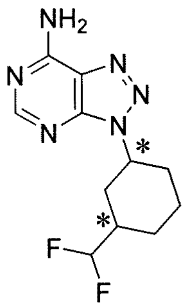 cis, racemate | MS (ESI) m/z; 269 [M+H]+ |
| 120 | 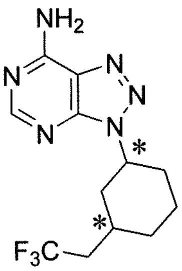 cis, racemate | MS(ESI) m/z; 301 [M+H]+ |
| 121 | 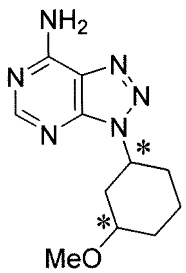 cis, racemate | MS(ESI) m/z; 249 [M+H]+ |
| 123 | 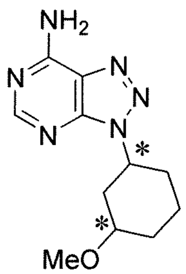 trans, racemate | MS(ESI) m/z; 249 [M+H]+ |

(continued)

| Example | Structural formula | Physical property etc. |
|---|---|---|
| 124 | 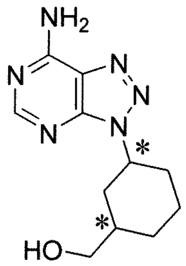 cis, racemate | MS(ESI) m/z; 249 [M+H]+ |
| 125 | 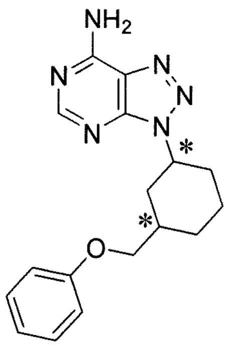 cis, racemate | MS(ESI) m/z; 325 [M+H]+ |
| 126 | 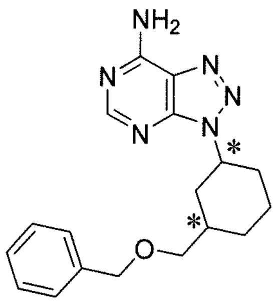 cis, racemate | MS(ESI) m/z; 339 [M+H]+ |
| 127 | 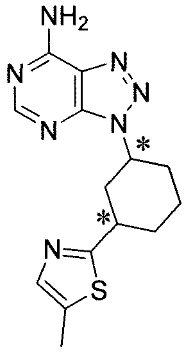 cis, racemate | MS(ESI) m/z; 316 [M+H]+ |

Example 128

Preparation of 3-(3,3-dimethylcyclohexyl)-3H-[1,2,3]triazolo[4,5-d]pyrimidin-7-amine

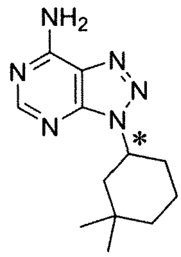

racemate

A mixture of N-(2,4-dimethoxybenzyl)-N-(3,3-dimethylcyclohexyl)-3-(4-methoxybenzyl)-3H-[1,2,3]triazolo[4,5-d]pyrimidin-7-amine (520 mg) prepared in the Reference Example 128-1, triethylsilane (0.8 mL), and trifluoroacetic acid (5 mL) was stirred at 70°C for 1 day. The reaction mixture was allowed to cool to room temperature, and concentrated under reduced pressure. To the resulting residues was added a saturated aqueous solution of sodium hydrogen carbonate, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, the resulting residues were washed with a mixed solvent of diethyl ether and hexane, collected by filtration, and dried under reduced pressure to give the title compound (114 mg) (yield 46%) as a yellow powder.
MS(APCI) m/z: 247 $[M+H]^+$

Examples 129 to 131:

A corresponding starting compound was treated in a similar manner to the Example 128 to give each compound described in the following Table 9.

Table 9

| Example | Structural formula | Physical property etc. |
|---|---|---|
| 129 | 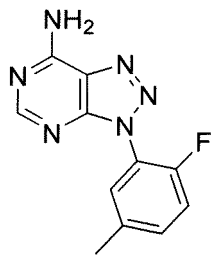 | MS(APCI) m/z; 245 $[M+H]^+$ |
| 130 | 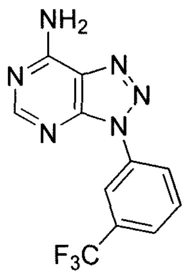 | MS(APCI) m/z; 281 $[M+H]^+$ |

(continued)

| Example | Structural formula | Physical property etc. |
|---|---|---|
| 131 | 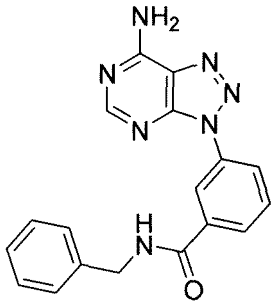 | MS(APCI) m/z; 346 [M+H]+ |

Example 132

Preparation of 3-[(1S,2R,5R)-2-methyl-5-(propan-2-yl)cyclohexyl]-3H-[1,2,3]triazolo[4,5-d]pyrimidin-7-amine

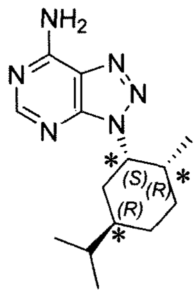

To a mixture of 3-[(1S,2R,5R)-2-methyl-5-(prop-1-en-2-yl)cyclohexyl]-3H-[1,2,3]triazolo[4,5-d]pyrimidin-7-amine (72 mg) prepared in the Example 106, ethanol (5 mL), and tetrahydrofuran (5 mL) was added 10% palladium carbon (50 mg), and the resulting mixture was stirred under hydrogen atmosphere at room temperature for 5 hours and 30 minutes. The reaction mixture was subjected to nitrogen replacement, then the insoluble matters were removed by filtration, and the resulting filtrate was concentrated under reduced pressure. The resulting residues were washed with a mixed solvent of hexane and ethyl acetate, the resulting solid was collected by filtration, and dried under reduced pressure to give the title compound (56 mg) (yield 77%) as a colorless powder.
MS(APCI) m/z: 275 [M+H]+

Example 133

Preparation of 3-(3-methylcyclobutyl)-[1,2,4]triazolo[4,3-a]pyrazin-8-amine

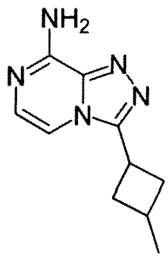

mixture of cis isomer and trans isomer

(1) To an eggplant flask were added 3-(3-methylenecyclobutyl)-[1,2,4]triazolo[4,3-a]pyrazin-8-amine (40 mg) prepared in the Example 19 and ethanol (2 mL), acetic acid was added thereto until the reaction mixture became homogeneously transparent, 5% palladium carbon (manufactured by NE CHEMCAT Corporation, BNA-Type (trade name)) (8.8 mg) was added thereto, and the resulting mixture was stirred under hydrogen balloon atmosphere at 60°C for 5 hours. The reaction mixture was subjected to nitrogen replacement, then filtered, and the resulting filtrate was concentrated under reduced pressure to give a crude product of title compound (33 mg) (yield 82%) as a white solid.
(2) To an eggplant flask were added the resulting crude product (31 mg), ethanol (1 mL), and water (0.5 mL), and the resulting mixture was stirred at room temperature for 2 hours. The resulting solid was filtered, and dried under reduced pressure to give the title compound (5.6 mg) (yield 18%) as a white solid.
MS(CI) m/z: 204 $[M+H]^+$

Example 134

Preparation of 3-[(1R,2S,5S)-2-methyl-5-(propan-2-yl)cyclohexyl]-3H-[1,2,3]triazolo[4,5-d]pyrimidin-7-amine

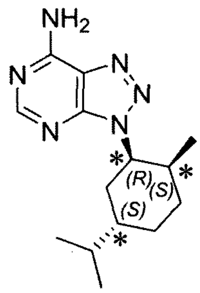

3-[(1R,2S,5S)-2-methyl-5-(prop-1-en-2-yl)cyclohexyl]-3H-[1,2,3]triazolo[4,5-d]pyrimidin-7-amine prepared in the Example 107 was reacted in a similar manner to the Example 132 to give the title compound.
MS(APCI) m/z: 275 $[M+H]^+$

Example 135

Preparation of 3-[(3S)-1-methylpiperidin-3-yl]-3H-[1,2,3]triazolo[4,5-d]pyrimidin-7-amine

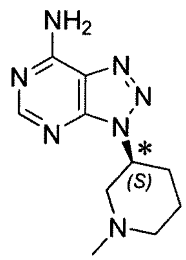

To a mixture of 3-[(3S)-piperidin-3-yl]-3H-[1,2,3]triazolo[4,5-d]pyrimidin-7-amine dihydrochloride (200 mg) prepared in the Reference Example 135-1, N,N-diisopropylethylamine (0.296 mL), formaldehyde (0.0555 mL), and dichloromethane (5 mL) was added sodium triacetoxyborohydride (290 mg), and the resulting mixture was stirred for 4 days. To the reaction mixture was added a saturated aqueous solution of sodium hydrogen carbonate, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, the resulting residues were washed with a mixed solvent of hexane and ethyl acetate, then the resulting solid was collected by filtration, and dried under reduced pressure to give the title compound (92 mg) (yield 58%) as a pale red powder.

MS(APCI) m/z: 234 [M+H]+

Example 136

Preparation of 3-[(3R)-1-phenylpiperidin-3-yl]-3H-[1,2,3]triazolo[4,5-d]pyrimidin-7-amine

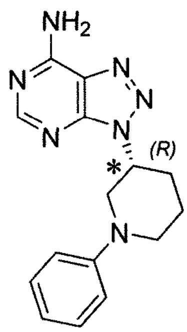

A mixture of 3-[(3R)-piperidin-3-yl]-3H-[1,2,3]triazolo[4,5-d]pyrimidin-7-amine dihydrochloride (200 mg) prepared in the Reference Example 136-1, copper(II) acetate (249 mg), phenylboronic acid (167 mg), N,N-diisopropylethylamine (0.951 mL), and dichloromethane (7 mL) was stirred at room temperature for 8 hours. To the reaction mixture were additionally added copper(II) acetate (249 mg) and phenylboronic acid (167 mg), and the resulting mixture was stirred at room temperature overnight. The reaction mixture was filtered, and the resulting residues were washed with ethyl acetate. The resulting filtrate was concentrated under reduced pressure, and the resulting residues were purified by NH-silica gel column chromatography (solvent: hexane/ethyl acetate = 70/30 to 30/70) to give the title compound (26 mg) (yield 13%) as a colorless powder.
MS(APCI) m/z: 296 [M+H]+

Example 137

Preparation of 3-[(3S)-1-phenylpiperidin-3-yl]-3H-[1,2,3]triazolo[4,5-d]pyrimidin-7-amine

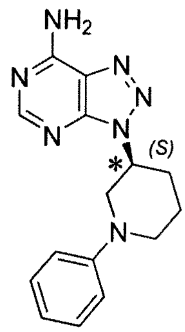

3-[(3S)-piperidin-3-yl]-3H-[1,2,3]triazolo[4,5-d]pyrimidin-7-amine dihydrochloride prepared in the Reference Example 135-1 was reacted in a similar manner to the Example 136 to give the title compound.
MS(APCI) m/z: 296 [M+H]+

Example 138

Preparation of 3-[(3S)-1-benzylpiperidin-3-yl]-3H-[1,2,3]triazolo[4,5-d]pyrimidin-7-amine

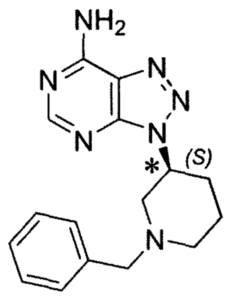

3-[(3S)-piperidin-3-yl]-3H-[1,2,3]triazolo[4,5-d]pyrimidin-7-amine dihydrochloride prepared in the Reference Example 135-1 and benzaldehyde were reacted in a similar manner to the Example 135 to give the title compound.
MS(APCI) m/z: 310 $[M+H]^+$

Example 139

Preparation of 3-[3-(benzyloxy)phenyl]-3H-[1,2,3]triazolo[4,5-d]pyrimidin-7-amine

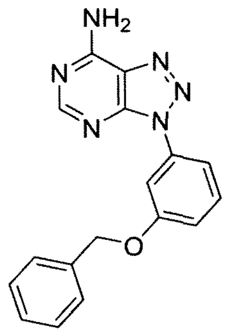

To a suspension of 3-(7-amino-3H-[1,2,3]triazolo[4,5-d]pyrimidin-3-yl)phenol (58 mg) prepared in the Reference Example 139-1, triphenylphosphine (134 mg), benzylalcohol (0.53 mL), and tetrahydrofuran (3 mL) was added diisopropyl azodicarboxylate (a 40% solution in toluene) (0.27 mL), and the resulting mixture was stirred at room temperature for 1 hour. To the reaction mixture was added water, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 70/30 to 30/70) to give the title compound (15 mg) (yield 18%) as a colorless powder.
MS(APCI) m/z: 319 $[M+H]^+$

Example 140

Preparation of 3-[cis-3-methylcyclohexyl][1,2,4]triazolo[4,3-a]pyrazin-8-amine

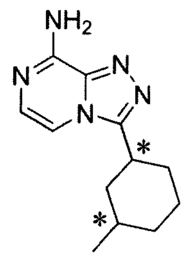

cis, racemate

A solution of 8-chloro-3-(cis-3-methylcyclohexyl)[1,2,4]triazolo[4,3-a]pyrazine (308 mg) prepared in the Reference Example 140-1 in a 2.0 mol/L ammonia/isopropanol (10 mL) was sealed in a tube, and the resulting mixture was stirred at 100°C for 7 hours. The reaction mixture was allowed to cool to room temperature, and the resulting mixture was concentrated under reduced pressure. The resulting residues were washed with water and a small amount of ethanol, the resulting solid was collected by filtration, and dried under reduced pressure to give the title compound (250 mg) (yield 88%) as a colorless powder.
MS(APCI) m/z: 232 $[M+H]^+$

Examples 141 to 170:

A corresponding starting compound was treated in a similar manner to the Example 140 to give each compound described in the following Table 10.

Table 10

| Example | Structural formula | Physical property etc. |
|---|---|---|
| 141 | 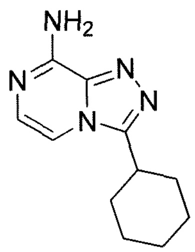 | MS(ESI) m/z; 218 $[M+H]^+$ |
| 142 | 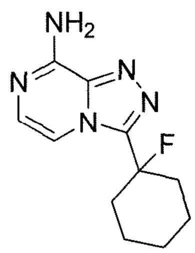 | MS(ESI) m/z; 236 $[M+H]^+$ |
| 143 | 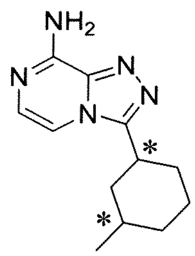 trans, racemate | MS(ESI) m/z; 232 $[M+H]^+$ |

(continued)

| Example | Structural formula | Physical property etc. |
|---|---|---|
| 144 | 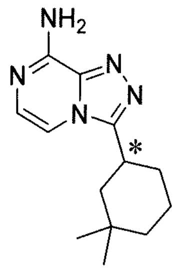 racemate | MS(ESI) m/z; 246 $[M+H]^+$ |
| 145 | 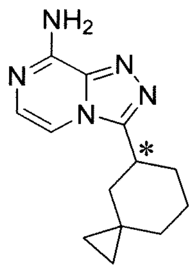 racemate | MS(ESI) m/z; 244 $[M+H]^+$ |
| 146 | 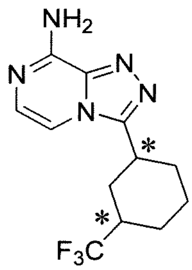 cis, racemate | MS(ESI) m/z; 286 $[M+H]^+$ |
| 147 | 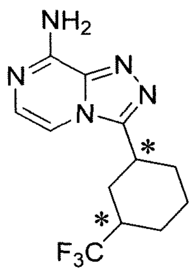 trans, racemate | MS(ESI) m/z; 286 $[M+H]^+$ |
| 148 | 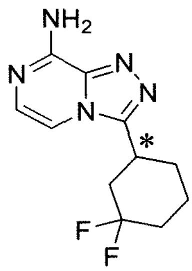 racemate | MS(APCI) m/z; 254 $[M+H]^+$ |

(continued)

| Example | Structural formula | Physical property etc. |
|---|---|---|
| 149 | 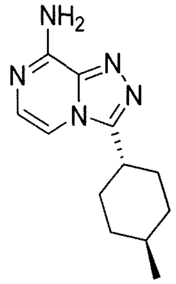 trans | MS(ESI) m/z; 232 $[M+H]^+$ |
| 150 | 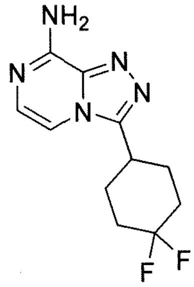 | MS(ESI) m/z; 254 $[M+H]^+$ |
| 151 | 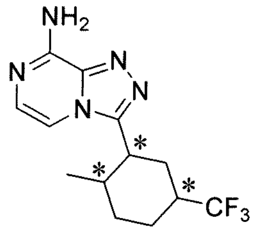 relative configuration (1R*, 2S*, 5R*), racemate | MS(APCI) m/z; 300 $[M+H]^+$ |
| 152 | 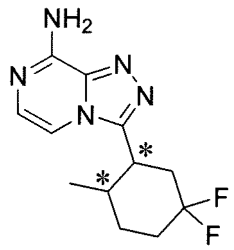 cis, racemate | MS(ESI) m/z; 268 $[M+H]^+$ |
| 153 | 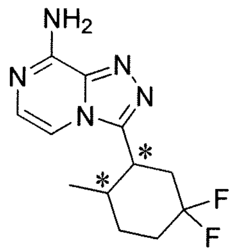 trans, racemate | MS (ESI) m/z; 268 $[M+H]^+$ |

(continued)

| Example | Structural formula | Physical property etc. |
|---|---|---|
| 154 | 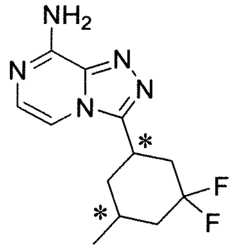 cis, racemate | MS (ESI) m/z; 268 [M+H]+ |
| 155 | 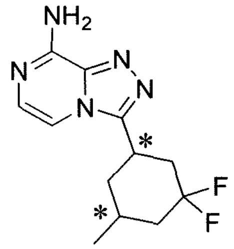 trans, racemate | MS(ESI) m/z; 268 [M+H]+ |
| 156 | 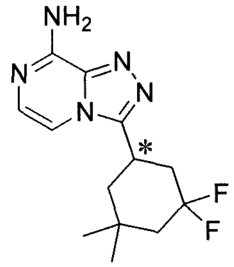 racemate | MS(ESI) m/z; 282 [M+H]+ |
| 157 | 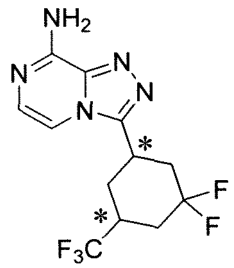 cis, racemate | MS (ESI) m/z; 322 [M+H]+ |
| 158 | 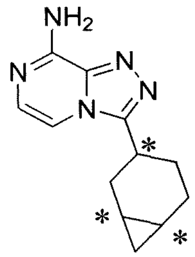 cyclopropane in bicyclo[4,1,0]heptane ring is cis isomer, mixture of four types of stereoisomers | MS(ESI) m/z; 230 [M+H]+ |

(continued)

| Example | Structural formula | Physical property etc. |
|---|---|---|
| 159 | 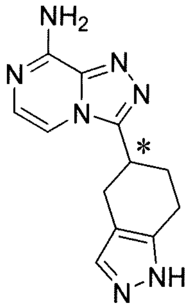 racemate | MS(ESI) m/z; 256 [M+H]+ |
| 160 | 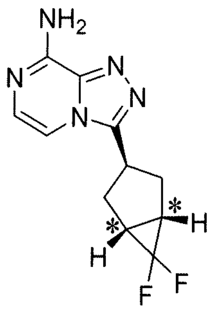 | MS (ESI) m/z; 252 [M+H]+ |
| 161 | 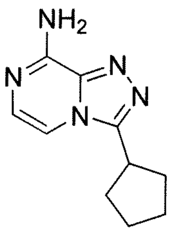 | MS(ESI) m/z; 204 [M+H]+ |
| 162 | 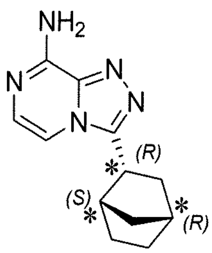 | MS (ESI) m/z; 230 [M+H]+ |
| 163 | 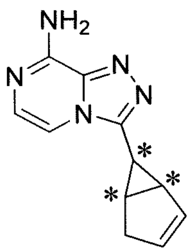 relative configuration (1S*, 5R*, 6S*), racemate | MS(ESI) m/z; 214 [M+H]+ |

(continued)

| Example | Structural formula | Physical property etc. |
|---|---|---|
| 164 | 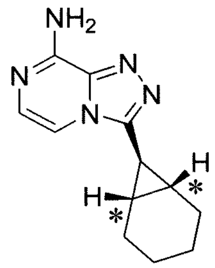 | MS(ESI) m/z; 230 [M+H]+ |
| 165 | 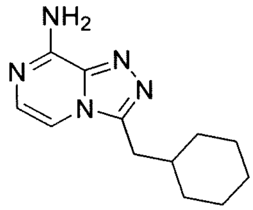 | MS(ESI) m/z; 232 [M+H]+ |
| 166 | 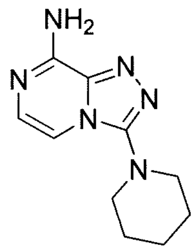 | MS(ESI) m/z; 219 [M+H]+ |
| 167 | 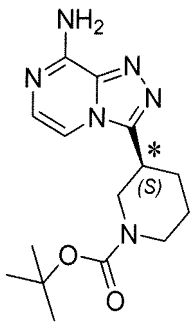 | MS(APCI) m/z; 319 [M+H]+ |
| 168 | 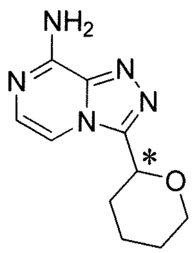 racemate | MS(ESI) m/z; 220 [M+H]+ |

(continued)

| Example | Structural formula | Physical property etc. |
|---|---|---|
| 169 | 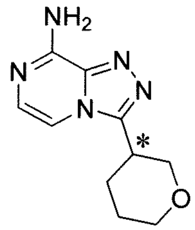 racemate | MS(ESI) m/z; 220 [M+H]+ |
| 170 | 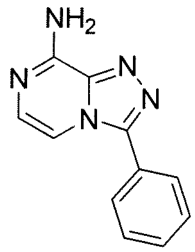 | MS(ESI) m/z; 212 [M+H]+ |

Example 171

Preparation of 3-cyclohexyl-5-methyl[1,2,4]triazolo[4,3-a]pyrazin-8-amine

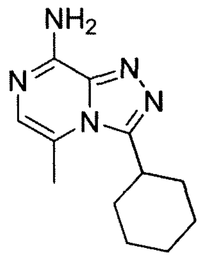

To a reaction container for microwave were added 3-bromo-5-methyl-pyrazin-2-amine (570 mg), cyclohexanecarbohydrazide (530 mg), triethylamine (625 μL), and N-methylpyrrolidone (3 mL), the container was sealed, and the resulting mixture was stirred under microwave radiation at 225°C for 3 hours. The reaction solution was purified by silica gel column chromatography (solvent: hexane/ethyl acetate = 25/75 to 0/100 to solvent: ethyl acetate/methanol = 100/0 to 80/20) to give the title compound (13.9 mg) (yield 2%) as a pale yellow solid. MS(ESI) m/z: 232 [M+H]+

Example 172

Preparation of 3-[cis-2,2-difluoro-5-(trifluoromethyl)cyclohexyl][1,2,4]triazolo[4,3-a]pyrazin-8-amine

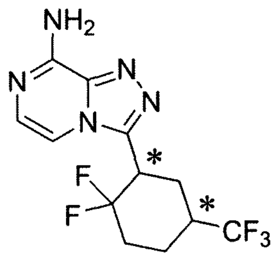

cis, racemate

A mixture of 3-[cis-2,2-difluoro-5-(trifluoromethyl)cyclohexyl]-N,N-bis(4-methoxybenzyl)[1,2,4]triazolo[4,3-a]pyrazin-8-amine (23 mg) prepared in Reference Example 172-1, triethylsilane (0.04 mL), and trifluoroacetic acid (2 mL) was stirred at 70°C for 2 days. The reaction mixture was allowed to cool to room temperature, concentrated under reduced pressure, and the resulting residues were purified by NH silica gel column chromatography (solvent: hexane/ethyl acetate = 40/60 to 0/100) to give the title compound (5.9 mg) (yield 45%) as a colorless solid.
MS(APCI) m/z: 322 $[M+H]^+$

Examples 173 to 178:

A corresponding starting compound was treated in a similar manner to the Example 172 to give each compound described in the following Table 11.

Table 11

| Example | Structural formula | Physical property etc. |
|---|---|---|
| 173 | 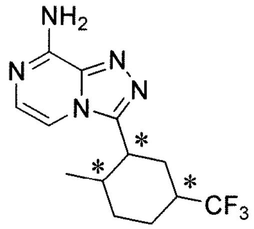 relative configuration (1R*, 2S*, 5R*), single enantiomer wherein the absolute configuration is derived from Reference Example 173-1 | MS(APCI) m/z; 300 $[M+H]^+$ |
| 174 | 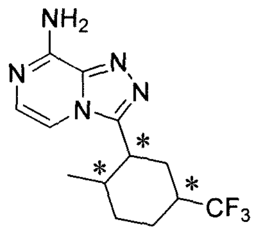 relative configuration (1R*, 2S*, 5R*), single enantiomer wherein the absolute configuration is derived from Reference Example 174-1 | MS(APCI) m/z; 300 $[M+H]^+$ |
| 175 | 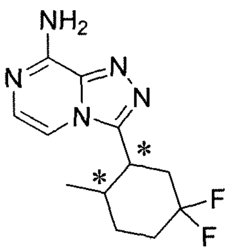 cis, single enantiomer wherein the absolute configuration is derived from Reference Example 175-1 | MS(ESI) m/z; 268 $[M+H]^+$ |

(continued)

| Example | Structural formula | Physical property etc. |
|---|---|---|
| 176 | 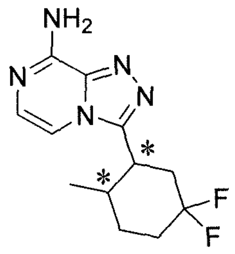 cis, single enantiomer wherein the absolute configuration is derived from Reference Example 176-1 | MS(ESI) m/z; 268 [M+H]$^+$ |
| 177 | 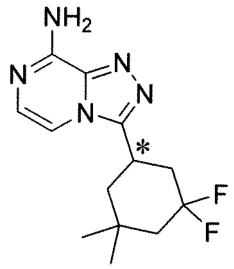 single enantiomer wherein the absolute configuration is derived from Reference Example 177-1 | MS(APCI) m/z; 282 [M+H]$^+$ |
| 178 | 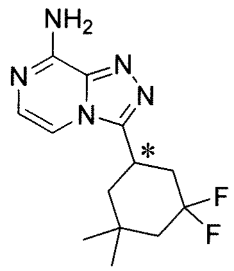 single enantiomer wherein the absolute configuration is derived from Reference Example 178-1 | MS(APCI) m/z; 282 [M+H]$^+$ |

Example 179

Preparation of 3-[(1R,5S,6r)-bicyclo[3.1.0]hex-6-yl][1,2,4]triazolo[4,3-a]pyrazin-8-amine

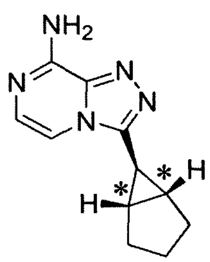

A mixture of 3-[(1S*,5R*,6S*)-bicyclo[3.1.0]hex-2-en-6-yl][1,2,4]triazolo[4,3-a]pyrazin-8-amine (80 mg) prepared in the Example 163 and ethanol (20 mL) was subjected to nitrogen replacement, then 10% palladium carbon (40 mg) was added thereto, and the resulting mixture was stirred under hydrogen atmosphere at room temperature for 2

hours. The reaction mixture was subjected to nitrogen replacement, and then the insoluble matters were removed by Celite filtration. The insoluble matters were washed with ethanol, and the resulting filtrate was concentrated under reduced pressure. The resulting residues were purified by silica gel column chromatography (solvent: ethyl acetate/methanol = 100/0 to 90/10), and then purified by reverse-phase HPLC (solvent: 0.05% solution of trifluoroacetic acid in water/0.05% solution of trifluoroacetic acid in acetonitrile = 90/10 to 65/35) to give the title compound (47.6 mg) (yield 59%) as a colorless powder.
MS(ESI) m/z: 216 $[M+H]^+$

Example 180

Preparation of 3-cyclohexylimidazo[1,5-a]pyrazin-8-amine

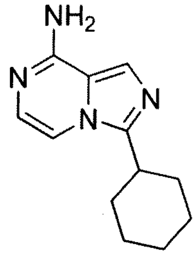

To a reaction container for microwave were added 8-chloro-3-cyclohexylimidazo[1,5-a]pyrazine (51.8 mg) prepared in the Reference Example 180-1 and a 7 mol/L ammonia-methanol solution (2.5 mL), and the resulting mixture was stirred under microwave radiation at 150°C for 3 hours. The reaction mixture was allowed to cool to room temperature, and the reaction solution was purified by silica gel column chromatography (solvent: ethyl acetate/methanol = 100/0 to 85/15) to give the title compound (32.2 mg) (yield 68%) as a colorless powder. MS(ESI) m/z: 217 $[M+H]^+$

Examples 181 to 185:

A corresponding starting compound was treated in a similar manner to the Example 180 to give each compound described in the following Table 12.

Table 12

| Example | Structural formula | Physical property etc. |
|---|---|---|
| 181 | 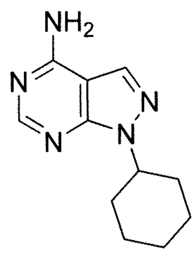 | MS(ESI) m/z; 218 $[M+H]^+$ |
| 182 | 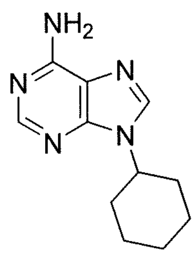 | MS(ESI) m/z; 218 $[M+H]^+$ |

(continued)

| Example | Structural formula | Physical property etc. |
|---|---|---|
| 183 | 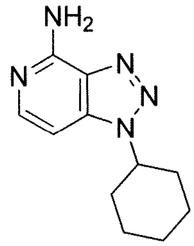 | MS(ESI) m/z; 218 $[M+H]^+$ |
| 184 | 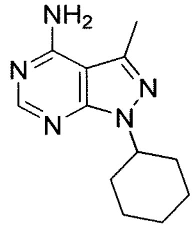 | MS(ESI) m/z; 232 $[M+H]^+$ |
| 185 | 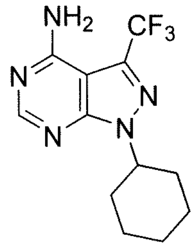 | MS (ESI) m/z; 286 $[M+H]^+$ |

Example 186

Preparation of 3-cyclohexyl[1,2,4]triazolo[4,3-a]pyridin-8-amine

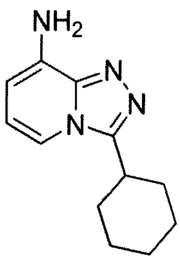

To a mixture of 3-cyclohexyl-8-nitro[1,2,4]triazolo[4,3-a]pyridine (2.0 g) prepared in the Reference Example 186-1 and methanol (90 mL) was added 10% palladium carbon (300 mg), and the resulting mixture was stirred under hydrogen atmosphere at room temperature for 5 hours. The insoluble matters were removed by filtration, and the resulting filtrate was concentrated under reduced pressure. The resulting residues were purified by silica gel column chromatography (solvent: chloroform/methanol = 100/0 to 98/2), and then purified by NH-silica gel column chromatography (solvent: ethyl acetate) again to give the title compound (960 mg) (yield 55%) as a brown powder.
MS(ESI) m/z: 217 $[M+H]^+$

Example 187

Preparation of 3-cyclohexyl-1-methyl-1H-pyrazolo[4,3-d]pyrimidin-7-amine

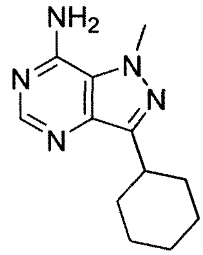

(1) To a 25 mL eggplant flask were added 3-(cyclohex-1-en-1-yl)-N,N-bis(2,4-dimethoxybenzyl)-1-methyl-1H-pyrazolo[4,3-d]pyrimidin-7-amine (235 mg) prepared in the Reference Example 187-1, 10% palladium carbon (117 mg), and ethanol (1.9 mL), and the resulting mixture was stirred under hydrogen atmosphere at room temperature overnight. The reaction mixture was subjected to nitrogen replacement, the insoluble matters were removed by Celite filtration, and the resulting filtrate was concentrated under reduced pressure to give a crude product of 3-cyclohexyl-N,N-bis(2,4-dimethoxybenzyl)-1-methyl-1H-pyrazolo[4,3-d]pyrimidin-7-amine.

(2) To 3-cyclohexyl-N,N-bis(2,4-dimethoxybenzyl)-1-methyl-1H-pyrazolo[4,3-d]pyrimidin-7-amine prepared in the above (1) were added chloroform (1.9 mL), triethylsilane (309 μL), and trifluoroacetic acid (1.9 mL), the resulting mixture was stirred at 50°C overnight, then chloroform (1.9 mL) and triethylsilane (309 μL) were additionally added thereto, and the resulting mixture was stirred at 50°C overnight. The reaction mixture was allowed to cool to room temperature, a saturated aqueous solution of sodium hydrogen carbonate was added thereto, and the resulting mixture was extracted three times with chloroform. The resulting organic layers were combined, and dried over anhydrous sodium sulfate. The insoluble matters were removed by filtration, and the resulting filtrate was concentrated under reduced pressure. The resulting residues were purified by silica gel column chromatography (solvent: ethyl acetate/methanol = 100/0 to 75/25) to give the title compound (73 mg) (yield 82%) as a colorless powder.
MS(ESI) m/z: 232 $[M+H]^+$

Example 188

Preparation of 3-cyclohexylisoxazolo[4,5-d]pyrimidin-7-amine

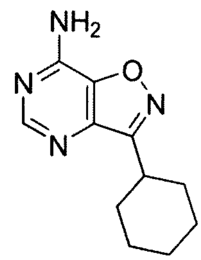

3-Cyclohexyl-N-(2,4-dimethoxybenzyl)isoxazolo[4,5-d]pyrimidin-7-amine prepared in the Reference Example 188-1 was reacted in a similar manner to the Example 172 to give the title compound.
MS(APCI) m/z: 219 $[M+H]^+$

Example 189

Preparation of 3-cyclohexyl[1,2,4]triazolo[4,3-c]pyrimidin-8-amine

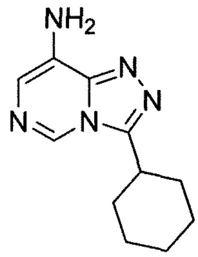

N'-(5-aminopyrimidin-4-yl)cyclohexanecarbohydrazide prepared in the Reference Example 189-1 was reacted in a similar manner to the Reference Example 142-1 to give the title compound.
MS(APCI) m/z: 218 $[M+H]^+$

Examples 190 to 247:

A racemic mixture or a diastereomer mixture prepared in each of the above Example was resolved by chiral high performance liquid chromatography (chiral HPLC) or chiral supercritical fluid chromatography (chiral SFC) to give each compound described in the following Table 13.

Table 13

| Ex. | Structural formula | Physical property etc. | Analysis conditions etc. |
|---|---|---|---|
| 190 | 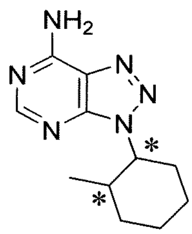 cis, single enantiomer | MS (ESI) m/z; 233 $[M+H]^+$ | Column: CHIRALCEL OJ-3 (4.6 × 150 mm) Mobile phase: methanol/diethylamine (100/0.1) Flow rate: 0.5 mL/min Temperature: 25°C Analysis channel: PDA 280.0 nm Retention time (min.): 6.196 |
| 191 | 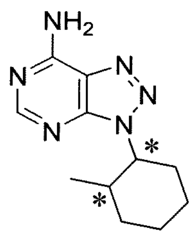 cis, single enantiomer opposite to Example 190 | MS(ESI) m/z; 233 $[M+H]^+$ | Column: CHIRALCEL OJ-3 (4.6 × 150 mm) Mobile phase: methanol/diethylamine (100/0.1) Flow rate: 0.5 mL/min Temperature: 25°C Analysis channel: PDA 280.0 nm Retention time (min.): 7.630 |
| 192 | 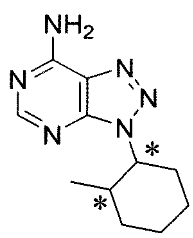 trans, single enantiomer | MS(APCI) m/z; 233 $[M+H]^+$ | Column: CHIRALPAKIA-3 (4.6 × 150 mm) Mobile phase: methanol/diethylamine (100/0.1) Flow rate: 0.5 mL/min Temperature: 25°C Analysis channel: PDA 278.0 nm Retention time (min.): 6.169 |

(continued)

| Ex. | Structural formula | Physical property etc. | Analysis conditions etc. |
|---|---|---|---|
| 193 | 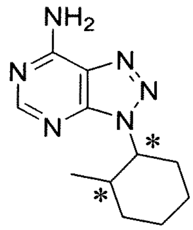 trans, single enantiomer opposite to Example 192 | MS(APCI) m/z; 233 [M+H]+ | Column: CHIRALPAKIA-3 (4.6 × 150 mm) Mobile phase: methanol/diethylamine (100/0.1) Flow rate: 0.5 mL/min Temperature: 25°C Analysis channel: PDA 278.0 nm Retention time (min.): 7.704 |
| 194 | 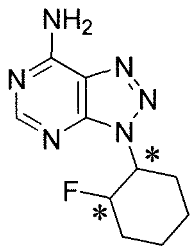 cis, single enantiomer | MS(APCI) m/z; 237 [M+H]+ | Column: CHIRALPAKIA-3 (4.6 × 150 mm) Mobile phase: methanol/diethylamine (100/0.1) Flow rate: 0.5 mL/min Temperature: 25°C Analysis channel: PDA 278.0 nm Retention time (min.): 9.711 |
| 195 | 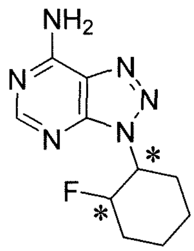 cis, single enantiomer opposite to Example 194 | MS (APCI) m/z; 237 [M+H]+ | Column: CHIRALPAKIA-3 (4.6 × 150 mm) Mobile phase: methanol/diethylamine (100/0.1) Flow rate: 0.5 mL/min Temperature: 25°C Analysis channel: PDA 278.0 nm Retention time (min.): 11.725 |
| 196 | 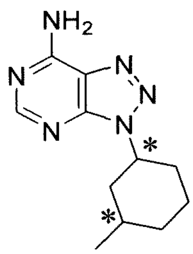 cis, single enantiomer | MS (APCI) m/z; 233 [M+H]+ | Column: CHIRALPAKID-3 (4.6 × 150 mm) Mobile phase: hexane/ethanol/ tetrahydrofuran/diethylamine (80/12.5/7.5/0.1) Flow rate: 0.5 mL/min Temperature: 25°C Analysis channel: PDA 280.0 nm Retention time (min.): 7.924 |

(continued)

| Ex. | Structural formula | Physical property etc. | Analysis conditions etc. |
|---|---|---|---|
| 197 | 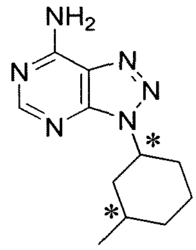<br>cis, single enantiomer opposite to Example 196 | MS (APCI)<br>m/z;<br>233 $[M+H]^+$ | Column: CHIRALPAKID-3 (4.6 × 150 mm)<br>Mobile phase: hexane/ethanol/ tetrahydrofuran/diethylamine (80/12.5/7.5/0.1)<br>Flow rate: 0.5 mL/min<br>Temperature: 25°C<br>Analysis channel: PDA 280.0 nm<br>Retention time (min.): 9.666 |
| 198 | 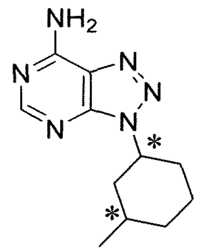<br>trans, single enantiomer | MS (APCI)<br>m/z;<br>233 $[M+H]^+$ | Column: CHIRALPAKIF-3 (4.6 × 150 mm)<br>Mobile phase: methanol/diethylamine (100/0.1)<br>Flow rate: 0.5 mL/min<br>Temperature: 25°C<br>Analysis channel: PDA 280.0 nm<br>Retention time (min.): 9.911 |
| 199 | 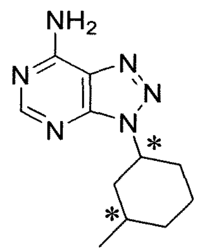<br>trans, single enantiomer opposite to Example 198 | MS (APCI)<br>m/z;<br>233 $[M+H]^+$ | Column: CHIRALPAKIF-3 (4.6 × 150 mm)<br>Mobile phase: methanol/diethylamine (100/0.1)<br>Flow rate: 0.5 mL/min<br>Temperature: 25°C<br>Analysis channel: PDA 280.0 nm<br>Retention time (min.): 12.173 |
| 200 | 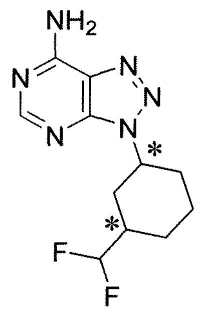<br>cis, single enantiomer | MS (ESI)<br>m/z;<br>269 $[M+H]^+$ | Column: CHIRALPAKIA-3 (4.6 × 150 mm)<br>Mobile phase: methanol/ tetrahydrofuran/diethylamine (80/20/0.1)<br>Flow rate: 0.5 mL/min<br>Temperature: 25°C<br>Analysis channel: PDA 278.0 nm<br>Retention time (min.): 6.157 |

(continued)

| Ex. | Structural formula | Physical property etc. | Analysis conditions etc. |
|---|---|---|---|
| 201 | 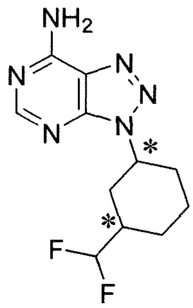 cis, single enantiomer opposite to Example 200 | MS (ESI) m/z; 269 [M+H]+ | Column: CHIRALPAKIA-3 (4.6 × 150 mm) Mobile phase: methanol/ tetrahydrofuran/diethylamine (80/20/0.1) Flow rate: 0.5 mL/min Temperature: 25°C Analysis channel: PDA 278.0 nm Retention time (min.): 9.412 |
| 202 | 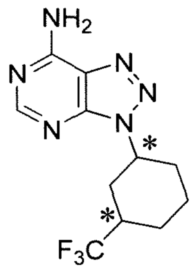 cis, single enantiomer | MS(ESI) m/z; 287 [M+H]+ | Column: CHIRALPAKIA-3 (4.6 × 150 mm) Mobile phase: hexane/methanol/ tetrahydrofuran/diethylamine (60/20/20/0.1) Flow rate: 0.5 mL/min Temperature: 25°C Analysis channel: PDA 280.0 nm Retention time (min.): 5.703 |
| 203 | 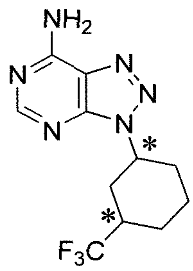 cis, single enantiomer opposite to Example 202 | MS(ESI) m/z; 287 [M+H]+ | Column: CHIRALPAKIA-3 (4.6 × 150 mm) Mobile phase: hexane/methanol/ tetrahydrofuran/diethylamine (60/20/20/0.1) Flow 0.5 mL/min rate: Temperature: 25°C Analysis channel: PDA 280.0 nm Retention time (min.): 10.170 |
| 204 | 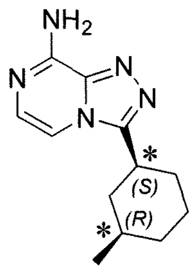 | MS(ESI) m/z; 232 [M+H]+ | Column: CHIRALPAKIA-3 (4.6 × 150 mm) Mobile phase: hexane/ethanol/ tetrahydrofuran/diethylamine (55/25/20/0.1) Flow rate: 0.5 mL/min Temperature: 25°C Analysis channel: PDA 290.0 nm Retention time (min.): 7.813 |

(continued)

| Ex. | Structural formula | Physical property etc. | Analysis conditions etc. |
|---|---|---|---|
| 205 | 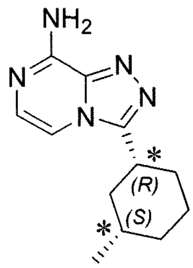 | MS(ESI)<br>m/z; 2<br>32 [M+H]+ | Column: CHIRALPAKIA-3 (4.6 × 150 mm)<br>Mobile phase: hexane/ethanol/ tetrahydrofuran/diethylamine (55/25/20/0.1)<br>Flow rate: 0.5 mL/min<br>Temperature: 25°C<br>Analysis channel: PDA 290.0 nm<br>Retention time (min.): 10.140 |
| 206 | 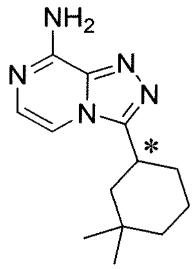<br>single enantiomer | MS(ESI)<br>m/z;<br>246 [M+H]+ | Column: CHIRALPAKID-3 (4.6 × 150 mm)<br>Mobile phase: methyl tert-butyl ether/ ethanol/diethylamine (80/20/0.1)<br>Flow rate: 0.5 mL/min<br>Temperature: 25°C<br>Analysis channel: PDA 231.0 nm<br>Retention time (min.): 10.995 |
| 207 | 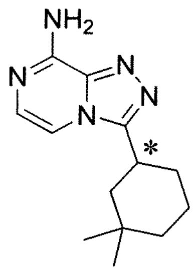<br>single enantiomer opposite to Example 206 | MS(ESI)<br>m/z;<br>246 [M+H]+ | Column: CHIRALPAKID-3 (4.6 × 150 mm)<br>Mobile phase: methyl tert-butyl ether/ ethanol/diethylamine (80/20/0.1)<br>Flow rate: 0.5 mL/min<br>Temperature: 25°C<br>Analysis channel: PDA 231.0 nm<br>Retention time (min.): 8.032 |
| 208 | 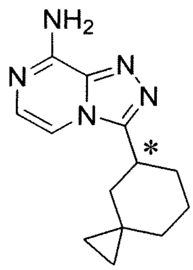<br>single enantiomer | MS(APCI)<br>m/z;<br>244 [M+H]+ | Column: CHIRALPAK IC-3 (4.6 × 150 mm)<br>Mobile phase: hexane/2-propanol/ diethylamine (10/90/0.1)<br>Flow rate: 0.5 mL/min<br>Temperature: 25°C<br>Analysis channel: PDA 231.0 nm<br>Retention time (min.): 10.439 |

(continued)

| Ex. | Structural formula | Physical property etc. | Analysis conditions etc. |
|---|---|---|---|
| 209 | 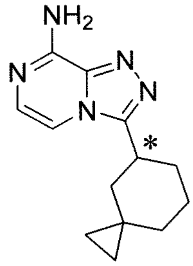 single enantiomer opposite to Example 208 | MS(APCI) m/z; 244 $[M+H]^+$ | Column: CHIRALPAK IC-3 (4.6 × 150 mm) Mobile phase: hexane/2-propanol/ diethylamine (10/90/0.1) Flow rate: 0.5 mL/min Temperature: 25°C Analysis channel: PDA 231.0 nm Retention time (min.): 13.042 |
| 210 | 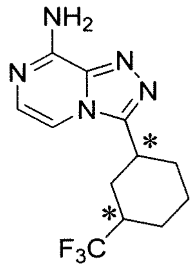 cis, single enantiomer | MS(ESI) m/z; 286 $[M+H]^+$ | Column: CHIRALPAKIE-3 (4.6 × 150 mm) Mobile phase: methanol/diethylamine (100/0.1) Flow rate: 0.5 mL/min Temperature: 25°C Analysis channel: PDA 230 nm Retention time (min.): 6.662 |
| 211 | 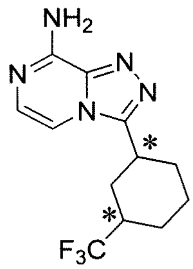 cis, single enantiomer opposite to Example 210 | MS(ESI) m/z; 286 $[M+H]^+$ | Column: CHIRALPAKIE-3 (4.6 × 150 mm) Mobile phase: methanol/diethylamine (100/0.1) Flow rate: 0.5 mL/min Temperature: 25°C Analysis channel: PDA 230 nm Retention time (min.): 9.254 |
| 212 | 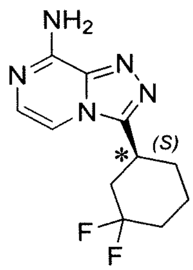 | MS(APCI) m/z; 254 $[M+H]^+$ | Column: CHIRALPAKIA-3 (4.6 × 150 mm) Mobile phase: methanol/acetonitrile/ diethylamine (70/30/0.1) Flow rate: 0.5 mL/min Temperature: 25°C Analysis channel: PDA 230.0 nm Retention time (min.): 8.299 |
| 213 | 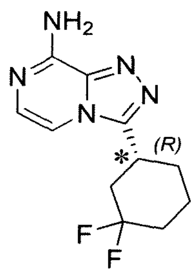 | MS(APCI) m/z; 254 $[M+H]^+$ | Column: CHIRALPAKIA-3 (4.6 × 150 mm) Mobile phase: methanol/acetonitrile/ diethylamine (70/30/0.1) Flow rate: 0.5 mL/min Temperature: 25°C Analysis channel: PDA 230.0 nm Retention time (min.): 13.495 |

(continued)

| Ex. | Structural formula | Physical property etc. | Analysis conditions etc. |
|---|---|---|---|
| 214 | 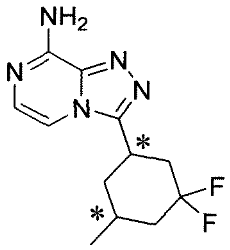<br>cis, single enantiomer | MS(APCI)<br>m/z;<br>268 [M+H]+ | Column: CHIRALPAKIF-3 (4.6 × 150 mm)<br>Mobile phase: ethanol/acetonitrile/ diethylamine (60/40/0.1)<br>Flow rate: 0.5 mL/min<br>Temperature: 25°C<br>Analysis channel: PDA 291.0 nm<br>Retention time (min.): 10.447 |
| 215 | 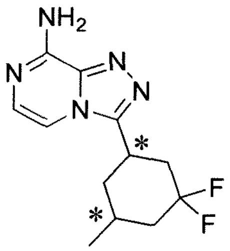<br>cis, single enantiomer opposite to Example 214 | MS(APCI)<br>m/z;<br>268 [M+H]+ | Column: CHIRALPAKIF-3 (4.6 × 150 mm)<br>Mobile phase: ethanol/acetonitrile/ diethylamine (60/40/0.1)<br>Flow rate: 0.5 mL/min<br>Temperature: 25°C<br>Analysis channel: PDA 291.0 nm<br>Retention time (min.): 12.584 |
| 216 | 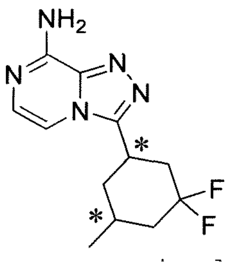<br>trans, single enantiomer | MS(ESI)<br>m/z;<br>268 [M+H]+ | Column: CHIRALPAKIA-3 (4.6 × 150 mm)<br>Mobile phase: methanol/diethylamine (100/0.1)<br>Flow rate: 0.5 mL/min<br>Temperature: 25°C<br>Analysis channel: PDA 292.0 nm<br>Retention time (min.): 8.198 |
| 217 | 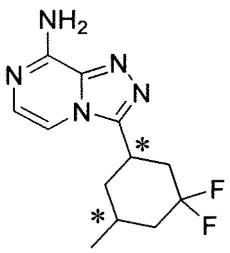<br>trans, single enantiomer opposite to Example 216 | MS(ESI)<br>m/z;<br>268 [M+H]+ | Column: CHIRALPAKIA-3 (4.6 × 150 mm)<br>Mobile phase: methanol/diethylamine (100/0.1)<br>Flow rate: 0.5 mL/min<br>Temperature: 25°C<br>Analysis channel: PDA 292.0 nm<br>Retention time (min.): 13.119 |

(continued)

| Ex. | Structural formula | Physical property etc. | Analysis conditions etc. |
|---|---|---|---|
| 218 | 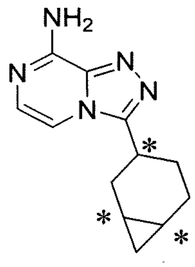 relative configuration (1R*, 3S*, 6R*), single enantiomer | MS(ESI) m/z; 230 [M+H]$^+$ | Column: CHIRALPAKIA-3 (4.6 × 150 mm) Mobile phase: methanol/diethylamine (100/0.1) Flow rate: 0.5 mL/min Temperature: 25°C Analysis channel: PDA 291.0 nm Retention time (min.): 6.974 |
| 219 | 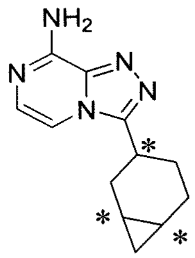 relative configuration (1R*, 3S*, 6R*), single enantiomer opposite to Example 218 | MS(ESI) m/z; 230 [M+H]$^+$ | Column: CHIRALPAKIA-3 (4.6 × 150 mm) Mobile phase: methanol/diethylamine (100/0.1) Flow rate: 0.5 mL/min Temperature: 25°C Analysis channel: PDA 291.0 nm Retention time (min.): 8.969 |
| 220 | 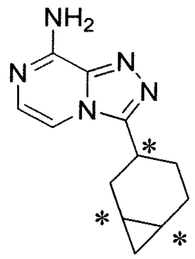 relative configuration (1S*, 3S*, 6S*), single enantiomer | MS(ESI) m/z; 230 [M+H]$^+$ | Column: CHIRALPAKIA-3 (4.6 × 150 mm) Mobile phase: methanol/diethylamine (100/0.1) Flow rate: 0.5 mL/min Temperature: 25°C Analysis channel: PDA 291.0 nm Retention time (min.): 10.926 |
| 221 | 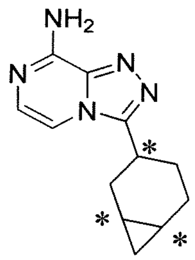 relative configuration (1S* 3S*, 6S*), single enantiomer opposite to Example 220 | MS(ESI) m/z; 230 [M+H]$^+$ | Column: CHIRALPAKIA-3 (4.6 × 150 mm) Mobile phase: methanol/diethylamine (100/0.1) Flow rate: 0.5 mL/min Temperature: 25°C Analysis channel: PDA 291.0 nm Retention time (min.): 12.801 |

(continued)

| Ex. | Structural formula | Physical property etc. | Analysis conditions etc. |
|---|---|---|---|
| 222 | 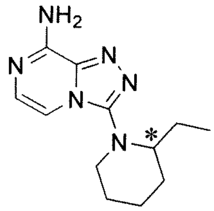 single enantiomer | MS (APCI) m/z; 247 [M+H]+ | Column: CHIRALPAKIF-3 (4.6 × 150 mm) Mobile phase: methyl tert-butyl ether/ methanol/diethylamine (94/6/0.1) Flow rate: 0.5 mL/min Temperature: 25°C Analysis channel: PDA 253.0 nm Retention time (min.): 11.309 |
| 223 | 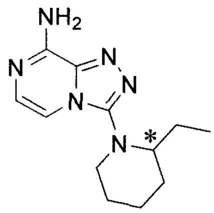 single enantiomer opposite to Example 222 | MS (APCI) m/z; 247 [M+H]+ | Column: CHIRALPAKIF-3 (4.6 × 150 mm) Mobile phase: methyl tert-butyl ether/ methanol/diethylamine (94/6/0.1) Flow rate: 0.5 mL/min Temperature: 25°C Analysis channel: PDA 253.0 nm Retention time (min.): 12.935 |
| 224 | 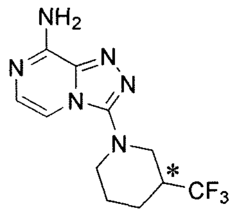 single enantiomer | MS (APCI) m/z; 287 [M+H]+ | Column: CHIRALPAK IC-3 (4.6 × 150 mm) Mobile phase: methyl tert-butyl ether/ ethanol/diethylamine (90/10/0.1) Flow rate: 0.5 mL/min Temperature: 25°C Analysis channel: PDA 247.0 nm Retention time (min.): 10.244 |
| 225 | 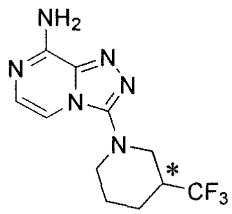 single enantiomer opposite to Example 224 | MS (APCI) m/z; 287 [M+H]+ | Column: CHIRALPAK IC-3 (4.6 × 150 mm) Mobile phase: methyl tert-butyl ether/ ethanol/diethylamine (90/10/0.1) Flow rate: 0.5 mL/min Temperature: 25°C Analysis channel: PDA 247.0 nm Retention time (min.): 12.419 |
| 226 | 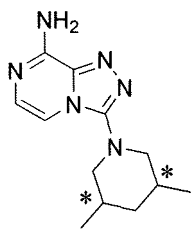 trans, single enantiomer | MS(APCI) m/z; 247 [M+H]+ | Column: CHIRALPAKIF-3 (4.6 × 150 mm) Mobile phase: ethanol/diethylamine (100/0.1) Flow rate: 0.5 mL/min Temperature: 25°C Analysis channel: PDA 254.0 nm Retention time (min.): 8.274 |

(continued)

| Ex. | Structural formula | Physical property etc. | Analysis conditions etc. |
|---|---|---|---|
| 227 | 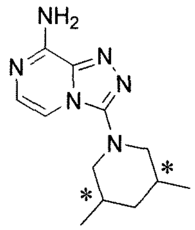<br>trans, single enantiomer opposite to Example 226 | MS(APCI)<br>m/z;<br>247 $[M+H]^+$ | Column: CHIRALPAKIF-3 (4.6 × 150 mm)<br>Mobile phase: ethanol/diethylamine (100/0.1)<br>Flow rate: 0.5 mL/min<br>Temperature: 25°C<br>Analysis channel: PDA 254.0 nm<br>Retention time (min.): 14.741 |
| 228 | 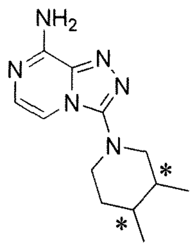<br>unknown relative configuration single enantiomer | MS (APCI)<br>m/z;<br>247 $[M+H]^+$ | Column: CHIRALPAKIF-3 (4.6 × 150 mm)<br>Mobile phase: methanol/acetonitrile/ diethylamine (95/5/0.1)<br>Flow rate: 0.5 mL/min<br>Temperature: 25°C<br>Analysis channel: PDA 303.0 nm<br>Retention time (min.): 9.605 |
| 229 | 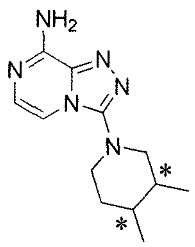<br>relative configuration different from Example 228 single enantiomer | MS (APCI)<br>m/z;<br>247 $[M+H]^+$ | Column: CHIRALPAKIF-3 (4.6 × 150 mm)<br>Mobile phase: methanol/acetonitrile/ diethylamine (95/5/0.1)<br>Flow rate: 0.5 mL/min<br>Temperature: 25°C<br>Analysis channel: PDA 303.0 nm<br>Retention time (min.): 12.473 |
| 230 | 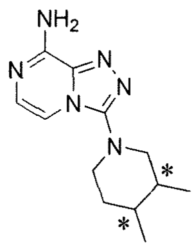<br>unknown relative configuration single enantiomer opposite to Example 229 | MS (APCI)<br>m/z;<br>247 $[M+H]^+$ | Column: CHIRALPAKIF-3 (4.6 × 150 mm)<br>Mobile phase: methanol/acetonitrile/ diethylamine (95/5/0.1)<br>Flow rate: 0.5 mL/min<br>Temperature: 25°C<br>Analysis channel: PDA 303.0 nm<br>Retention time (min.): 14.256 |
| 231 | 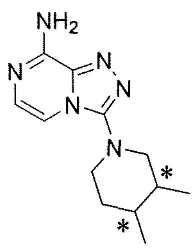<br>unknown relative configuration single enantiomer opposite to Example 228 | MS (APCI)<br>m/z;<br>247 $[M+H]^+$ | Column: CHIRALPAKIF-3 (4.6 × 150 mm)<br>Mobile phase: methanol/acetonitrile/ diethylamine (95/5/0.1)<br>Flow rate: 0.5 mL/min<br>Temperature: 25°C<br>Analysis channel: PDA 303.0 nm<br>Retention time (min.): 15.670 |

(continued)

| Ex. | Structural formula | Physical property etc. | Analysis conditions etc. |
|---|---|---|---|
| 232 | 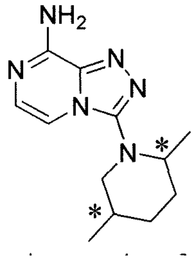<br>cis, single enantiomer | MS (APCI)<br>m/z;<br>247 [M+H]$^+$ | Column: CHIRALPAKIA-3 (4.6 × 150 mm)<br>Mobile phase: methyl tert-butyl ether/2-propanol/methanol/diethylamine (94/3/3/0.1)<br>Flow rate: 0.5 mL/min<br>Temperature: 25°C<br>Analysis channel: PDA 253.0 nm<br>Retention time (min.): 8.809 |
| 233 | 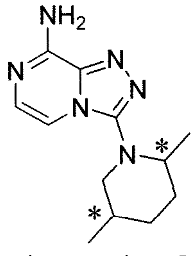<br>cis, single enantiomer opposite to Example 232 | MS(APCI)<br>m/z;<br>247 [M+H]$^+$ | Column: CHIRALPAKIA-3 (4.6 × 150 mm)<br>Mobile phase: methyl tert-butyl ether/2-propanol/methanol/diethylamine (94/3/3/0.1)<br>Flow rate: 0.5 mL/min<br>Temperature: 25°C<br>Analysis channel: PDA 253.0 nm<br>Retention time (min.): 12.744 |
| 234 | 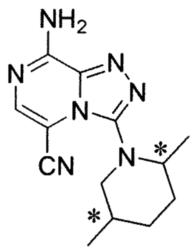<br>cis, single enantiomer | MS (APCI)<br>m/z;<br>272 [M+H]$^+$ | Column: CHIRALPAKIA-3 (4.6 × 150 mm)<br>Mobile phase: methyl tert-butyl ether/2-propanol/diethylamine (98/2/0.1)<br>Flow rate: 0.5 mL/min<br>Temperature: 25°C<br>Analysis channel: PDA 274.0 nm<br>Retention time (min.): 8.380 |
| 235 | 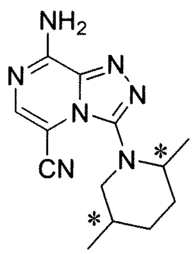<br>cis, single enantiomer opposite to Example 234 | MS (APCI)<br>m/z;<br>272 [M+H]$^+$ | Column: CHIRALPAKIA-3 (4.6 × 150 mm)<br>Mobile phase: methyl tert-butyl ether/2-propanol/diethylamine (98/2/0.1)<br>Flow rate: 0.5 mL/min<br>Temperature: 25°C<br>Analysis channel: PDA 274.0 nm<br>Retention time (min.): 11.863 |

(continued)

| Ex. | Structural formula | Physical property etc. | Analysis conditions etc. |
|---|---|---|---|
| 236 | 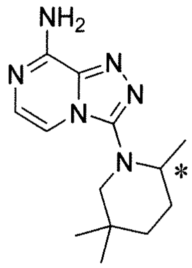 single enantiomer | MS(APCI) m/z; 269 [M+H]+ | Column: CHIRALPAK IC-3 (4.6 × 150 mm) Mobile phase: hexane/2-propanol/ diethylamine (40/60/0.1) Flow rate: 0.5 mL/min Temperature: 25°C Analysis channel: PDA 298.0 nm Retention time (min.): 10.294 |
| 237 | 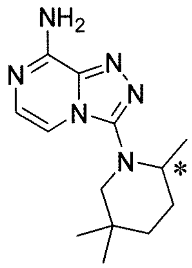 single enantiomer opposite to Example 236 | MS (APCI) m/z; 269 [M+H]+ | Column: CHIRALPAK IC-3 (4.6 × 150 mm) Mobile phase: hexane/2-propanol/ diethylamine (40/60/0.1) Flow rate: 0.5 mL/min Temperature: 25°C Analysis channel: PDA 298.0 nm Retention time (min.): 13.251 |
| 238 | 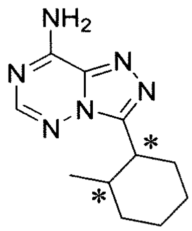 relative configuration is derived from Example 69, single enantiomer | MS (APCI) m/z; 233 [M+H]+ | Column: CHIRALPAKIF-3 (4.6 × 150 mm) Mobile phase: methanol/diethylamine (100/0.1) Flow rate: 0.5 mL/min Temperature: 25°C Analysis channel: PDA 280.0 nm Retention time (min.): 6.100 |
| 239 | 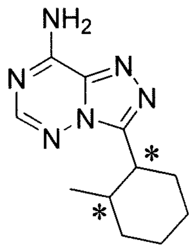 relative configuration is derived from Example 69, single enantiomer opposite to Example 238 | MS (APCI) m/z; 233 [M+H]+ | Column: CHIRALPAKIF-3 (4.6 × 150 mm) Mobile phase: methanol/diethylamine (100/0.1) Flow rate: 0.5 mL/min Temperature: 25°C Analysis channel: PDA 280.0 nm Retention time (min.): 10.114 |

(continued)

| Ex. | Structural formula | Physical property etc. | Analysis conditions etc. |
|---|---|---|---|
| 240 | 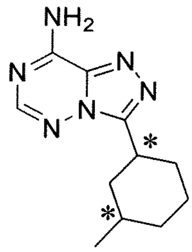 relative configuration is derived from Example 71, single enantiomer | MS(APCI) m/z; 233 [M+H]+ | Column: CHIRALPAKIF-3 (4.6 × 150 mm) Mobile phase: methanol/ tetrahydrofuran/diethylamine (90/10/0.1) Flow rate: 0.5 mL/min Temperature: 25°C Analysis channel: PDA 280.0 nm Retention time (min.): 7.634 |
| 241 | 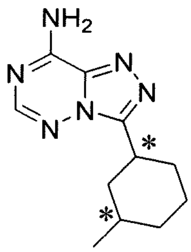 relative configuration is derived from Example 71, single enantiomer opposite to Example 240 | MS (APCI) m/z; 233 [M+H]+ | Column: CHIRALPAKIF-3 (4.6 × 150 mm) Mobile phase: methanol/ tetrahydrofuran/diethylamine (90/10/0.1) Flow rate: 0.5 mL/min Temperature: 25°C Analysis channel: PDA 280.0 nm Retention time (min.): 12.486 |
| 242 | 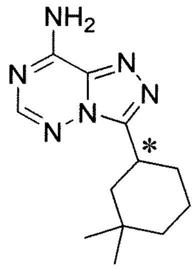 single enantiomer | MS(APCI) m/z; 247 [M+H]+ | Column: CHIRALPAKIA-3 (4.6 × 150 mm) Mobile phase: ethanol/methanol/ diethylamine (50/50/0.1) Flow rate: 0.5 mL/min Temperature: 25°C Analysis channel: PDA 280.0 nm Retention time (min.): 6.874 |
| 243 | 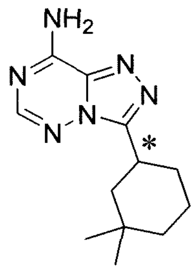 single enantiomer opposite to Example 242 | MS(APCI) m/z; 247 [M+H]+ | Column: CHIRALPAKIA-3 (4.6 × 150 mm) Mobile phase: ethanol/methanol/ diethylamine (50/50/0.1) Flow rate: 0.5 mL/min Temperature: 25°C Analysis channel: PDA 280.0 nm Retention time (min.): 11.479 |

(continued)

| Ex. | Structural formula | Physical property etc. | Analysis conditions etc. |
|---|---|---|---|
| 244 | 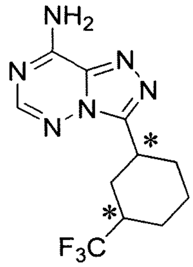 relative configuration is derived from Example 74, single enantiomer | MS (APCI) m/z; 287 $[M+H]^+$ | Column: CHIRALPAKIF-3 (4.6 × 150 mm) Mobile phase: methanol/ tetrahydrofuran/diethylamine (80/20/0.1) Flow rate: 0.5 mL/min Temperature: 25°C Analysis channel: PDA 280.0 nm Retention time (min.): 4.557 |
| 245 | 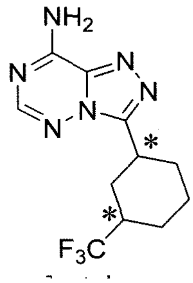 relative configuration is derived from Example 74, single enantiomer opposite to Example 244 | MS(APCI) m/z; 287 $[M+H]^+$ | Column: CHIRALPAKIF-3 (4.6 × 150 mm) Mobile phase: methanol/ tetrahydrofuran/diethylamine (80/20/0.1) Flow rate: 0.5 mL/min Temperature: 25°C Analysis channel: PDA 280.0 nm Retention time (min.): 9.403 |
| 246 | 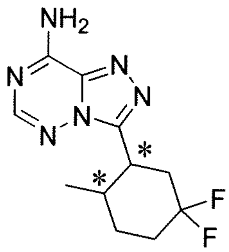 cis, single enantiomer | MS(APCI) m/z; 269 $[M+H]^+$ | Column: CHIRALPAKIA-3 (4.6 × 150 mm) Mobile phase: methanol/diethylamine (100/0.1) Flow rate: 0.5 mL/min Temperature: 25°C Analysis channel: PDA 280.0 nm Retention time (min.): 6.169 |
| 247 | 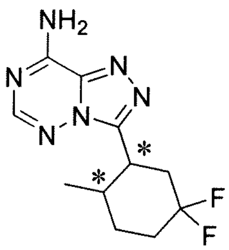 cis, single enantiomer opposite to Example 246 | MS(APCI) m/z; 269 $[M+H]^+$ | Column: CHIRALPAKIA-3 (4.6 × 150 mm) Mobile phase: methanol/diethylamine (100/0.1) Flow rate: 0.5 mL/min Temperature: 25°C Analysis channel: PDA 280.0 nm Retention time (min.): 11.554 |

Example 248

Preparation of 5-chloro-3-(3,3-dimethylpiperidin-1-yl)-[1,2,4]triazolo[4,3-a]pyrazin-8-amine

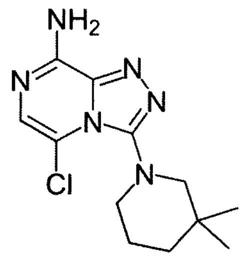

To a 10 mL cylindrical flask were added 3-(3,3-dimethylpiperidin-1-yl)-[1,2,4]triazolo[4,3-a]pyrazin-8-amine (50 mg) prepared in the Example 26, tetrahydrofuran (0.5 mL), and N-chlorosuccinimide (32 mg) under argon gas flow at room temperature, and the resulting mixture was stirred for 24 hours. After the reaction was completed, to the resulting reaction solution was added a saturated aqueous solution of sodium hydrogen carbonate, and the resulting mixture was extracted with dichloromethane. The resulting organic layer was washed with saturated brine, dried over anhydrous magnesium sulfate, and the resulting mixture was concentrated under reduced pressure. The resulting residues were subjected to silica gel column chromatography (dichloromethane: methanol = 98 : 2 to 92 : 8) using YAMAZEN medium pressure preparative (Silica M (16 g)), the fractions comprising the target compound (Rf value = 0.6 (dichloromethane : methanol = 10 : 1) were collected, and concentrated under reduced pressure to give a slightly yellow solid. To the resulting solid was added ethyl acetate, the resulting mixture was stirred for 30 minutes, and filtered to give the title compound (16 mg) (yield 28%) as a white solid.
MS(CI) m/z: 281 $[M+H]^+$

Example 249

Preparation of 3-(1-methylpiperidin-2-yl)-[1,2,4]triazolo[4,3-a]pyrazin-8-amine

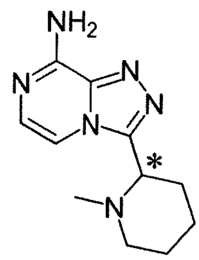

racemate

To a 20 mL cylindrical flask were added 3-(piperidin-2-yl)-[1,2,4]triazolo[4,3-a]pyrazin-8-amine trihydrochloride (66.9 mg) prepared in the Reference Example 249-1, acetonitrile (1 mL), methyl iodide (0.022 mL), and potassium carbonate (127 mg), and the resulting mixture was stirred at 50°C for 3 hours. After the reaction was completed, to the reaction solution was added water, the resulting solution was concentrated under reduced pressure, and the precipitated solid was filtered. The resulting solid was washed with water to give the title compound (13.5 mg) (yield 25%) as a colorless solid.
MS(CI) m/z: 233 $[M+H]^+$

Example 250

Preparation of 3-(1-(pyrimidin-2-yl)piperidin-2-yl)-[1,2,4]triazolo[4,3-a]pyrazin-8-amine

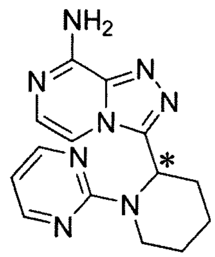

racemate

To a 20 mL cylindrical flask were added 3-(piperidin-2-yl)-[1,2,4]triazolo[4,3-a]pyrazin-8-amine trihydrochloride (0.29 g) prepared in the Reference Example 249-1, potassium carbonate (0.21 g), and dimethyl sulfoxide (DMSO) (1 mL), the resulting mixture was stirred at room temperature for 1 hour, and then filtered.

The resulting filtrate was added to a 20 mL cylindrical flask, 2-chloropyrimidine (0.14 g) and diisopropylethylamine (0.39 g) were added thereto, and the resulting mixture was stirred under heating at 140°C for 8 hours. After the reaction was completed, to the resulting reaction solution was added water (10 mL), and the precipitated solid was collected by filtration. The resulting solid was washed with a mixed solution of dichloromethane and methanol (dichloromethane : methanol = 9 : 1) to give the title compound (56.2 mg) (yield 19%) as a pale brown solid.
MS(CI) m/z: 297 $[M+H]^+$

Example 251

Preparation of methyl 2-(8-amino-[1,2,4]triazolo[4,3-a]pyrazin-3-yl)-piperidine-1-carboxylate

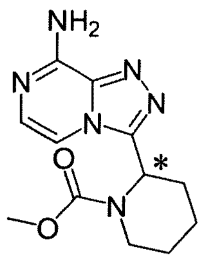

racemate

To a 20 mL cylindrical flask were added 3-(piperidin-2-yl)-[1,2,4]triazolo[4,3-a]pyrazin-8-amine trihydrochloride (58.2 mg) prepared in the Reference Example 249-1, triethylamine (0.14 mL), dichloromethane (2 mL), and methyl chloroformate (23.18 mg), and the resulting mixture was stirred at room temperature for 3 hours. After the reaction was completed, to the resulting reaction solution were added water and methanol, the resulting mixture was concentrated under reduced pressure, and the precipitated solid was filtered. The resulting solid was washed with water and ethanol to give the title compound (22.6 mg) (yield 41%) as a colorless solid.
MS(CI) m/z: 277 $[M+H]^+$

Example 252

Preparation of 3-(3,3-dimethylpiperidin-1-yl)-5-(trifluoromethyl)-[1,2,4]triazolo[4,3-a]pyrazin-8-amine

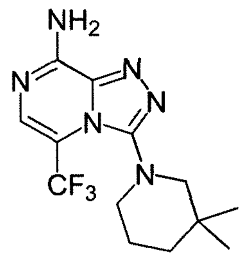

(1) To a 10 mL cylindrical flask was added 3-(3,3-dimethylpiperidin-1-yl)-8-methoxy-5-(trifluoromethyl)-[1,2,4]triazolo[4,3-a]pyrazine (38 mg) prepared in the Reference Example 252-1, phosphorus oxychloride (500 μL) was added thereto under argon gas flow at room temperature with stirring, and the resulting mixture was stirred at 130°C for 5 hours.
After the reaction was completed, to the resulting reaction solution was added a saturated aqueous solution of sodium hydrogen carbonate, and the resulting mixture was extracted with ethyl acetate. The resulting organic layer was washed with a saturated aqueous solution of sodium chloride, dried over anhydrous sodium sulfate, and concentrated under reduced pressure to give a crude product of 8-chloro-3-(3,3-dimethylpiperidin-1-yl)-5-(trifluoromethyl)-[1,2,4]triazolo[4,3-a]pyrazine (58 mg) as a brown oil.
(2) To a 0.5 to 2 mL cylindrical flask for microwave were added 8-chloro-3-(3,3-dimethylpiperidin-1-yl)-5-(trifluoromethyl)-[1,2,4]triazolo[4,3-a]pyrazine (58 mg) prepared in the above (1) and 2-propanol (1 mL), and ammonium hydroxide (180 mg) was added thereto at room temperature. Said mixture was stirred under microwave radiation at 100°C for 1 hour. After the reaction was completed, to the reaction solution was added water, and the mixed solution was extracted with ethyl acetate. The resulting organic layer was washed with saturated brine, dried over anhydrous sodium sulfate, filtered, and the resulting mixture was concentrated under reduced pressure. The resulting residues were subjected to silica gel column chromatography (hexane : ethyl acetate = 67 : 33 to 46 : 54) using YAMAZEN medium pressure preparative (Silica (16 g)), the fractions comprising the target compound (Rf value = 0.45 (hexane : ethyl acetate = 1 : 1)) were collected, and concentrated under reduced pressure. To the resulting residues was added hexane, and the resulting mixture was filtered to give the title compound (5 mg) (yield 9%) as a white solid.
MS(CI) m/z: 315 $[M+H]^+$

Example 253

Preparation of 3-(cyclohexyl)-isoxazolo[4,3-d]pyrimidin-8-amine

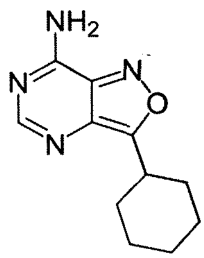

To a 30 mL cylindrical flask were added 3-(cyclohexyl)-isoxazolo[4,3-d]pyrimidin-7-ol (54.6 mg) prepared in the Reference Example 253-1 and phosphorus oxychloride (11.6 mL), and the resulting mixture was stirred at 100°C for 10 hours. After the reaction was completed, the resulting reaction solution was added dropwise to a 14% aqueous ammonia so that the temperature would not exceed 25°C, and the resulting mixture was extracted with ethyl acetate. The resulting organic layer was washed with a saturated aqueous solution of sodium chloride, dried over anhydrous magnesium sulfate, and concentrated under reduced pressure. The resulting residues were subjected to silica gel column chromatography (dichloromethane : methanol = 100 : 0 to 95 : 5) using Moritex medium pressure preparative (Purif-Pack SI size 20 (10 g)), and the fractions comprising the target compound were concentrated under reduced pressure. To the precipitated solid was added ethyl acetate, the resulting mixture was filtered, and washed with ethyl acetate to give the title compound (9.8 mg) (yield 18%) as a colorless solid.

MS(CI) m/z: 219 [M+H]$^+$

(Reference Examples)

Next, Reference Examples are described.

Reference Example 4-2

Preparation of 6-chloro-N$^4$-cyclohexyl-2-methylpyrimidine-4,5-diamine

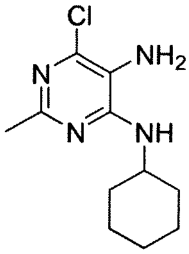

A mixture of 5-amino-4,6-dichloro-2-methylpyrimidine (1.0 g), cyclohexylamine (770 μL), N,N-diisopropylethylamine (1.2 mL), and N-methylpyrrolidone (5 mL) was stirred at 120°C overnight. To the reaction mixture were additionally added cyclohexylamine (770 μL) and N,N-diisopropylethylamine (1.2 mL), and the resulting mixture was stirred at 120°C overnight. The reaction mixture was allowed to cool to room temperature, water was added thereto, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 90/10 to 70/30) to give the title compound (1.35 g) (yield 100%) as a brown powder. MS(APCI) m/z: 241/243 [M+H]$^+$

Reference Example 1-2 etc.:

A corresponding starting compound was treated in a similar manner to the Reference Example 4-2 to give each compound described in the following Table 14.

Table 14

| Reference Example | Structural formula | Physical property etc. |
|---|---|---|
| 3-2 | 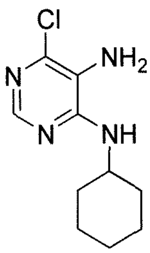 | MS(ESI) m/z; 227/229 [M+H]$^+$ |
| 5-3 | 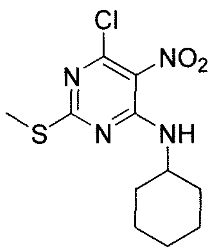 | MS(APCI) m/z; 303/305 [M+H]$^+$ |

(continued)

| Reference Example | Structural formula | Physical property etc. |
|---|---|---|
| 6-2 | 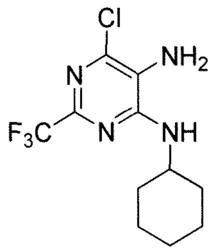 | MS(APCI) m/z; 295/297 [M+H]$^+$ |
| 7-2 | 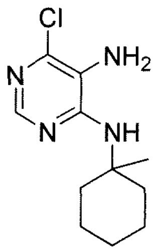 | MS(APCI) m/z; 241/243 [M+H]$^+$ |
| 1-2 | 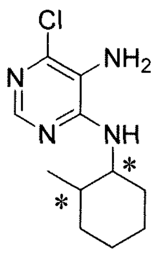 trans, racemate | MS(APCI) m/z; 241/243 [M+H]$^+$ |
| 112-4 | 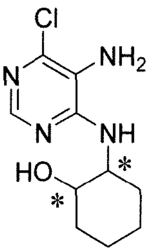 cis, racemate | MS(APCI) m/z; 243/245 [M+H]$^+$ |
| 116-4 | 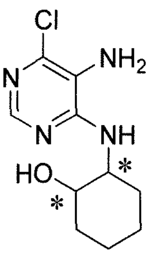 trans, racemate | MS(APCI) m/z; 243/245 [M+H]$^+$ |

(continued)

| Reference Example | Structural formula | Physical property etc. |
|---|---|---|
| 117-6 | 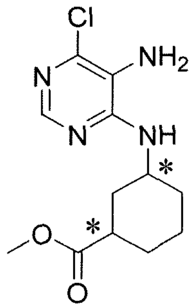<br>cis, racemate | MS(ESI) m/z; 285/287 [M+H]+ |
| 128-3 | 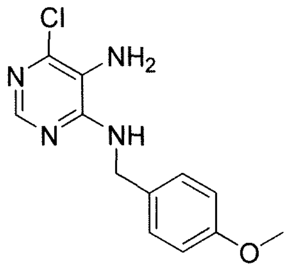 | MS(ESI) m/z; 265/267 [M+H]+ |
| 87-2 | 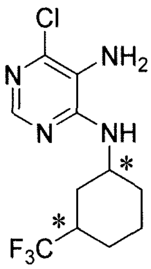<br>cis, racemate | MS(ESI) m/z; 295/297 [M+H]+ |
| 86-2 | 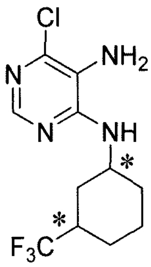<br>trans, racemate | MS(ESI) m/z; 295/297 [M+H]+ |
| 88-2 | 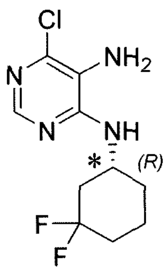 | MS(ESI) m/z; 263/265 [M+H]+ |

(continued)

| Reference Example | Structural formula | Physical property etc. |
|---|---|---|
| 89-2 | 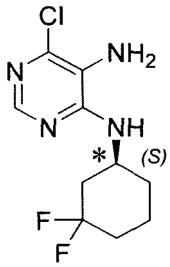 | MS(ESI) m/z; 263/265 [M+H]$^+$ |
| 121-4 | 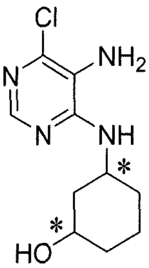 mixture of four types of stereoisomers | MS(ESI) m/z; 243/245 [M+H]$^+$ |
| 8-2 | 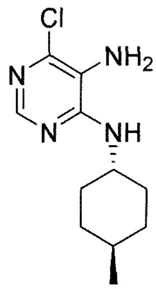 trans | MS(ESI) m/z; 241/243 [M+H]$^+$ |
| 9-2 | 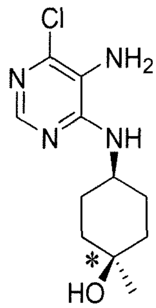 cis | MS(ESI) m/z; 257/259 [M+H]$^+$ |
| 10-2 | 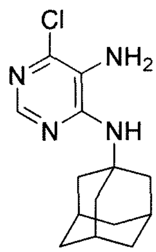 | MS(APCI) m/z; 279/281 [M+H]$^+$ |

(continued)

| Reference Example | Structural formula | Physical property etc. |
|---|---|---|
| 90-2 | 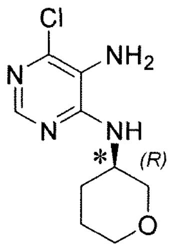 | MS(ESI) m/z; 229/231 [M+H]$^+$ |
| 91-2 | 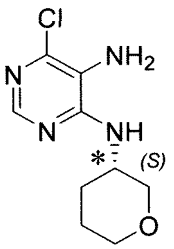 | MS(ESI) m/z; 229/231 [M+H]$^+$ |
| 11-2 | 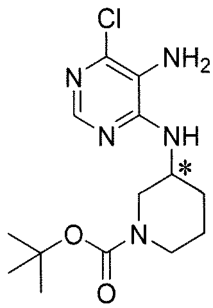 racemate | MS(APCI) m/z; 328/330 [M+H]$^+$ |
| 12-2 | 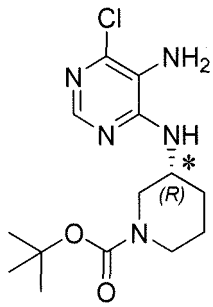 | MS(ESI) m/z; 328/330 [M+H]$^+$ |
| 13-2 | 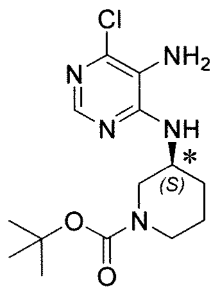 | MS(APCI) m/z; 328/330 [M+H]$^+$ |

(continued)

| Reference Example | Structural formula | Physical property etc. |
|---|---|---|
| 15-2 | 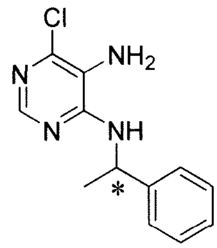 racemate | MS(ESI) m/z; 249/251 $[M+H]^+$ |
| 16-2 | 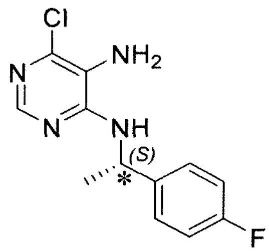 | MS(ESI) m/z; 267/269 $[M+H]^+$ |
| 33-2 | 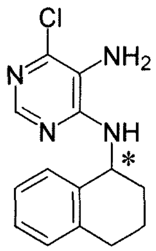 racemate | MS(CI) m/z; 275/277 $[M+H]^+$ |
| 34-2 | 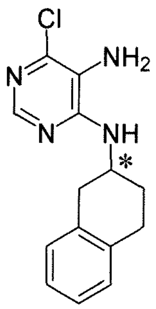 racemate | MS(CI) m/z; 275/277 $[M+H]^+$ |
| 35-2 | 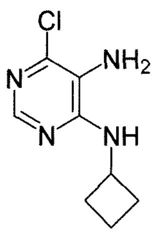 | MS(CI) m/z; 199/201 $[M+H]^+$ |

Reference Example 14-2

Preparation of 6-chloro-$N^4$-(2,6-difluorophenyl)pyrimidine-4,5-diamine

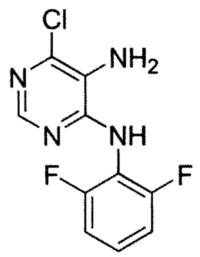

A mixture of 5-amino-4,6-dichloropyrimidine (500 mg), 2,6-difluoroaniline (1.54 mL), and N-methylpyrrolidone (1 mL) was stirred under microwave radiation at 150°C for 2 hours, and stirred at 180°C for 3 hours. The reaction mixture was allowed to cool to room temperature, water was added thereto, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 80/20 to 50/50) to give the title compound (359 g) (yield 46%) as a yellow powder. MS(APCI) m/z: 257/259 $[M+H]^+$

Reference Example 5-2

Preparation of 6-chloro-$N^4$-cyclohexyl-2-(methylsulfanyl)pyrimidine-4,5-diamine

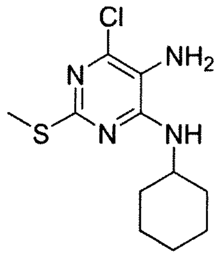

A mixture of 6-chloro-N-cyclohexyl-2-(methylsulfanyl)-5-nitropyrimidin-4-amine (910 mg) prepared in the Reference Example 5-3, tin(II) chloride dihydrate (2.71 g), and ethanol (15 mL) was stirred with heating under reflux for 2 hours. The reaction mixture was allowed to cool to room temperature, and concentrated under reduced pressure. To the resulting residues were added a saturated aqueous solution of sodium hydrogen carbonate and ethyl acetate to separate them, and the resulting aqueous layer was extracted with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 95/5 to 80/20) to give the title compound (510 mg) (yield 62%) as an orange oil. MS(APCI) m/z: 273/275 $[M+H]^+$

Reference Example 6-1

Preparation of 7-chloro-3-cyclohexyl-5-(trifluoromethyl)-3H-[1,2,3]triazolo[4,5-d]pyrimidine

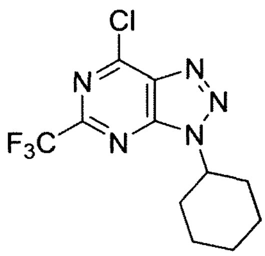

To a solution of 6-chloro-$N^4$-cyclohexyl-2-(trifluoromethyl)pyrimidine-4,5-diamine (288 mg) prepared in the Reference Example 6-2, acetic acid (2 mL), and dichloromethane (2 mL) was added dropwise an aqueous solution (0.4 mL) comprising sodium nitrite (87 mg) under ice-cooling, and the resulting mixture was stirred for 1 hour. The reaction mixture was added dropwise to a saturated aqueous solution of sodium hydrogen carbonate under ice-cooling. The resulting mixture was extracted twice with chloroform, the resulting organic layers were combined, washed with saturated brine, dried over anhydrous magnesium sulfate, and the insoluble matters were removed by filtration. The resulting filtrate was concentrated under reduced pressure to give the title compound (282 mg) (yield 94%) as a brown powder. MS(APCI) m/z: 306/308 $[M+H]^+$

Reference Example 1-1 etc.:

A corresponding starting compound was treated in a similar manner to the Reference Example 6-1 to give each compound described in the following Table 15.

Table 15

| Reference Example | Structural formula | Physical property etc. |
|---|---|---|
| 3-1 | 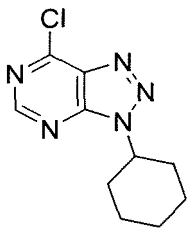 | MS(ESI) m/z; 238/240 $[M+H]^+$ |
| 4-1 | 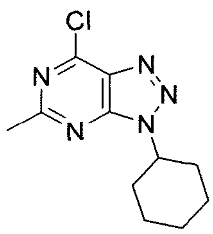 | MS(APCI) m/z; 252/254 $[M+H]^+$ |
| 5-1 | 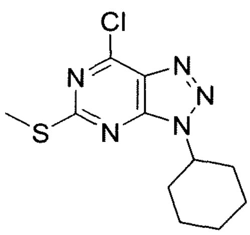 | MS(APCI) m/z; 284/286 $[M+H]^+$ |

(continued)

| Reference Example | Structural formula | Physical property etc. |
|---|---|---|
| 7-1 | 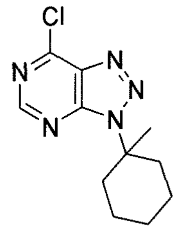 | MS(APCI) m/z; 252/254 [M+H]+ |
| 1-1 | 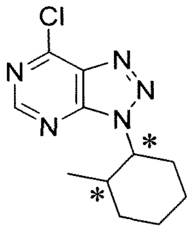 trans, racemate | MS(APCI) m/z; 252/254 [M+H]+ |
| 112-3 | 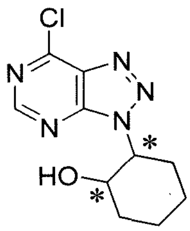 cis, racemate | MS(APCI) m/z; 254/256 [M+H]+ |
| 116-3 | 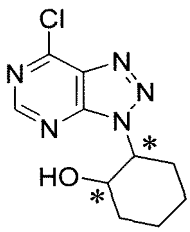 trans, racemate | MS(APCI) m/z; 254/256 [M+H]+ |
| 117-5 | 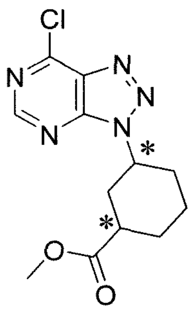 cis, racemate | MS(ESI) m/z; 296/298 [M+H]+ |
| 128-2 | 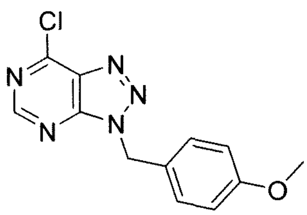 | MS(ESI) m/z; 276/278 [M+H]+ |

(continued)

| Reference Example | Structural formula | Physical property etc. |
|---|---|---|
| 8-1 | 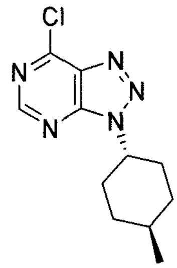 trans | MS(ESI) m/z; 252/254 [M+H]$^+$ |
| 9-1 | 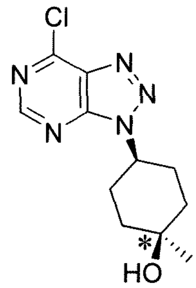 cis | MS(ESI) m/z; 268/270 [M+H]$^+$ |
| 10-1 | 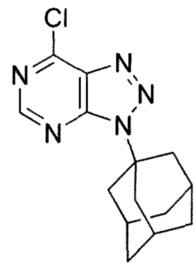 | MS(APCI) m/z; 290/292 [M+H]$^+$ |
| 11-1 | 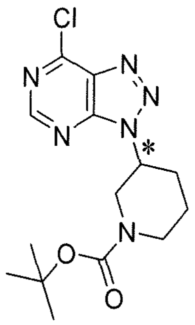 racemate | MS(APCI) m/z; 339/341 [M+H]$^+$ |
| 12-1 | 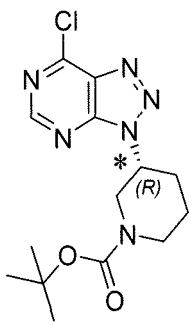 | MS(ESI) m/z; 283/285 [M+2H-tBu]$^+$ |

(continued)

| Reference Example | Structural formula | Physical property etc. |
|---|---|---|
| 13-1 | 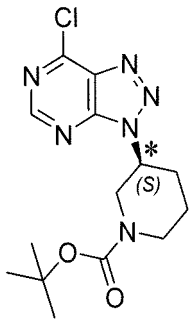 | MS(APCI) m/z; 339/341 $[M+H]^+$ |
| 14-1 | 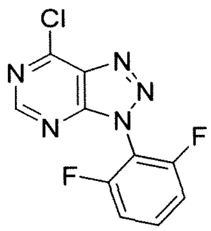 | MS(APCI) m/z; 268/270 $[M+H]^+$ |
| 15-1 | 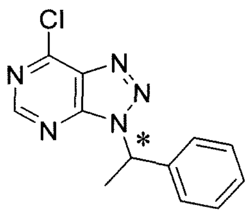 racemate | MS(ESI) m/z; 260/262 $[M+H]^+$ |
| 16-1 | 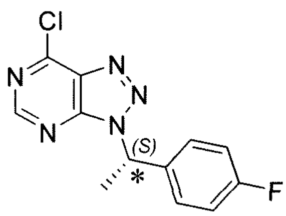 | MS(ESI) m/z; 278/280 $[M+H]^+$ |
| 33-1 | 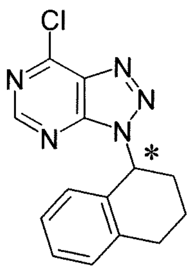 racemate | MS(CI) m/z; 286/288 $[M+H]^+$ |

(continued)

| Reference Example | Structural formula | Physical property etc. |
|---|---|---|
| 34-1 | 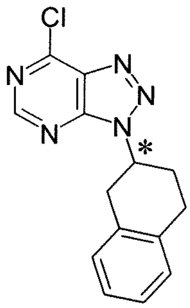 racemate | MS(CI) m/z; 286/288 [M+H]$^+$ |
| 35-1 | 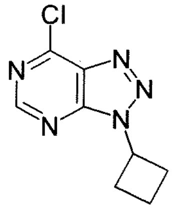 | MS(CI) m/z; 210/212 [M+H]$^+$ |

Reference Example 93-1

Preparation of 3-cyclohexyl-5-(methylsulfinyl)-3H-[1,2,3]triazolo[4,5-d]pyrimidin-7-amine

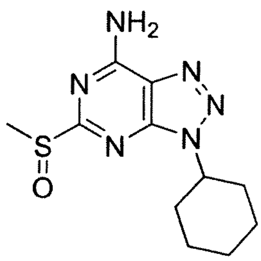

To a solution of 3-cyclohexyl-5-(methylsulfanyl)-3H-[1,2,3]triazolo[4,5-d]pyrimidin-7-amine (427 mg) prepared in the Example 5 in dichloromethane (20 mL) was added m-chloroperbenzoic acid (wetted with ca. 30% water) (444 mg) under ice-cooling, and the resulting mixture was stirred under ice-cooling for 2 hours. To the reaction mixture was added a saturated aqueous solution of sodium hydrogen carbonate, and the resulting mixture was extracted twice with chloroform. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 30/70 to 0/100 to solvent: ethyl acetate/methanol = 90/10) to give the title compound (242 mg) (yield 53%) as a pale yellow powder.
MS(APCI) m/z: 281 [M+H]$^+$

Reference Example 112-2

Preparation of cis-2-{7-[bis(2,4-dimethoxybenzyl)amino]-3H-[1,2,3]triazolo[4,5-d]pyrimidin-3-yl}cyclohexanol

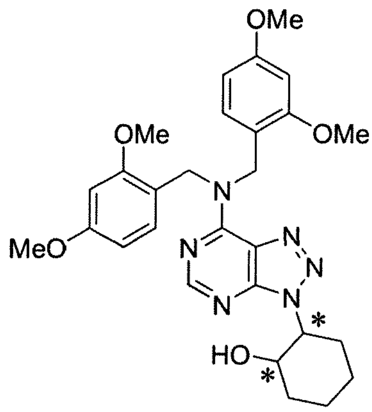

cis, racemate

A mixture of cis-2-(7-chloro-3H-[1,2,3]triazolo[4,5-d]pyrimidin-3-yl)cyclohexanol (550 mg) prepared in the Reference Example 112-3, bis(2,4-dimethoxybenzyl)amine (826 mg), N,N-diisopropylethylamine (0.755 mL), and tetrahydrofuran (7 mL) was stirred at room temperature overnight. To the reaction mixture was added a 20% aqueous solution of citric acid, and the resulting mixture was extracted twice with ethyl acetate. The resulting organic layers were combined, washed sequentially with a saturated aqueous solution of sodium hydrogen carbonate and saturated brine, dried over anhydrous magnesium sulfate, and the insoluble matters were removed by filtration. The resulting filtrate was concentrated under reduced pressure to give the title compound (1.19 g) as a pale yellow powder. MS(APCI) m/z: 535 $[M+H]^+$

Reference Example 116-2 etc.:

A corresponding starting compound was treated in a similar manner to the Reference Example 112-2 to give each compound described in the following Table 16.

Table 16

| Reference Example | Structural formula | Physical property etc. |
|---|---|---|
| 116-2 | 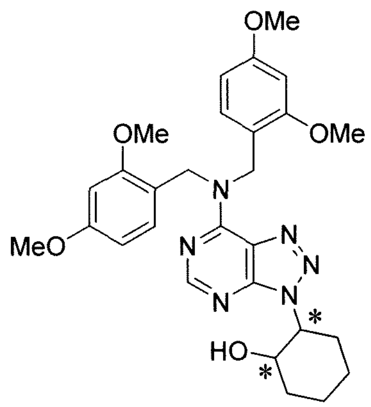 trans, racemate | MS(APCI) m/z; 535 $[M+H]^+$ |

(continued)

| Reference Example | Structural formula | Physical property etc. |
|---|---|---|
| 117-4 | 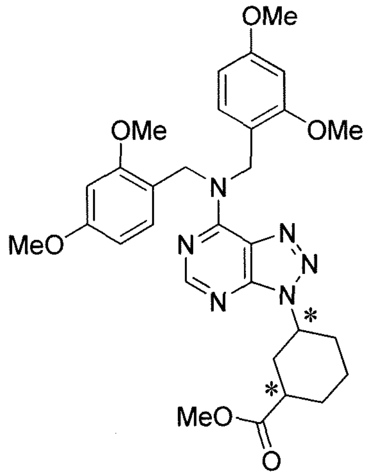 cis, racemate | MS(ESI) m/z; 577 $[M+H]^+$ |

Reference Example 112-1

Preparation of N,N-bis(2,4-dimethoxybenzyl)-3-[trans-2-fluorocyclohexyl]-3H-[1,2,3]triazolo[4,5-d]pyrimidin-7-amine

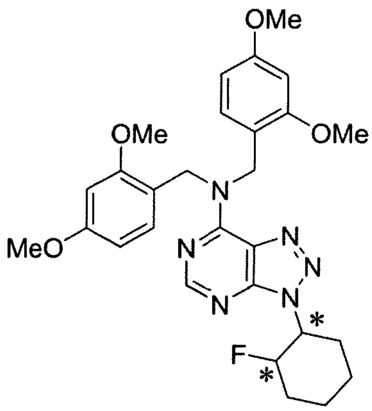

trans, racemate

To a solution of cis-2-{7-[bis(2,4-dimethoxybenzyl)amino]-3H-[1,2,3]triazolo[4,5-d]pyrimidin-3-yl}cyclohexanol (600 mg) prepared in the Reference Example 112-2 in dichloromethane (10 mL) was added (diethylamino)sulfur trifluoride (0.222 mL), and the resulting mixture was stirred at room temperature for 5 hours and 30 minutes. To the reaction mixture was additionally added (diethylamino)sulfur trifluoride (0.222 mL), and the resulting mixture was stirred at room temperature overnight. To the reaction mixture was added water, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 80/20 to 60/40) to give the title compound (189 mg) (yield 31%) as a colorless powder.
MS(APCI) m/z: 537 $[M+H]^+$

Reference Example 113-2

Preparation of 2-{7-[bis(2,4-dimethoxybenzyl)amino]-3H-[1,2,3]triazolo[4,5-d]pyrimidin-3-yl}cyclohexanone

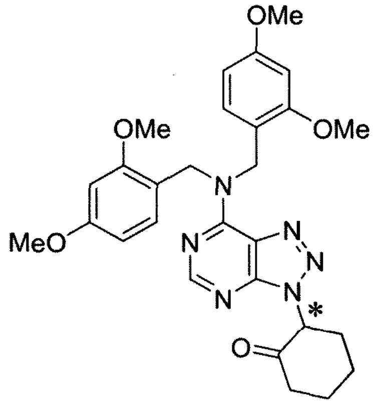

racemate

To a solution of cis-2-{7-[bis(2,4-dimethoxybenzyl)amino]-3H-[1,2,3]triazolo[4,5-d]pyrimidin-3-yl}cyclohexanol (200 mg) prepared in the Reference Example 112-2 in dichloromethane (8 mL) was added 1,1,1-triacetoxy-1,1-dihydro-1,2-benziodoxol-3-(1H)-one (476 mg), and the resulting mixture was stirred at room temperature for 3 hours. To the reaction mixture was added a 1 mol/L aqueous solution of sodium hydroxide, the resulting mixture was stirred at room temperature for 20 minutes, and then extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 70/30 to 40/60) to give the title compound (196 mg) (yield 98%) as a colorless oil. MS(APCI) m/z: 533 $[M+H]^+$

Reference Example 113-1

Preparation of 3-(2,2-difluorocyclohexyl)-N,N-bis(2,4-dimethoxybenzyl)-3H-[1,2,3]triazolo[4,5-d]pyrimidin-7-amine

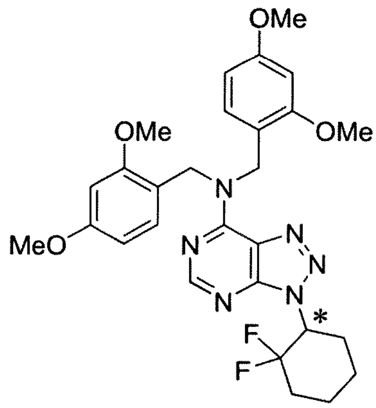

racemate

2-{7-[Bis(2,4-dimethoxybenzyl)amino]-3H-[1,2,3]triazolo[4,5-d]pyrimidin-3-yl}cyclohexanone prepared in the Reference Example 113-2 was reacted in a similar manner to the Reference Example 112-1 to give the title compound. MS(APCI) m/z: 555 $[M+H]^+$

Reference Example 114-1 etc.:

3-(2,2-Difluorocyclohexyl)-N,N-bis(2,4-dimethoxybenzyl)-3H-[1,2,3]triazolo[4,5-d]pyrimidin-7-amine prepared in the Reference Example 113-1 was optically resolved by chiral HPLC to give each compound described in the following Table 17.

Table 17

| Ref. Ex. | Structural formula | Physical property etc. | Analysis conditions etc. |
|---|---|---|---|
| 114-1 | 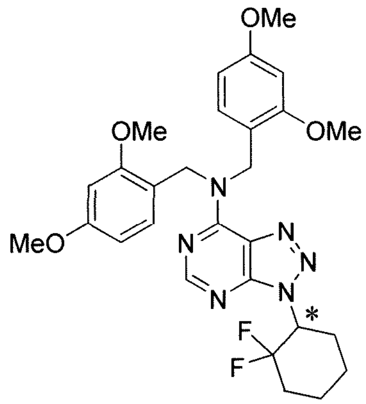 single enantiomer | MS(APCI) m/z; 555 [M+H]+ | Column: CHIRALPAK IF-3 (4.6 × 150 mm)<br>Mobile phase: methanol/acetonitrile/ diethylamine (95/5/0.1)<br>Flow rate: 0.5 mL/min<br>Temperature: 25°C<br>Analysis channel: PDA 298.0 nm<br>Retention time (min.): 8.332 |
| 115-1 | 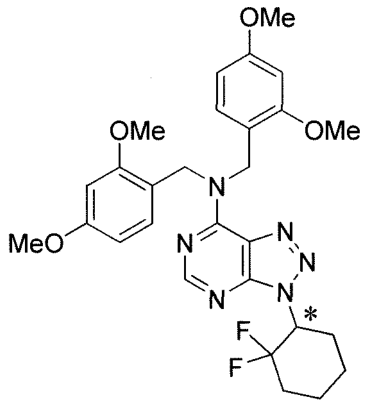 single enantiomer opposite to Reference Example 114-1 | MS (APCI) m/z; 555 [M+H]+ | Column: CHIRALPAK IF-3 (4.6 × 150 mm)<br>Mobile phase: methanol/acetonitrile/ diethylamine (95/5/0.1)<br>Flow rate: 0.5 mL/min<br>Temperature: 25°C<br>Analysis channel: PDA 298.0 nm<br>Retention time (min.): 12.122 |

Reference Example 111-1

Preparation of N,N-bis(2,4-dimethoxybenzyl)-3-[cis-2-methoxycyclohexyl]-3H-[1,2,3]triazolo[4,5-d]pyrimidin-7-amine

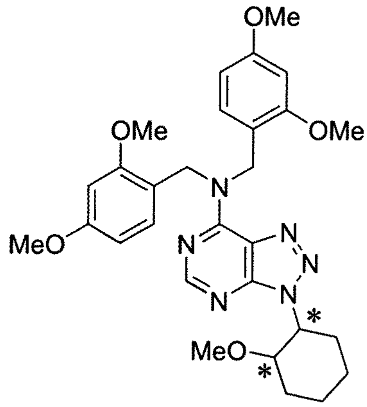

cis, racemate

To a solution of cis-2-{7-[bis(2,4-dimethoxybenzyl)amino]-3H-[1,2,3]triazolo[4,5-d]pyrimidin-3-yl}cyclohexanol (224 mg) prepared in the Reference Example 112-2 in tetrahydrofuran (5 mL) was added sodium hydride (60%) (20.1 mg) under ice-cooling, the resulting mixture was stirred for 5 minutes, then methyl iodide (0.031 mL) was added thereto, and the resulting mixture was stirred at room temperature overnight. To the reaction mixture were additionally added sodium hydride (60%) (20.1 mg) and methyl iodide (0.031 mL), and the resulting mixture was stirred for 2 hours and 30 minutes. To the reaction mixture was added water, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure to give the title compound (240 mg) (yield 104%) as a pale yellow oil. MS(APCI) m/z: 549 $[M+H]^+$

Reference Example 116-1

Preparation of N,N-bis(2,4-dimethoxybenzyl)-3-[trans-2-methoxycyclohexyl]-3H-[1,2,3]triazolo[4,5-d]pyrimidin-7-amine

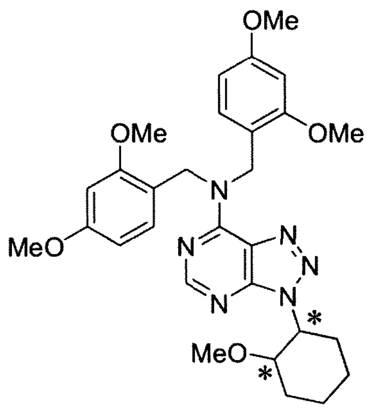

trans, racemate

Trans-2-{7-[bis(2,4-dimethoxybenzyl)amino]-3H-[1,2,3]triazolo[4,5-d]pyrimidin-3-yl}cyclohexanol prepared in the Reference Example 116-2 was reacted in a similar manner to the Reference Example 111-1 to give the title compound. MS(APCI) m/z: 549 $[M+H]^+$

Reference Example 117-3

Preparation of [cis-3-{7-[bis(2,4-dimethoxybenzyl)amino]-3H-[1,2,3]triazolo[4,5-d]pyrimidin-3-yl}cyclohexyl]methanol

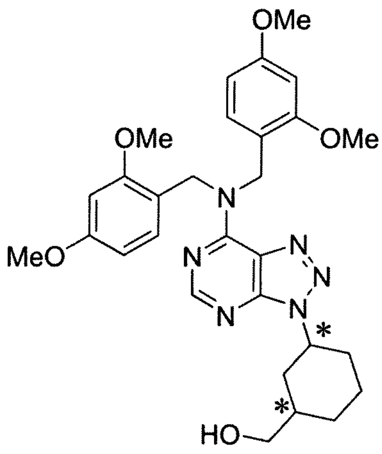

cis, racemate

A solution of methyl cis-3-{7-[bis(2,4-dimethoxybenzyl)amino]-3H-[1,2,3]triazolo[4,5-d]pyrimidin-3-yl}cyclohexanecarboxylate (8.88 g) prepared in the Reference Example 117-4 in dichloromethane (75 mL) was subjected to nitrogen replacement, and then diisobutylaluminium hydride (1.0 mol/L solution in toluene) (45 mL) was added dropwise thereto under ice-cooling over 15 minutes. The reaction mixture was stirred for 2 hours with gradually warming to room temperature. To the reaction mixture was added an aqueous solution of potassium sodium tartrate, the resulting mixture was stirred overnight, and then extracted twice with ethyl acetate. The resulting organic layers were combined, washed with saturated brine, dried over anhydrous sodium sulfate, and the insoluble matters were removed by filtration. The resulting filtrate was concentrated under reduced pressure, and the resulting residues were purified by silica gel column chromatography (solvent: ethyl acetate/methanol = 100/0 to 85/15) to give the title compound (7.03 g) (yield 86%).
MS(ESI) m/z: 549 $[M+H]^+$

Reference Example 117-2

Preparation of cis-3-{7-[bis(2,4-dimethoxybenzyl)amino]-3H-[1,2,3]triazolo[4,5-d]pyrimidin-3-yl}cyclohexanecarbaldehyde

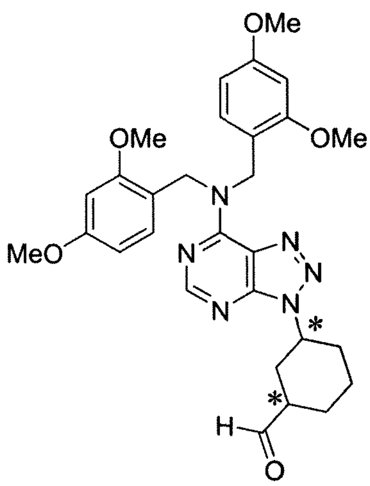

cis, racemate

To a 300 mL eggplant flask were added [cis-3-{7-[bis(2,4-dimethoxybenzyl)amino]-3H-[1,2,3]triazolo[4,5-d]pyrimidin-3-yl}cyclohexyl]methanol (5.49 g) prepared in the Reference Example 117-3, tetrakis(acetonitrile)copper(I) hexafluorophosphate (187 mg), 2,2'-bipyridine (78.6 mg), 2,2,6,6-tetramethylpiperidin-1-oxyl free radical (81.6 mg), 1-meth-

ylimidazole (78.9 μL), and acetonitrile (25 mL), and the resulting mixture was stirred at room temperature overnight. The reaction solution was concentrated under reduced pressure, and the resulting residues were purified by silica gel column chromatography (solvent: hexane/ethyl acetate = 50/50 to 0/100) to give the title compound (3.72 g) (yield 68%) as a pale yellow amorphous.
MS(ESI) m/z: 547 $[M+H]^+$

Reference Example 117-1

Preparation of N,N-bis(2,4-dimethoxybenzyl)-3-[cis-3-ethenylcyclohexyl]-3H-[1,2,3]triazolo[4,5-d]pyrimidin-7-amine

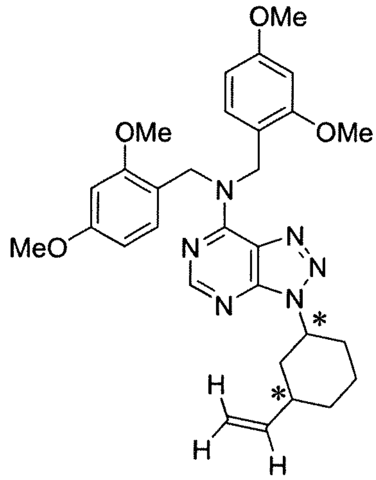

cis, racemate

To a 25 mL eggplant flask were added methyltriphenylphosphonium bromide (300 mg), potassium tert-butoxide (91 mg), and toluene (2 mL), the resulting mixture was subjected to nitrogen atmosphere, and stirred at room temperature for 30 minutes. To the reaction mixture was added a solution of cis-3-{7-[bis(2,4-dimethoxybenzyl)amino]-3H-[1,2,3]triazolo[4,5-d]pyrimidin-3-yl}cyclohexanecarbaldehyde (230 mg) prepared in the Reference Example 117-2 in tetrahydrofuran (8.2 mL), and the resulting mixture was stirred at room temperature for 30 minutes. To the reaction mixture was added a saturated aqueous solution of ammonium chloride, and the resulting mixture was extracted twice with ethyl acetate. The resulting organic layers were combined, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 80/20 to 50/50) to give the title compound (102 mg) (yield 45%) as a colorless amorphous.
MS(ESI) m/z: 545 $[M+H]^+$

Reference Example 118-2

Preparation of [cis-3-{7-[bis(2,4-dimethoxybenzyl)amino]-3H-[1,2,3]triazolo[4,5-d]pyrimidin-3-yl}cyclohexyl]methyl methanesulfonate

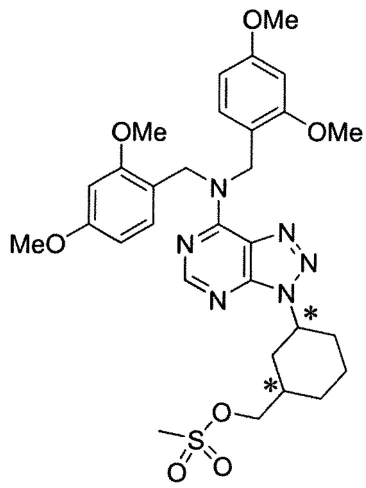

cis, racemate

To a 25 mL eggplant flask were added [cis-3-{7-[bis(2,4-dimethoxybenzyl)amino]-3H-[1,2,3]triazolo[4,5-d]pyrimidin-3-yl}cyclohexyl]methanol (308 mg) prepared in the Reference Example 117-3, ethyl acetate (2.8 mL), and triethylamine (156 μL), and the resulting mixture was cooled to 0°C in an ice bath. To the mixture was added methanesulfonyl chloride (65.4 μL), and the resulting mixture was stirred for 30 minutes. To the reaction mixture was added a saturated aqueous solution of sodium hydrogen carbonate, and the resulting mixture was extracted three times with ethyl acetate. The resulting organic layers were combined, dried over anhydrous sodium sulfate, and silica gel was added thereto. The insoluble matters were removed by filtration, and the resulting filtrate was concentrated under reduced pressure to give the title compound (363 mg) as a colorless amorphous.
MS(ESI) m/z: 627 $[M+H]^+$

Reference Example 118-1

Preparation of N,N-bis(2,4-dimethoxybenzyl)-3-[cis-3-(fluoromethyl)cyclohexyl]-3H-[1,2,3]triazolo[4,5-d]pyrimidin-7-amine

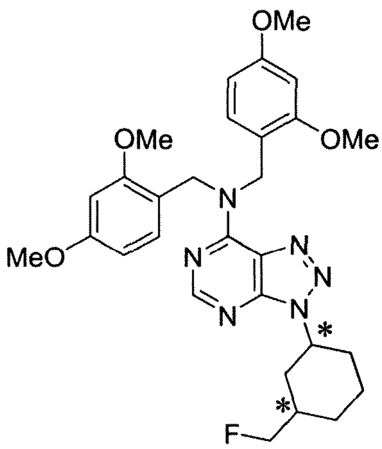

cis, racemate

To a 100 mL eggplant flask were added [cis-3-{7-[bis(2,4-dimethoxybenzyl)amino]-3H-[1,2,3]triazolo[4,5-d]pyrimidin-3-yl}cyclohexyl]methyl methanesulfonate (175 mg) prepared in the Reference Example 118-2, cesium fluoride (205 mg), acetonitrile (1.4 mL), water (24.4 μL), and 1-butyl-3-methylimidazolium tetrafluoroborate (1.4 mL), and the

resulting mixture was stirred at 100°C for 5 hours. The reaction mixture was allowed to cool to room temperature, and purified by silica gel column chromatography (solvent: hexane/ethyl acetate = 90/10 to 25/75) to give the title compound (105 mg) (yield 70%) as a colorless amorphous.
MS(ESI) m/z: 551 [M+H]+

Reference Example 119-1

Preparation of 3-[cis-3-(difluoromethyl)cyclohexyl]-N,N-bis(2,4-dimethoxybenzyl)-3H-[1,2,3]triazolo[4,5-d]pyrimidin-7-amine

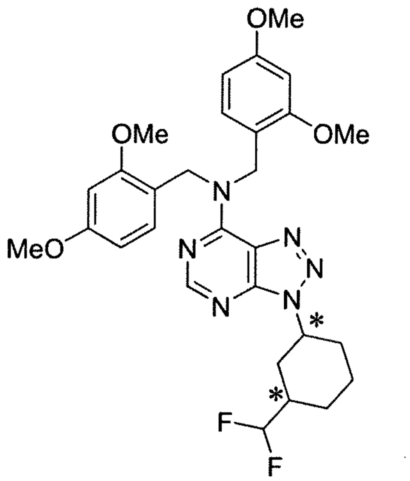

cis, racemate

Cis-3-{7-[bis(2,4-dimethoxybenzyl)amino]-3H-[1,2,3]triazolo[4,5-d]pyrimidin-3-yl}cyclohexanecarbaldehyde prepared in the Reference Example 117-2 was reacted in a similar manner to the Reference Example 154-3(2) to give the title compound. MS(ESI) m/z: 569 [M+H]+

Reference Example 120-3

Preparation of 1-[cis-3-{7-[bis(2,4-dimethoxybenzyl)amino]-3H-[1,2,3]triazolo[4,5-d]pyrimidin-3-yl}cyclohexyl]-2,2,2-trifluoroethanol

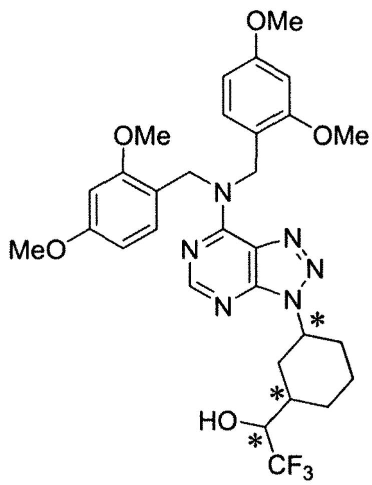

relative configuration of cyclohexane is cis, mixture of four types of stereoisomers

To a 200 mL eggplant flask were added cis-3-{7-[bis(2,4-dimethoxybenzyl)amino]-3H-[1,2,3]triazolo[4,5-d]pyrimidin-3-yl}cyclohexanecarbaldehyde (369 mg) prepared in the Reference Example 117-2, (trifluoromethyl)trimethylsilane (198 μL), cesium fluoride (122 mg), and tetrahydrofuran (3.4 mL), and the resulting mixture was stirred at room temperature for 6 days. To the reaction mixture was added saturated brine, and the resulting mixture was extracted twice with ethyl acetate. The resulting organic layers were combined, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 75/25 to 0/100) to give the title compound (306 mg) (yield 73%) as a colorless amorphous.

MS(ESI) m/z: 617 $[M+H]^+$

Reference Example 120-2

Preparation of O-{1-[cis-3-{7-[bis(2,4-dimethoxybenzyl)amino]-3H-[1,2,3]triazolo[4,5-d]pyrimidin-3-yl}cyclohexyl]-2,2,2-trifluoroethyl} O-phenyl thiocarbonate

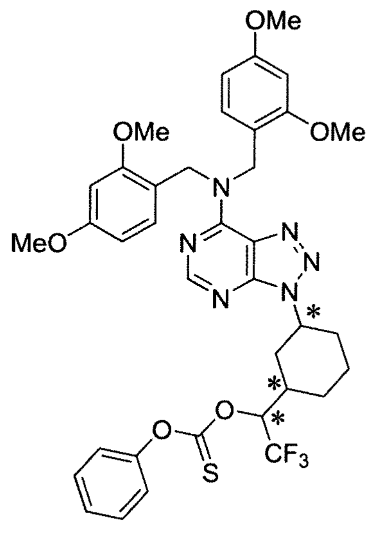

relative configuration of cyclohexane is cis, mixture of four types of stereoisomers

To a 25 mL flask were added 1-[cis-3-{7-[bis(2,4-dimethoxybenzyl)amino]-3H-[1,2,3]triazolo[4,5-d]pyrimidin-3-yl}cyclohexyl]-2,2,2-trifluoroethanol (306 mg) prepared in the Reference Example 120-3, 4-dimethylaminopyridine (89 mg), and phenyl chlorothionoformate (103 μL), and the resulting mixture was stirred at room temperature for 2 hours. To the reaction mixture were additionally added 4-dimethylaminopyridine (97.9 mg) and phenyl chlorothionoformate

(103 μL), and the resulting mixture was stirred overnight. Ethyl acetate (22.5 mL) and a 5% aqueous solution of citric acid (10 mL) were added thereto, the resulting mixture was separated, and the resulting aqueous layer was extracted with ethyl acetate. The resulting organic layers were combined, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 90/10 to 50/50) to give the title compound (343 mg) (yield 92%) as a colorless powder.
MS(ESI) m/z: 753 $[M+H]^+$

Reference Example 120-1

Preparation of N,N-bis(2,4-dimethoxybenzyl)-3-[cis-3-(2,2,2-trifluoroethyl)cyclohexyl]-3H-[1,2,3]triazolo[4,5-d]pyrimidin-7-amine

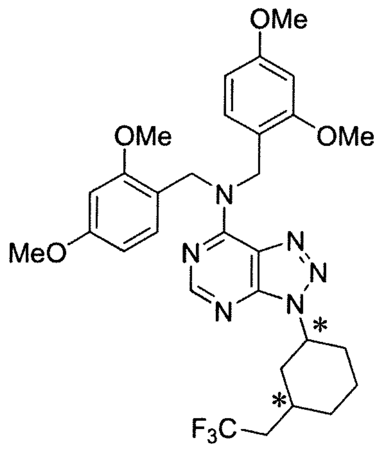

cis, racemate

To a 25 mL eggplant flask were added O-{1-[cis-3-{7-[bis(2,4-dimethoxybenzyl)amino]-3H-[1,2,3]triazolo[4,5-d]pyrimidin-3-yl}cyclohexyl]-2,2,2-trifluoroethyl} O-phenyl thiocarbonate (343 mg) prepared in the Reference Example 120-2, tributyltin hydride (612 μL), 2,2'-azobis(isobutyronitrile) (9 mg), and toluene (2.3 mL), and the resulting mixture was stirred under nitrogen atmosphere at 80°C for 17 hours. The reaction mixture was allowed to cool to room temperature, and purified by NH-silica gel column chromatography (solvent: hexane/ethyl acetate = 100/0 to 60/40) to give the title compound (234 mg) (yield 85%) as a colorless amorphous.
MS(ESI) m/z: 601 $[M+H]^+$

Preparation of Reference Example 88-4

benzyl N-[(1R)-3,3-difluorocyclohexyl]carbamate and Reference Example 89-4 benzyl N-[(1S)-3,3-difluorocyclohexyl]carbamate

Benzyl N-(3,3-difluoromethylcyclohexyl)carbamate was resolved by chiral HPLC to give the title compound. (Table 18)
The absolute configuration was determined by converting the title compound into a benzimidazole derivative, then obtaining crystals, and carrying out X-ray structural analysis.

Table 18

| Ref. Ex. | Structural formula | Physical property etc. | Analysis conditions etc. |
|---|---|---|---|
| 88-4 | 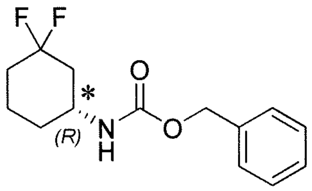 | MS(ESI) m/z; 270 [M+H]+ | Column: CHIRALPAK AD-3 (4.6 × 150 mm)<br>Mobile phase: hexane/ethanol/diethyl amine (90/10/0.1)<br>Flow rate: 0.5 mL/min<br>Temperature: 25°C<br>Analysis channel: PDA 254.0 nm<br>Retention time (min.): 11.248 |
| 89-4 | 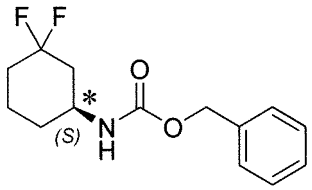 | MS (ESI) m/z; 270 [M+H]+ | Column: CHIRALPAK AD-3 (4.6 × 150 mm)<br>Mobile phase: hexane/ethanol/diethyl amine (90/10/0.1)<br>Flow rate: 0.5 mL/min<br>Temperature: 25°C<br>Analysis channel: PDA 254.0 nm<br>Retention time (min.): 12.466 |

Reference Example 88-3

Preparation of (1R)-3,3-difluorohexylamine hydrochloride

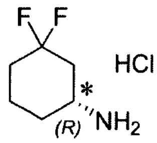

To a 100 mL eggplant flask were added benzyl N-[(1R)-3,3-difluorocyclohexyl]carbamate (1.0 g) prepared in the Reference Example 88-4, ethanol (7.5 mL), a 4 mol/L solution of hydrogen chloride in 1,4-dioxane (1 mL), and 10% palladium carbon (495 mg), and the resulting mixture was stirred under hydrogen atmosphere at room temperature for 19 hours. The reaction mixture was subjected to nitrogen replacement, then the insoluble matters were removed by Celite filtration, and the resulting filtrate was concentrated under reduced pressure to give the title compound (572 mg) (yield 90%).
MS(ESI) m/z: 136 $[M+H]^+$

Reference Example 89-3

Preparation of (1S)-3,3-difluorocyclohexylamine p-toluenesulfonate

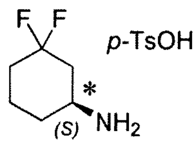

To a 200 mL eggplant flask were added benzyl N-[(1S)-3,3-difluorocyclohexyl]carbamate (5.0 g) prepared in the Reference Example 89-4, ethanol (50 mL), and 10% palladium carbon (1.06 g), and the resulting mixture was stirred under hydrogen atmosphere at room temperature for 1 hour and 30 minutes. The reaction mixture was subjected to nitrogen replacement, then the insoluble matters were removed by Celite filtration, and the resulting filtrate was concentrated under reduced pressure. To the resulting residues was added ethanol (8 mL), then was added a solution of p-toluenesulfonic acid monohydrate (3.74 g) in ethanol (8 mL), and the resulting mixture was stirred at room temperature for 70 minutes. The reaction mixture was concentrated under reduced pressure, to the resulting residues was added diethyl ether (40 mL), the resulting mixture was stirred at room temperature for 15 minutes, then the resulting solid was collected by filtration, and dried under reduced pressure to give the title compound (4.34 g) (yield 76%).
MS(APCI) m/z: 136 $[M+H]^+$

Reference Example 121-2

Preparation of cis-3-{7-[bis(2,4-dimethoxybenzyl)amino]-3H-[1,2,3]triazolo[4,5-d]pyrimidin-3-yl}cyclohexanol

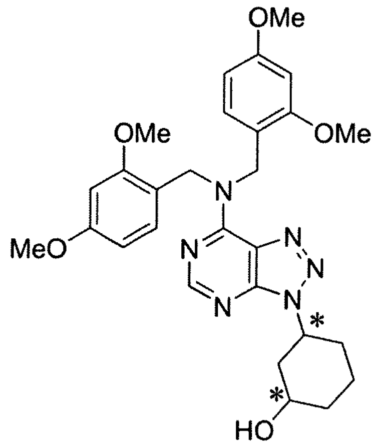

cis, racemate

and Reference Example 123-2

trans-3-{7-[bis(2,4-dimethoxybenzyl)amino]-3H-[1,2,3]triazolo[4,5-d]pyrimidin-3-yl}cyclohexanol

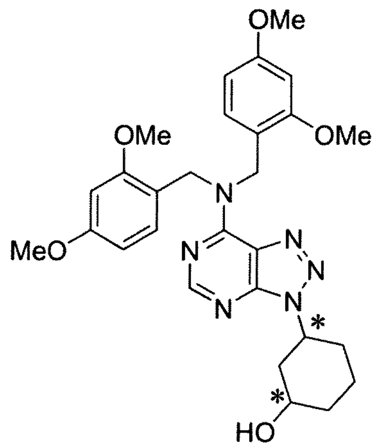

trans, racemate

To a 200 mL eggplant flask were added 3-[5-amino-6-chloropyrimidin-4-yl]amino]cyclohexanol (3.19 g) prepared in the Reference Example 121-4, dichloromethane (26 mL), and acetic acid (26 mL), an aqueous solution (5.3 mL) comprising sodium nitrite (1.18 g) was added dropwise thereto under ice-cooling, and the resulting mixture was stirred for 1 hour. To the reaction mixture were added ethyl acetate (130 mL) and water (130 mL), the resulting mixture

was separated, the resulting organic layer was washed with a saturated aqueous solution of sodium hydrogen carbonate and a saturated aqueous solution of sodium chloride, dried over anhydrous magnesium sulfate, and the insoluble matters were removed by filtration. The resulting filtrate was concentrated under reduced pressure. To the resulting residues were added bis(2,4-dimethoxybenzyl)amine (4.61 g), N,N-diisopropylethylamine (3.4 mL), and tetrahydrofuran (26 mL), and the resulting mixture was stirred at room temperature overnight. To the reaction mixture was added a 1 mol/L aqueous solution of sodium hydroxide (26 mL), and the resulting mixture was stirred for 5 hours. To the reaction mixture were added citric acid monohydrate (14 g) and saturated brine (100 mL), the resulting mixture was separated, and the resulting aqueous layer was extracted with ethyl acetate. The resulting organic layers were combined, washed with water and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 50/50 to 0/100, then ethyl acetate/methanol = 90/10) to give cis-3-{7-[bis(2,4-dimethoxybenzyl)amino]-3H-[1,2,3]triazolo[4,5-d]pyrimidin-3-yl}cyclohexanol (2.99 g) (yield 42%) and trans-3-{7-[bis(2,4-dimethoxybenzyl)amino]-3H-[1,2,3]triazolo[4,5-d]pyrimidin-3-yl}cyclohexanol (1.09 g) (yield 15%).
cis-3-{7-[bis(2,4-dimethoxybenzyl)amino]-3H-[1,2,3]triazolo[4,5-d]pyrimidin-3-yl}cyclohexanol
MS(ESI) m/z: 535 $[M+H]^+$
trans-3-{7-[bis(2,4-dimethoxybenzyl)amino]-3H-[1,2,3]triazolo[4,5-d]pyrimidin-3-yl}cyclohexanol
MS(ESI) m/z: 535 $[M+H]^+$

Reference Examples 121-1 and 123-1

A corresponding starting compound was reacted in a similar manner to the Reference Example 111-1 to give each compound described in the following Table 19.

Table 19

| Reference Example | Structural formula | Physical property etc. |
|---|---|---|
| 121-1 | 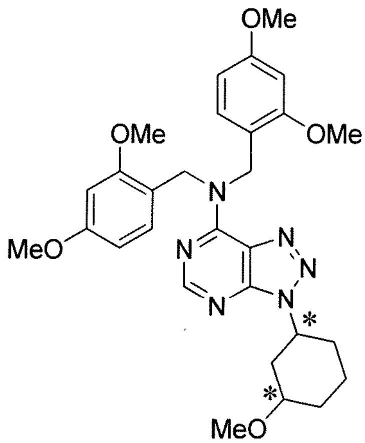 cis, racemate | MS(ESI) m/z; 549 $[M+H]^+$ |
| 123-1 | 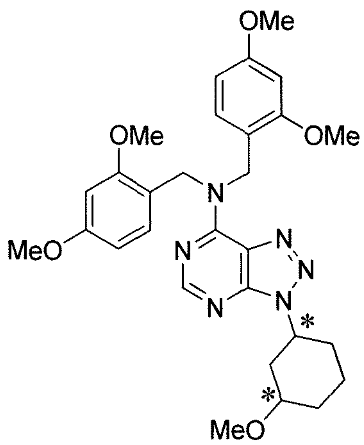 trans, racemate | MS(ESI) m/z; 549 $[M+H]^+$ |

Reference Example 125-1

Preparation of N,N-bis(2,4-dimethoxybenzyl)-3-[cis-3-(phenoxymethyl)cyclohexyl]-3H-[1,2,3]triazolo[4,5-d]pyrimidin-7-amine

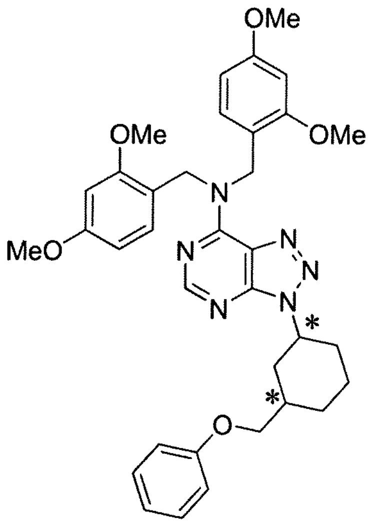

cis, racemate

To a 25 mL eggplant flask were added [cis-3-{7-[bis(2,4-dimethoxybenzyl)amino]-3H-[1,2,3]triazolo[4,5-d]pyrimidin-3-yl}cyclohexyl]methanol (182 mg) prepared in the Reference Example 117-3, phenol (50.4 mg), triphenylphosphine (137 mg), and tetrahydrofuran (1.7 mL), to the resulting suspension was added diisopropyl azodicarboxylate (99 μL) under stirring, and the resulting mixture was stirred at room temperature for 2 hours. Triphenylphosphine (55.9 mg) and diisopropyl azodicarboxylate (33 μL) were additionally added thereto, and the resulting mixture was stirred at room temperature for 1 hour and 30 minutes. The reaction mixture was concentrated under reduced pressure, and the resulting residues were purified by silica gel column chromatography (solvent: hexane/ethyl acetate = 85/15 to 0/100) to give the title compound (267 mg) (yield 99%) as a colorless oil.
MS(ESI) m/z: 625 $[M+H]^+$

Reference Example 126-1

Preparation of 3-{cis-3-[(benzyloxy)methyl]cyclohexyl}-N,N-bis(2,4-dimethoxybenzyl)-3H-[1,2,3]triazolo[4,5-d]pyrimidin-7-amine

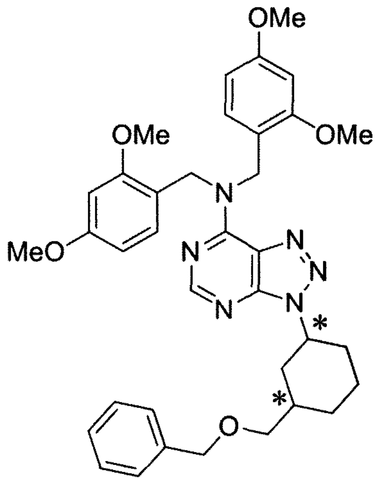

cis, racemate

To a 25 mL eggplant flask were added [cis-3-{7-[bis(2,4-dimethoxybenzyl)amino]-3H-[1,2,3]triazolo[4,5-d]pyrimidin-3-yl}cyclohexyl]methanol (185 mg) prepared in the Reference Example 117-3 and N,N-dimethylformamide (1.7 mL), sodium hydride (60%) (17 mg) was added thereto, and the resulting mixture was stirred for 1 hour. To the reaction mixture were added benzyl bromide (30 μL) and sodium iodide (54.3 mg), the resulting mixture was stirred at room temperature for 1 hour and 30 minutes, then warmed to 50°C, and stirred for 1 hour. To the reaction mixture was additionally added benzyl bromide (30 μL), the resulting mixture was stirred at 50°C for 1 hour, and then stirred overnight with gradually cooling to room temperature. To the reaction mixture was added a saturated aqueous solution of ammonium chloride, and the resulting mixture was extracted three times with ethyl acetate. The resulting organic layers were combined, washed with water and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 85/15 to 0/100) to give the title compound (91.4 mg) (yield 43%) as a yellow oil.
MS(ESI) m/z: 639 $[M+H]^+$

Reference Example 127-3

Preparation of cis-3-{7-[bis(2,4-dimethoxybenzyl)amino]-3H-[1,2,3]triazolo[4,5-d]pyrimidin-3-yl}cyclohexanecarboxylic acid

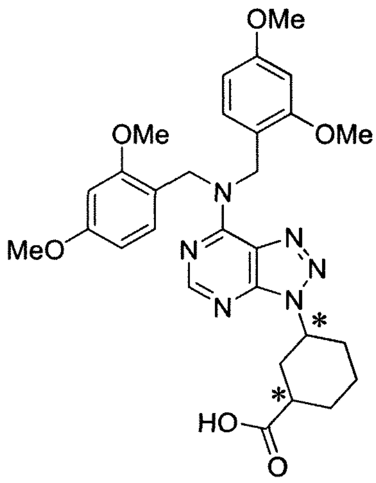

cis, racemate

To a 300 mL eggplant flask were added methyl cis-3-{7-[bis(2,4-dimethoxybenzyl)amino]-3H-[1,2,3]triazolo[4,5-d]pyrimidin-3-yl}cyclohexanecarboxylate (5.01 g) prepared in the Reference Example 117-4, tetrahydrofuran (42 mL), and a 1 mol/L aqueous solution of sodium hydroxide (17 mL), and the resulting mixture was stirred at room temperature for 6 hours. To the reaction mixture was added citric acid monohydrate (1.84 g) to be acidified, then saturated brine was added thereto, and the resulting mixture was extracted twice with ethyl acetate. The resulting organic layers were combined, washed sequentially with water and saturated brine, dried over anhydrous sodium sulfate, and the insoluble matters were removed by filtration. The resulting filtrate was concentrated under reduced pressure to give the title compound (4.95 g) (yield 100%) as a pale yellow powder.
MS(ESI) m/z: 563 $[M+H]^+$

Reference Example 127-2

Preparation of cis-3-{7-[bis(2,4-dimethoxybenzyl)amino]-3H-[1,2,3]triazolo[4,5-d]pyrimidin-3-yl}-N-(2-oxopropyl)cyclohexanecarboxamide

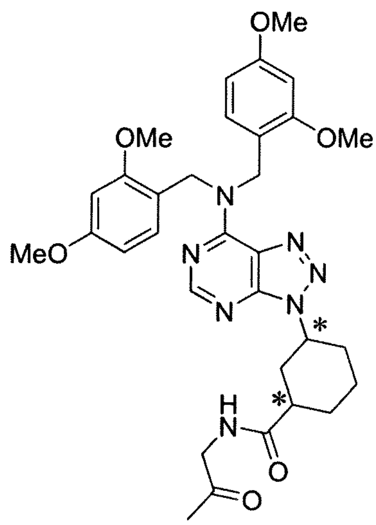

cis, racemate

To a 25 mL eggplant flask were added cis-3-{7-[bis(2,4-dimethoxybenzyl)amino]-3H-[1,2,3]triazolo[4,5-d]pyrimidin-3-yl}cyclohexanecarboxylic acid (284 mg) prepared in the Reference Example 127-3, aminoacetone hydrochloride

(181 mg), 1-hydroxybenzotriazole (97.9 mg), and chloroform (2.44 mL), 1-ethyl-3-(3-dimethylaminopropyl)carbodiimide hydrochloride (142.8 mg) was added thereto under stirring, and the resulting mixture was stirred at room temperature for 20 minutes. To the reaction mixture was added triethylamine (364 μL), and the resulting mixture was stirred at room temperature overnight. The reaction mixture was purified by silica gel column chromatography (solvent: ethyl acetate/methanol = 100/0 to 80/20) to give the title compound (228 mg) (yield 76%) as a pale yellow powder.
MS(ESI) m/z: 618 $[M+H]^+$

Reference Example 127-1

Preparation of N,N-bis(2,4-dimethoxybenzyl)-3-[cis-3-(5-methyl-1,3-thiazol-2-yl)cyclohexyl]-3H-[1,2,3]triazolo[4,5-d]pyrimidin-7-amine

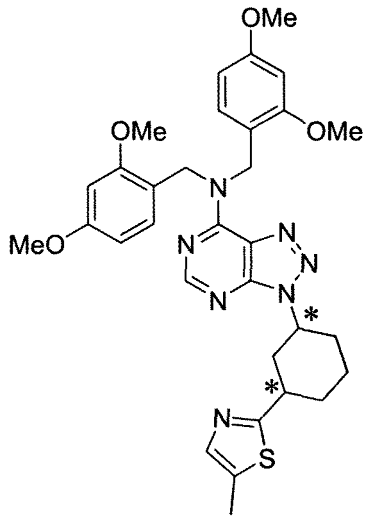

cis, racemate

To a 25 mL eggplant flask were added cis-3-{7-[bis(2,4-dimethoxybenzyl)amino]-3H-[1,2,3]triazolo[4,5-d]pyrimidin-3-yl}-N-(2-oxopropyl)cyclohexanecarboxamide (228 mg) prepared in the Reference Example 127-2, 2,4-bis(4-methoxyphenyl)-1,3-dithia-2,4-diphosphetane 2,4-disulfide (299 mg), and tetrahydrofuran (3.6 mL), and the resulting mixture was stirred at 80°C for 1 hour. The reaction mixture was allowed to cool to room temperature, a saturated aqueous solution of sodium hydrogen carbonate was added thereto, and the resulting mixture was extracted three times with ethyl acetate. The resulting organic layers were combined, washed with saturated brine, dried over anhydrous sodium sulfate, and the insoluble matters were removed by filtration. The resulting filtrate was concentrated under reduced pressure, and the resulting residues were purified by NH-silica gel column chromatography (solvent: hexane/ethyl acetate = 75/25 to 25/75) to give the title compound (172 mg) (yield 76%) as a colorless powder.
MS(APCI) m/z: 616 $[M+H]^+$

Reference Example 136-1

Preparation of 3-[(3R)-piperidin-3-yl]-3H-[1,2,3]triazolo[4,5-d]pyrimidin-7-amine dihydrochloride

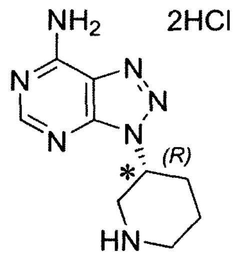

To a mixture of tert-butyl (3R)-3-(7-amino-3H-[1,2,3]triazolo[4,5-d]pyrimidin-3-yl)piperidine-1-carboxylate (3.00 g) prepared in the Example 12 and ethyl acetate (25 mL) was added a 4 mol/L solution of hydrogen chloride in ethyl acetate (15 mL), and the resulting mixture was stirred at room temperature overnight. The resulting precipitates were collected by filtration, washed with ethyl acetate, and then dried under reduced pressure to give the title compound (2.87 g) (yield 105%) as a colorless powder.
MS(APCI) m/z: 220 $[M+H]^+$

Reference Example 135-1

Preparation of 3-[(3S)-piperidin-3-yl]-3H-[1,2,3]triazolo[4,5-d]pyrimidin-7-amine dihydrochloride

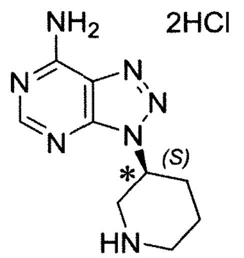

Tert-butyl (3S)-3-(7-amino-3H-[1,2,3]triazolo[4,5-d]pyrimidin-3-yl)piperidine-1-carboxylate prepared in the Example 13 was reacted in a similar manner to the Reference Example 136-1 to give the title compound.
MS(APCI) m/z: 220 $[M+H]^+$

Reference Example 128-4

Preparation of N-(2,4-dimethoxybenzyl)-3,3-dimethylcyclohexaneamine

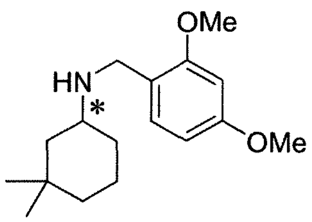

racemate

To a mixture of 3,3-dimethylcyclohexanone (1.00 g), 2,4-dimethoxybenzylamine (1.60 g), acetic acid (0.45 mL), and 1,2-dichloroethane (15 mL) was added sodium triacetoxyborohydride (5.00 g), and the resulting mixture was stirred at room temperature for 3 days. To the reaction mixture was added a 1 mol/L aqueous solution of sodium hydroxide to be basified, and then the resulting mixture was extracted twice with chloroform. The resulting organic layers were

combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 95/5 to 70/30) to give a crude product of the title compound (2.44 g) (yield 110%) as a colorless oil.
MS(APCI) m/z: 278 $[M+H]^+$

Reference Example 128-1

Preparation of N-(2,4-dimethoxybenzyl)-N-(3,3-dimethylcyclohexyl)-3-(4-methoxybenzyl)-3H-[1,2,3]triazolo[4,5-d]pyrimidin-7-amine

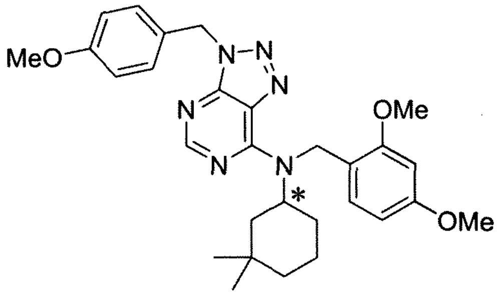

racemate

A mixture of 7-chloro-3-(4-methoxybenzyl)-3H-[1,2,3]triazolo[4,5-d]pyrimidine (300 mg) prepared in the Reference Example 128-2, N-(2,4-dimethoxybenzyl)-3,3-dimethylcyclohexaneamine (362 mg) prepared in the Reference Example 128-4, triethylamine (0.227 mL), and tetrahydrofuran (6 mL) was stirred at room temperature for 2 hours and 30 minutes. To the reaction mixture was added water, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 95/5 to 70/30) to give the title compound (520 mg) (yield 93%) as a colorless powder.
MS(APCI) m/z: 517 $[M+H]^+$

Reference Example 129-1

Preparation of N-(2-fluoro-5-methylphenyl)-3-(4-methoxybenzyl)-3H-[1,2,3]triazolo[4,5-d]pyrimidin-7-amine

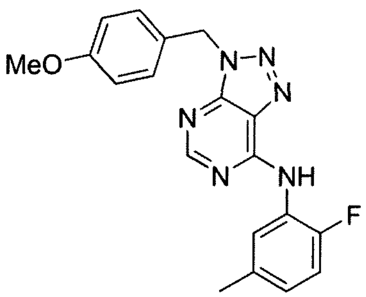

A mixture of 7-chloro-3-(4-methoxybenzyl)-3H-[1,2,3]triazolo[4,5-d]pyrimidine (200 mg) prepared in the Reference Example 128-2, 2-fluoro-5-methyl-aniline (0.164 mL), a 4 mol/L solution of hydrogen chloride in 1,4-dioxane (0.02 mL), and tert-butyl alcohol (4 mL) was stirred at 80°C for 4 hours and 30 minutes. The reaction mixture was allowed to cool to room temperature, a saturated aqueous solution of sodium hydrogen carbonate was added thereto, and the resulting mixture was extracted twice with ethyl acetate. The resulting organic layers were combined, washed with

saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 95/5 to 70/30) to give the title compound (75 mg) (yield 28%) as a colorless powder.
MS(APCI) m/z: 365 $[M+H]^+$

Reference Example 130-1 etc.:

A corresponding starting compound was treated in a similar manner to the Reference Example 129-1 to give each compound described in the following Table 20.

Table 20

| Reference Example | Structural formula | Physical property etc. |
|---|---|---|
| 130-1 | 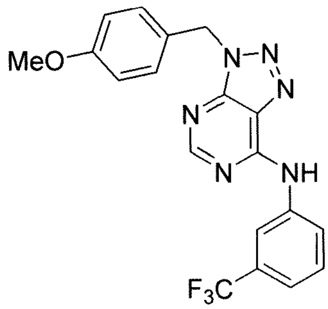 | MS(APCI) m/z; 401 $[M+H]^+$ |
| 139-2 | 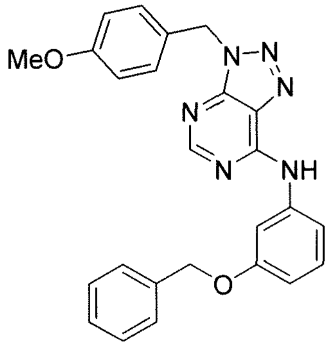 | MS(APCI) m/z; 439 $[M+H]^+$ |
| 131-3 | 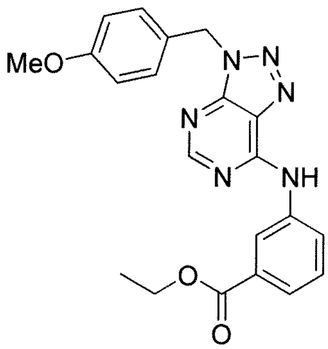 | MS(APCI) m/z; 405 $[M+H]^+$ |

Reference Example 139-1

Preparation of 3-(7-amino-3H-[1,2,3]triazolo[4,5-d]pyrimidin-3-yl)phenol

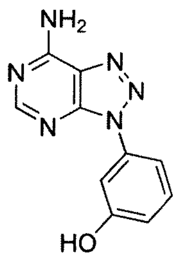

N-[3-(benzyloxy)phenyl]-3-(4-methoxybenzyl)-3H-[1,2,3]triazolo[4,5-d]pyrimidin-7-amine prepared in the Reference Example 139-2 was reacted in a similar manner to the Example 128 to give the title compound.
MS(APCI) m/z: 229 $[M+H]^+$

Reference Example 131-2

Preparation of 3-{[3-(4-methoxybenzyl)-3H-[1,2,3]triazolo[4,5-d]pyrimidin-7-yl]amino}benzoic acid

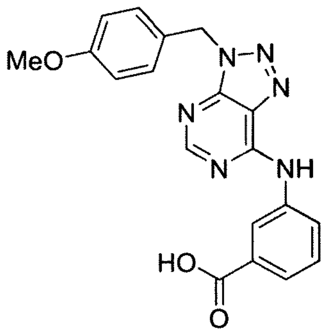

A mixture of ethyl 3-{[3-(4-methoxybenzyl)-3H-[1,2,3]triazolo[4,5-d]pyrimidin-7-yl]amino}benzoate (300 mg) prepared in the Reference Example 131-3, a 1 mol/L aqueous solution of sodium hydroxide (0.9 mL), and ethanol (6 mL) was stirred at room temperature for 1 hour and 30 minutes. The reaction mixture was heated to 60°C, stirred for 1 hour and 30 minutes, and then stirred at room temperature overnight. To the reaction mixture was additionally added a 1 mol/L aqueous solution of sodium hydroxide (1.8 mL), and the resulting mixture was stirred at 60°C for 5 hours. The reaction mixture was allowed to cool to room temperature, 1 mol/L hydrochloric acid (2.7 mL) was added thereto, and the resulting precipitates were collected by filtration. The precipitates were washed with water and ethanol, and dried under reduced pressure to give the title compound (260 mg) (yield 93%) as a colorless powder.
MS(APCI) m/z: 377 $[M+H]^+$

Reference Example 131-1

Preparation of N-benzyl-3-{[3-(4-methoxybenzyl)-3H-[1,2,3]triazolo[4,5-d]pyrimidin-7-yl]amino}benzamide

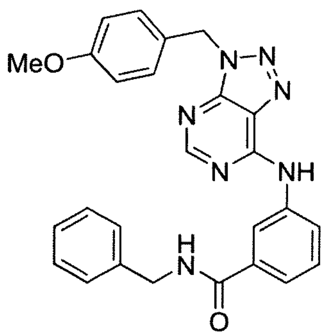

3-{[3-(4-Methoxybenzyl)-3H-[1,2,3]triazolo[4,5-d]pyrimidin-7-yl]amino}benzoic acid prepared in the Reference Example 131-2 was reacted in a similar manner to the Reference Example 127-2 using a corresponding reagent to give the title compound.
MS(APCI) m/z: 466 $[M+H]^+$

Reference Example 146-2

Preparation of cis-N'-(3-chloropyrazin-2-yl)-3-(trifluoromethyl)cyclohexanecarbohydrazide

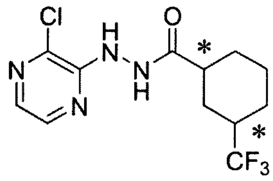

cis, racemate

and Reference Example 147-2

trans-N'-(3-chloropyrazin-2-yl)-3-(trifluoromethyl)cyclohexanecarbohydrazide

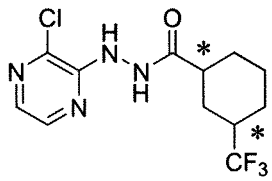

trans, racemate

To a mixture of 3-trifluoromethylcyclohexanecarboxylic acid (516 mg), 1-hydroxybenzotriazole (429 mg), 1-(3-dimethylaminopropyl)-3-ethylcarbodiimide hydrochloride (621 mg), and chloroform (13 mL) in a 50 mL eggplant flask was added 2-chloro-3-hydrazinylpyrazine (418 mg), and the resulting mixture was stirred at room temperature overnight. The reaction mixture was purified by silica gel column chromatography (solvent: hexane/ethyl acetate = 75/25 to 40/60) to give cis-N'-(3-chloropyrazin-2-yl)-3-(trifluoromethyl)cyclohexanecarbohydrazide (548.6 mg) (yield 64%) and trans-N'-(3-chloropyrazin-2-yl)-3-(trifluoromethyl)cyclohexanecarbohydrazide (222.5 mg) (yield 26%) respectively as a colorless solid. cis-N'-(3-chloropyrazin-2-yl)-3-(trifluoromethyl)cyclohexanecarbohydrazide
MS(ESI) m/z: 323/325 $[M+H]^+$
trans-N'-(3-chloropyrazin-2-yl)-3-(trifluoromethyl)cyclohexanecarbohydrazide
MS(ESI) m/z: 323/325 $[M+H]^+$

Reference Example 140-2 etc.:

A corresponding starting compound was reacted in a similar manner to the Reference Example 146-2 to give each compound described in the following Table 21.

Table 21

| Reference Example | Structural formula | Physical property etc. |
|---|---|---|
| 142-2 | 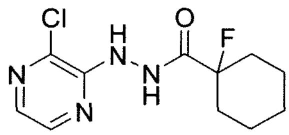 | MS(APCI) m/z; 273/275 $[M+H]^+$ |
| 140-2 | 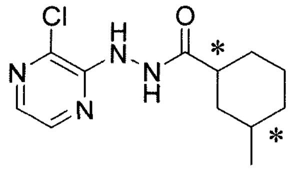 cis, racemate | MS(ESI) m/z; 269/271 $[M+H]^+$ |
| 143-2 | 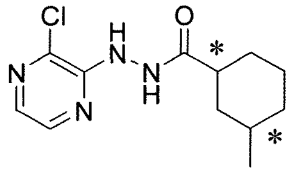 trans, racemate | MS(ESI) m/z; 269/271 $[M+H]^+$ |
| 144-2 | 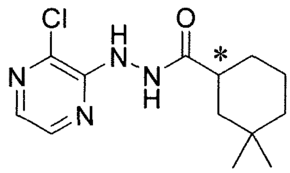 racemate | MS(ESI) m/z; 283/285 $[M+H]^+$ |
| 145-2 | 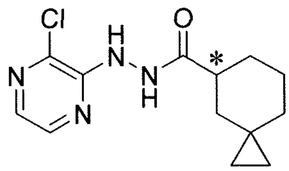 racemate | MS(ESI) m/z; 281/283 $[M+H]^+$ |
| 148-2 | 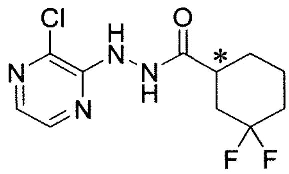 racemate | MS(APCI) m/z; 291/293 $[M+H]^+$ |
| 149-2 | 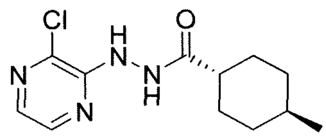 trans | MS(ESI) m/z; 269/271 $[M+H]^+$ |
| 150-2 | 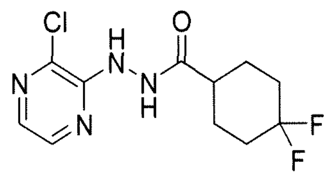 | MS(ESI) m/z; 291/293 $[M+H]^+$ |

(continued)

| Reference Example | Structural formula | Physical property etc. |
|---|---|---|
| 151-2 | 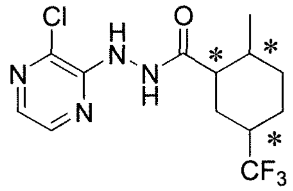 relative configuration (1R*, 2S*, 5R*), racemate | MS(APCI) m/z; 337/339 [M+H]+ |
| 152-2 | 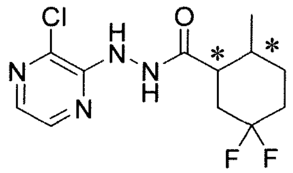 cis, racemate | MS(ESI) m/z; 305/307 [M+H]+ |
| 153-2 | 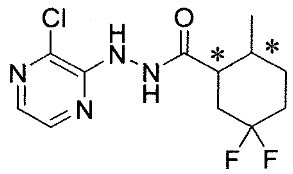 trans, racemate | MS(ESI) m/z; 305/307 [M+H]+ |
| 154-2 | 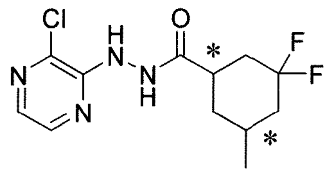 cis, racemate | MS(ESI) m/z; 305/307 [M+H]+ |
| 155-2 | 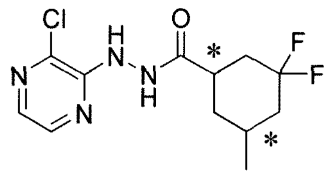 trans, racemate | MS(ESI) m/z; 305/307 [M+H]+ |
| 156-2 | 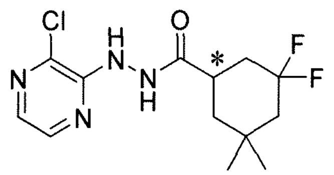 racemate | MS(ESI) m/z; 319/321 [M+H]+ |
| 157-2 | 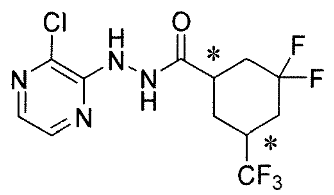 cis, racemate | MS(ESI) m/z; 359/361 [M+H]+ |

(continued)

| Reference Example | Structural formula | Physical property etc. |
|---|---|---|
| 172-5 | 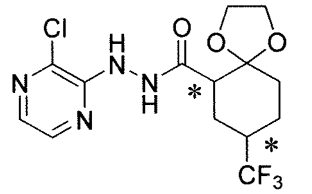 cis, racemate | MS(APCI) m/z; 381/383 [M+H]+ |
| 158-2 | 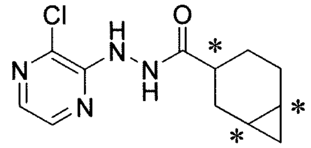 cyclopropane in bicyclo[4,1,0]hep tane ring is cis isomer, mixture of four types of stereoisomers | MS(ESI) m/z; 267/269 [M+H]+ |
| 159-2 | 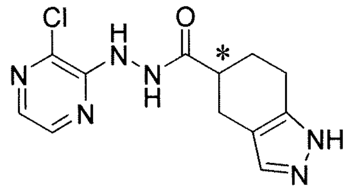 racemate | MS(ESI) m/z; 293/295 [M+H]+ |
| 162-2 | 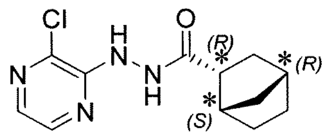 | MS(ESI) m/z; 267/269 [M+H]+ |
| 163-2 | 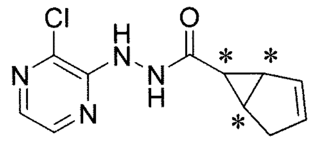 relative configuration (1S*, 5R*, 6S*), racemate | MS(ESI) m/z; 251/253 [M+H]+ |
| 164-2 | 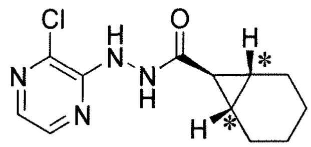 | MS(ESI) m/z; 267/269 [M+H]+ |
| 167-2 | 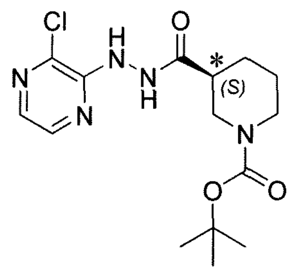 | MS(APCI) m/z; 356/358 [M+H]+ |

(continued)

| Reference Example | Structural formula | Physical property etc. |
|---|---|---|
| 168-2 | 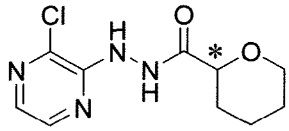 racemate | MS(ESI) m/z; 257/259 $[M+H]^+$ |
| 169-2 | 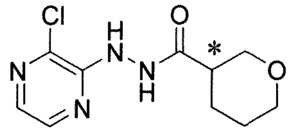 racemate | MS(ESI) m/z; 257/259 $[M+H]^+$ |

Reference Example 68-4

Preparation of 6-amino-3-cyclohexyl-[1,2,4]triazolo[3,4-f] [1,2,4]triazin-8(7H)-one

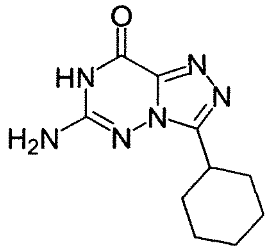

(1) To a 100 mL eggplant flask were added 3-amino-6-hydrazinyl-1,2,4-triazin-5(4H)-one (1.42 g), cyclohexanecarboxylic acid (1.92 g), 1-ethyl-3-(3-dimethylaminopropyl)carbodiimide hydrochloride (2.93 g), 1-hydroxybenzotriazole (0.68 g), and dimethylformamide (30 mL), triethylamine (1.82 g) was added thereto under argon atmosphere under ice-cooling with stirring, and the resulting mixture was stirred at 50°C for 3 hours. After the reaction was completed, to the mixture was added water (100 mL), the precipitated solid was collected by filtration, and washed with water to give N'-(3-amino-5-oxo-4,5-dihydro-1,2,4-triazin-6-yl)cyclohexanecarbohydrazide (1.82 g) as a slightly yellow crystal.

(2) To a 100 mL cylindrical flask were added N'-(3-amino-5-oxo-4,5-dihydro-1,2,4-triazin-6-yl)cyclohexanecarbohydrazide (1.82 g) prepared in the above (1) and ethylene glycol (20 mL), and the resulting mixture was stirred at 180°C for 3 hours. After the reaction was completed, the mixture was cooled to room temperature, ethyl acetate and ethanol were added thereto, the precipitated solid was filtered, and the filtered residues were washed with ethanol to give the title compound (1.13 g) (yield 96%) as a slightly yellow solid.

MS(CI) m/z: 235 $[M+H]^+$

Reference Example 69-4 etc.:

A corresponding starting compound was reacted in a similar manner to the Reference Example 68-4 to give each compound described in the following Table 22.

Table 22

| Reference Example | Structural formula | Physical property etc. |
|---|---|---|
| 69-4 | 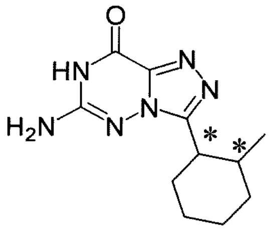 mixture of four types of stereoisomers | MS(CI) m/z; 249 [M+H]+ |
| 71-4 | 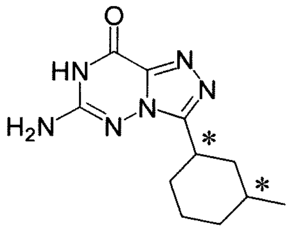 mixture of four types of stereoisomers | MS(CI) m/z; 249 [M+H]+ |
| 73-4 | 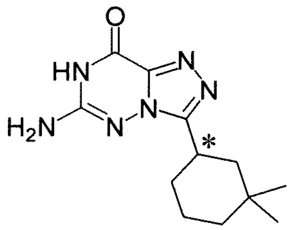 racemate | MS(CI) m/z; 263 [M+H]+ |
| 74-4 | 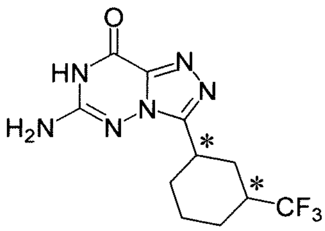 mixture of four | MS(CI) m/z; 303 [M+H]+ |
| | types of stereoisomers | |
| 82-4 | 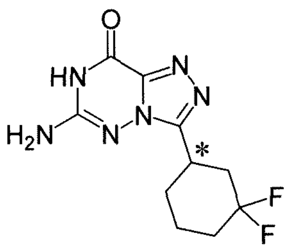 racemate | MS(DUIS) m/z; 271 [M+H]+ |

(continued)

| Reference Example | Structural formula | Physical property etc. |
|---|---|---|
| 76-4 | 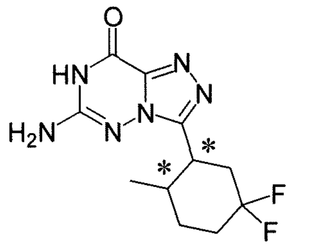 cis, racemate | MS(CI) m/z; 285 $[M+H]^+$ |
| 83-4 | 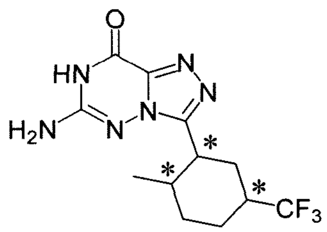 relative configuration (1R*, 2S*, 5R*), racemate | MS(CI) m/z; 317 $[M+H]^+$ |

Reference Example 17-2

Preparation of N'-(3-chloropyrazin-2-yl)-1,2,3,4-tetrahydronaphthalene-1-carbohydrazide

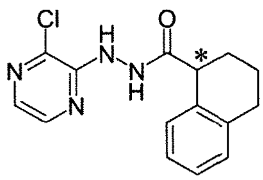

racemate

To a 30 mL cylindrical flask were added 2-chloro-3-hydrazinylpyrazine (470 mg), tetrahydrofuran (5 mL), 1,2,3,4-tetrahydronaphthalene-1-carboxylic acid (573 mg), triethylamine (550 μL), and 1-(3-dimethylaminopropyl)-3-ethylcarbodiimide hydrochloride (700 mg) under argon atmosphere, and the resulting mixture was stirred at room temperature for 3 hours. To the reaction mixture were additionally added 1-(3-dimethylaminopropyl)-3-ethylcarbodiimide hydrochloride (350 mg) and triethylamine (280 μL), and the resulting mixture was stirred at room temperature for additional 2 hours. The reaction mixture was added to a saturated aqueous solution of sodium hydrogen carbonate, and the resulting mixture was extracted with ethyl acetate. The resulting organic layer was washed with saturated brine, dried over anhydrous magnesium sulfate, and concentrated under reduced pressure. The resulting residues were subjected to silica gel column chromatography (Rf value = 0.23 (solvent: hexane/ethyl acetate = 1 : 1)) (Silica L (40 g), hexane/ethyl acetate = 52/48 to 31/69) using YAMAZEN medium pressure preparative column, and the fractions comprising the target compound were concentrated under reduced pressure to give the title compound (308 mg) (yield 31%) as a white solid.
MS(CI) m/z: 303/305 $[M+H]^+$

Reference Example 18-2 etc.:

A corresponding starting compound was reacted in a similar manner to the Reference Example 17-2 to give each compound described in the following Table 23.

Table 23

| Reference Example | Structural formula | Physical property etc. |
|---|---|---|
| 77-2 | 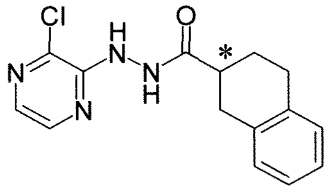 racemate | MS(CI) m/z; 303/305 [M+H]$^+$ |
| 18-2 | 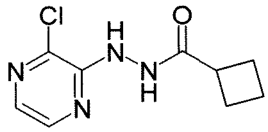 | MS(CI) m/z; 227/229 [M+H]$^+$ |
| 84-2 | 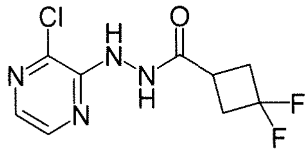 | MS(CI) m/z; 263/265 [M+H]$^+$ |
| 19-2 | 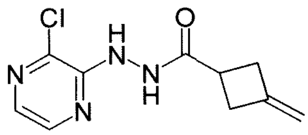 | MS(CI) m/z; 239/241 [M+H]$^+$ |
| 20-2 | 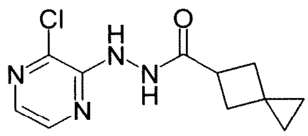 | MS(DUIS) m/z; 253/255 [M+H]$^+$ |
| 32-2 | 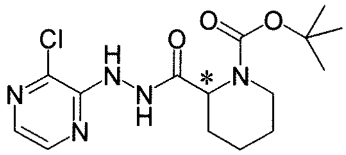 racemate | MS(DUIS) m/z; 356/358 [M+H]$^+$ |

Reference Example 17-1

Preparation of 8-chloro-3-(1,2,3,4-tetrahydronaphthalen-1-yl)-[1,2,4]triazole[4,3-a]pyrazine

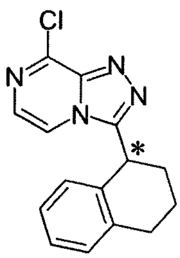

racemate

To a 30 mL cylindrical flask were added N'-(3-chloropyrazin-2-yl)-1,2,3,4-tetrahydronaphthalene-1-carbohydrazide (300 mg) prepared in the Reference Example 17-2, tetrahydrofuran (1.5 mL), and methyl N-(triethylammonium)carbamate (Burgess reagent) (470 mg), and the resulting mixture was stirred with heating under reflux for 5 hours. After the reaction was completed, to the resulting reaction solution was added a saturated aqueous solution of sodium hydrogen carbonate, and the resulting mixture was extracted with ethyl acetate. The resulting organic layer was washed with saturated brine, dried over anhydrous magnesium sulfate, and concentrated under reduced pressure. The resulting residues were subjected to silica gel column chromatography (Silica L (40 g), hexane/ethyl acetate = 50/50 to 30/70) using YAMAZEN medium pressure preparative column, and the fractions comprising the target compound were concentrated under reduced pressure to give the title compound (202 mg) (yield 72%) as a white solid. MS(CI) m/z: 285/287 $[M+H]^+$

Reference Example 18-1 etc.:

A corresponding starting compound was reacted in a similar manner to the Reference Example 17-1 to give each compound described in the following Table 24.

Table 24

| Reference Example | Structural formula | Physical property etc. |
|---|---|---|
| 77-1 | 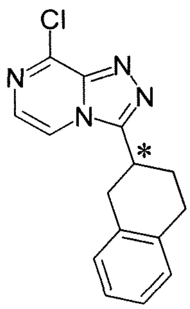 racemate | MS(CI) m/z; 285/287 $[M+H]^+$ |
| 18-1 | 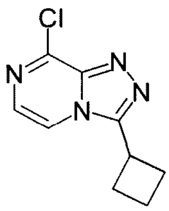 | MS(CI) m/z; 209/211 $[M+H]^+$ |
| 84-1 | 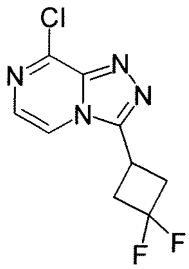 | MS(CI) m/z; 245/247 $[M+H]^+$ |
| 19-1 | 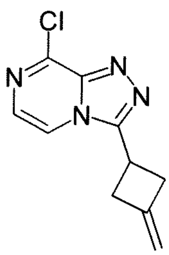 | MS(CI) m/z; 221/223 $[M+H]^+$ |

Reference Example 141-2

Preparation of N'-(3-chloropyrazin-2-yl)cyclohexanecarbohydrazide

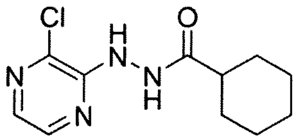

To a mixture of 2-chloro-3-hydrazinylpyrazine (1.10 g), triethylamine (1.27 mL), and chloroform (38 mL) in a 200 mL eggplant flask was added cyclohexanecarbonyl chloride (1.13 mL) under ice-cooling, and the resulting mixture was stirred at room temperature for 1 hour. To the reaction mixture were added a saturated aqueous solution of sodium hydrogen carbonate (40 mL), saturated brine (40 mL), and ethyl acetate (120 mL), and the resulting mixture was stirred for a while. The resulting organic layer was separated, washed with saturated brine, dried over anhydrous sodium sulfate, and the insoluble matters were removed by filtration. The resulting filtrate was concentrated under reduced pressure to give the title compound (1.53 g) (yield 79%) as a pale yellow solid. MS(ESI) m/z: 255/257 $[M+H]^+$

Reference Example 161-2 etc.:

A corresponding starting compound was reacted in a similar manner to the Reference Example 141-2 to give each compound described in the following Table 25.

Table 25

| Reference Example | Structural formula | Physical property etc. |
|---|---|---|
| 161-2 | 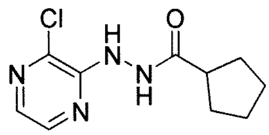 | MS(ESI) m/z; 241/243 $[M+H]^+$ |
| 165-2 | 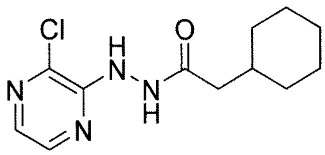 | MS(ESI) m/z; 269/271 $[M+H]^+$ |
| 166-2 | 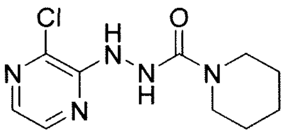 | MS(ESI) m/z; 256/258 $[M+H]^+$ |
| 170-2 | 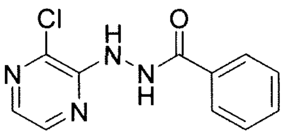 | MS(ESI) m/z; 249/251 $[M+H]^+$ |

Reference Example 54-6

Preparation of 4-nitrophenyl 3,5-dimethylpiperidine-1-carboxylate

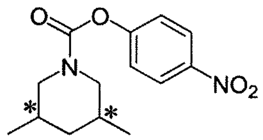

trans, racemate

To a 300 mL eggplant flask were added 3,5-dimethylpiperidine (5 g), dichloromethane (200 mL), and triethylamine (18.5 mL), and the resulting mixture was stirred under ice-cooling. Then, 4-nitrophenyl chloroformate (9.8 g) was added dividedly under ice-cooling, and the resulting mixture was stirred for 1 hour. After the reaction was completed, water was added thereto to separate an organic layer, and the organic layer was washed with a saturated aqueous solution of ammonium chloride. The organic layer was dried over anhydrous sodium sulfate, and concentrated under reduced pressure. To the resulting residues was added diisopropyl ether, the precipitated solid was filtered off, and the resulting filtrate was concentrated. To the resulting residues was added diisopropyl ether, and the precipitated solid was filtered off. The resulting filtrate was concentrated, the resulting residues were subjected to silica gel column chromatography (Silica 2L (55 g)) using YAMAZEN medium pressure preparative column, and the fractions comprising the target compound (Rf value = 0.5 (solvent: hexane/ethyl acetate = 9 : 1)) were concentrated under reduced pressure to give the title compound (0.563 g) (yield 4.6%) as a colorless oil.
MS(CI) m/z: 279 $[M+H]^+$

Reference Example 60-6

A corresponding starting compound was reacted in a similar manner to the Reference Example 54-6 to give the compound described in the following Table 26.

Table 26

| Reference Example | Structural formula | Physical property etc. |
|---|---|---|
| 60-6 | 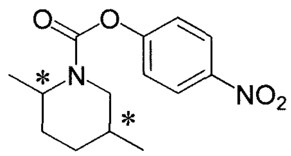 cis, racemate | MS(ESI) m/z; 279 $[M+H]^+$ |

Reference Example 54-5

Preparation of ethyl 3,5-dimethylpiperidine-1-carboxylate (trans configuration, racemate)

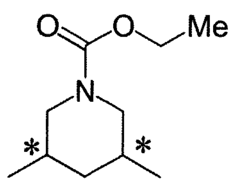

trans, racemate

To a 100 mL eggplant flask were added 4-nitrophenyl 3,5-dimethylpiperidine-1-carboxylate (trans configuration, racemate) (550 mg) prepared in the Reference Example 54-6, tetrahydrofuran (10 mL), and sodium ethoxide (1.345 g) at room temperature, and the resulting mixture was stirred overnight. The reaction solution was added to a mixed solution of diisopropyl ether/water to separate an organic layer, and the organic layer was washed with a saturated aqueous solution of sodium hydrogen carbonate and then saturated brine. The resulting organic layer was dried over anhydrous

magnesium sulfate, and concentrated under reduced pressure to give the title compound (0.322 g) (yield 88%) as a slightly brown oil.
MS(CI) m/z: 186 $[M+H]^+$

Reference Example 60-5

A corresponding starting compound was reacted in a similar manner to the Reference Example 54-5 to give the compound described in the following Table 27.

Table 27

| Reference Example | Structural formula | Physical property etc. |
|---|---|---|
| 60-5 | 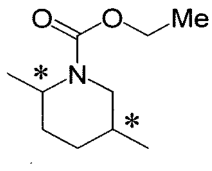 cis, racemate | MS(CI) m/z; 186 $[M+H]^+$ |

Reference Example 39-3

Preparation of 2-trifluoromethylpiperidine-1-carbonylchloride

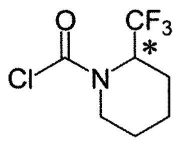

racemate

To a 200 mL eggplant flask were added triphosgene (0.97 g) and dichloromethane (50 mL) under argon gas flow, a solution of pyridine (0.79 mL) in dichloromethane (2 mL) was added thereto with stirring at 0°C, and the resulting mixture was stirred at room temperature for 0.5 hours. Then, a solution of 2-trifluoromethylpiperidine (1.50 g) in dichloromethane (4 mL) was added dropwise thereto at 0°C, and the resulting mixture was stirred at 0°C for 1 hour.

After the reaction was completed, 1N hydrochloric acid was added thereto, and the resulting mixture was extracted with dichloromethane. The resulting organic layer was washed with a saturated aqueous solution of sodium hydrogen carbonate, then dried over anhydrous magnesium sulfate, and concentrated under reduced pressure to give the title compound (1.98 g) (yield 94%) as a slightly red oil. MS(DUIS) m/z: 216/218 $[M+H]^+$

Reference Example 40-3 etc.:

A corresponding starting compound was reacted in a similar manner to the Reference Example 39-3 to give each compound described in the following Table 28.

Table 28

| Reference Example | Structural formula | Physical property etc. |
|---|---|---|
| 40-3 | 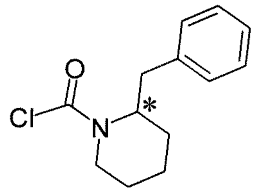 racemate | MS(CI) m/z; 238/240 [M+H]$^+$ |
| 45-3 | 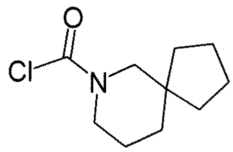 | MS(CI) m/z; 202/204 [M+H]$^+$ |

Reference Example 79-3

Preparation of 2-propylpiperidine-1-carbonylchloride

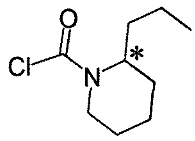

racemate

To a 200 mL eggplant flask were added triphosgene (0.49 g) and dichloromethane (25 mL), a solution of pyridine (0.41 mL) in dichloromethane (2 mL) was added thereto under argon gas flow with stirring at 0°C, and the resulting mixture was stirred at room temperature for 30 minutes. Then, to the resulting reaction solution was added dropwise a solution of 2-propylpiperidinehydrochloride (0.82 g) and diisopropylethylamine (0.65 g) in dichloromethane (30 mL) at 0°C, and the resulting mixture was stirred at the same temperature for 1 hour. After the reaction was completed, 1N hydrochloric acid was added thereto, and the resulting mixture was extracted with dichloromethane. The resulting organic layer was washed with a saturated aqueous solution of sodium hydrogen carbonate, then dried over anhydrous magnesium sulfate, and concentrated under reduced pressure to give the title compound (0.82 g) (yield 86%) as a slightly red oil.
MS(CI) m/z: 190/192 [M+H]$^+$

Reference Example 37-3 etc.:

A corresponding starting compound was reacted in a similar manner to the Reference Example 79-3 to give each compound described in the following Table 29.

Table 29

| Reference Example | Structural formula | Physical property etc. |
|---|---|---|
| 37-3 | 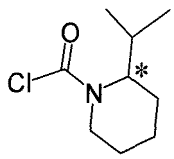 racemate | MS(CI) m/z; 190/192 [M+H]$^+$ |

(continued)

| Reference Example | Structural formula | Physical property etc. |
|---|---|---|
| 38-3 | 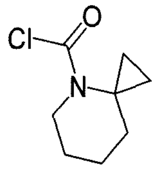 | MS(CI) m/z; 174/176 $[M+H]^+$ |
| 41-3 | 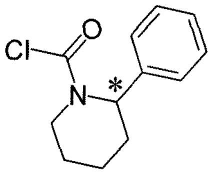 racemate | MS(CI) m/z; 224/226 $[M+H]^+$ |
| 63-3 | 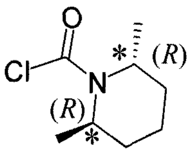 | MS(CI) m/z; 176/178 $[M+H]^+$ |
| 65-3 | 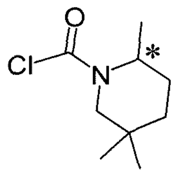 racemate | MS(CI) m/z; 190/192 $[M+H]^+$ |
| 66-3 | 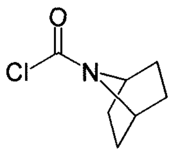 | MS(CI) m/z; 160/162 $[M+H]^+$ |

Reference Example 21-2

Preparation of (S)-N'-(3-chloropyrazin-2-yl)-2-methylpiperidine-1-carbohydrazide

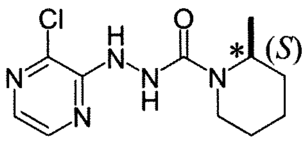

(1) To a 100 mL eggplant flask were added (S)-2-methylpiperidine (0.30 g), pyridine (0.24 mL), and dichloromethane (9 mL), and a solution of triphosgene (0.29 g) in dichloromethane (4 mL) was added thereto under argon gas flow with stirring at 0°C. Then, the resulting mixture was stirred at room temperature for 2 hours. After the reaction was completed, 2N hydrochloric acid was added thereto, and the resulting mixture was extracted with dichloromethane. The resulting organic layer was washed with a saturated aqueous solution of sodium hydrogen carbonate, then dried over anhydrous magnesium sulfate, and concentrated under reduced pressure to give (S)-2-methylpiperidine-1-carbonylchloride (0.53 g) as a slightly red oil.
(2) To a 100 mL eggplant flask were added the above slightly red oil of (S)-2-methylpiperidine-1-carbonylchloride (0.53 g), diisopropylethylamine (1.57 mL), and dichloromethane (20 mL), 2-chloro-3-hydrazinylpyrazine (0.44 g)

was added thereto under argon gas flow with stirring at room temperature, and the resulting mixture was stirred at the same temperature for 24 hours. After the reaction was completed, water was added thereto, and the resulting mixture was extracted with dichloromethane. The resulting organic layer was washed with saturated brine, dried over anhydrous magnesium sulfate, and concentrated under reduced pressure. The resulting residues were subjected to Moritex medium pressure preparative (Purif-Pack, SI size 20 (10 g), hexane : ethyl acetate = 70 : 30 to 0 : 100), the fractions comprising the title compound were collected, concentrated under reduced pressure, ethyl acetate and diisopropyl ethyl ether were added thereto, the precipitated solid was collected by filtration, and washed with diisopropyl ethyl ether to give the title compound (23.7 mg) (yield 2.9%) as a colorless solid.
MS(DUIS) m/z: 270/272 [M+H]$^+$

Reference Example 26-2 etc.:

A corresponding starting compound was reacted in a similar manner to the Reference Example 21-2 to give each compound described in the following Table 30.

Table 30

| Reference Example | Structural formula | Physical property etc. |
|---|---|---|
| 26-2 | 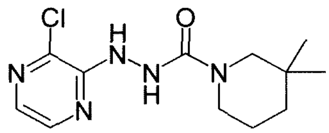 | MS(CI) m/z; 284/286 [M+H]$^+$ |
| 36-2 | 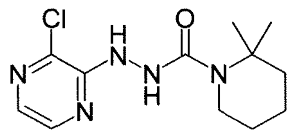 | MS(DUIS) m/z; 284/286 [M+H]$^+$ |
| 80-4 | 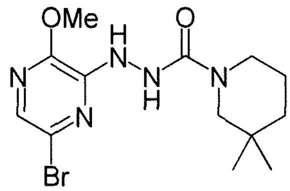 | MS(DUIS) m/z; 358/360 [M+H]$^+$ |

Reference Example 44-2

Preparation of N'-(3-chloropyrazin-2-yl)-5-azaspiro[2,5]octane-5-carbohydrazide

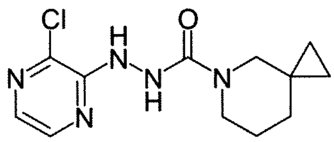

(1) To a 100 mL three-necked flask were added triphosgene (0.29 g) and dichloromethane (15 mL), pyridine (0.262 mL) was added dropwise thereto under argon gas flow with stirring so that the temperature would not exceed 10°C, and the resulting mixture was stirred at 0°C for 1 hour. Then, a dichloromethane solution comprising 5-azaspiro[2,5]octane (300 mg) prepared in the Reference Example 44-3 was added dropwise thereto so that the temperature would not exceed 10°C, and the resulting mixture was stirred at room temperature for 95 minutes.
After the reaction was completed, 1N hydrochloric acid (50 mL) was added thereto, and the resulting mixture was extracted with dichloromethane. The resulting organic layer was washed with a saturated aqueous solution of sodium hydrogen carbonate, then dried over anhydrous magnesium sulfate, and concentrated under reduced pressure to give 5-azaspiro[2.5]octane-5-carbonyl chloride (440 mg) as a brown oil.
(2) To a 100 mL eggplant flask were added 2-chloro-3-hydrazinylpyrazine (370 mg), diisopropylethylamine (1.3 mL),

acetonitrile (15 mL), and the above 5-azaspiro[2.5]octane-5-carbonyl chloride (435 mg) under argon gas flow, and the resulting mixture was stirred at 80°C for 100 minutes.

After the reaction was completed, the mixture was concentrated under reduced pressure, the resulting residues were subjected to silica gel chromatography using YAMAZEN medium pressure preparative (Silica L (40 g)), the fractions comprising the target compound (Rf value = 0.4 (hexane : ethyl acetate = 1 : 1)) were collected, and concentrated under reduced pressure to give the title compound (330 mg) (yield 47%) as a slightly yellow foam.
MS(CI) m/z: 282/284 [M+H]$^+$

Reference Example 46-2 etc.:

A corresponding starting compound was reacted in a similar manner to the Reference Example 44-2 to give each compound described in the following Table 31.

Table 31

| Reference Example | Structural formula | Physical property etc. |
|---|---|---|
| 46-2 | 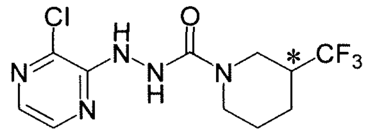 racemate | MS(CI) m/z; 324/326 [M+H]$^+$ |
| 47-2 | 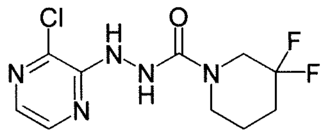 | MS(CI) m/z; 290/292 [M+H]$^+$ |
| 64-2 | 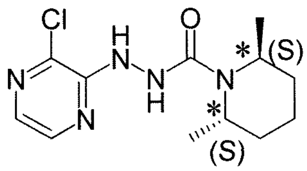 | MS(CI) m/z; 284/286 [M+H]$^+$ |
| 67-2 | 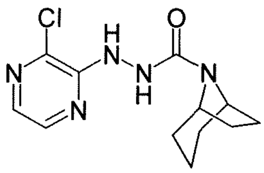 | MS(CI) m/z; 282/284 [M+H]$^+$ |
| 58-2 | 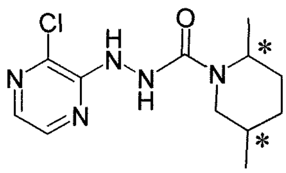 mixture of four types of stereoisomers | MS(CI) m/z; 284/286 [M+H]$^+$ |

Reference Example 22-3

Preparation of (R)-ethyl 2-methylpiperidine-1-carboxylate

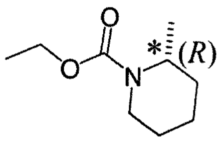

To a 100 mL eggplant flask were added (R)-2-methylpiperidine (1.00 g), dimethylaminopyridine (1.49 g), and dichloromethane (10 mL), ethyl chloroformate (1.37 g) was added dropwise thereto under argon gas flow with stirring under water-cooling, and then the resulting mixture was stirred at room temperature for 16 hours. After the reaction was completed, water was added thereto, and the resulting mixture was extracted with diisopropyl ether. The resulting organic layer was dried over anhydrous magnesium sulfate, and concentrated under reduced pressure to give the title compound (1.42 g) (yield 81%) as a colorless oil.
MS(CI) m/z: 172 $[M+H]^+$

Reference Example 23-4 etc.:

A corresponding starting compound was reacted in a similar manner to the Reference Example 22-3 to give each compound described in the following Table 32.

Table 32

| Reference Example | Structural formula | Physical property etc. |
|---|---|---|
| 23-4 | 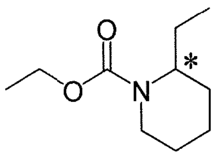 racemate | MS(CI) m/z; 186 $[M+H]^+$ |
| 24-4 | 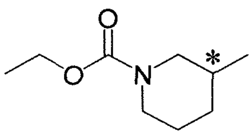 racemate | MS(DUIS) m/z; 172 $[M+H]^+$ |
| 25-3 | 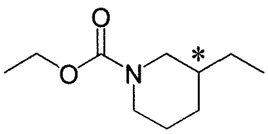 racemate | MS(CI) m/z; 186 $[M+H]^+$ |
| 27-4 | 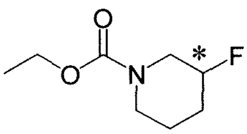 racemate | MS(CI) m/z; 176 $[M+H]^+$ |
| 48-4 | 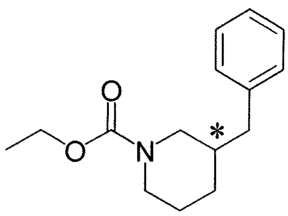 racemate | MS(CI) m/z; 248 $[M+H]^+$ |

(continued)

| Reference Example | Structural formula | Physical property etc. |
|---|---|---|
| 49-4 | 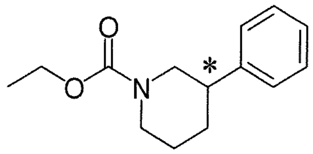 racemate | MS(CI) m/z; 234 [M+H]$^+$ |
| 28-4 | 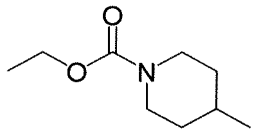 | MS(DUIS) m/z; 172 [M+H]$^+$ |
| 50-4 | 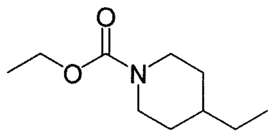 | MS(CI) m/z; 186 [M+H]$^+$ |
| 51-3 | 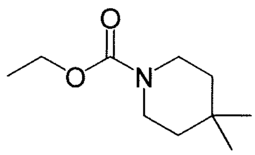 | MS(CI) m/z; 186 [M+H]$^+$ |
| 78-3 | 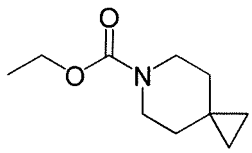 | MS(DUIS) m/z; 184 [M+H]$^+$ |
| 52-3 | 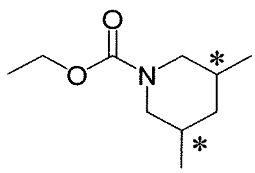 mixture of four types of stereoisomers | MS(CI) m/z; 186 [M+H]$^+$ |
| 55-4 | 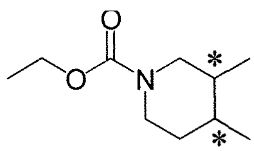 mixture of four types of stereoisomers | MS(CI) m/z; 186 [M+H]$^+$ |
| 56-4 | 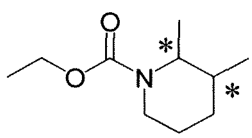 mixture of four types of stereoisomers | MS(CI) m/z; 186 [M+H]$^+$ |
| 61-4 | 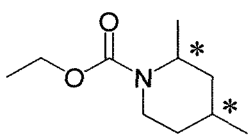 mixture of four types of stereoisomers | MS(CI) m/z; 186 [M+H]$^+$ |

(continued)

| Reference Example | Structural formula | Physical property etc. |
|---|---|---|
| 29-4 | 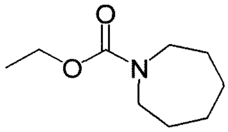 | MS(CI) m/z; 172 [M+H]$^+$ |
| 30-4 | 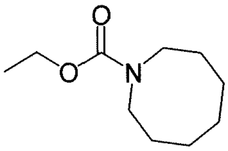 | MS(CI) m/z; 186 [M+H]$^+$ |
| 31-4 | 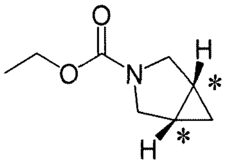 | MS(CI) m/z; 156 [M+H]$^+$ |

Reference Example 23-3

Preparation of 2-ethylpiperidine-1-carbonylchloride

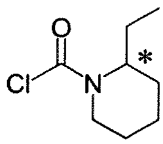

To a 200 mL eggplant flask were added ethyl 2-ethylpiperidine-1-carboxylate (2.29 g) prepared in the Reference Example 23-4, acetonitrile (25 mL), and phosphoryl chloride (9.87 g) under argon gas flow at room temperature, and the resulting mixture was stirred at 100°C for 7.5 hours. After the reaction was completed, the reaction solution was poured into ice, the resulting mixture was stirred for 30 minutes, and extracted with dichloromethane. The resulting organic layer was washed with water and a saturated aqueous solution of sodium chloride, then dried over anhydrous magnesium sulfate, and concentrated under reduced pressure to give the title compound (2.18 g) (yield 100%) as a yellow oil.
MS(CI) m/z: 176/178 [M+H]$^+$

Reference Example 24-3 etc.:

A corresponding starting compound was reacted in a similar manner to the Reference Example 23-3 to give each compound described in the following Table 33.

Table 33

| Reference Example | Structural formula | Physical property etc. |
|---|---|---|
| 24-3 | 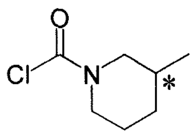 racemate | MS(CI) m/z; 162/164 [M+H]$^+$ |

(continued)

| Reference Example | Structural formula | Physical property etc. |
|---|---|---|
| 27-3 | 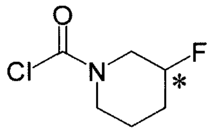 racemate | MS(CI) m/z; 166/168 $[M+H]^+$ |
| 48-3 | 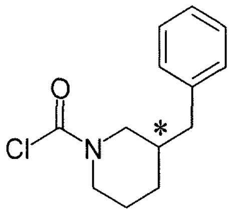 racemate | MS(CI) m/z; 238/240 $[M+H]^+$ |
| 49-3 | 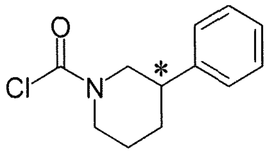 racemate | MS(CI) m/z; 224/226 $[M+H]^+$ |
| 28-3 | 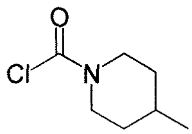 | MS(CI) m/z; 176/178 $[M+H]^+$ |
| 50-3 | 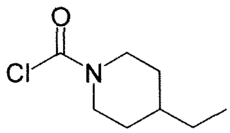 | MS(CI) m/z; 162/164 $[M+H]^+$ |
| 54-4 | 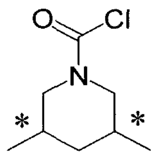 trans, racemate | MS(CI) m/z; 176/178 $[M+H]^+$ |
| 55-3 | 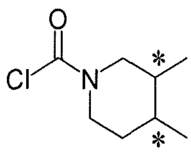 mixture of four types of stereoisomers | MS(CI) m/z; 176/178 $[M+H]^+$ |
| 56-3 | 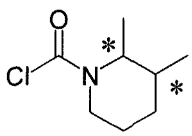 mixture of four types of stereoisomers | MS(CI) m/z; 176/178 $[M+H]^+$ |

(continued)

| Reference Example | Structural formula | Physical property etc. |
|---|---|---|
| 60-4 | 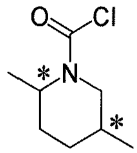 cis, racemate | MS(CI) m/z; 176/178 [M+H]$^+$ |
| 61-3 | 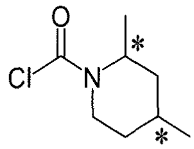 mixture of four types of stereoisomers | MS(CI) m/z; 176/178 [M+H]$^+$ |
| 29-3 | 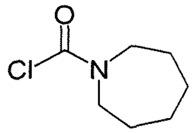 | MS(CI) m/z; 162/164 [M+H]$^+$ |
| 30-3 | 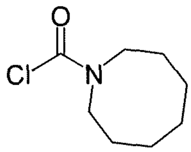 | MS(CI) m/z; 176/178 [M+H]$^+$ |
| 31-3 | 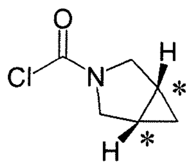 | MS(CI) m/z; 146/148 [M+H]$^+$ |

Reference Example 25-2

Preparation of N'-(3-chloropyrazin-2-yl)-3-ethylpiperidine-1-carbohydrazide

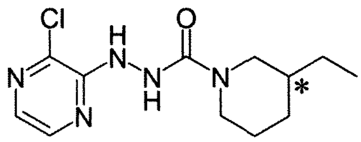

racemate

(1) To a 100 mL eggplant flask were added ethyl 3-ethylpiperidine-1-carboxylate (1.8 g) prepared in the Reference Example 25-3, acetonitrile (15 mL), and phosphorus oxychloride (4.1 mL), and the resulting mixture was stirred at 105°C in a bath for 8 hours.

After the reaction was completed, toluene was added thereto, and the resulting mixture was concentrated under reduced pressure. To the resulting residues was added dichloromethane, the resulting mixture was washed with a saturated aqueous solution of sodium hydrogen carbonate, dried over anhydrous sodium sulfate, and concentrated under reduced pressure. To the resulting residues was added toluene, and the resulting mixture was concentrated under reduced pressure to give a yellow oil (1.65 g).

(2) To a 100 mL eggplant flask were added the yellow oil (1.65 g) prepared in the above step, 2-chloro-3-hydrazinylpyrazine (1.26 g), and diisopropylethylamine (4.56 mL) under argon gas flow, and the resulting mixture was stirred at 80°C for 4 hours.

After the reaction was completed, the mixture was concentrated under reduced pressure, water was added thereto, and the resulting mixture was extracted with ethyl acetate. The resulting organic layer was dried over anhydrous magnesium sulfate, and concentrated under reduced pressure. The resulting residues were subjected to silica gel chromatography (hexane : ethyl acetate = 70 : 30 to 50 : 50) using YAMAZEN medium pressure preparative (Silica L (40 g)), the fractions comprising the target compound (Rf value = 0.25 (hexane : ethyl acetate = 1 : 1)) were collected, concentrated under reduced pressure, to the resulting residues was added diisopropyl ether, the precipitated solid was filtered, and dried under reduced pressure to give the title compound (1.53 g) (yield 62%) as a white solid.
MS(CI) m/z: 284/286 $[M+H]^+$

Reference Example 22-2 etc.:

A corresponding starting compound was reacted in a similar manner to the Reference Example 25-2 to give each compound described in the following Table 34.

Table 34

| Reference Example | Structural formula | Physical property etc. |
|---|---|---|
| 22-2 | 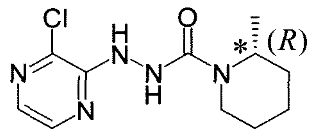 | MS(CI) m/z; 270/272 $[M+H]^+$ |
| 51-2 | 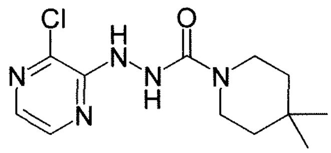 | MS(CI) m/z; 284/286 $[M+H]^+$ |
| 52-2 | 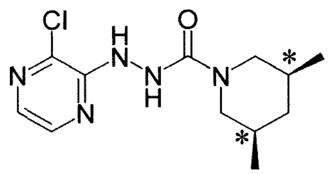 cis | MS(CI) m/z; 284/286 $[M+H]^+$ |
| 53-2 | 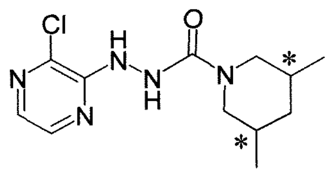 trans, racemate | MS(CI) m/z; 284/286 $[M+H]^+$ |
| 78-2 | 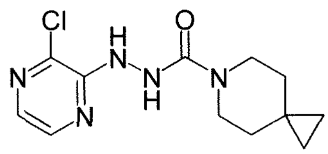 | MS(CI) m/z; 282/284 $[M+H]^+$ |

Reference Example 23-2

Preparation of N'-(3-chloropyrazin-2-yl)-2-ethylpiperidine-1-carbohydrazide

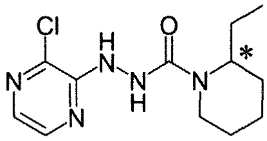

racemate

To a 100 mL eggplant flask were added 2-ethylpiperidine-1-carbonylchloride (509 mg) prepared in the Reference Example 23-3, diisopropylethylamine (1.11 g), acetonitrile (10 mL), and 2-chloro-3-hydrazinylpyrazine (947 mg) under argon gas flow at room temperature, and the resulting mixture was stirred at 80°C for 1 hour. After the reaction was completed, the reaction solution was concentrated under reduced pressure. The resulting residues were subjected to silica gel chromatography (hexane : ethyl acetate) using YAMAZEN medium pressure preparative (Silica L (40 g)), the fractions comprising the target compound (Rf value = 0.70 (ethyl acetate)) were collected, and concentrated under reduced pressure to give the title compound (332 mg) (yield 36%) as a yellow foam. MS(CI) m/z: 284/286 $[M+H]^+$

Reference Example 24-2 etc.:

A corresponding starting compound was reacted in a similar manner to the Reference Example 23-2 to give each compound described in the following Table 35.

Table 35

| Reference Example | Structural formula | Physical property etc. |
|---|---|---|
| 79-2 | 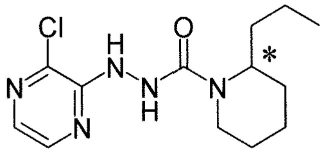 racemate | MS(CI) m/z; 298/300 $[M+H]^+$ |
| 37-2 | 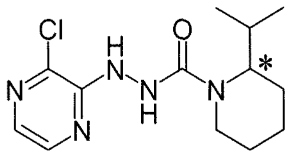 racemate | MS(CI) m/z; 298/300 $[M+H]^+$ |
| 38-2 | 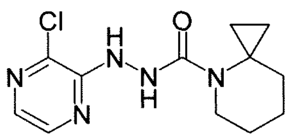 | MS(CI) m/z; 282/284 $[M+H]^+$ |
| 39-2 | 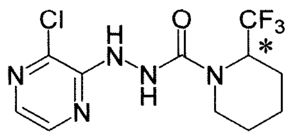 racemate | MS(CI) m/z; 324/326 $[M+H]^+$ |
| 40-2 | 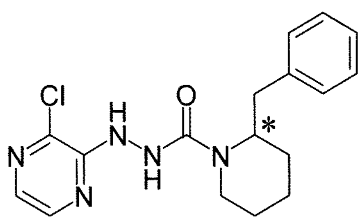 racemate | MS(CI) m/z; 346/348 $[M+H]^+$ |

(continued)

| Reference Example | Structural formula | Physical property etc. |
|---|---|---|
| 41-2 | 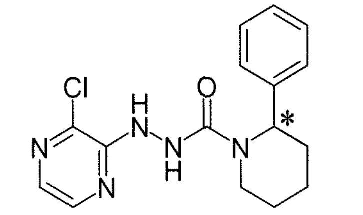 racemate | MS(CI) m/z; 332/334 [M+H]+ |
| 24-2 | 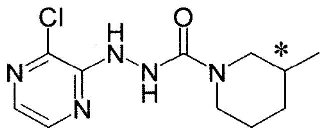 racemate | MS(CI) m/z; 270/272 [M+H]+ |
| 45-2 | 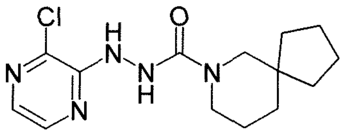 | MS(CI) m/z; 310/312 [M+H]+ |
| 27-2 | 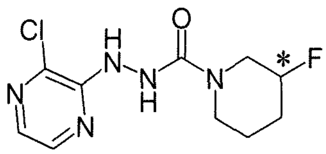 racemate | MS(CI) m/z; 274/276 [M+H]+ |
| 48-2 | 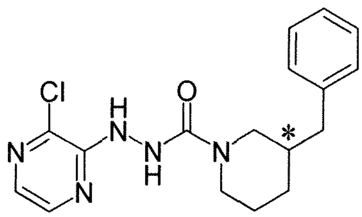 racemate | MS(CI) m/z; 346/348 [M+H]+ |
| 49-2 | 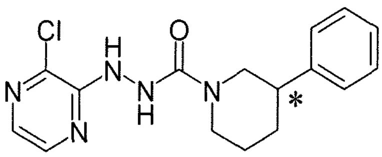 racemate | MS(CI) m/z; 332/334 [M+H]+ |
| 28-2 | 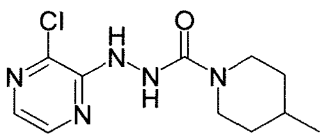 | MS(CI) m/z; 270/272 [M+H]+ |
| 50-2 | 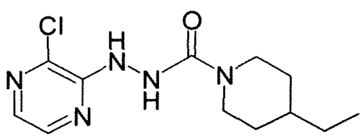 | MS(CI) m/z; 284/286 [M+H]+ |

(continued)

| Reference Example | Structural formula | Physical property etc. |
|---|---|---|
| 54-3 | 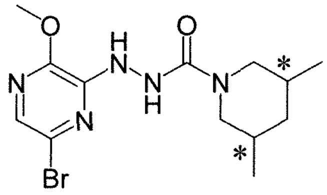 trans, racemate | MS(CI) m/z; 358/360 [M+H]$^+$ |
| 55-2 | 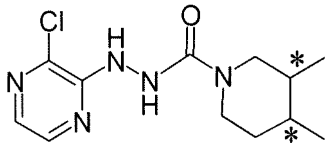 mixture of four types of stereoisomers | MS(CI) m/z; 284/286 [M+H]$^+$ |
| 56-2 | 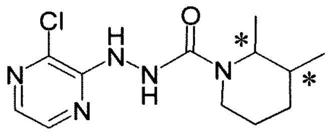 mixture of four types of stereoisomers | MS(CI) m/z; 284/286 [M+H]$^+$ |
| 60-3 | 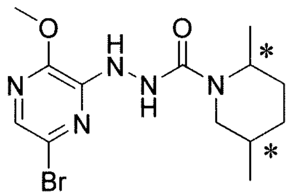 cis, racemate | MS(CI) m/z; 358/360 [M+H]$^+$ |
| 61-2 | 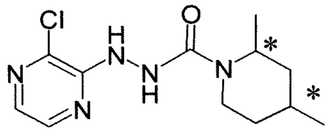 mixture of four types of stereoisomers | MS(CI) m/z; 284/286 [M+H]$^+$ |
| 63-2 | 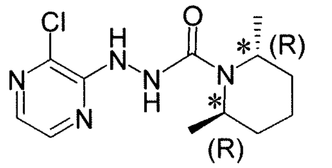 | MS(CI) m/z; 284/286 [M+H]$^+$ |
| 65-2 | 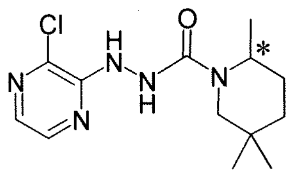 racemate | MS(CI) m/z; 298/300 [M+H]$^+$ |
| 29-2 | 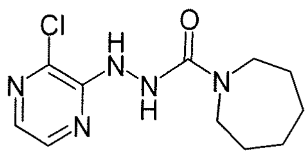 | MS(CI) m/z; 270/272 [M+H]$^+$ |

(continued)

| Reference Example | Structural formula | Physical property etc. |
|---|---|---|
| 30-2 | 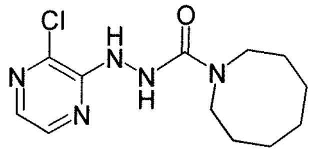 | MS(CI) m/z; 284/286 $[M+H]^+$ |
| 31-2 | 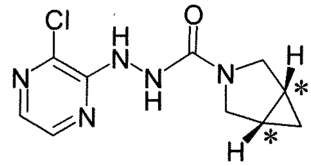 | MS(CI) m/z; 254/256 $[M+H]^+$ |
| 66-2 | 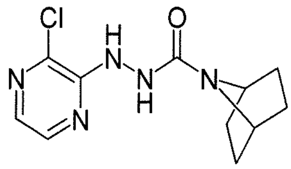 | MS(CI) m/z; 268/270 $[M+H]^+$ |

Reference Example 142-1

Preparation of 8-chloro-3-(1-fluorocyclohexyl) [1,2,4]triazolo[4,3-a]pyrazine

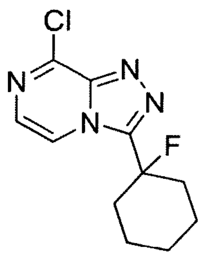

To a mixture of N'-(3-chloropyrazin-2-yl)-1-fluorocyclohexanecarbohydrazide (388 mg) prepared in the Reference Example 142-2, triethylamine (0.79 mL), triphenylphosphine (746 mg), and tetrahydrofuran (8 mL) was added hexachloroethane (674 mg) in two additions under ice-cooling. The reaction mixture was allowed to cool to room temperature, and stirred for 3 hours and 30 minutes. To the reaction mixture was added water, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 90/10 to 70/30) to give the title compound (338 mg) (yield 93%) as a colorless powder. MS(APCI) m/z: 255/257 $[M+H]^+$

Reference Example 144-1 etc.:

A corresponding starting compound was reacted in a similar manner to the Reference Example 142-1 to give each compound described in the following Table 36.

Table 36

| Reference Example | Structural formula | Physical property etc. |
|---|---|---|
| 144-1 | 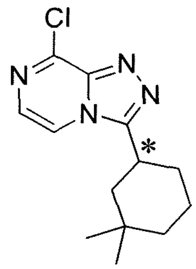 racemate | MS(ESI) m/z; 265/267 [M+H]+ |
| 145-1 | 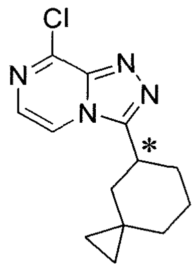 racemate | MS(ESI) m/z; 263/265 [M+H]+ |
| 146-1 | 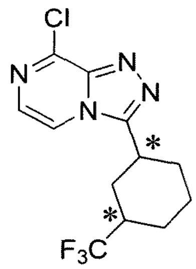 cis, racemate | MS(ESI) m/z; 305/307 [M+H]+ |
| 147-1 | 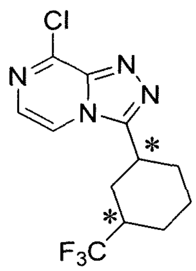 trans, racemate | MS(ESI) m/z; 305/307 [M+H]+ |
| 148-1 | 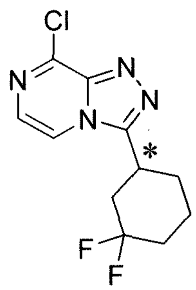 racemate | MS(ESI) m/z; 273/275 [M+H]+ |

(continued)

| Reference Example | Structural formula | Physical property etc. |
|---|---|---|
| 150-1 | 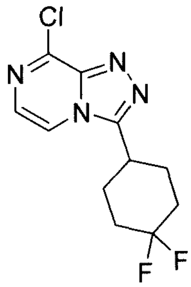 | MS(ESI) m/z; 273/275 [M+H]+ |
| 151-1 | 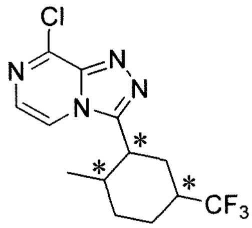 relative configuration (1R*, 2S*, 5R*), racemate | MS(APCI) m/z; 319/321 [M+H]+ |
| 152-1 | 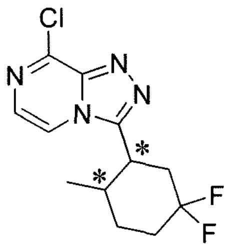 cis, racemate | MS(ESI) m/z; 287/289 [M+H]+ |
| 153-1 | 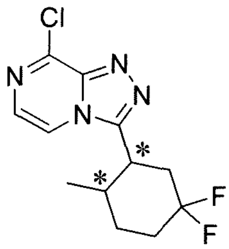 trans, racemate | MS(ESI) m/z; 287/289 [M+H]+ |
| 154-1 | 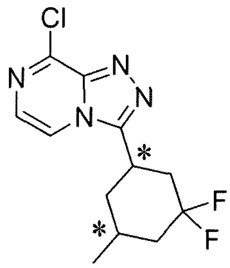 cis, racemate | MS(ESI) m/z; 287/289 [M+H]+ |

(continued)

| Reference Example | Structural formula | Physical property etc. |
|---|---|---|
| 155-1 | 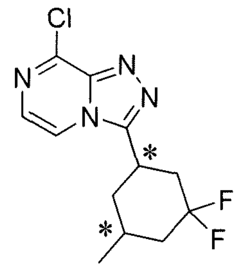 trans, racemate | MS(ESI) m/z; 287/289 [M+H]+ |
| 156-1 | 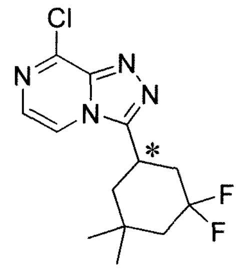 racemate | MS(ESI) m/z; 301/303 [M+H]+ |
| 157-1 | 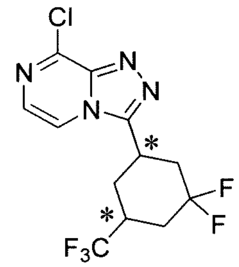 cis, racemate | MS(ESI) m/z; 341/343 [M+H]+ |
| 172-4 | 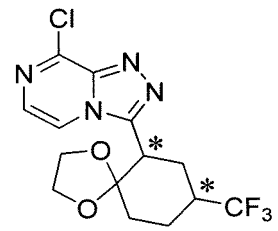 cis, racemate | MS(APCI) m/z; 363/365 [M+H]+ |
| 158-1 | 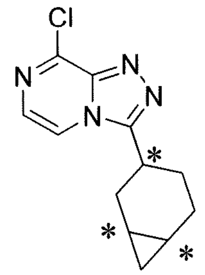 cyclopropane in bicyclo[4,1,0]hep tane ring is cis isomer mixture of four types of stereoisomers | MS(ESI) m/z; 249/251 [M+H]+ |

(continued)

| Reference Example | Structural formula | Physical property etc. |
|---|---|---|
| 159-1 | 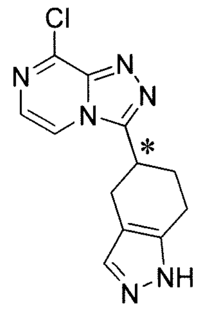 racemate | MS(ESI) m/z; 275/277 [M+H]+ |
| 160-1 | 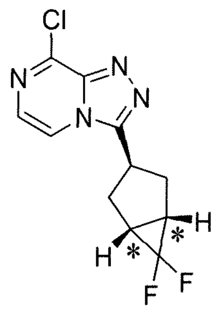 | MS(ESI) m/z; 271/273 [M+H]+ |
| 162-1 | 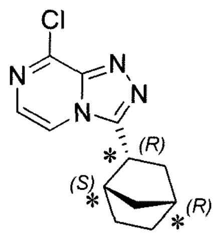 | MS(ESI) m/z; 249/251 [M+H]+ |
| 163-1 | 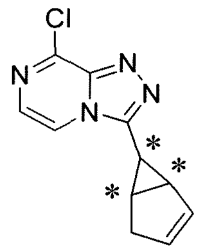 relative configuration (1S*, 5R*, 6S*) racemate | MS(ESI) m/z; 233/235 [M+H]+ |
| 164-1 | 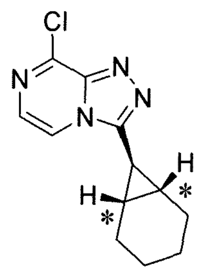 | MS(ESI) m/z; 249/251 [M+H]+ |

(continued)

| Reference Example | Structural formula | Physical property etc. |
|---|---|---|
| 167-1 | 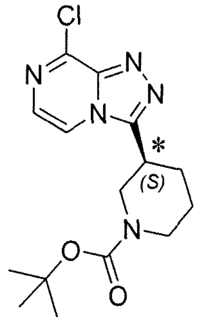 | MS(APCI) m/z; 338/340 $[M+H]^+$ |
| 168-1 | 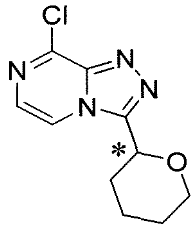 racemate | MS(ESI) m/z; 239/241 $[M+H]^+$ |
| 169-1 | 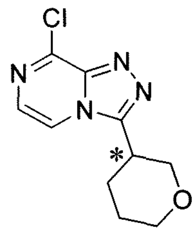 racemate | MS(ESI) m/z; 239/241 $[M+H]^+$ |

Reference Example 20-1

Preparation of 8-chloro-3-(spiro[2.3]hexan-5-yl)-[1,2,4]triazolo[4,3-a]pyrazine

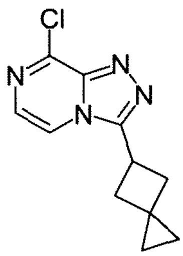

To a 100 mL eggplant flask were added dividedly N'-(3-chloropyrazin-2-yl)spiro[2.3]hexane-5-carbohydrazide (660 mg) prepared in the Reference Example 20-2, triphenylphosphine (1.37 g), triethylamine (1052.7 mg), tetrahydrofuran (10 mL), and hexachloroethane (1.24 g) under argon atmosphere at room temperature, and the resulting mixture was stirred at room temperature for 3 hours. After the reaction was completed, water was added thereto, and the resulting mixture was extracted with ethyl acetate. The resulting organic layer was washed with saturated brine, dried over anhydrous magnesium sulfate, and the resulting mixture was concentrated under reduced pressure. The resulting residues were subjected to silica gel column chromatography (Rf value = 0.3 (solvent: hexane/ethyl acetate = 2 : 1)) (Silica

L (40 g)) using YAMAZEN medium pressure preparative column, the fractions comprising the target compound were concentrated under reduced pressure, to the resulting residues was added hexane, the resulting mixture was subjected to sonication, filtered, washed with hexane, and dried to give the title compound (440 mg) (yield 72%) as a white solid. MS(DUIS) m/z: 235/237 [M+H]+

Reference Example 21-1 etc.:

A corresponding starting compound was reacted in a similar manner to the Reference Example 20-1 to give each compound described in the following Table 37.

Table 37

| Reference Example | Structural formula | Physical property etc. |
|---|---|---|
| 21-1 | 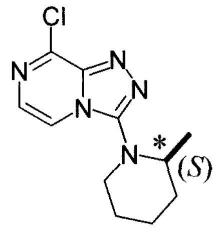 | MS(CI) m/z; 252/254 [M+H]+ |
| 22-1 | 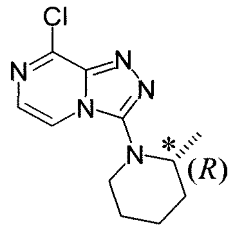 | MS(CI) m/z; 252/254 [M+H]+ |
| 36-1 | 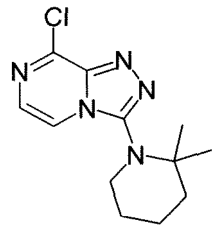 | MS(CI) m/z; 266/268 [M+H]+ |
| 23-1 | 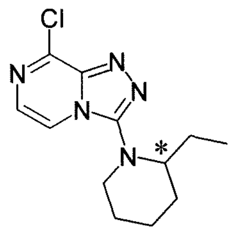 racemate | MS(CI) m/z; 266/268 [M+H]+ |
| 79-1 | 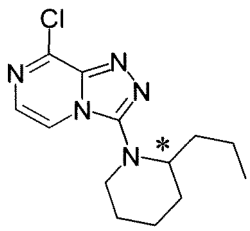 racemate | MS(CI) m/z; 280/282 [M+H]+ |

(continued)

| Reference Example | Structural formula | Physical property etc. |
|---|---|---|
| 37-1 | 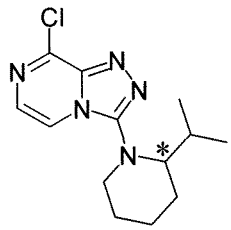 racemate | MS(CI) m/z; 280/282 $[M+H]^+$ |
| 38-1 | 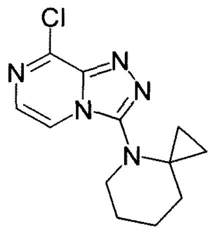 | MS(CI) m/z; 264/266 $[M+H]^+$ |
| 39-1 | 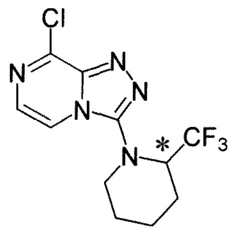 racemate | MS(CI) m/z; 306/308 $[M+H]^+$ |
| 40-1 | 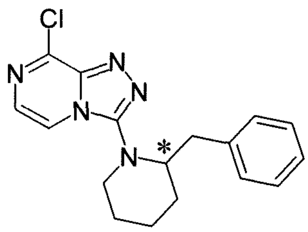 racemate | MS(CI) m/z; 328/330 $[M+H]^+$ |
| 41-1 | 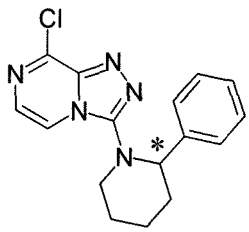 racemate | MS(CI) m/z; 314/316 $[M+H]^+$ |
| 24-1 | 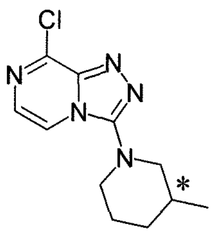 racemate | MS(CI) m/z; 252/254 $[M+H]^+$ |

(continued)

| Reference Example | Structural formula | Physical property etc. |
|---|---|---|
| 25-1 | 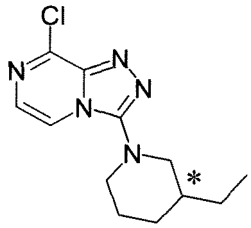 racemate | MS(CI) m/z; 266/268 [M+H]$^+$ |
| 26-1 | 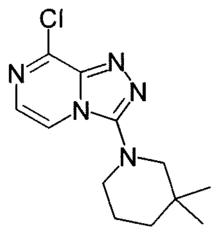 | MS(CI) m/z; 266/268 [M+H]$^+$ |
| 80-3 | 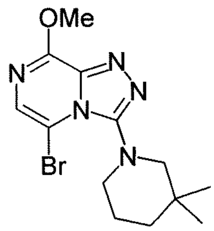 | MS(DUIS) m/z; 340/342 [M+H]$^+$ |
| 44-1 | 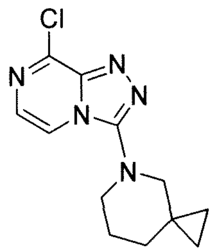 | MS(CI) m/z; 264/266 [M+H]$^+$ |
| 45-1 | 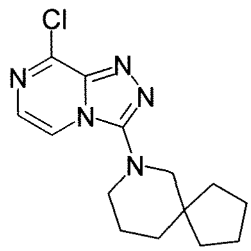 | MS(CI) m/z; 292/294 [M+H]$^+$ |
| 46-1 | 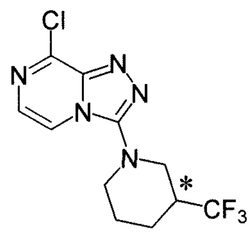 racemate | MS(CI) m/z; 306/308 [M+H]$^+$ |

(continued)

| Reference Example | Structural formula | Physical property etc. |
|---|---|---|
| 27-1 | 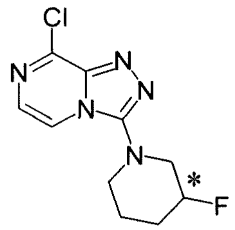 racemate | MS(CI) m/z; 256/258 [M+H]+ |
| 47-1 | 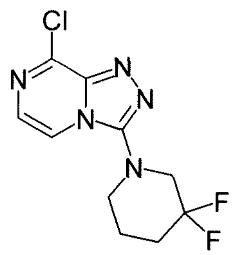 | MS(CI) m/z; 274/276 [M+H]+ |
| 48-1 | 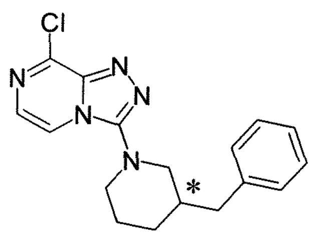 racemate | MS(CI) m/z; 328/330 [M+H]+ |
| 49-1 | 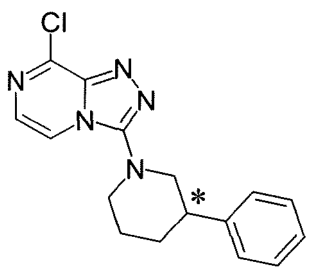 racemate | MS(CI) m/z; 314/316 [M+H]+ |
| 28-1 | 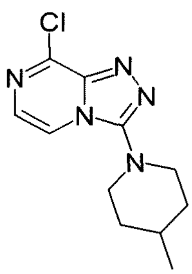 | MS(CI) m/z; 252/254 [M+H]+ |

(continued)

| Reference Example | Structural formula | Physical property etc. |
|---|---|---|
| 50-1 | 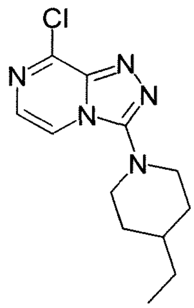 | MS(CI) m/z; 266/268 [M+H]+ |
| 51-1 | 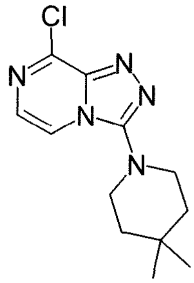 | MS(CI) m/z; 266/268 [M+H]+ |
| 78-1 | 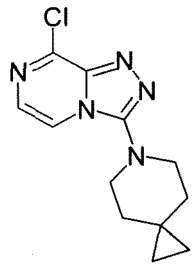 | MS(DUIS) m/z; 264/266 [M+H]+ |
| 52-1 | 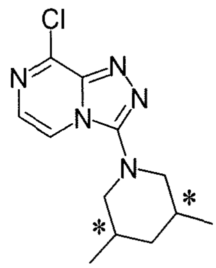 cis | MS(CI) m/z; 266/268 [M+H]+ |
| 53-1 | 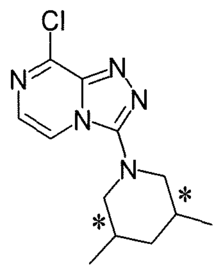 trans, racemate | MS(CI) m/z; 266/268 [M+H]+ |

(continued)

| Reference Example | Structural formula | Physical property etc. |
|---|---|---|
| 54-2 | 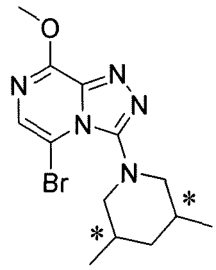<br>trans, racemate | MS(CI) m/z; 340/342 $[M+H]^+$ |
| 55-1 | 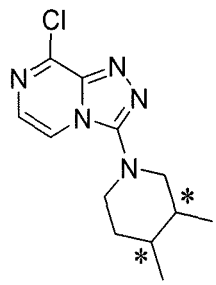<br>mixture of four types of stereoisomers | MS(CI) m/z; 266/268 $[M+H]^+$ |
| 56-1 | 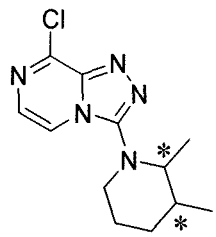<br>unknown relative configuration single diastereomer racemate | MS(CI) m/z; 266/268 $[M+H]^+$ |
| 57-1 | 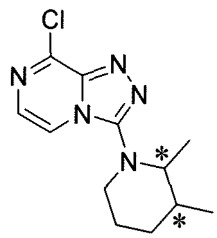<br>unknown relative configuration single diastereomer different from Reference Example 56-1 racemate | MS(CI) m/z; 266/268 $[M+H]^+$ |
| 58-1 | 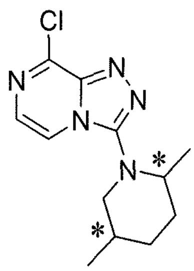<br>cis, racemate | MS(CI) m/z; 266/268 $[M+H]^+$ |

(continued)

| Reference Example | Structural formula | Physical property etc. |
|---|---|---|
| 59-1 | 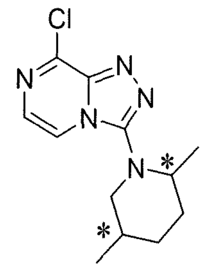 trans, racemate | MS(CI) m/z; 266/268 [M+H]+ |
| 60-2 | 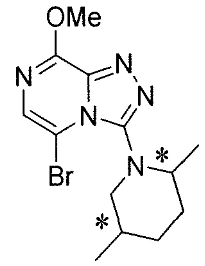 cis, racemate | MS(DUIS) m/z; 340/342 [M+H]+ |
| 61-1 | 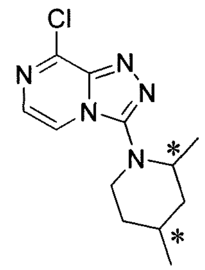 trans, racemate | MS(CI) m/z; 266/268 [M+H]+ |
| 62-1 | 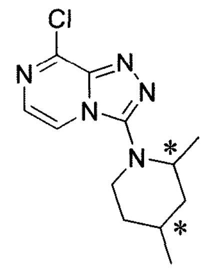 cis, racemate | MS(CI) m/z; 266/268 [M+H]+ |
| 63-1 | 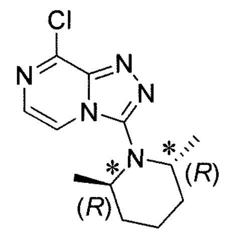 | MS(CI) m/z; 266/268 [M+H]+ |

(continued)

| Reference Example | Structural formula | Physical property etc. |
|---|---|---|
| 64-1 | 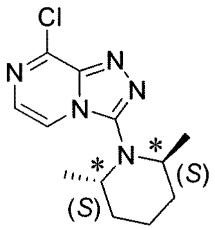 | MS(CI) m/z; 266/268 [M+H]+ |
| 65-1 | 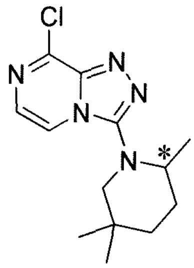 racemate | MS(CI) m/z; 280/282 [M+H]+ |
| 29-1 | 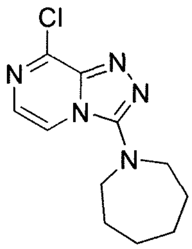 | MS(CI) m/z; 252/254 [M+H]+ |
| 30-1 | 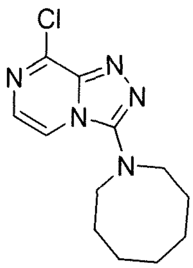 | MS(CI) m/z; 266/268 [M+H]+ |
| 31-1 | 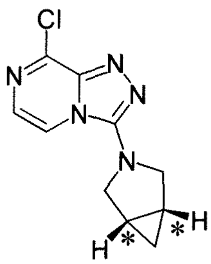 | MS(CI) m/z; 236/238 [M+H]+ |
| 66-1 | 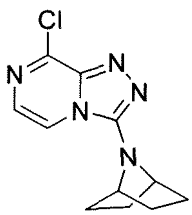 | MS(CI) m/z; 250/252 [M+H]+ |

(continued)

| Reference Example | Structural formula | Physical property etc. |
|---|---|---|
| 67-1 | 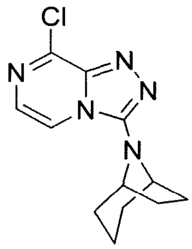 | MS(CI) m/z; 264/266 $[M+H]^+$ |
| 32-1 | 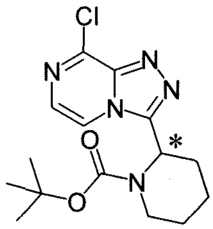<br>racemate | MS(CI) m/z; 338/340 $[M+H]^+$ |

Reference Example 249-1

Preparation of 3-(piperidin-2-yl)-[1,2,4]triazolo[4,3-a]pyrazin-8-amine trihydrochloride

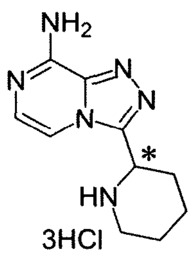

racemate

To a 20 mL cylindrical flask were added tert-butyl 2-(8-amino-[1,2,4]triazolo[4,3-a]pyrazin-3-yl)piperidine-1-carboxylate (160 mg) prepared in the Example 32 and 4N hydrogen chloride/1,4-dioxane (2.5 mL), and the resulting mixture was stirred at room temperature for 10 minutes. Then, concentrated hydrochloric acid (2 mL) was added thereto, and the resulting mixture was stirred at room temperature for 1 hour. After the reaction was completed, the solvent was concentrated under reduced pressure, ethanol was added thereto, the resulting mixture was stirred, then the resulting solid was collected by filtration, and washed with ethanol to give the title compound (150 mg) (yield 91% (as trihydrochloride)) as a white solid.
MS(DUIS) m/z: 219 $[M+H]^+$

Reference Example 80-2

Preparation of 3-(3,3-dimethylpiperidin-1-yl)-8-methoxy-5-methyl-[1,2,4]triazolo[4,3-a]pyrazine

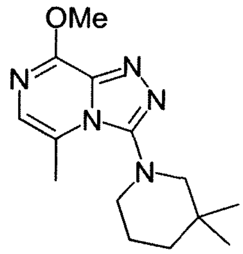

To a 10 mL cylindrical flask were added 5-bromo-3-(3,3-dimethylpiperidin-1-yl)-8-methoxy-[1,2,4]triazolo[4,3-a]pyrazine (89 mg) prepared in the Reference Example 80-3, 1,4-dioxane (1780 μL), [1,1'-bis(diphenylphosphino)ferrocene]palladium(II) dichloride ($PdCl_2$(dppf)) (3 mg), potassium carbonate (110 mg), and trimethylboroxine (40.41 mg) under argon gas flow at room temperature, and the resulting mixture was stirred at 110°C for 7 hours. After the reaction was completed, water was added thereto, and the resulting mixture was extracted with ethyl acetate. The resulting organic layer was washed with saturated brine, dried over anhydrous magnesium sulfate, and concentrated under reduced pressure. The resulting residues were subjected to silica gel chromatography (hexane : ethyl acetate = 45 : 55 to 24 : 76) using YAMAZEN medium pressure preparative column (Silica M (16 g)), the fractions comprising the target compound (Rf value = 0.13 (hexane : ethyl acetate = 1 : 1)) were collected, and concentrated under reduced pressure to give the title compound (54 mg) (yield 75%) as a pale brown amorphous. MS(DUIS) m/z: 276 $[M+H]^+$

Reference Example 42-2

Preparation of 3-(3,3-dimethylpiperidin-1-yl)-5-ethyl-8-methoxy-[1,2,4]triazolo[4,3-a]pyrazine

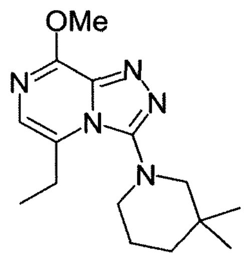

To a 0.5 to 2 mL flask were added 5-bromo-3-(3,3-dimethylpiperidin-1-yl)-8-methoxy-[1,2,4]triazolo[4,3-a]pyrazine (400 mg) prepared in the Reference Example 80-3, tetrahydrofuran (4.00 mL), and iron(III) acetylacetonate (21 mg) under argon gas flow, ethylmagnesium bromide (320 mg) was added dropwise thereto at -78°C, and the resulting mixture was stirred at -78°C for 15 minutes. Then, the mixture was warmed to room temperature. After the reaction was completed, 1N hydrochloric acid was added thereto, and the resulting mixture was extracted twice with ethyl acetate. The resulting organic layer was washed with a saturated aqueous solution of sodium hydrogen carbonate, dried over anhydrous sodium sulfate, filtered, and concentrated under reduced pressure. The resulting residues were subjected to silica gel chromatography (hexane : ethyl acetate = 60 : 40 to 35 : 65) using YAMAZEN medium pressure preparative (Silica L (40 g)), the fractions comprising the target compound (Rf value = 0.25 (hexane : ethyl acetate = 1 : 2)) were collected, and concentrated under reduced pressure to give the title compound (35 mg) (yield 10%) as a yellow oil.
MS(DUIS) m/z: 290 $[M+H]^+$

Reference Example 43-2

Preparation of 5-cyclopropyl-3-(3,3-dimethylpiperidin-1-yl)-8-methoxy-[1,2,4]triazolo[4,3-a]pyrazine

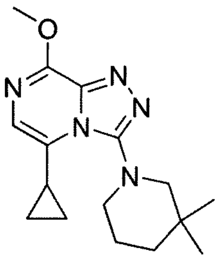

To a 30 mL cylindrical flask were added 5-bromo-3-(3,3-dimethylpiperidin-1-yl)-8-methoxy-[1,2,4]triazolo[4,3-a]pyrazine (200 mg) prepared in the Reference Example 80-3, cyclopropylboronic acid (80 mg), tricyclohexylphosphine (17 mg), potassium phosphate (400 mg), a toluene solution (2 mL), and water (0.25 mL), and the resulting mixture was subjected to nitrogen replacement. Then, palladium(II) acetate (10 mg) was added thereto, and the resulting mixture was heated with stirring at 100°C. After the reaction was completed, water was added thereto, and the resulting mixture was extracted with ethyl acetate. The resulting organic layer was washed sequentially with water and saturated brine, dried over anhydrous magnesium sulfate, and concentrated under reduced pressure. The resulting residues were subjected to silica gel chromatography (Rf value = 0.5 (hexane : ethyl acetate = 50 : 50)) using YAMAZEN medium pressure preparative (Silica L (40 g)), the fractions comprising the target compound were collected, and concentrated under reduced pressure to give the title compound (133 mg) (yield 75%) as a slightly yellow solid. MS(CI) m/z: 302 $[M+H]^+$

Reference Example 81-2

Preparation of 3-(3,3-dimethylpiperidin-1-yl)-8-methoxy-5-phenyl-[1,2,4]triazolo[4,3-a]pyrazine

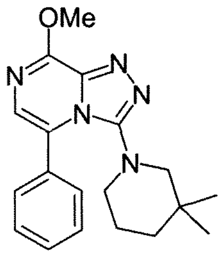

To a 20 mL cylindrical flask were added 5-bromo-3-(3,3-dimethylpiperidin-1-yl)-8-methoxy-[1,2,4]triazolo[4,3-a]pyrazine (100 mg) prepared in the Reference Example 80-3, 1,4-dioxane (500 μL), phenylboronic acid (45 mg), potassium carbonate (81 mg), and tetrakistriphenylphosphinepalladium (35 mg) under argon gas flow, and the resulting mixture was stirred at 110°C for 7 hours. After the reaction was completed, water was added thereto, and the resulting mixture was extracted with ethyl acetate. The resulting organic layer was washed with saturated brine, dried over anhydrous magnesium sulfate, and concentrated under reduced pressure. The resulting residues were subjected to silica gel chromatography (hexane : ethyl acetate = 50 : 50 to 30 : 70) using YAMAZEN medium pressure preparative (Silica M (16 g)), the fractions comprising the target compound (Rf value = 0.42 (hexane : ethyl acetate = 1 : 2)) were collected, and concentrated under reduced pressure to give the title compound (81 mg) (yield 82%) as a slightly yellow oil. MS(DUIS) m/z: 338 $[M+H]^+$

Reference Example 2-1

Preparation of 3-(3,3-dimethylpiperidin-1-yl)-8-methoxy-[1,2,4]triazolo[4,3-a]pyrazine-5-carbonitrile

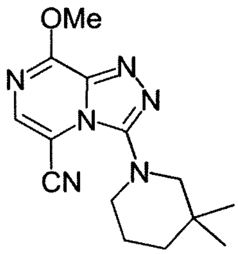

To a 10 to 20 mL cylindrical flask for microwave were added 5-bromo-3-(3,3-dimethylpiperidin-1-yl)-8-methoxy-[1,2,4]triazolo[4,3-a]pyrazine (400 mg) prepared in the Reference Example 80-3, N,N-dimethylformamide (8 mL), zinc dicyanide (85 mg), and $PdCl_2$(dppf) dichloromethane adduct (10 mg) under argon gas flow at room temperature, and the resulting mixture was stirred under microwave radiation at 120°C for 1 hour. To the resulting reaction solution was additionally added $PdCl_2$(dppf) dichloromethane adduct (10 mg), and the resulting mixture was stirred under microwave radiation at 120°C for 1 hour. Then, to the resulting reaction solution were added zinc (80 mg), tris(dibenzylideneacetone)dipalladium(0) ($Pd_2(dba)_3$) (110 mg), and 1,1'-bis(diphenylphosphino)ferrocene (abbreviated as dppf) (80 mg), and the resulting mixture was stirred under microwave radiation at 120°C for 1 hour. After the reaction was completed, a saturated aqueous solution of sodium hydrogen carbonate was added thereto, and the resulting mixture was extracted with ethyl acetate. The resulting organic layer was washed with saturated brine, dried over anhydrous sodium sulfate, and concentrated under reduced pressure. The resulting residues were subjected to silica gel chromatography using YAMAZEN medium pressure preparative column (Silica M (40 g)), the fractions comprising the target compound (Rf value = 0.4 (hexane : ethyl acetate = 1 : 2)) were collected, and concentrated under reduced pressure to give the title compound (260 mg) (yield 77%) as a pale orange solid.
MS(DUIS) m/z: 287 $[M+H]^+$

Reference Example 54-1 etc.:

A corresponding starting compound was reacted in a similar manner to the Reference Example 2-1 to give each compound described in the following Table 38.

Table 38

| Reference Example | Structural formula | Physical property etc. |
|---|---|---|
| 54-1 | 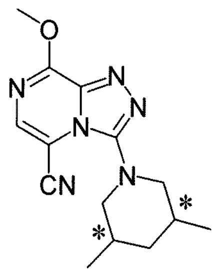 trans, racemate | MS(CI) m/z; 287 $[M+H]^+$ |
| 60-1 | 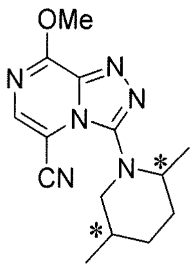 cis, racemate | MS(DUIS) m/z; 287 $[M+H]^+$ |

Reference Example 65-4

Preparation of 2,5,5-trimethylpiperidine hydrochloride

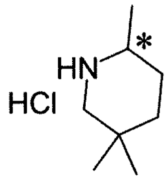

racemate

To a 50 mL eggplant flask were added a solution of 1-benzyl-2,5,5-trimethylpiperidine (100 mg) described in Tetrahedron, 2012, Vol.68, #15, p.3172-3178 in ethanol (30 mL) and 10% palladium-carbon (wetted with water) (20 mg) under argon gas flow at room temperature, the resulting mixture was subjected to hydrogen replacement, and then stirred at room temperature for 7 hours. 10% Palladium-carbon (wetted with water) (40 mg) was additionally added thereto, and the resulting mixture was stirred at 45°C. Separately, 1-benzyl-2,5,5-trimethylpiperidine (1.19 g) described in Tetrahedron, 2012, Vol.68, #15, p.3172-3178 was used to carry out a similar reaction. The two reaction solutions were combined to carry out the subsequent reactions. The resulting reaction solution was subjected to Celite filtration, 4N hydrochloric acid in dioxane (2 mL) was added thereto, and the resulting mixture was concentrated under reduced pressure. Isopropyl ether was added thereto, the precipitated solid was collected by filtration, and washed with isopropyl ether to give the title compound (683 mg) (yield 70%) as a white solid. MS(CI) m/z: 128 $[M+H]^+$

Reference Example 80-1

Preparation of 8-chloro-3-(3,3-dimethylpiperidin-1-yl)-5-methyl-[1,2,4]triazolo[4,3-a]pyrazine

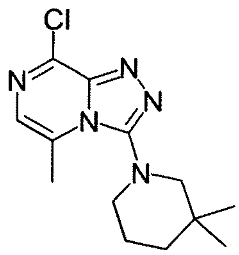

To a 10 mL cylindrical flask was added 3-(3,3-dimethylpiperidin-1-yl)-8-methoxy-5-methyl-[1,2,4]triazolo[4,3-a]pyrazine (20 mg) prepared in the Reference Example 80-2 under argon gas flow, phosphoryl chloride (492 mg) was added thereto with stirring at room temperature, and the resulting mixture was stirred at 130°C for 1.5 hours. After the reaction was completed, the reaction solution was added dropwise to iced water, and the resulting mixture was extracted with ethyl acetate. The resulting organic layer was washed sequentially with a saturated aqueous solution of sodium hydrogen carbonate and saturated brine, dried over anhydrous sodium sulfate, and concentrated under reduced pressure. The resulting residues were subjected to silica gel chromatography (hexane : ethyl acetate = 53 : 47 to 32 : 68) using YAMAZEN medium pressure preparative (Silica S (7 g)), the fractions comprising the target compound (Rf value = 0.5 (hexane : ethyl acetate = 1 : 2)) were collected, and concentrated under reduced pressure to give the title compound (14 mg) (yield 69%) as a yellow solid.
MS(CI) m/z: 280/282 $[M+H]^+$

Reference Example 42-1 etc.:

A corresponding starting compound was reacted in a similar manner to the Reference Example 80-1 to give each compound described in the following Table 39.

Table 39

| Reference Example | Structural formula | Physical property etc. |
| --- | --- | --- |
| 42-1 | 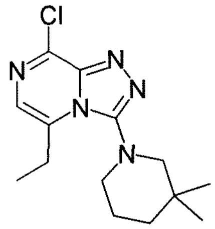 | MS(DUIS) m/z; 294/296 $[M+H]^+$ |
| 43-1 | 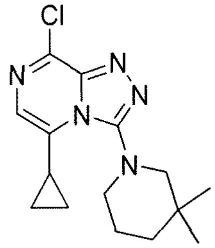 | MS(DUIS) m/z; 306/308 $[M+H]^+$ |
| 81-1 | 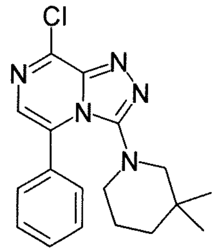 | MS(DUIS) m/z; 342/344 $[M+H]^+$ |

Reference Example 68-3

Preparation of 8-chloro-3-cyclohexyl-[1,2,4]triazolo[3,4-f][1,2,4]triazin-6-amine

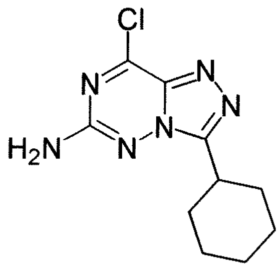

To a 50 mL eggplant flask were added 6-amino-3-cyclohexyl-[1,2,4]triazolo[3,4-f][1,2,4]triazin-8(7H)-one (0.36 g) prepared in the Reference Example 68-4 and phosphoryl chloride (11.84 g), and the resulting mixture was stirred at 100°C for 8 hours. After the reaction was completed, the reaction mixture was poured into iced water comprising sodium hydrogen carbonate (20 g), the resulting mixture was stirred for 1 hour, and then extracted with ethyl acetate. The resulting organic layer was dried over anhydrous magnesium sulfate, and concentrated under reduced pressure. The resulting residues were subjected to silica gel chromatography (hexane : ethyl acetate = 70 : 30 to 0 : 100) using Moritex medium pressure preparative column (Purif-Pack SI size 60 (30 g)), the fractions comprising the target compound were collected, and concentrated under reduced pressure to give the title compound (0.17 g) (yield 44%) as a colorless solid. MS(DUIS) m/z: 253/255 $[M+H]^+$

Reference Example 69-3 etc.:

A corresponding starting compound was reacted in a similar manner to the Reference Example 68-3 to give each compound described in the following Table 40.

Table 40

| Reference Example | Structural formula | Physical property etc. |
|---|---|---|
| 69-3 | 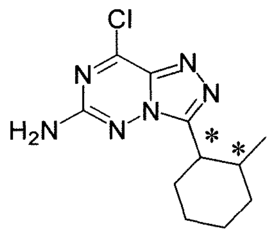 mixture of four types of stereoisomers | MS(CI) m/z; 267/269 $[M+H]^+$ |
| 71-3 | 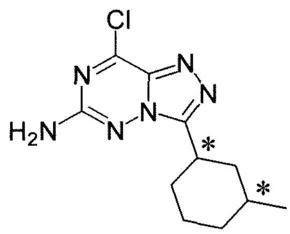 mixture of four types of stereoisomers | MS(CI) m/z; 267/269 $[M+H]^+$ |
| 73-3 | 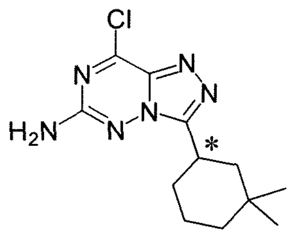 racemate | MS(CI) m/z; 281/283 $[M+H]^+$ |
| 74-3 | 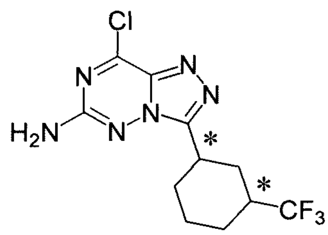 unknown relative configuration single diastereomer racemate | MS(CI) m/z; 321/323 $[M+H]^+$ |
| 75-3 | 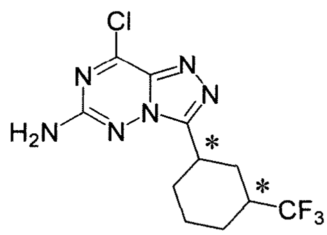 unknown relative configuration single diastereomer different from Reference Example 74-3 racemate | MS(CI) m/z; 321/323 $[M+H]^+$ |

(continued)

| Reference Example | Structural formula | Physical property etc. |
|---|---|---|
| 82-3 | 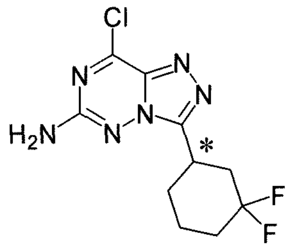<br>racemate | MS(CI) m/z; 289/291 [M+H]$^+$ |
| 76-3 | 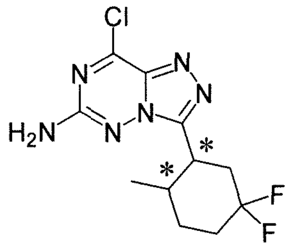<br>cis, racemate | MS(DUIS) m/z; 303/305 [M+H]$^+$ |
| 83-3 | 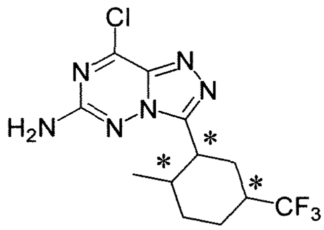<br>relative configuration (1R*, 2S*, 5R*) racemate | MS(CI) m/z; 335/337 [M+H]$^+$ |

Reference Example 68-2

Preparation of 3-cyclohexyl-8-(methylthio)-[1,2,4]triazolo[3,4-f][1,2,4]triazin-6-amine

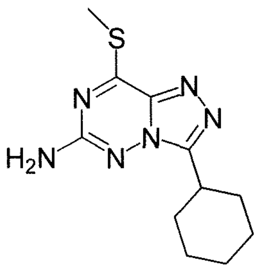

To a 20 mL cylindrical flask were added 8-chloro-3-cyclohexyl-[1,2,4]triazolo[3,4-f][1,2,4]triazin-6-amine (0.155 g) prepared in the Reference Example 68-3, tetrahydrofuran (3 mL), and sodium methyl mercaptide (0.34 g), and the resulting mixture was stirred at room temperature for 1 hour. After the reaction was completed, water was added thereto, and the resulting mixture was extracted with ethyl acetate. The resulting organic layer was washed with saturated solution of sodium hydrogen carbonate, dried over anhydrous magnesium sulfate, and concentrated under reduced pressure to give the title compound (153.5 mg) (yield 95%) as a colorless solid.
MS(CI) m/z: 265 [M+H]$^+$

Reference Example 69-2 etc.:

A corresponding starting compound was reacted in a similar manner to the Reference Example 68-2 to give each compound described in the following Table 41.

Table 41

| Reference Example | Structural formula | Physical property etc. |
|---|---|---|
| 69-2 | 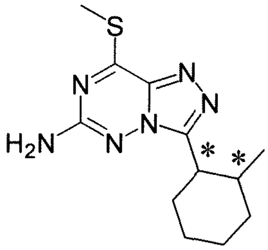 mixture of four types of stereoisomers | MS(CI) m/z; 279 $[M+H]^+$ |
| 71-2 | 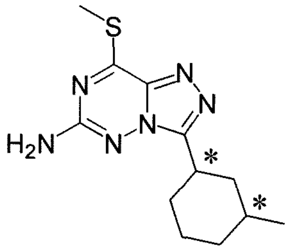 mixture of four types of stereoisomers | MS(CI) m/z; 279 $[M+H]^+$ |
| 73-2 | 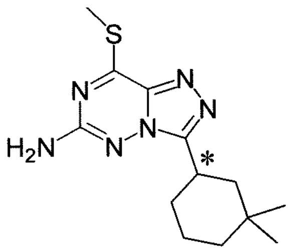 racemate | MS(CI) m/z; 293 $[M+H]^+$ |
| 74-2 | 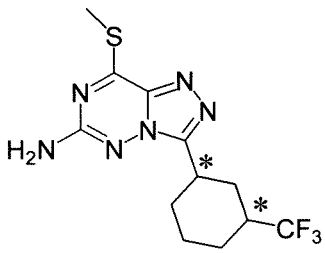 unknown relative configuration single diastereomer racemate | MS(CI) m/z; 333 $[M+H]^+$ |

(continued)

| Reference Example | Structural formula | Physical property etc. |
|---|---|---|
| 75-2 | 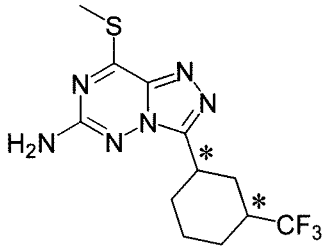 unknown relative configuration single diastereomer different from Reference Example 74-2 racemate | MS(CI) m/z; 333 $[M+H]^+$ |
| 82-2 | 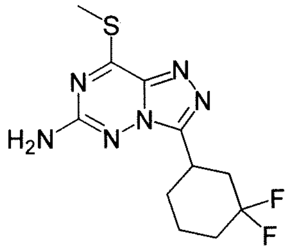 racemate | MS(CI) m/z; 301 $[M+H]^+$ |
| 76-2 | 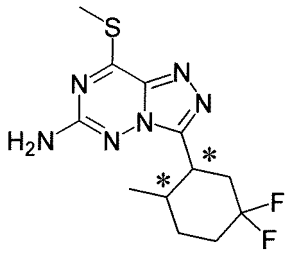 cis, racemate | MS(DUIS) m/z; 315 $[M+H]^+$ |
| 83-2 | 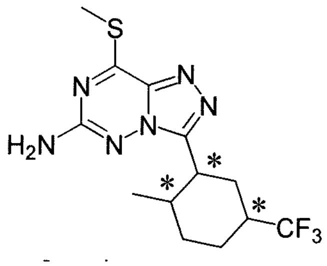 relative configuration (1R*, 2S*, 5R*) racemate | MS(CI) m/z; 347 $[M+H]^+$ |

Reference Example 68-1

Preparation of 3-cyclohexyl-8-(methylthio)-[1,2,4]triazolo[3,4-f] [1,2,4]triazine

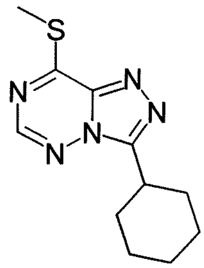

To a 20 mL cylindrical flask were added 3-cyclohexyl-8-(methylthio)-[1,2,4]triazolo[3,4-f] [1,2,4]triazin-6-amine (153 mg) prepared in the Reference Example 68-2 and toluene, and the resulting mixture was concentrated under reduced pressure. Then, tetrahydrofuran (10 mL) was added thereto, and the resulting mixture was degassed by argon. Then, isoamyl nitrite (677 mg) was added thereto at room temperature, and the resulting mixture was stirred at 65°C for 12 hours. After the reaction was completed, the mixture was concentrated under reduced pressure. The resulting residues were subjected to silica gel column chromatography (hexane : ethyl acetate = 80 : 20 to 0 : 100) using Moritex medium pressure preparative (Purif-Pack SI size 20 (10 g)), the fractions comprising the target compound were collected, and said fractions were concentrated under reduced pressure to give the title compound (68 mg) (yield 47%) as a slightly yellow solid. MS(CI) m/z: 250 $[M+H]^+$

Reference Example 69-1 etc.:

A corresponding starting compound was reacted in a similar manner to the Reference Example 68-1 to give each compound described in the following Table 42.

Table 42

| Reference Example | Structural formula | Physical property etc. |
|---|---|---|
| 69-1 | 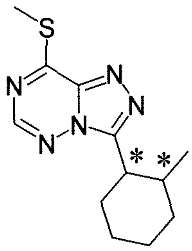 unknown relative configuration single diastereomer racemate | MS(CI) m/z; 264 $[M+H]^+$ |
| 70-1 | 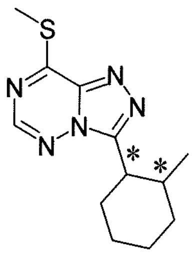 single diastereomer different from Reference Example 69-1 racemate | MS(CI) m/z; 264 $[M+H]^+$ |
| 71-1 | 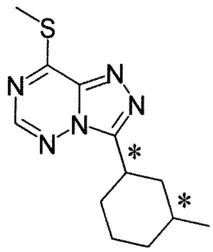 unknown relative configuration single diastereomer, racemate | MS(CI) m/z; 264 $[M+H]^+$ |

(continued)

| Reference Example | Structural formula | Physical property etc. |
|---|---|---|
| 72-1 | 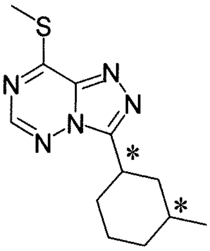 unknown relative configuration single diastereomer different from Reference Example 71-1 racemate | MS(CI) m/z; 264 $[M+H]^+$ |
| 73-1 | 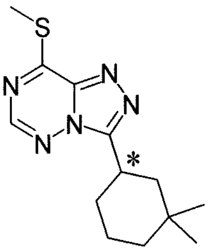 racemate | MS(CI) m/z; 278 $[M+H]^+$ |
| 74-1 | 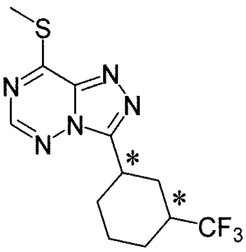 unknown relative configuration single diastereomer racemate | MS(CI) m/z; 318 $[M+H]^+$ |
| 75-1 | 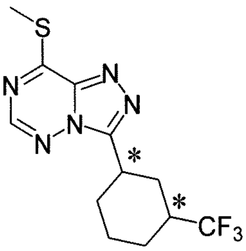 unknown relative configuration single diastereomer different from Reference Example 74-1 racemate | MS(CI) m/z; 318 $[M+H]^+$ |
| 82-1 | 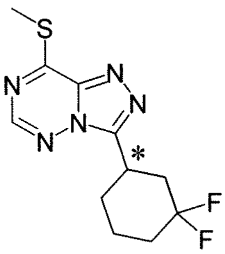 racemate | MS(DUIS) m/z; 286 $[M+H]^+$ |

(continued)

| Reference Example | Structural formula | Physical property etc. |
|---|---|---|
| 76-1 | 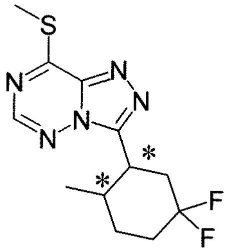 cis, racemate | MS(DUIS) m/z; 300 [M+H]$^+$ |
| 83-1 | 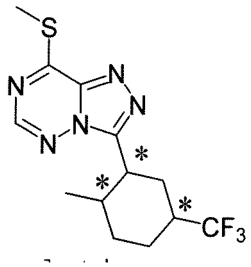 relative configuration (1R*, 2S*, 5R*) racemate | MS(CI) m/z; 332 [M+H]$^+$ |

Reference Example 140-1

Preparation of 8-chloro-3-[cis-3-methylcyclohexyl] [1,2,4]triazolo[4,3-a]pyrazine

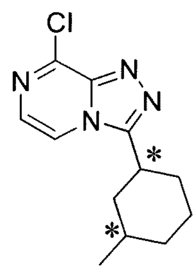

cis, racemate

A solution of cis-N'-(3-chloropyrazin-2-yl)-3-methylcyclohexanecarbohydrazide (500 mg) prepared in the Reference Example 140-2 and (methoxycarbonylsulfamoyl)triethylammoniumhydroxide inner salt (665 mg) in tetrahydrofuran (8 mL) was heated under reflux for 1 hour, then (methoxycarbonylsulfamoyl)triethylammoniumhydroxide inner salt (180 mg) was added thereto, and the resulting mixture was heated under reflux for additional 1 hour. The reaction mixture was allowed to cool to room temperature, water was added thereto, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 70/30 to 40/60) to give the title compound (395 mg) (yield 85%) as a colorless powder.
MS(APCI) m/z: 251/253 [M+H]$^+$

Reference Example 141-1 etc.:

A corresponding starting compound was reacted in a similar manner to the Reference Example 140-1 to give

each compound described in the following Table 43.

Table 43

| Reference Example | Structural formula | Physical property etc. |
|---|---|---|
| 141-1 | 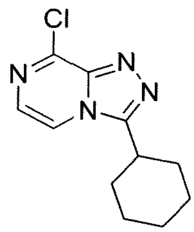 | MS(ESI) m/z; 237/239 [M+H]$^+$ |
| 143-1 | 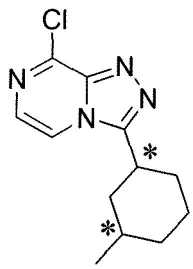<br>trans, racemate | MS(ESI) m/z; 251/253 [M+H]$^+$ |
| 149-1 | 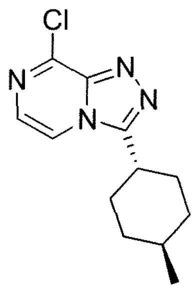<br>trans | MS(ESI) m/z; 251/253 [M+H]$^+$ |
| 161-1 | 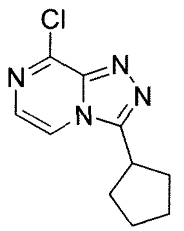 | MS(ESI) m/z; 223/225 [M+H]$^+$ |
| 165-1 | 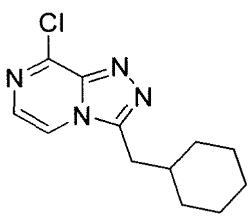 | MS(ESI) m/z; 251/253 [M+H]$^+$ |
| 166-1 | 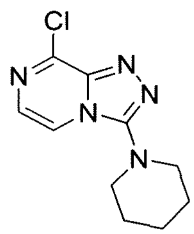 | MS(ESI) m/z; 238/240 [M+H]$^+$ |

(continued)

| Reference Example | Structural formula | Physical property etc. |
|---|---|---|
| 170-1 | 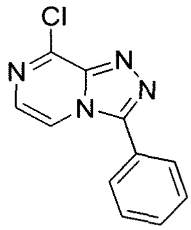 | MS(ESI) m/z; 231/233 [M+H]$^+$ |

Reference Example 174-2

Preparation of N,N-bis(2,4-dimethoxybenzyl)-3-[(1R*,2S*,5R*)-2-methyl-5-(trifluoromethyl)cyclohexyl] [1,2,4]triazolo[4,3-a]pyrazin-8-amine

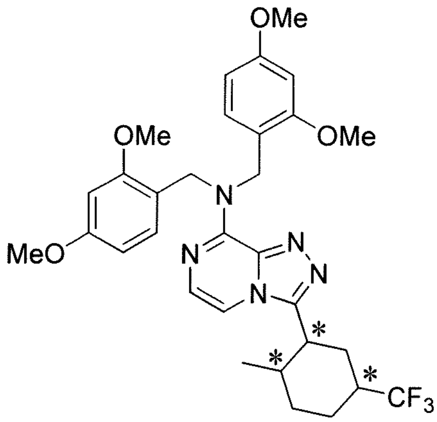

relative configuration (1R*,2S*,5R*), racemate

A mixture of 8-chloro-3-[(1R*,2S*,5R*)-2-methyl-5-(trifluoromethyl)cyclohexyl] [1,2,4]triazolo[4,3-a]pyrazine (366 mg) prepared in the Reference Example 151-1, bis(2,4-dimethoxybenzyl)amine (437 mg), N,N-diisopropylethylamine (0.3 mL), and 1,4-dioxane (4 mL) was stirred under microwave radiation at 150°C for 2 hours and 30 minutes. The reaction mixture was allowed to cool to room temperature, and water was added thereto. The resulting mixture was extracted twice with ethyl acetate, the resulting organic layers were combined, washed with saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 80/20 to 50/50) to give the title compound (696 mg) (yield 101%) as a colorless oil.
MS(APCI) m/z: 600 [M+H]$^+$

Reference Example 172-3 etc.:

A corresponding starting compound was reacted in a similar manner to the Reference Example 174-2 to give each compound described in the following Table 44.

Table 44

| Reference Example | Structural formula | Physical property etc. |
|---|---|---|
| 175-2 | 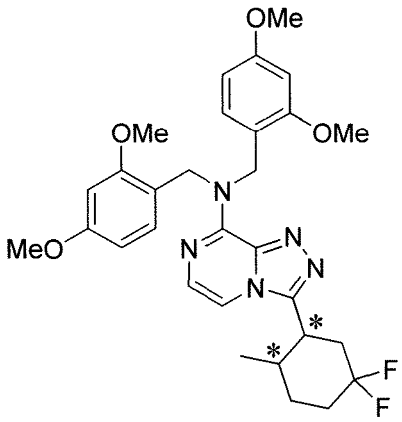 cis, racemate | MS(ESI) m/z; 568 [M+H]+ |
| 177-2 | 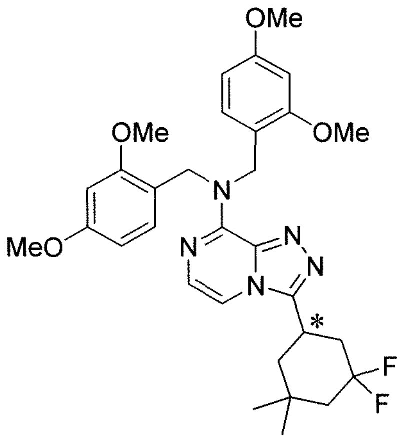 racemate | MS(ESI) m/z; 582 [M+H]+ |
| 172-3 | 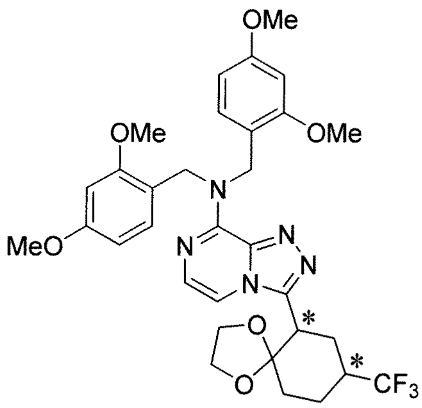 cis, racemate | MS(APCI) m/z; 584 [M+H]+ |

Reference Example 173-1 etc.:

The racemic mixture prepared in each of the above Reference Examples was resolved by chiral high performance liquid chromatography (chiral HPLC) or chiral supercritical fluid chromatography (chiral SFC) to give each compound described in the following Table 45.

Table 45

| Ref. Ex. | Structural formula | Physical property etc. | Analysis conditions etc. |
|---|---|---|---|
| 173-1 | 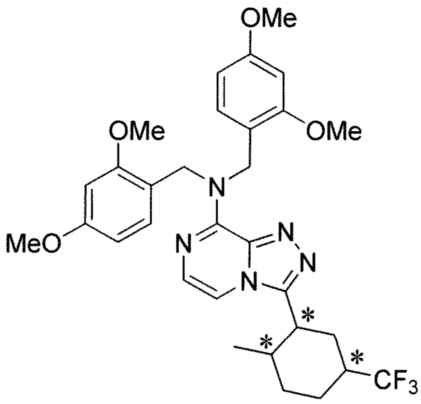 relative configuration (1R*, 2S*, 5R*), single enantiomer | MS (APCI) m/z; 600 $[M+H]^+$ | Column: CHIRALPAC IF-3(4.6 × 150 mm) Mobile phase: hexane/2-propanol/diethylamine (65/35/0.1) Flow rate: 0.5 mL/min Temperature: 25°C Analysis channel: PDA 284.0 nm Retention time (min.): 9.741 |
| 174-1 | 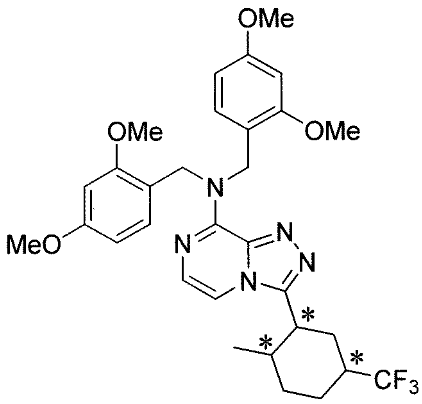 relative configuration (1R*, 2S*, 5R*), single enantiomer opposite to Reference Example 173-1 | MS (APCI) m/z; 600 $[M+H]^+$ | Column: CHIRALPAC IF-3 (4.6 × 150 mm) Mobile phase: hexane/2-propanol/diethylamine (65/35/0.1) Flow rate: 0.5 mL/min Temperature: 25°C Analysis channel: PDA 284.0 nm Retention time (min.): 11.181 |
| 175-1 | 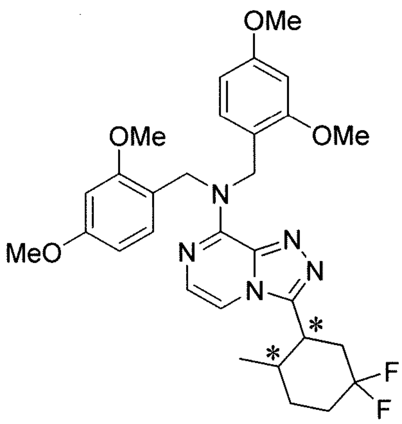 cis, single enantiomer | MS(ESI) m/z; 568 $[M+H]^+$ | Column: CHIRALPAC IC-3(4.6 × 150 mm) Mobile phase: hexane/ethanol/ diethy lamine (35/65/0.1) Flow rate: 0.5 mL/min Temperature: 25°C Analysis channel: PDA 283.0 nm Retention time (min.): 7.505 |

(continued)

| Ref. Ex. | Structural formula | Physical property etc. | Analysis conditions etc. |
|---|---|---|---|
| 176-1 | 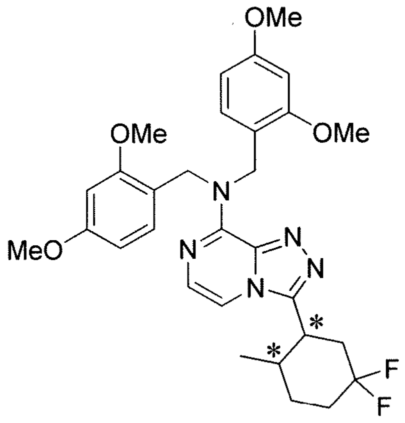 cis, single enantiomer opposite to Reference Example 175-1 | MS(ESI) m/z; 568 [M+H]+ | Column: CHIRALPAC IC-3 (4.6 × 150 mm)<br>Mobile phase: hexane/ethanol/ diethy lamine (35/65/0.1)<br>Flow rate: 0.5 mL/min<br>Temperature: 25°C<br>Analysis channel: PDA 283.0 nm<br>Retention time (min.): 11.691 |
| 177-1 | 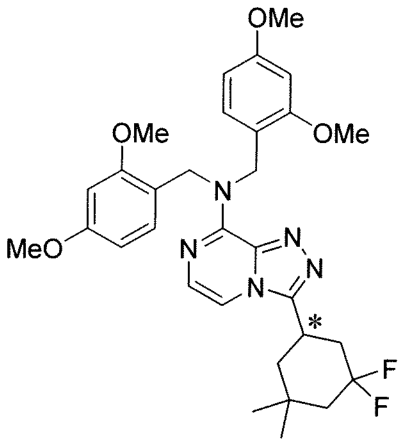 single enantiomer | MS(APCI) m/z; 582 [M+H]+ | Column: CHIRALPAC IC-3 (4.6 × 150 mm)<br>Mobile phase: methanol/ diethylamine (100/0.1)<br>Flow rate: 0.5 mL/min<br>Temperature: 25°C<br>Analysis channel: PDA 283.0 nm<br>Retention time (min.): 9.541 |
| 178-1 | 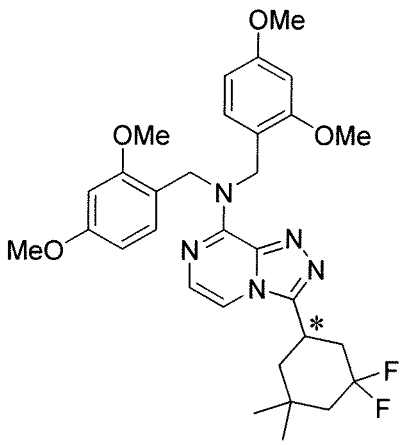 single enantiomer opposite to Reference Example 177-1 | MS(APCI) m/z; 582 [M+H]+ | Column: CHIRALPAC IC-3 (4.6 × 150 mm)<br>Mobile phase: methanol/ diethylamine (100/0.1)<br>Flow rate: 0.5 mL/min<br>Temperature: 25°C<br>Analysis channel: PDA 283.0 nm<br>Retention time (min.): 11.766 |

Reference Example 152-6

Preparation of benzyl 5,5-difluoro-2-oxocyclohexanecarboxylate

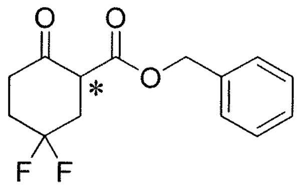

racemate

To a solution of 4,4-difluorohexanone (4.29 g) in tetrahydrofuran (60 mL) was added dropwise lithium bis(trimethylsilyl)amide (1.1 mol/L solution in tetrahydrofuran) (35 mL) under cooling in a dry ice/acetone bath, then to the reaction mixture was added a solution of benzyl cyanoformate (6.18 g) in tetrahydrofuran (20 mL), and the resulting mixture was stirred under the same conditions for 2 hours and 30 minutes. To the reaction mixture was added water, and the resulting mixture was extracted twice with ethyl acetate. The resulting organic layers were combined, washed sequentially with 1 mol/L hydrochloric acid and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 96/4 to 60/40) to give the title compound (5.09 g) (yield 59%) as a colorless oil.
MS(APCI) m/z: 286 $[M+NH_4]^+$

Reference Example 152-4

Preparation of benzyl 5,5-difluoro-2-methylcyclohex-1-ene-1-carboxylate

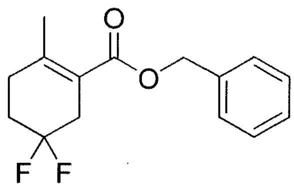

(1) A solution of benzyl 5,5-difluoro-2-oxocyclohexanecarboxylate (5.09 g) prepared in the Reference Example 152-6 in dichloromethane (192 mL) was subjected to nitrogen replacement, sodium hydride (60%) (2.28 g) was added thereto under ice-cooling, and the resulting mixture was stirred for 10 minutes. To the reaction mixture was added trifluoromethanesulfonic anhydride (9.57 mL), and the resulting mixture was stirred with gradually warming to room temperature overnight. The reaction mixture was ice-cooled, a saturated aqueous solution of sodium hydrogen carbonate was added thereto, and then the resulting mixture was extracted twice with chloroform. The resulting organic layers were combined, washed with saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 95/5 to 50/50) to give benzyl 5,5-difluoro-2-(trifluoromethylsulfonyloxy)cyclohex-1-ene-1-carboxylate (6.87 g) (yield 91%) as a colorless crystal.

(2) To a solution of benzyl 5,5-difluoro-2-(trifluoromethylsulfonyloxy)cyclohex-1-ene-1-carboxylate (2.8 g) prepared in the above (1) in tetrahydrofuran (140 mL) was added 1,1'-bis(diphenylphosphino)ferrocene-palladium(II) dichloride dichloromethane adduct (570 mg), and dimethylzinc (1.01 mol/L solution in heptane) (10 mL) was added thereto under nitrogen atmosphere. The reaction mixture was heated to 60°C, and stirred for 2 hours and 30 minutes. The reaction mixture was allowed to cool to room temperature, saturated brine was added thereto, and the resulting mixture was extracted twice with ethyl acetate. The resulting organic layers were combined, dried over anhydrous magnesium sulfate, and the insoluble matters were removed by filtration. The resulting filtrate was concentrated under reduced pressure, and the resulting residues were purified by silica gel column chromatography (solvent:

hexane/ethyl acetate = 95/5 to 50/50) to give the title compound (1.97 g) (yield 98%) as a colorless oil.
MS(ESI) m/z: 267 $[M+H]^+$

Reference Example 152-3

Preparation of 5,5-difluoro-2-methylcyclohexanecarboxylic acid

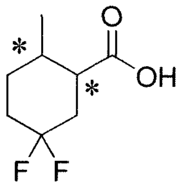

mixture of four types of stereoisomers

A mixture of benzyl 5,5-difluoro-2-methylcyclohex-1-ene-1-carboxylate (1.97 g) prepared in the Reference Example 152-4, 10% palladium carbon (1.24 g), and ethanol (76 mL) was stirred under hydrogen pressure (800 kPa) overnight. The reaction mixture was subjected to nitrogen replacement, and then the insoluble matters were removed by filtration. The insoluble matters were washed with ethyl acetate, and the resulting filtrate was concentrated under reduced pressure to give the title compound (1.13 g) (yield 91%) as a colorless oil.
MS(ESI) m/z: 177 $[M-H]^-$

Reference Example 20-3 etc.:

A corresponding starting compound was reacted in a similar manner to the Reference Example 152-3 except that the hydrogen pressure was set to be 1 atm to give each compound described in the following Table 46.

Table 46

| Reference Example | Structural formula | Physical property etc. |
|---|---|---|
| 20-3 | 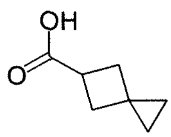 | MS(CI) m/z; 127 $[M+H]^+$ |
| 44-3 | 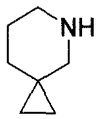 | MS(CI) m/z; 112 $[M+H]^+$ |

Reference Example 151-6

Preparation of benzyl 2-oxo-5-(trifluoromethyl)cyclohexanecarboxylate

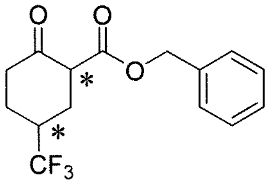

mixture of stereoisomers

4-Trifluoromethylcyclohexanone was reacted in a similar manner to the Reference Example 152-6 to give the

title compound.
MS(APCI) m/z: 318 $[M+NH_4]^+$

Reference Example 151-4

Preparation of benzyl 2-methyl-5-(trifluoromethyl)cyclohex-1-ene-1-carboxylate

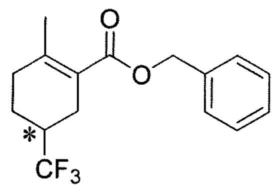

racemate

Benzyl 2-oxo-5-(trifluoromethyl)cyclohexanecarboxylate prepared in the Reference Example 151-6 was reacted in a similar manner to the Reference Example 152-4 (1) and (2) to give the title compound.
MS(APCI) m/z: 316 $[M+NH_4]^+$

Reference Example 151-3

Preparation of 2-methyl-5-(trifluoromethyl)cyclohexanecarboxylic acid

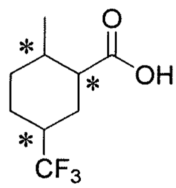

mixture of stereoisomers

Benzyl 2-methyl-5-(trifluoromethyl)cyclohex-1-ene-1-carboxylate prepared in the Reference Example 151-4 was reacted in a similar manner to the Reference Example 152-3 to give the title compound.
MS(APCI) m/z: 209 $[M-H]^-$

Reference Example 154-11

Preparation of benzyl cis-5-{[tert-butyl(dimethyl)silyl]oxy}cyclohex-3-ene-1-carboxylate

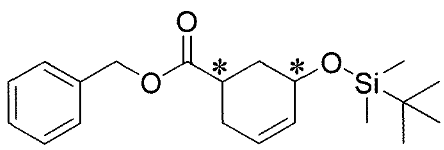

cis, racemate

To a 200 mL flask were added benzyl cis-5-hydroxycyclohex-3-ene-1-carboxylate (3.70 g), imidazole (2.20 g), 4-dimethylaminopyridine (100.6 mg), tert-butyldimethylchlorosilane (3.57 g), and N,N-dimethylformamide (16 mL), and the resulting mixture was stirred at room temperature for 3 hours. To the reaction mixture was added water (1.43 mL),

and the resulting mixture was stirred at room temperature for 20 minutes. To the reaction mixture were added a saturated aqueous solution of sodium hydrogen carbonate (80 mL) and ethyl acetate (160 mL), the resulting mixture was separated, the resulting organic layer was washed sequentially with water and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 100/0 to 90/10) to give the title compound (5.38 g) (yield 97%) as a colorless oil.
MS(ESI) m/z: 347 $[M+H]^+$

Reference Example 154-10

Preparation of benzyl (1S*, 3S*, 5S*, 6S*)-5-{[tert-butyl(dimethyl)silyl]oxy}-7-oxabicyclo[4.1.0]heptane-3-carboxylate

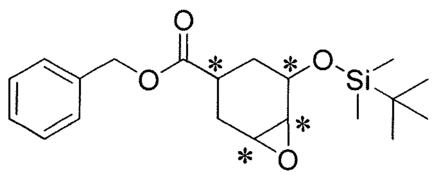

relative configuration (1S*, 3S*, 5S*, 6S*), racemate

To a 300 mL eggplant flask were added benzyl cis-5-{[tert-butyl(dimethyl)silyl]oxy}cyclohex-3-ene-1-carboxylate (2.88 g) prepared in the Reference Example 154-11 and dichloromethane (42 mL), m-chloroperbenzoic acid (wetted with ca. 30% water) (5.54 g) was added dividedly thereto under ice-cooling, and the resulting mixture was stirred with gradually warming to room temperature overnight. To the reaction mixture was added ethyl acetate, then a mixture of sodium thiosulfate pentahydrate (5.29 g), water (41 mL), and a saturated aqueous solution of sodium hydrogen carbonate (41 mL) was added thereto, and the resulting mixture was stirred at room temperature for 30 minutes. To the reaction mixture was added ethyl acetate to be separated, the resulting organic layer was washed sequentially with a saturated aqueous solution of sodium hydrogen carbonate and saturated brine, and NH-silica gel (11.5 g), silica gel (11.5 g), and anhydrous sodium sulfate were added thereto. The insoluble matters were removed by filtration, the resulting filtrate was concentrated under reduced pressure, and the resulting residues were purified by silica gel column chromatography (solvent: hexane/ethyl acetate = 99/1 to 85/15) to give the title compound (2.51 g) (yield 83%) as a colorless oil.
MS(ESI) m/z: 363 $[M+H]^+$

Reference Example 154-9

Preparation of benzyl (1S*, 3S*, 4S*, 5R*)-3-{[tert-butyl(dimethyl)silyl]oxy}-4-hydroxy-5-methylcyclohexanecarboxylate

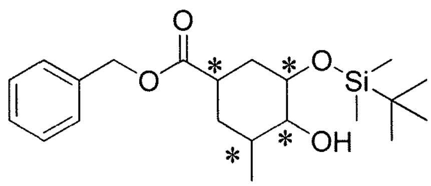

relative configuration (1S*, 3S*, 4S*, 5R*), racemate

To a 500 mL flask were added copper(I) cyanide (3.10 g) and tetrahydrofuran (50 mL), the resulting mixture was subjected to nitrogen replacement, then cooled in a dry ice/acetone bath, and methyllithium (1.0 mol/L solution in diethyl ether) (61 mL) was added dropwise thereto under stirring. The reaction mixture was gradually warmed to - 15°C, the contents were dissolved, and then the resulting mixture was cooled to -78°C again. To the reaction mixture was added dropwise a solution of boron trifluoride etherate (1.75 mL) in tetrahydrofuran (9.7 mL), and the resulting mixture was stirred for 15 minutes. To the reaction mixture was added dropwise a solution of benzyl (1S*, 3S*, 5S*, 6S*)-5-{[tert-butyl(dimethyl)silyl]oxy}-7-oxabicyclo[4.1.0]heptane-3-carboxylate (2.50 g) prepared in the Reference Example 154-10 in tetrahydrofuran (40 mL) over 5 minutes, and the resulting mixture was stirred at - 78°C for 3 hours. To the reaction mixture was added a solution of triethylamine (20 mL) in methanol (20 mL), then the mixture was warmed to room

temperature, ethyl acetate (300 mL) was added thereto, and the resulting mixture was separated. The resulting organic layer was washed sequentially with a mixed solution of a saturated aqueous solution of ammonia/a saturated aqueous solution of ammonium carbonate (1/9), saturated brine, a 5% aqueous solution of acetic acid, a saturated aqueous solution of sodium hydrogen carbonate, and saturated brine, silica gel (27.8 g) was added thereto, then dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 100/0 to 75/25) to give the title compound (2.11 g) (yield 80%) as a colorless oil.
MS(ESI) m/z: 379 $[M+H]^+$

Reference Example 154-8

Preparation of benzyl (1S*, 3S*, 4S*, 5R*)-3-{[tert-butyl(dimethyl)silyl]oxy}-4-[(1H-imidazol-1-ylcarbonothionyl)oxy]-5-methylcyclohexanecarboxylate

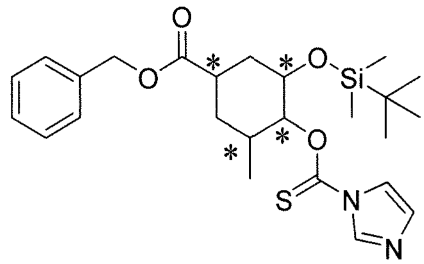

relative configuration (1S*, 3S*, 4S*, 5R*), racemate

To a 100 mL flask was added chloroform (3.4 mL), thiophosgene (431 μL) was added thereto under ice-cooling, and the resulting mixture was stirred. To the mixture was added dropwise a solution of benzyl (1S*, 3S*, 4S*, 5R*)-3-{[tert-butyl(dimethyl)silyl]oxy}-4-hydroxy-5-methylcyclohexanecarboxylate (1.07 g) prepared in the Reference Example 154-9 and pyridine (1.14 mL) in chloroform (11 mL), then to the reaction mixture was added 4-dimethylaminopyridine (34.6 mg), the resulting mixture was warmed to room temperature, stirred at room temperature for 6 hours and 30 minutes, then imidazole (769 mg) was added thereto, and the resulting mixture was stirred overnight. To the reaction mixture was added a saturated aqueous solution of sodium hydrogen carbonate, and then the insoluble matters were removed by filtration. The aqueous layer was extracted twice with ethyl acetate, the resulting organic layers were combined, silica gel (11.3 g) was added thereto, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 80/20 to 50/50) to give the title compound (419 mg) (yield 39%) as an orange oil.
MS(ESI) m/z: 489 $[M+H]^+$

Reference Example 154-7

Preparation of benzyl (1S*, 3R*, 5R*)-3-{[tert-butyl(dimethyl)silyl]oxy}-5-methylcyclohexanecarboxylate

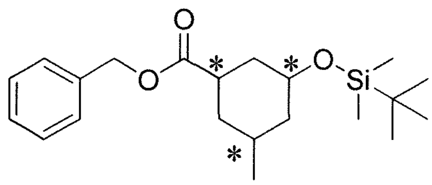

relative configuration (1S*, 3R*, 5R*), racemate

To a 300 mL eggplant flask were added benzyl (1S*, 3S*, 4S*, 5R*)-3-{[tert-butyl(dimethyl)silyl]oxy}-4-[(1H-imidazol-1-ylcarbonothionyl)oxy]-5-methylcyclohexanecarboxylate (591 mg) prepared in the Reference Example 154-8, tributyltin hydride (977 μL), 2,2'-azobis(isobutyronitrile) (21.9 mg), and toluene (6 mL), and the resulting mixture was

stirred at 110°C for 2 hours. The reaction mixture was allowed to cool to room temperature, NH-silica gel was added thereto, and a mixed solution (30 mL) of hexane and ethyl acetate (hexane : ethyl acetate = 1 : 1) was added thereto. The insoluble matters were removed by filtration. The resulting filtrate was concentrated under reduced pressure, and the resulting residues were purified by NH-silica gel column chromatography (solvent: hexane/ethyl acetate = 100/0 to 95/5) to give the title compound (211 mg) (yield 48%) as a colorless oil.
MS(ESI) m/z: 363 $[M+H]^+$

Reference Example 154-6

Preparation of benzyl (1S*, 3R*, 5R*)-3-hydroxy-5-methylcyclohexanecarboxylate

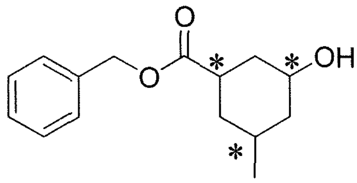

relative configuration (1S*, 3R*, 5R*), racemate

To a 200 mL eggplant flask were added benzyl (1S*, 3R*, 5R*)-3-{[tert-butyl(dimethyl)silyl]oxy}-5-methylcyclohexanecarboxylate (211 mg) prepared in the Reference Example 154-7, tetrabutylammonium fluoride (ca. 1.0 mol/L solution in tetrahydrofuran) (1.2 mL), and tetrahydrofuran (2.9 mL), and the resulting mixture was stirred at room temperature overnight. The reaction mixture was concentrated under reduced pressure, and the resulting residues were purified by silica gel column chromatography (solvent: hexane/ethyl acetate = 82/18 to 45/55) to give the title compound (118 mg) (yield 81%) as a colorless oil.
MS(ESI) m/z: 249 $[M+H]^+$

Reference Example 154-3

Preparation of cis-3,3-difluoro-5-methylcyclohexanecarboxylic acid

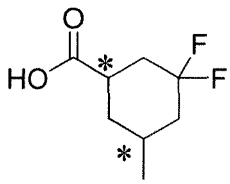

cis, racemate

(1) To a 300 mL eggplant flask were added benzyl (1S*, 3R*, 5R*)-3-hydroxy-5-methylcyclohexanecarboxylate (118 mg) prepared in the Reference Example 154-6, Molecular Sieves 4A (119 mg), and dichloromethane (5.8 mL), N-methylmorpholine N-oxide (112 mg) and tetrapropylammonium perruthenate (15.4 mg) were added thereto under ice-cooling, and the resulting mixture was stirred with gradually warming to room temperature overnight. The reaction mixture was purified by silica gel column chromatography (solvent: hexane/ethyl acetate = 100/0 to 65/35) to give benzyl cis-3-methyl-5-oxocyclohexanecarboxylate (111 mg) (yield 95%) as a colorless solid.
(2) To a 200 mL eggplant flask were added benzyl cis-3-methyl-5-oxocyclohexanecarboxylate (106 mg) prepared in the above (1), dichloromethane (4.3 mL), and ethanol (7.6 pL), bis(2-methoxyethyl)aminosulfur trifluoride (266 pL) was added thereto under ice-cooling, the resulting mixture was warmed to room temperature, stirred for 6 hours, then bis(2-methoxyethyl)aminosulfur trifluoride (133 pL) was added thereto, and the resulting mixture was stirred overnight. To the reaction mixture was added a saturated aqueous solution of sodium hydrogen carbonate, and the resulting mixture was extracted three times with ethyl acetate. The resulting organic layers were combined, dried over anhydrous sodium sulfate, and the insoluble matters were removed by filtration. The resulting filtrate was concentrated under reduced pressure, and the resulting residues were purified by silica gel column chromatography

(solvent: hexane/ethyl acetate = 100/0 to 93/7) to give benzyl cis-3,3-difluoro-5-methylcyclohexanecarboxylate (91 mg) (yield 79%) as a colorless oil.

(3) To a 100 mL eggplant flask were added benzyl cis-3,3-difluoro-5-methylcyclohexanecarboxylate (91 mg) prepared in the above (2) and tetrahydrofuran (3.4 mL), the resulting mixture was subjected to nitrogen replacement, then 10% palladium carbon (45.7 mg) was added thereto, and the resulting mixture was stirred under hydrogen atmosphere at room temperature for 5 hours. The reaction mixture was subjected to nitrogen replacement, then the insoluble matters were removed by Celite filtration, and the resulting filtrate was concentrated under reduced pressure to give the title compound (56.6 mg) (yield 93%) as a colorless powder.

MS(ESI) m/z: 177 $[M-H]^-$

Reference Example 155-6

Preparation of benzyl 5-oxocyclohex-3-ene-1-carboxylate

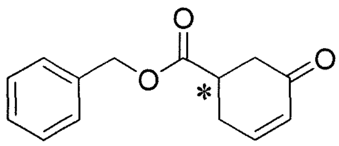

racemate

Benzyl cis-5-hydroxycyclohex-3-ene-1-carboxylate was reacted in a similar manner to the Reference Example 154-3 (1) to give the title compound.

MS(ESI) m/z: 231 $[M+H]^+$

Reference Example 155-5

Preparation of benzyl trans-3-methyl-5-oxocyclohexanecarboxylate

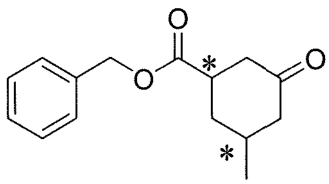

trans, racemate

To a 300 mL four-necked flask were added copper(I) iodide (2.58 g) and tetrahydrofuran (34 mL), the resulting mixture was subjected to nitrogen replacement, and then ice-cooled. To the mixture was added dropwise methyllithium (1.0 mol/L solution in diethyl ether) (24 mL) under stirring over 10 minutes. The resulting mixture was stirred under the same conditions for 30 minutes. The reaction mixture was cooled to -78°C in a dry ice/acetone bath, stirred for 5 minutes, then a solution of benzyl 5-oxocyclohex-3-ene-1-carboxylate (1.56 g) prepared in the Reference Example 155-6 in tetrahydrofuran (24 mL) was added dropwise thereto over 7 minutes, the resulting mixture was stirred for 30 minutes, and then stirred with gradually warming to room temperature for 2 hours. To the reaction mixture were added a saturated aqueous solution of ammonium chloride (68 mL), water (68 mL), and ethyl acetate (136 mL), the resulting mixture was stirred, and the insoluble matters were removed by filtration. The resulting filtrate was separated, and the resulting aqueous layer was extracted with ethyl acetate. The resulting organic layers were combined, sequentially washed with water, a saturated aqueous solution of sodium hydrogen carbonate, and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 95/5 to 74/26) to give the title compound (1.53 g) (yield 92%) as a colorless oil.

MS(ESI) m/z: 247 $[M+H]^+$

Reference Example 155-4

Preparation of benzyl trans-3,3-difluoro-5-methylcyclohexanecarboxylate

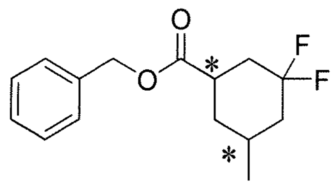

trans, racemate

Benzyl trans-3-methyl-5-oxocyclohexanecarboxylate prepared in the Reference Example 155-5 was reacted in a similar manner to the Reference Example 154-3 (2) to give the title compound.
MS(APCI) m/z: 286 $[M+NH_4]^+$

Reference Example 155-3

Preparation of trans-3,3-difluoro-5-methylcyclohexanecarboxylic acid

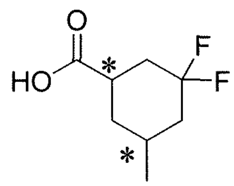

trans, racemate

Benzyl trans-3,3-difluoro-5-methylcyclohexanecarboxylate prepared in the Reference Example 155-4 was reacted in a similar manner to the Reference Example 154-3 (3) to give the title compound. MS(ESI) m/z: 177 $[M-H]^-$

Reference Example 157-6

Preparation of benzyl (1R*,3R*,5S*)-3-hydroxy-5-(trifluoromethyl)cyclohexanecarboxylate

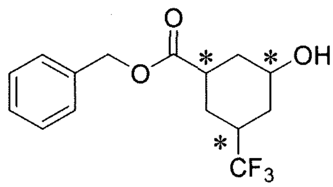

relative configuration (1R*,3R*,5S*), racemate

(1) A mixture of 3-hydroxy-5-trifluoromethylbenzoic acid (930 mg), platinum(IV) oxide (174 mg), and acetic acid (18 mL) was stirred under hydrogen pressure (7.9 atm) at 60°C overnight. The reaction mixture was subjected to nitrogen replacement, then the catalyst was removed by filtration, and the resulting solution was concentrated under reduced pressure to give a crude product of (1R*,3R*,5S*)-3-hydroxy-5-(trifluoromethyl)cyclohexanecarboxylic acid (874

mg) .

(2) To a 100 mL eggplant flask were added a crude product of (1R*,3R*,5S*)-3-hydroxy-5-(trifluoromethyl)cyclohexanecarboxylic acid (857 mg) prepared in the above (1), benzyl bromide (0.698 mL), cesium carbonate (1.62 g), and N,N-dimethylformamide (9.57 mL), and the resulting mixture was stirred at room temperature for 3 hours. To the reaction mixture was added ethyl acetate, then added water, and the resulting mixture was separated. The resulting organic layer was washed with saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 100/0 to 50/50) to give the title compound (155 mg) (yield 11% (two steps)) as an orange oil.

MS(ESI) m/z: 303 $[M+H]^+$

Reference Example 157-3

Preparation of cis-3,3-difluoro-5-(trifluoromethyl)cyclohexanecarboxylic acid

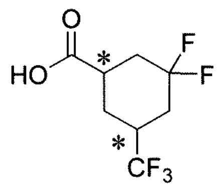

cis, racemate

Benzyl (1R*,3R*,5S*)-3-hydroxy-5-(trifluoromethyl)cyclohexanecarboxylate prepared in the Reference Example 157-6 was reacted in a similar manner to the Reference Example 154-3 (1), (2), and (3) to give the title compound.

MS(APCI) m/z: 231 $[M-H]^-$

Reference Example 158-5

Preparation of benzyl cyclohex-3-ene-1-carboxylate

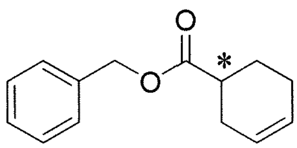

racemate

Cyclohex-3-ene-1-carboxylic acid (926 μL), 4-dimethylaminopyridine (96.6 mg), 1-(3-dimethylaminopropyl)-3-ethylcarbodiimide hydrochloride (1.98 g), and chloroform (16 mL) were mixed, and the resulting mixture was stirred at room temperature for 15 minutes. To the reaction mixture was added benzylalcohol (984 μL), the resulting mixture was stirred overnight, then benzylalcohol (246 μL) was additionally added thereto, and the resulting mixture was stirred overnight. The reaction mixture was concentrated under reduced pressure until the volume was reduced by approximately half, and the resulting residues were purified by silica gel column chromatography (solvent: hexane/ethyl acetate = 100/0 to 67/33) to give the title compound (1.64 g) (yield 96%) as a colorless oil.

MS(ESI) m/z: 217 $[M+H]^+$

Reference Example 158-4

Preparation of benzyl bicyclo[4.1.0]heptane-3-carboxylate

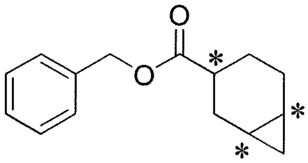

cyclopropane in bicyclo[4,1,0]heptane ring is cis isomer, mixture of four types of stereoisomers

Dichloromethane (4.8 mL) was added to a 100 mL eggplant flask, subjected to nitrogen replacement, and then ice-cooled. Diethylzinc (ca. 1 mol/L solution in toluene) (5.7 mL) and diiodomethane (460 μL) were added thereto, and the resulting mixture was stirred. To the reaction mixture was added a solution of benzyl cyclohexan-3-ene-1-carboxylate (415 mg) prepared in the Reference Example 158-5 in dichloromethane (4.8 mL), and the resulting mixture was stirred with gradually warming to room temperature overnight. To the reaction mixture were sequentially additionally added diethylzinc (ca. 1 mol/L solution in toluene) (5.7 mL) and diiodomethane (460 μL), and the resulting mixture was stirred at room temperature for 3 days. To the reaction mixture was added a saturated aqueous solution of ammonium chloride, and then citric acid was added thereto to be acidified. Ethyl acetate was added to the resulting mixture to be separated. The resulting organic layer was washed with a saturated aqueous solution of sodium hydrogen carbonate and saturated brine, and dried over anhydrous sodium sulfate. The insoluble matters were removed by filtration, and the resulting filtrate was concentrated under reduced pressure to give a crude product of the title compound (522 mg).

To a 200 mL eggplant flask were added the resulting crude product (522 mg), N-methylmorpholine N-oxide (226.5 mg), osmium tetroxide (2.5% solution in tert-butyl alcohol) (98 μL), acetone (7.7 mL), and water (1.9 mL), and the resulting mixture was stirred at room temperature overnight. To the reaction mixture were added ethyl acetate (40 mL), saturated brine (20 mL), water (20 mL), and sodium thiosulfate pentahydrate (968 mg), and the resulting mixture was stirred for 1 hour. The mixture was separated, the resulting organic layer was dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 100/0 to 67/33) to give the title compound (339 mg) (yield 77%) as a pale yellow oil.
MS(ESI) m/z: 231 $[M+H]^+$

Reference Example 158-3

Preparation of bicyclo[4.1.0]heptane-3-carboxylic acid

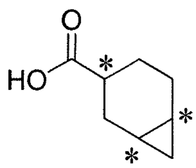

cyclopropane in bicyclo[4,1,0]heptane ring is cis isomer, mixture of four types of stereoisomers

Benzyl bicyclo[4.1.0]heptane-3-carboxylate prepared in the Reference Example 158-4 was reacted in a similar manner to the Reference Example 154-3 (3) to give the title compound.
MS(ESI) m/z: 141 $[M+H]^+$

Reference Example 20-4

Preparation of benzyl spiro[2.3]hexane-5-carboxylate

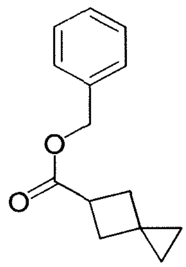

To a 200 mL eggplant flask were added benzyl 3-methylenecyclobutanecarboxylate (1.75 g) and dichloromethane (60 mL) under argon atmosphere, then diethylzinc (2.72 g) was added dropwise thereto at 0°C, and the resulting mixture was stirred at 0°C for 20 minutes. Then, chloroiodomethane (6.08 g) was added dropwise thereto at 0°C, and the resulting mixture was stirred at room temperature overnight. After the reaction was completed, the reaction solution was poured into water, and the resulting mixture was extracted with dichloromethane. The resulting organic layer was washed sequentially with a saturated aqueous solution of ammonium chloride and saturated brine, dried over anhydrous magnesium sulfate, and concentrated under reduced pressure to give the title compound (1.39 g) (yield 74%) as a colorless oil.
MS(ESI) m/z: 231 $[M+H]^+$

Reference Example 44-4

A corresponding starting compound was reacted in a similar manner to the Reference Example 20-4 to give the compound described in the following Table 47.

Table 47

| Reference Example | Structural formula | Physical property etc. |
|---|---|---|
| 44-4 | 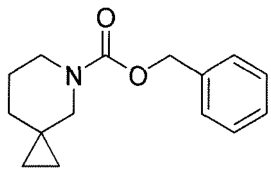 | MS(ESI) m/z; 582 $[M+H]^+$ |

Reference Example 160-2

Preparation of (1R,3S,5S)-N'-(3-chloropyrazin-2-yl)-6,6-difluorobicyclo[3.1.0]hexane-3-carbohydrazide

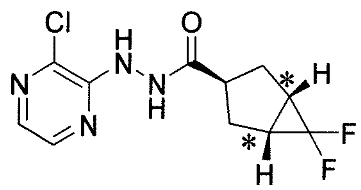

(1) A mixture of methyl 3-cyclopentene-1-carboxylate (2.52 g) and diethylene glycol dimethyl ether (25 mL) was stirred under heating at 180°C. To the mixture was added dropwise a mixture of sodium chlorodifluoroacetate (15.25 g) and diethylene glycol dimethyl ether (110 mL) over 2 hours and 40 minutes. After the addition was completed, the reaction mixture was allowed to cool to room temperature, and poured into water. The resulting mixture was extracted with hexane, the resulting organic layer was washed five times with water, then washed with saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 97/3 to 80/20) to give methyl (lR,3S,5S)-6,6-difluorobicyclo[3.1.0]hexane-3-carboxylate (2.00 g) (yield 57%) as an oil.

(2) To a mixture of methyl (1R,3S,5S)-6,6-difluorobicyclo[3.1.0]hexane-3-carboxylate (2.00 g) prepared in the above (1), tetrahydrofuran (20 mL), and methanol (20 mL) was added a solution of lithium hydroxide (1.9 g) in water (20 mL), and the resulting mixture was stirred at room temperature overnight. The reaction mixture was concentrated under reduced pressure, 1 mol/L hydrochloric acid was added thereto until pH of the mixture became 3, the mixture was separated by a mixed solvent of chloroform and ethanol (chloroform/ethanol = 5/1), and the resulting organic layer was concentrated under reduced pressure. To the resulting residues was added toluene, and the resulting residues were concentrated under reduced pressure to give (1R,3S,5S)-6,6-difluorobicyclo[3.1.0]hexane-3-carboxylic acid (1.60 g) (yield 87%) as a white powder.

(3) (1R,3S,5S)-6,6-difluorobicyclo[3.1.0]hexane-3-carboxylic acid prepared in the above (2) was reacted in a similar manner to the Reference Example 146-2 to give the title compound.

MS(ESI) m/z: 289/291 $[M+H]^+$

Reference Example 172-7

Preparation of benzyl cis-8-(trifluoromethyl)-1,4-dioxaspiro[4.5]decane-6-carboxylate

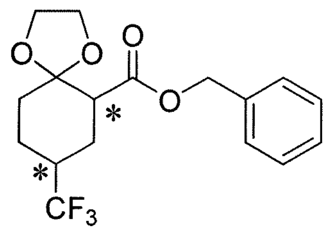

cis, racemate

A mixture of benzyl 2-oxo-5-(trifluoromethyl)cyclohexanecarboxylate (300 mg) prepared in the Reference Example 151-6, p-toluenesulfonic acid (57 mg), ethylene glycol (1 mL), and toluene (2 mL) was heated under reflux for 5 hours. The reaction mixture was allowed to cool to room temperature, a saturated aqueous solution of sodium hydrogen carbonate was added thereto, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 95/5 to 80/20) to give the title compound (171 mg) (yield 50%) as a colorless oil.

MS(APCI) m/z: 345 $[M+H]^+$

Reference Example 172-6

Preparation of cis-8-(trifluoromethyl)-1,4-dioxaspiro[4.5]decane-6-carboxylic acid

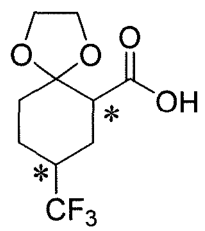

cis, racemate

Benzyl cis-8-(trifluoromethyl)-1,4-dioxaspiro[4.5]decane-6-carboxylate prepared in the Reference Example 172-7 was reacted in a similar manner to the Reference Example 154-3 (3) to give the title compound. MS(APCI) m/z:

255 [M+H]+

Reference Example 172-2

Preparation of cis-2-{8-[bis(4-methoxybenzyl)amino][1,2,4]triazolo[4,3-a]pyrazin-3-yl}-4-(trifluoromethyl)cyclohexanone

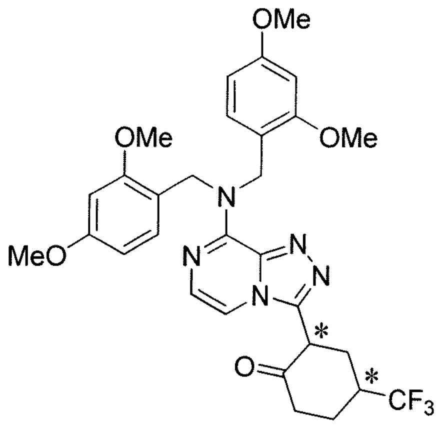

cis, racemate

A mixture of N,N-bis(4-methoxybenzyl)-3-[cis-8-(trifluoromethyl)-1,4-dioxaspiro[4.5]dec-6-yl][1,2,4]triazolo[4,3-a]pyrazin-8-amine (176 mg) prepared in the Reference Example 172-3, 1 mol/L hydrochloric acid (1 mL), and tetrahydrofuran (1 mL) was stirred at 60°C for 6 hours and 30 minutes. The reaction mixture was allowed to cool to room temperature, water was added thereto, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 80/20 to 50/50) to give the title compound (69 mg) (yield 42%) as a pale yellow oil.
MS(APCI) m/z: 540 [M+H]+

Reference Example 172-1

Preparation of 3-[cis-2,2-difluoro-5-(trifluoromethyl)cyclohexyl]-N,N-bis(4-methoxybenzyl)[1,2,4]triazolo[4,3-a]pyrazin-8-amine

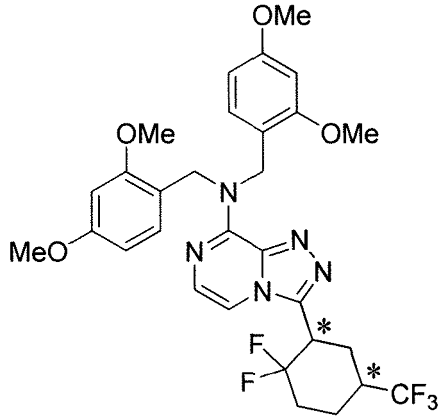

cis, racemate

To a solution of cis-2-{8-[bis(4-methoxybenzyl)amino][1,2,4]triazolo[4,3-a]pyrazin-3-yl}-4-(trifluoromethyl)cyclohexanone (136 mg) prepared in the Reference Example 172-2 in dichloromethane (4 mL) was added (diethylamino)sulfur trifluoride (0.133 mL), and the resulting mixture was stirred at room temperature overnight. To the reaction mixture was additionally added (diethylamino)sulfur trifluoride (0.133 mL), and the resulting mixture was stirred at room temperature for 2 hours. To the reaction mixture was added a saturated aqueous solution of sodium hydrogen carbonate, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 80/20 to 60/40) to give the title compound (24.5 mg) (yield 17%) as a pale yellow oil.
MS(APCI) m/z: 562 $[M+H]^+$

Reference Example 204-4

Preparation of (3R)-3-methylcyclohexanecarbonitrile

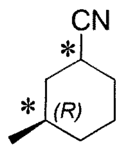

mixture of cis and trans isomers

To a mixture of (R)-3-methylcyclohexanone (300 mg), p-toluenesulfonylmethyl isocyanide (1.04 g), 1,2-dimethoxyethane (9 mL), and ethanol (0.3 mL) was added dividedly potassium tert-butoxide (1.05 g) under ice-cooling. The reaction mixture was stirred under ice-cooling for 1 hour, and then stirred at room temperature overnight. To the reaction mixture was added water, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 60/40 to 30/70) to give the title compound (172 mg) (yield 52%) as a yellow oil.
MS(APCI) m/z: 124 $[M+H]^+$

Reference Example 204-3

Preparation of (3R)-3-methylcyclohexanecarboxylic acid

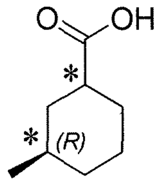

mixture of cis and trans isomers

A mixture of (3R)-3-methylcyclohexanecarbonitrile (154 mg) prepared in the Reference Example 204-4 and concentrated hydrochloric acid (2 mL) was stirred at 100°C for 1 day. The reaction mixture was allowed to cool to room temperature, added to water, and the resulting mixture was extracted twice with diethyl ether. The resulting organic layers were combined, washed with saturated brine, dried over anhydrous magnesium sulfate, and the insoluble matters were removed by filtration. The resulting filtrate was concentrated under reduced pressure to give the title compound (157 mg) (yield 88%) as a brown oil.
MS(APCI) m/z: 141 $[M-H]^-$

Reference Example 204

Preparation of 3-[(1S,3R)-3-methylcyclohexyl] [1,2,4]triazolo[4,3-a]pyrazin-8-amine

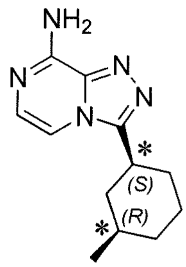

(3R)-3-methylcyclohexanecarboxylic acid prepared in the Reference Example 204-3 was reacted in a similar manner to the Reference Example 146-2, Reference Example 142-1, and Example 140 to give the title compound. A comparative analysis by chiral HPLC using said compound as an authentic sample was carried out to determine the absolute configuration of the Example 204 as (1S,3R), and determine the absolute configuration of the opposite enantiomer, Example 205 as (1R,3S).

Reference Example 212

Preparation of 3-[(1S)-3,3-difluorocyclohexyl] [1,2,4]triazolo[4,3-a]pyrazin-8-amine

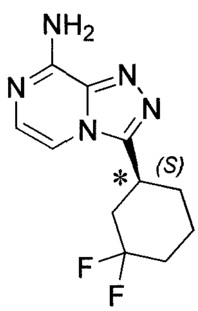

(1S)-3,3-difluorocyclohexanecarboxylic acid was reacted in a similar manner to the Reference Example 146-2, Reference Example 140-1, and Example 140 to give the title compound. A comparative analysis by chiral HPLC using said compound as an authentic sample was carried out to determine the absolute configuration of the Example 212 as

(S), and determine the absolute configuration of the opposite enantiomer, Example 213 as (R).

Reference Example 181-1

Preparation of 4-chloro-1-cyclohexyl-1H-pyrazolo[3,4-d]pyrimidine

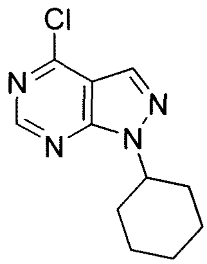

To a 300 mL eggplant flask were added 4,6-dichloropyrimidine-5-carbaldehyde (4.49 g), cyclohexylhydrazine hydrochloride (3.83 g), and tetrahydrofuran (130 mL), triethylamine (7.1 mL) was added thereto under ice-cooling, and the resulting mixture was stirred at room temperature overnight. The insoluble matters were removed by Celite filtration, and the resulting filtrate was concentrated under reduced pressure. The resulting residues were purified by silica gel column chromatography (solvent: hexane/ethyl acetate = 100/0 to 75/25) to give the title compound (3.91 g) (yield 65%) as a colorless powder.
MS(ESI) m/z: 237/239 $[M+H]^+$

Reference Example 182-1

Preparation of 6-chloro-9-cyclohexyl-9H-purine

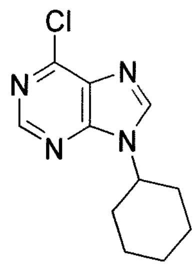

To a 25 mL eggplant flask were added 6-chloro-$N^4$-cyclohexyl-pyrimidine-4,5-diamine (377 mg) prepared in the Reference Example 3-2, p-toluenesulfonic acid (31.1 mg), and triethyl orthoformate (3.3 mL), and the resulting mixture was stirred at 110°C for 16 hours. The reaction mixture was allowed to cool to room temperature, and purified by silica gel column chromatography (solvent: hexane/ethyl acetate = 71/29 to 50/50) to give the title compound (350 mg) (yield 89%) as a colorless powder.
MS(ESI) m/z: 237/239 $[M+H]^+$

Reference Example 183-3

Preparation of 2-chloro-N-cyclohexyl-3-nitropyrimidin-4-amine

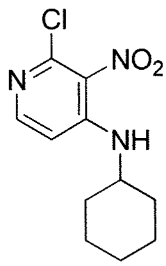

To a 200 mL eggplant flask were added 2,4-dichloro-3-nitropyridine (3.87 g), triethylamine (3.1 mL), and N,N-dimethylformamide (25 mL), cyclopropylamine (2.4 mL) was added thereto under ice-cooling, then the resulting mixture was warmed to room temperature, and stirred for 3 hours and 30 minutes. To the reaction mixture were added water and ethyl acetate, and the resulting mixture was separated. The resulting organic layer was washed sequentially with water and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 97/3 to 76/24) to give the title compound (2.78 g) (yield 54%) as a yellow oil.
MS(ESI) m/z: 256/258 $[M+H]^+$

Reference Example 183-2

Preparation of 2-chloro-$N^4$-cyclohexylpyridine-3,4-diamine

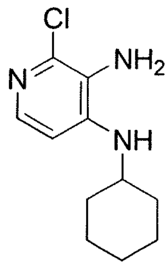

2-Chloro-N-cyclohexyl-3-nitropyrimidin-4-amine prepared in the Reference Example 183-3 was reacted in a similar manner to the Reference Example 5-2 to give the title compound.
MS(ESI) m/z: 226/228 $[M+H]^+$

Reference Example 183-1

Preparation of 4-chloro-1-cyclohexyl-1H-[1,2,3]triazolo[4,5-c]pyrimidine

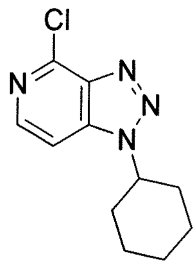

2-Chloro-$N^4$-cyclohexylpyridine-3,4-diamine prepared in the Reference Example 183-2 was reacted in a similar manner to the Reference Example 6-1 to give the title compound.

MS(ESI) m/z: 237/239 [M+H]+

Reference Example 180-2

Preparation of N-[(3-chloropyrazin-2-yl)methyl]cyclohexanecarboxamide

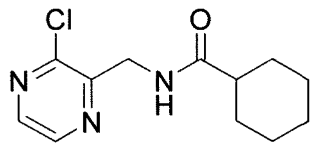

A corresponding starting compound was reacted in a similar manner to the Reference Example 141-2 to give the title compound.
MS(ESI) m/z: 254/256 [M+H]+

Reference Example 180-1

Preparation of 8-chloro-3-cyclohexylimidazo[1,5-a]pyrazine

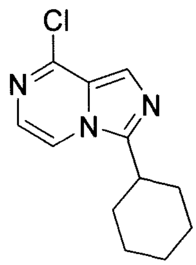

N-[(3-chloropyrazin-2-yl)methyl]cyclohexanecarboxamide prepared in the Reference Example 180-2 was reacted in a similar manner to the Reference Example 140-1 to give the title compound.
MS(ESI) m/z: 236/238 [M+H]+

Reference Example 184-1

Preparation of 4-chloro-1-cyclohexyl-3-methyl-1H-pyrazolo[3,4-d]pyrimidine

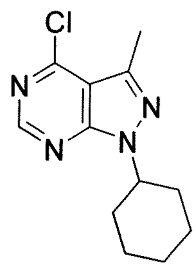

A corresponding starting compound was reacted in a similar manner to the Reference Example 181-1 to give the title compound.
MS(ESI) m/z: 251/253 [M+H]+

Reference Example 185-2

Preparation of 1-cyclohexyl-3-(trifluoromethyl)-1,5-dihydro-4H-pyrazolo[3,4-d]pyrimidin-4-one

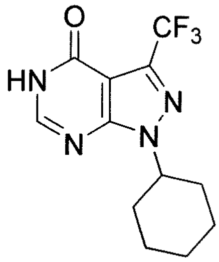

To a 200 mL eggplant flask were added 4-chloro-1-cyclohexyl-1H-pyrazolo[3,4-d]pyrimidine (504 mg) prepared in the Reference Example 181-1, sodium trifluoromethanesulfinate (2.81 g), dimethyl sulfoxide (15 mL), and water (6 mL), tert-butyl peroxide (70% aqueous solution) (2.9 mL) was added dropwise thereto over 7 minutes, and then the resulting mixture was stirred at room temperature overnight. To the reaction mixture were additionally added sodium sulfite (2.95 g) and water (60 mL), and the resulting mixture was stirred. To the reaction mixture was added ethyl acetate, the resulting mixture was separated, and the resulting aqueous layer was extracted twice with ethyl acetate. The resulting organic layers were combined, washed sequentially with water and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 70/30 to 0/100) to give the title compound (131 mg) (yield 22%) as a pale yellow powder.
MS(ESI) m/z: 287 $[M+H]^+$

Reference Example 185-1

Preparation of 4-chloro-1-cyclohexyl-3-(trifluoromethyl)-1H-pyrazolo[3,4-d]pyrimidine

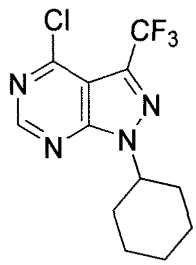

To a 25 mL eggplant flask were added 1-cyclohexyl-3-(trifluoromethyl)-1,5-dihydro-4H-pyrazolo[3,4-d]pyrimidin-4-one (131 mg) prepared in the Reference Example 185-2, triethylamine (70 μL), and chloroform (2.3 mL), N,N-dimethylformamide (177 μL) and thionyl chloride (100 μL) were sequentially added thereto, the resulting mixture was stirred at room temperature for 2 hours and 30 minutes, then heated to 60°C, and stirred overnight. To the reaction mixture was additionally added thionyl chloride (234 μL), and the resulting mixture was stirred at 80°C for 1 day. The reaction mixture was allowed to cool to room temperature, a saturated aqueous solution of sodium hydrogen carbonate and ethyl acetate were added thereto, the resulting mixture was stirred at room temperature for 2 hours, then separated, and the resulting aqueous layer was extracted twice with ethyl acetate. The resulting organic layers were combined, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 100/0 to 50/50) to give the title compound (30 mg) (yield 22%) as a yellow oil.
MS(ESI) m/z: 305/307 $[M+H]^+$

Reference Example 186-2

Preparation of N'-(3-nitropyridin-2-yl)cyclohexanecarbohydrazide

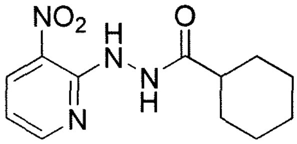

A corresponding starting compound was reacted in a similar manner to the Reference Example 141-2 to give the title compound.
MS(ESI) m/z: 265 $[M+H]^+$

Reference Example 186-1

Preparation of 3-cyclohexyl-8-nitro[1,2,4]triazolo[4,3-a]pyridine

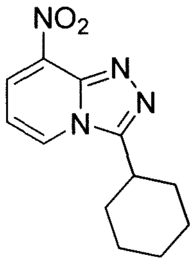

N'-(3-nitropyridin-2-yl)cyclohexanecarbohydrazide prepared in the Reference Example 186-2 was reacted in a similar manner to the Reference Example 140-1 to give the title compound.
MS(ESI) m/z: 247 $[M+H]^+$

Reference Example 187-5

Preparation of methyl 4-amino-3-bromo-1-methyl-1H-pyrazole-5-carboxylate

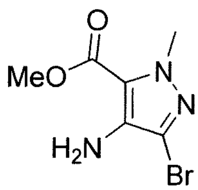

To a 200 mL eggplant flask were added methyl 4-amino-1-methyl-1H-pyrazole-5-carboxylate (1.84 g) and chloroform (24 mL), N-bromosuccinimide (2.31 g) was added thereto in ten additions under ice-cooling with stirring, and the resulting mixture was stirred for 1 hour. To the reaction mixture was added a saturated aqueous solution of sodium hydrogen carbonate, sodium sulfite (1.64 g) and ethyl acetate (72 mL) were added thereto, the resulting mixture was stirred for 10 minutes, then separated, and the resulting aqueous layer was extracted twice with ethyl acetate. The resulting organic layers were combined, washed sequentially with water and saturated brine, silica gel (7.4 g) was added thereto, then dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 90/10 to 50/50) to give the title compound (1.84 g) (yield 66%) as a brown

powder.
MS(ESI) m/z: 234/236 $[M+H]^+$

Reference Example 187-4

Preparation of methyl 4-amino-(3-cyclohex-1-en-1-yl)-1-methyl-1H-pyrazole-5-carboxylate

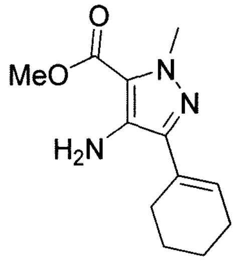

To a 300 mL eggplant flask were added methyl 4-amino-3-bromo-1-methyl-1H-pyrazole-5-carboxylate (2.24 g) prepared in the Reference Example 187-5, 1-cyclohexene boronic acid pinacol (2.49 mL), bis(di-tert-butyl(4-dimethylaminophenyl)phosphine)dichloropalladium(II) (338 mg), potassium carbonate (2.64 g), 1,4-dioxane (48 mL), and water (862 μL), the resulting mixture was subjected to nitrogen replacement, and then stirred at 100°C for 1 day. The reaction mixture was allowed to cool to room temperature, ethyl acetate (200 mL) and NH silica gel (2.4 g) were added thereto, and the resulting mixture was stirred for 30 minutes. The insoluble matters were removed by filtration, and the resulting filtrate was concentrated under reduced pressure. To the resulting residues was added chloroform, the insoluble matters were removed by filtration, and the resulting filtrate was concentrated under reduced pressure. The resulting residues were purified by silica gel column chromatography (solvent: hexane/ethyl acetate = 95/5 to 50/50) to give the title compound (1.12 g) (yield 50%) as a pale yellow solid. MS(ESI) m/z: 236 $[M+H]^+$

Reference Example 187-3

Preparation of 3-(cyclohex-1-en-1-yl)-1-methyl-1,6-dihydro-7H-pyrazolo[4,3-d]pyrimidin-7-one

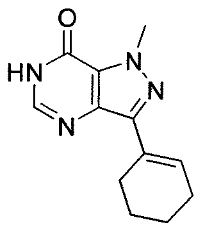

To a 200 mL eggplant flask were added methyl 4-amino-(3-cyclohex-1-en-1-yl)-1-methyl-1H-pyrazole-5-carboxylate (559 mg) prepared in the Reference Example 187-4, formamidine acetate (748 mg), N,N-diisopropylethylamine (1.24 mL), and ethanol (12 mL), and the resulting mixture was heated under reflux overnight. The reaction mixture was allowed to cool to room temperature, ethyl acetate and water were added thereto to be separated, and the resulting aqueous layer was extracted with ethyl acetate. The resulting organic layers were combined, washed sequentially with water and saturated brine, dried over anhydrous sodium sulfate, and the insoluble matters were removed by filtration. The resulting filtrate was concentrated under reduced pressure, and the resulting residues were purified by silica gel column chromatography (solvent: hexane/ethyl acetate = 60/40 to 30/70) to give the title compound (445 mg) (yield 81%) as a colorless powder.
MS(ESI) m/z: 231 $[M+H]^+$

Reference Example 187-2

Preparation of 7-chloro-3-(cyclohex-1-en-1-yl)-1-methyl-1H-pyrazolo[4,3-d]pyrimidine

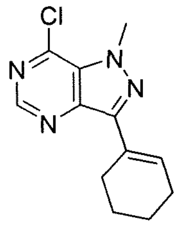

To a 100 mL eggplant flask were added 3-(cyclohex-1-en-1-yl)-1-methyl-1,6-dihydro-7H-pyrazolo[4,3-d]pyrimidin-7-one (223 mg) prepared in the Reference Example 187-3, oxalyl chloride (0.410 mL), N,N-dimethylformamide (0.3 mL), and chloroform (4.8 mL), and the resulting mixture was stirred at 80°C for 2 hours. The reaction mixture was allowed to cool to room temperature, ethyl acetate and water were added thereto to be separated, and the resulting aqueous layer was extracted twice with ethyl acetate. The resulting organic layers were combined, washed with saturated brine, dried over anhydrous sodium sulfate, and the insoluble matters were removed by filtration. The resulting filtrate was concentrated under reduced pressure to give the title compound (222 mg) (yield 92%) as an orange powder.
MS(ESI) m/z: 249/251 $[M+H]^+$

Reference Example 187-1

Preparation of 3-(cyclohex-1-en-1-yl)-N,N-bis(2,4-dimethoxybenzyl)-1-methyl-1H-pyrazolo[4,3-d]pyrimidin-7-amine

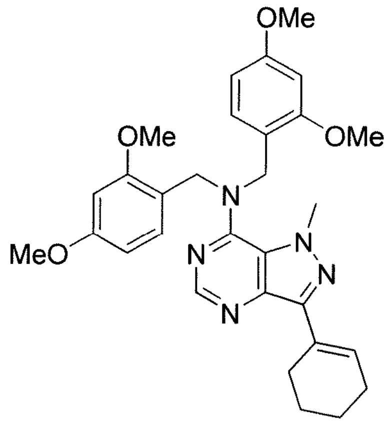

7-Chloro-3-(cyclohex-1-en-1-yl)-1-methyl-1H-pyrazolo[4,3-d]pyrimidine prepared in the Reference Example 187-2 was reacted in a similar manner to the Reference Example 112-2 to give the title compound.
MS(ESI) m/z: 530 $[M+H]^+$

Reference Example 188-3

Preparation of 3-cyclohexylisoxazolo[4,5-d]pyrimidin-7(6H)-one

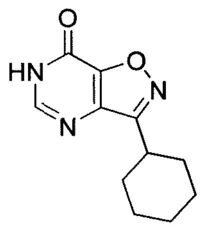

A corresponding starting compound was reacted in a similar manner to the Reference Example 187-3 to give the title compound.
MS(APCI) m/z: 220 [M+H]+

Reference Example 188-2

Preparation of 7-chloro-3-cyclohexylisoxazolo[4,5-d]pyrimidine

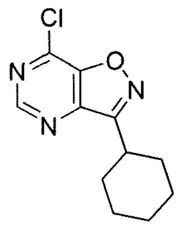

3-Cyclohexylisoxazolo[4,5-d]pyrimidin-7(6H)-one prepared in the Reference Example 188-3 was reacted in a similar manner to the Reference Example 187-2 to give the title compound.
MS(APCI) m/z: 238/240 [M+H]+

Reference Example 188-1

Preparation of 3-cyclohexyl-N-(2,4-dimethoxybenzyl)isoxazolo[4,5-d]pyrimidin-7-amine

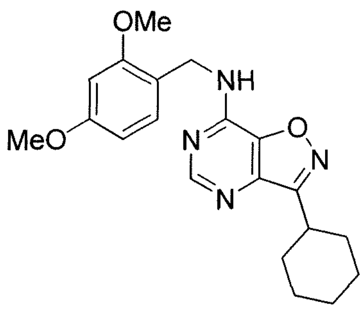

7-Chloro-3-cyclohexylisoxazolo[4,5-d]pyrimidine prepared in the Reference Example 188-2 and a corresponding starting compound were reacted in a similar manner to the Reference Example 112-2 to give the title compound.
MS(APCI) m/z: 369 [M+H]+

Reference Example 189-2

Preparation of N'-(6-chloro-5-nitropyrimidin-4-yl)cyclohexanecarbohydrazide

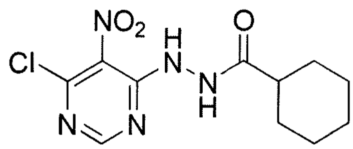

A mixture of 4,6-dichloro-5-nitropyrimidine (2.0 g), cyclohexanecarbohydrazide (1.5 g), triethylamine (1.7 mL), and tetrahydrofuran (30 mL) was stirred at room temperature for 1 hour and 30 minutes. To the reaction mixture was added water, and the resulting mixture was extracted twice with ethyl acetate. The resulting organic layers were combined, washed with saturated brine, dried over anhydrous magnesium sulfate, and the insoluble matters were removed by filtration. The resulting filtrate was concentrated under reduced pressure, and the resulting residues were purified by silica gel column chromatography (solvent: hexane/ethyl acetate = 90/10 to 70/30) to give the title compound (1.74 g) (yield 56%) as a pale red powder.
MS(APCI) m/z: 300/302 $[M+H]^+$

Reference Example 189-1

Preparation of N'-(5-aminopyrimidin-4-yl)cyclohexanecarbohydrazide

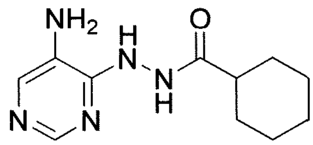

N'-(6-chloro-5-nitropyrimidin-4-yl)cyclohexanecarbohydrazide prepared in the Reference Example 189-2 was reacted in a similar manner to the Example 179 to give the title compound.
MS(APCI) m/z: 236 $[M+H]^+$

Reference Example 252-1

Preparation of 3-(3,3-dimethylpiperidin-1-yl)-8-methoxy-5-(trifluoromethyl)-[1,2,4]triazolo[4,3-a]pyrazine

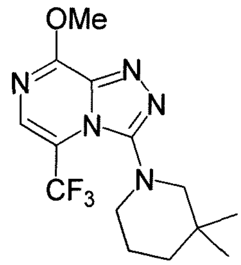

To a 20 mL cylindrical flask subjected to argon replacement were added copper(I) iodide (3 mg), phenanthroline (3 mg), and potassium fluoride (17 mg), and the resulting mixture was subjected argon replacement. N-methylpyrrolidone (250 μL), N,N-dimethylformamide (250 μL), 5-bromo-3-(3,3-dimethylpiperidin-1-yl)-8-methoxy-[1,2,4]triazolo[4,3-a]pyrazine (50 mg) prepared in the Reference Example 80-3, and (trifluoromethyl)trimethylsilane (43 mg) were added thereto, and the resulting mixture was stirred at room temperature. After the reaction was completed, a 1N aqueous solution of sodium hydroxide was added thereto, and the resulting mixture was extracted with ethyl acetate. The resulting organic layer was washed with saturated brine, dried over anhydrous magnesium sulfate, and concentrated under reduced pressure. The resulting residues were subjected to silica chromatography (hexane : ethyl acetate = 70 : 30 to 50 : 50) using YAMAZEN medium pressure preparative (Silica M (16 g)), the fractions comprising the target compound (Rf value = 0.55 (hexane : ethyl acetate = 50 : 50) were collected, and concentrated under reduced pressure to give the title compound (7 mg) (yield 15%) as a yellow oil.

MS(DUIS) m/z: 338 $[M+H]^+$

Reference Example 253-3

Preparation of ethyl 5-cyclohexyl-4-nitroisoxazole-3-carboxylate

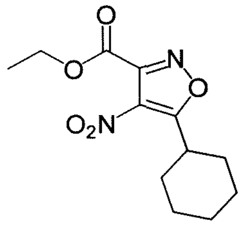

To a solution of 5-cyclohexylisoxazole-3-carboxylic acid (1.95 g) described in Bioorganic & Medicinal Chemistry Letters 23 Issues 23, 6346 (2013) and potassium nitrate (1.52 g) in concentrated sulfuric acid (20 mL) in a 100 mL eggplant flask was added potassium nitrate (1.52 g) under argon atmosphere with stirring at room temperature, and the resulting mixture was stirred at 50°C for 4 hours.
After the reaction was completed, water (100 mL) was added thereto, and the resulting mixture was extracted twice with ethyl acetate. The resulting organic layers were combined, dried over anhydrous magnesium sulfate, and concentrated under reduced pressure. To the resulting residues were added ethanol (30 mL) and concentrated sulfuric acid (2 mL), and the resulting mixture was stirred at 80°C for 3 hours. After the reaction was completed, the reaction solution was concentrated under reduced pressure, a saturated aqueous solution of sodium hydrogen carbonate was added thereto to neutralize the mixture, and the resulting mixture was extracted with ethyl acetate. The resulting organic layer was dried over anhydrous magnesium sulfate, and concentrated under reduced pressure to give the title compound (1.23 g) (yield 46%) as an orange oil. MS(CI) m/z: 269 $[M+H]^+$

Reference Example 253-2

Preparation of ethyl 4-amino-5-cyclohexylisoxazole-3-carboxylate

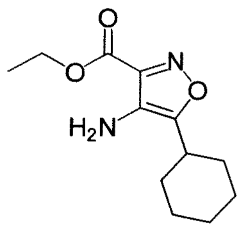

To a 100 mL eggplant flask were added zinc powder (1.49 g), acetic acid (1.37 g), and methanol (25 mL), a solution of ethyl 5-cyclohexyl-4-nitroisoxazole-3-carboxylate (1.23 g) prepared in the Reference Example 253-3 in methanol (5 mL) was added dividedly thereto with stirring at or below 28°C, and the resulting mixture was stirred at room temperature for 1 hour. Then, zinc powder (1.49 g) and acetic acid (1.37 g) were added thereto, and the resulting mixture was stirred at 50°C for 3 hours.
After the reaction was completed, the reaction solution was concentrated under reduced pressure, water was added thereto, and the resulting mixture was extracted with ethyl acetate. The resulting organic layer was washed with water, dried over anhydrous magnesium sulfate, and concentrated under reduced pressure. The resulting residues were subjected to silica gel column chromatography (hexane : ethyl acetate = 95 : 5 to 70 : 30) using Moritex medium pressure preparative (Purif-Pack SI size 60 (30 g)), the fractions comprising the target compound were collected, and concentrated under reduced pressure. To the resulting solid was added hexane, the resulting mixture was filtered, and washed with hexane to give the title compound (0.24 g) (yield 22%) as a colorless solid.
MS(CI) m/z: 239 $[M+H]^+$

Reference Example 253-1

Preparation of 3-cyclohexylisoxazolo[4,3-d]pyrimidin-7-ol

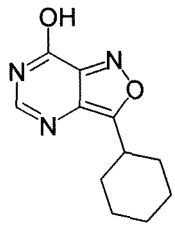

To a 30 mL cylindrical flask were added ethyl 4-amino-5-cyclohexylisoxazole-3-carboxylate (30 mg) prepared in the Reference Example 253-2, formamidine acetate (39 mg), ethanol (0.5 mL), and diisopropylethylamine (65 μL), and the resulting mixture was stirred at 90°C for 5 hours.

The same reaction as the above reaction was carried out except for changing the scale as follows.

To a 30 mL cylindrical flask were added ethyl 4-amino-5-cyclohexylisoxazole-3-carboxylate (0.23 g) prepared in the Reference Example 253-2, formamidine acetate (0.26 g), ethanol (4 mL), and diisopropylamine (0.44 mL), and the resulting mixture was stirred at 90°C for 5 hours.

The above two reaction solutions were combined, concentrated under reduced pressure, water was added thereto, the precipitated solid was filtered, and washed with water to give the title compound (190 mg) (yield 90%) as a colorless solid.
MS(CI) m/z: 220 $[M+H]^+$

Pharmacological Experimental Examples

Next, Pharmacological Experimental Examples are shown.

Experimental Example 1.

Measurement of PDE7 inhibitory activity

<Experimental method 1>

[Method for preparing samples 1]

Human PDE7B (hPDE7B) was isolated from COS-7 cells transfected with plasmids encoding hPDE7B according to the method described in a reference, Biochemical and Biophysical Research Communication, 271, p.575-583 (2000). The resulting enzyme solution was used in the PDE assay.

[Assay procedure 1]

PDE7 inhibitory assay was carried out by reacting the mixture of a compound, hPDE7B, and cAMP, and measuring the residual cAMP by a detection method using LANCE (registered trademark) Ultra cAMP Detection Kit (ParkinElmer). The compound was dissolved in DMSO, and diluted so that the concentration would become 50 times of the final concentration. The hPDE7B and cAMP were diluted with an assay buffer (50 mmol/L Tris-HCl, 1 mmol/L $MgCl_2$, 0.1% BSA, 0.5 mmol/L DTT, pH7.5) so that the hPDE7B would have an appropriate enzyme activity and the concentration of cAMP would become 6 nmol/L. To a 96 well plate were added the compound (2 μL) (DMSO final concentration: 2%), hPDE7B (48 μL), and cAMP (50 μL) (concentration at the reaction: 3 nmol/L), and the resulting mixture was reacted at room temperature for 60 minutes. The reaction solution (20 μL) was subjected to a TR-FRET method according to the protocol specified by the kit to measure the concentration of the residual cAMP. In the assay, to a tracer solution specified by the kit was added 0.5 mmol/L of 3-isobutyl-1-methylxanthine (IBMX, nonselective PDE inhibitor) in order to stop the PDE enzyme reaction after the completion of reaction until the measurement.

<Method for data calculation 1>

The reaction without a compound was defined as "0% inhibition", the reaction without hPDE7B was defined as "100% inhibition", and the inhibition rate of the compound was calculated by the following equation.

Inhibition rate of compound (%) = {(Residual cAMP concentration at the addition of compound - Residual cAMP concentration in 0% inhibition) / (Residual cAMP concentration in 100% inhibition - Residual cAMP concentration in 0% inhibition)} × 100

Each compound was evaluated at three or more concentrations with common ratio 10, and an approximate linear equation was prepared using two concentrations (in which one showed more than 50% of inhibition rate and the other showed less than 50% of inhibition rate) and the inhibition rates to calculate the $IC_{50}$ value.

<Experimental Results 1>

The results are shown in the following Table 48.

Table 48

| Test compound (Example No.) | PDE7 inhibitory activity $IC_{50}$ (μmol/L) |
|---|---|
| 1 | 0.08 |
| 2 | 0.0007 |
| 3 | 0.05 |
| 4 | 0.05 |
| 5 | 0.03 |
| 6 | 0.34 |
| 7 | 0.56 |
| 8 | 0.01 |
| 9 | 0.89 |
| 10 | 0.1 |
| 11 | 0.4 |
| 12 | 0.62 |
| 13 | 0.16 |
| 14 | 0.56 |
| 15 | 0.57 |
| 16 | 0.35 |
| 17 | 0.32 |
| 18 | 0.46 |
| 19 | 0.24 |
| 20 | 0.08 |
| 21 | 0.09 |

(continued)

| Test compound (Example No.) | PDE7 inhibitory activity $IC_{50}$ (μmol/L) |
|---|---|
| 22 | 0.04 |
| 23 | 0.04 |
| 24 | 0.07 |
| 25 | 0.19 |
| 26 | 0.02 |
| 27 | 0.32 |
| 28 | 0.09 |
| 29 | 0.08 |
| 30 | 0.39 |
| 31 | 0.48 |
| 32 | 0.08 |
| 33 | 0.52 |
| 34 | 0.46 |
| 35 | 0.2 |
| 36 | 0.38 |
| 37 | 0.49 |
| 38 | 0.08 |
| 39 | 0.22 |
| 40 | 0.73 |
| 41 | 0.29 |
| 42 | < 0.01 |
| 43 | 0.03 |
| 44 | 0.08 |
| 45 | 0.21 |
| 46 | 0.08 |
| 47 | 0.3 |
| 48 | 0.07 |
| 49 | 0.26 |
| 50 | 0.52 |
| 51 | 0.11 |
| 52 | 0.08 |
| 53 | 0.02 |
| 54 | 0.04 |
| 55 | 0.03 |
| 56 | 0.19 |
| 57 | 0.05 |
| 58 | 0.02 |
| 59 | 0.24 |

(continued)

| Test compound (Example No.) | PDE7 inhibitory activity $IC_{50}$ (μmol/L) |
|---|---|
| 60 | < 0.01 |
| 61 | 0.06 |
| 62 | 0.04 |
| 63 | 0.304 |
| 64 | 0.107 |
| 65 | < 0.01 |
| 66 | 0.36 |
| 67 | 0.09 |
| 68 | 0.02 |
| 69 | 0.01 |
| 70 | 0.025 |
| 71 | 0.004 |
| 72 | 0.01 |
| 73 | 0.001 |
| 74 | < 0.01 |
| 75 | 0.053 |
| 76 | < 0.01 |
| 77 | 0.31 |
| 78 | 0.14 |
| 79 | 0.26 |
| 80 | 0.003 |
| 81 | 0.16 |
| 82 | 0.04 |
| 83 | < 0.01 |
| 84 | 0.73 |
| 85 | 0.04 |
| 86 | 0.36 |
| 87 | 0.03 |
| 88 | 0.84 |
| 89 | 0.16 |
| 90 | 0.12 |
| 91 | 0.36 |
| 92 | < 0.01 |
| 93 | 0.02 |
| 94 | 0.06 |
| 95 | 0.04 |
| 96 | 0.95 |
| 97 | 0.05 |

(continued)

| Test compound (Example No.) | PDE7 inhibitory activity $IC_{50}$ (μmol/L) |
|---|---|
| 98 | 0.08 |
| 99 | 0.02 |
| 100 | 0.12 |
| 101 | 0.02 |
| 102 | 0.25 |
| 103 | 0.45 |
| 104 | 0.17 |
| 105 | 0.31 |
| 106 | 0.3 |
| 107 | > 1 (35% inhibition at 1 μmol/L) |
| 108 | 0.23 |
| 109 | 0.04 |
| 110 | 0.03 |
| 111 | 0.32 |
| 112 | 0.17 |
| 113 | 0.03 |
| 114 | 0.19 |
| 115 | < 0.01 |
| 116 | 0.38 |
| 117 | 0.08 |
| 118 | 0.08 |
| 119 | 0.02 |
| 120 | 0.38 |
| 121 | 0.87 |
| 123 | 0.42 |
| 124 | 0.26 |
| 125 | 0.21 |
| 126 | 0.28 |
| 127 | 0.73 |
| 128 | 0.02 |
| 129 | 0.75 |
| 130 | 0.49 |
| 131 | 0.25 |
| 132 | 0.67 |
| 133 | 0.16 |
| 134 | 0.33 |
| 135 | 0.31 |
| 136 | 0.68 |

(continued)

| Test compound (Example No.) | PDE7 inhibitory activity $IC_{50}$ (μmol/L) |
|---|---|
| 137 | 0.19 |
| 138 | 0.34 |
| 139 | 0.09 |
| 140 | 0.01 |
| 141 | 0.01 |
| 142 | 0.05 |
| 143 | 0.05 |
| 144 | 0.01 |
| 145 | 0.03 |
| 146 | 0.04 |
| 147 | 0.7 |
| 149 | 0.04 |
| 150 | 0.13 |
| 151 | 0.01 |
| 152 | < 0.01 |
| 153 | 0.07 |
| 154 | 0.11 |
| 155 | 0.09 |
| 156 | 0.03 |
| 157 | 0.92 |
| 158 | 0.07 |
| 159 | 0.06 |
| 160 | 0.08 |
| 161 | 0.11 |
| 162 | 0.17 |
| 163 | 0.22 |
| 164 | 0.03 |
| 165 | 0.36 |
| 166 | 0.16 |
| 167 | 0.06 |
| 168 | 0.44 |
| 169 | 0.09 |
| 170 | 0.27 |
| 171 | 0.07 |
| 172 | < 0.01 |
| 173 | > 1 (3% inhibition at 1 μmol/L) |
| 174 | 0.003 |
| 175 | < 0.01 |

(continued)

| Test compound (Example No.) | PDE7 inhibitory activity $IC_{50}$ (μmol/L) |
|---|---|
| 176 | > 1 (28% inhibition at 1 μmol/L) |
| 177 | 0.035 |
| 178 | > 1 (33% inhibition at 1 μmol/L) |
| 179 | 0.08 |
| 180 | 0.39 |
| 181 | 0.34 |
| 182 | 0.41 |
| 183 | 0.23 |
| 184 | 0.54 |
| 185 | 0.56 |
| 186 | 0.46 |
| 187 | 0.58 |
| 188 | 0.21 |
| 189 | 0.47 |
| 190 | 0.22 |
| 191 | < 0.01 |
| 192 | 0.04 |
| 193 | 0.43 |
| 194 | 0.04 |
| 195 | 0.05 |
| 196 | < 0.01 |
| 197 | 0.34 |
| 198 | 0.07 |
| 199 | 0.03 |
| 200 | 0.03 |
| 201 | > 1 (20% inhibition at 1 μmol/L) |
| 202 | 0.014 |
| 203 | > 1 (27% inhibition at 1 μmol/L) |
| 204 | < 0.01 |
| 205 | 0.66 |
| 206 | > 1 (44% inhibition at 1 μmol/L) |
| 207 | < 0.01 |
| 208 | < 0.01 |
| 209 | 0.13 |
| 210 | 0.013 |
| 211 | > 1(20% inhibition at 1 μmol/L) |
| 212 | 0.04 |
| 213 | 0.33 |

(continued)

| Test compound (Example No.) | PDE7 inhibitory activity $IC_{50}$ (μmol/L) |
|---|---|
| 214 | 0.06 |
| 215 | 0.53 |
| 216 | 0.05 |
| 217 | 0.1 |
| 218 | 0.08 |
| 219 | 0.05 |
| 220 | 0.11 |
| 221 | 0.06 |
| 222 | 0.08 |
| 223 | 0.19 |
| 224 | 0.17 |
| 225 | 0.54 |
| 226 | 0.01 |
| 227 | 0.19 |
| 228 | 0.54 |
| 229 | 0.31 |
| 230 | 0.08 |
| 231 | 0.01 |
| 232 | 0.003 |
| 233 | 0.1 |
| 234 | 0.00015 |
| 235 | 0.016 |
| 236 | 0.033 |
| 237 | 0.0028 |
| 238 | < 0.01 |
| 239 | 0.11 |
| 240 | < 0.01 |
| 241 | 0.23 |
| 242 | < 0.01 |
| 243 | 0.12 |
| 244 | < 0.01 |
| 245 | 0.85 |
| 246 | 0.003 |
| 247 | 0.629 |
| 248 | < 0.01 |
| 249 | 0.74 |
| 250 | 0.18 |
| 251 | 0.62 |

(continued)

| Test compound (Example No.) | PDE7 inhibitory activity $IC_{50}$ (μmol/L) |
|---|---|
| 252 | < 0.01 |
| 253 | 0.61 |

Experimental Example 2.

Measurement of PDE1 to 6 and 8 to 11 inhibitory activities (Ki) for determining PDE7 selectivity

The PDE7 selectivity was evaluated by comparing Ki values of a compound against PDE1 to 6 and 8 to 11 with Ki value of said compound against PDE7B.

<Experimental method 2>

[Method for preparing samples 2]

hPDE1A, hPDE2A, hPDE3A, hPDE4D, hPDE5A, and hPDE8B were purchased from SB Drug Discovery. hPDE7B was isolated by the same method as [Method for preparing samples 1], and hPDE9A, hPDE10A, and hPDE11A were isolated by the same method as hPDE7B, i.e., isolated from COS-7 cells transfected with plasmids encoding each PDE. PDE6 was purified and isolated from bovine retina (bovine PDE6).

[Assay procedure 2]

Prior to the calculation of Ki value, Km value of each PDE against cAMP or cGMP was calculated. Each PDE diluted with an assay buffer so that it would have an appropriate enzyme activity, and six or more concentrations of cAMP or cGMP were reacted at room temperature for 60 minutes. Regarding PDE using cAMP as a substrate, PDELight (trademark) HTS cAMP phosphodiesterase Kit (Lonza) was used to measure a degradation product, 5'-AMP. Also, regarding PDE using cGMP as a substrate, 0.04 μmol/L of perchloric acid was added to the mixture to stop the reaction, and the resulting mixture was subjected to LC-MS/MS to measure the residual cGMP concentration. The amount of degraded substrate in each substrate concentration was calculated, the concentration of the added substrate was plotted on the horizontal axis, the amount of degraded substrate was plotted on the vertical axis, and Km value was calculated by non-linear regression on the basis of Michaelis-Menten equation. Km value (substrate) of each PDE was hPDE1A: 4.3 (cGMP), hPDE2A: 36 (cAMP), hPDE3A: 0.11 (cAMP), hPDE4D: 0.90 (cAMP), hPDE5A: 3.9 (cGMP), bovine PDE6: 9.8 (cGMP), hPDE7B: 0.015 (cAMP), hPDE8B: 0.63 (cAMP), hPDE9A: 0.0037 (cGMP), hPDE10A: 0.051 (cAMP), and hPDE11A: 1.4 (cAMP) μmol/L, respectively.

Next, PDE inhibition assay of a compound was carried out using each PDE. The PDE inhibition assay was basically carried out by the same enzyme reaction method as [Assay procedure 1], and the degraded amount of cAMP or cGMP was measured by the same method as [Assay procedure 2]. In the assay, a concentration approximated to Km value of each PDE against cAMP or cGMP was used as a substrate concentration. Meanwhile, regarding hPDE5A, hPDE8B, and hPDE9A, IMAP (trademark) FP Phosphodiesterase Evaluation Assay Kit (Molecular Devices) was used to measure the degradation of FAM-cAMP or FAM-cGMP by fluorescence depolarization technique.

<Method for data calculation 2>

Each compound was evaluated at six or more concentrations with common ratio 10. Each inhibition rate was calculated according to <Method for data calculation 1>, and then each $IC_{50}$ value was calculated by sigmoid regression. The resulting $IC_{50}$ value was used in the following Cheng-Prusoff equation to calculate each Ki value. $Ki = IC_{50}/(1+[S]/Km)$, wherein [S] represents a substrate concentration used

<Experimental Results 2>

Each selectivity test (Ki value) of test compounds 3, 4, 141, 191, and 204 is shown in the following Table 49. PDE7 selectivity test (Ki value)

Table 49

| Test compound (Example No.) | | 3 | 4 | 141 | 191 | 204 |
|---|---|---|---|---|---|---|
| PDE inhibition constant Ki (μmol/L) | PDE7 | 0.031 | 0.027 | 0.031 | 0.012 | 0.0030 |
| | PDE1 | > 52 | > 5.2 | > 5.2 | 39 | 8.9 |
| | PDE2 | 27 | 6.6 | > 5.1 | 10 | 14 |
| | PDE3 | > 52 | > 5.2 | > 5.2 | > 52 | 43 |
| | PDE4 | 4.4 | 0.22 | > 4.8 | 4.2 | 1.2 |
| | PDE5 | > 98 | > 9.8 | > 9.8 | > 98 | > 98 |
| | PDE6 | > 49 | > 4.9 | > 4.9 | > 49 | > 49 |
| | PDE8 | 0.77 | 0.085 | 1.4 | 5.3 | 1.4 |
| | PDE9 | > 3.6 | > 0.36 | > 0.36 | > 3.6 | > 3.6 |
| | PDE10 | 12 | 1.1 | > 5.1 | 2.4 | 2.6 |
| | PDE11 | 29 | > 5.0 | > 5.0 | 49 | 46 |

Experimental Example 3.

Measurement of PDE4, 8, and 10 inhibitory activity ($IC_{50}$) for the prediction of PDE7 selectivity

The prediction of PDE7 selectivity was evaluated by comparing each $IC_{50}$ value of a compound against PDE4, 8, and 10 with the $IC_{50}$ value of said compound against PDE7B.

<Experimental method 3>

[Method for preparing samples 3]

hPDE4D, hPDE8B, and hPDE10A were prepared by the same method as [Method for preparing samples 2].

[Assay procedure 3]

PDE4, 8, and 10 inhibitory assays were carried out by the same method as [Assay procedure 1].

<Method for data calculation 3>

The reaction without a compound wad defined as "0% inhibition", the reaction without each PDE was defined as "100% inhibition", and the calculation of $IC_{50}$ value was carried out by the same method as <Method for data calculation 1>.

<Experimental Results 3>

$IC_{50}$ value(s) or inhibition rate(s) at prescribed concentration(s) in the PDE4, 8, and 10 inhibition assays of each test compound are shown in the following Table 50. PDE4, 8, and 10 inhibition assays ($IC_{50}$)

Table 50

| Test compound (Example No.) | PDE inhibition assay $IC_{50}$ (μmol/L) or inhibition rate (%) at prescribed concentration | | |
|---|---|---|---|
| | PDE4 | PDE8 | PDE10 |
| 2 | 61% at 1 μmol/L | 1.62 | 6.23 |
| 3 | 7.82 | 0.70 | 27% at 10 μmol/L |
| 4 | 0.54 | | 1.75 |
| 5 | 0.19 | | 2.76 |

(continued)

| Test compound (Example No.) | PDE inhibition assay $IC_{50}$ (μmol/L) or inhibition rate (%) at prescribed concentration | | |
|---|---|---|---|
| | PDE4 | PDE8 | PDE10 |
| 7 | | 50% at 10 μmol/L | |
| 8 | 2.13 | 0.24 | 48% at 10 μmol/L |
| 13 | | 0.22 | |
| 20 | 40% at 10 μmol/L | 1.58 | 22% at 10 μmol/L |
| 22 | 8.30 | 5.80 | 5.50 |
| 24 | 6.30 | 1.60 | 12.90 |
| 26 | 4.59 | 7.66 | 21.14 |
| 28 | 4.30 | 3.70 | 28.40 |
| 29 | 5.40 | 5.10 | 11.90 |
| 32 | 5.78 | 2.27 | 22.57 |
| 38 | 5.21 | 2.74 | 24.95 |
| 42 | 3.00 | 1.70 | 5.20 |
| 43 | 2.19 | 1.52 | 2.33 |
| 44 | 7.85 | 5.51 | 24.09 |
| 46 | 56.90 | 7.70 | 34.50 |
| 48 | 1.30 | 79% at 1 μmol/L | 52% at 1 μmol/L |
| 52 | 8.19 | 1.16 | 6.80 |
| 54 | 7.86 | 3.35 | 30% at 100 μmol/L |
| 57 | 6.40 | 9.90 | 8.70 |
| 61 | 5.80 | 18.40 | 16.30 |
| 62 | 4.30 | 21.30 | 7.10 |
| 67 | 20.99 | 15.01 | 31.79 |
| 68 | 4.13 | 0.50 | 34% at 10 μmol/L |
| 70 | 67% at 1 μmol/L | 64% at 1 μmol/L | 1.80 |
| 72 | 1.49 | 62% at 1 μmol/L | 8.91 |
| 75 | 27.10 | 4.33 | 44% at 100 μmol/L |
| 80 | 6.00 | 3.00 | 8.00 |
| 82 | 6.21 | 2.49 | 17.94 |
| 83 | 53% at 1 μmol/L | 1.33 | 3.04 |
| 89 | 37% at 10 μmol/L | 8.60 | 17% at 10 μmol/L |
| 90 | 3.70 | 59% at 1 μmol/L | 19.00 |
| 92 | 0.64 | | 2.97 |
| 99 | 1.60 | 0.12 | 38% at 10 μmol/L |
| 100 | | 1.80 | |
| 101 | 0.64 | 69% at 0.1 μmol/L | 5.84 |
| 104 | 9.45 | 2.06 | 25% at 10 μmol/L |
| 106 | | 4.38 | |

(continued)

| Test compound (Example No.) | PDE inhibition assay $IC_{50}$ (μmol/L) or inhibition rate (%) at prescribed concentration | | |
|---|---|---|---|
| | PDE4 | PDE8 | PDE10 |
| 109 | 99.20 | 12.90 | 24% at 100 μmol/L |
| 110 | 3.50 | 0.59 | 33% at 10 μmol/L |
| 115 | 11.90 | 52% at 1 μmol/L | 12.29 |
| 118 | 4.25 | 55% at 1 μmol/L | |
| 125 | | 1.11 | |
| 126 | | 2.65 | |
| 128 | 4.53 | 1.31 | 43% at 10 μmol/L |
| 135 | 17% at 10 μmol/L | 9.41 | 22% at 10 μmol/L |
| 137 | | 58% at 0.1 μmol/L | |
| 141 | 6.22 | 1.77 | 30% at 10 μmol/L |
| 142 | 5.72 | 49% at 10 μmol/L | 48% at 10 μmol/L |
| 149 | 2.20 | 1.66 | 46% at 10 μmol/L |
| 153 | 35.10 | 21.90 | 18.60 |
| 159 | 4.77 | 3.51 | 10.59 |
| 160 | 3.41 | 54% at 1 μmol/L | 34.89 |
| 161 | 5.50 | 1.77 | 27% at 10 μmol/L |
| 164 | 3.79 | 4.40 | 41% at 10 μmol/L |
| 166 | 3.87 | 8.89 | 39% at 10 μmol/L |
| 167 | 66% at 0.1 μmol/L | 98% at 0.1 μmol/L | 3.70 |
| 169 | 33% at 10 μmol/L | 1.74 | 20.80 |
| 171 | 48% at 10 μmol/L | 2.95 | 7.20 |
| 172 | 5.90 | 9.00 | 21.80 |
| 174 | 6.00 | 6.80 | 1.30 |
| 175 | 14.90 | 19.50 | 1.90 |
| 177 | 21.00 | 13.70 | 13.30 |
| 179 | 4.70 | 4.00 | 17.90 |
| 180 | 7.80 | 36% at 10 μmol/L | 41% at 10 μmol/L |
| 181 | 21% at 10 μmol/L | 24% at 10 μmol/L | 49% at 10 μmol/L |
| 182 | 3.42 | | 0.47 |
| 183 | 6.49 | 9.74 | 45% at 10 μmol/L |
| 184 | 48% at 10 μmol/L | 18% at 10 μmol/L | 0.50 |
| 185 | 1.84 | | 0.36 |
| 186 | -5% at 10 μmol/L | -1% at 10 μmol/L | 7% at 10 μmol/L |
| 187 | 38% at 10 μmol/L | | 2.72 |
| 188 | 38% at 10 μmol/L | 7.33 | 50% at 10 μmol/L |
| 189 | 22% at 10 μmol/L | 24% at 10 μmol/L | 0.9% at 10 μmol/L |
| 191 | 9.20 | 5.33 | 3.49 |

(continued)

| Test compound (Example No.) | PDE inhibition assay $IC_{50}$ (μmol/L) or inhibition rate (%) at prescribed concentration | | |
|---|---|---|---|
| | PDE4 | PDE8 | PDE10 |
| 192 | 45% at 10 μmol/L | 4.18 | 8.25 |
| 194 | 6.98 | 79% at 1 μmol/L | 12.16 |
| 195 | 5.70 | 1.41 | 30.58 |
| 196 | 2.49 | 0.29 | 49% at 10 μmol/L |
| 199 | 3.84 | 6.87 | 29% at 10 μmol/L |
| 200 | 0.54 | 0.99 | 16.60 |
| 202 | 5.31 | 1.16 | 27% at 10 μmol/L |
| 204 | 1.51 | 1.75 | 2.93 |
| 207 | 10.50 | 1.50 | 10.20 |
| 208 | 10.30 | 3.40 | 8.60 |
| 210 | 5.45 | 3.93 | 17.71 |
| 212 | 15.87 | 6.07 | 15.55 |
| 214 | 6.40 | 17.10 | 15.70 |
| 216 | 30.60 | 16.60 | 37.90 |
| 218 | 4.10 | 2.60 | 3.00 |
| 219 | 2.10 | 1.20 | 4.70 |
| 221 | 55% at 0.1 μmol/L | 69% at 0.1 μmol/L | 4.80 |
| 222 | 3.24 | 10.55 | 6.42 |
| 226 | 3.60 | 4.20 | 5.20 |
| 230 | 4.00 | 2.90 | 19.60 |
| 231 | 3.60 | 2.10 | 4.80 |
| 232 | 1.40 | 4.60 | 0.35 |
| 234 | 6.3 | 2.7 | 3.2 |
| 236 | 0.036 | 0.86 | 0.16 |
| 238 | 63% at 1 μmol/L | 51% at 1 μmol/L | 1.90 |
| 240 | 86% at 1 μmol/L | 95% at 1 μmol/L | 3.10 |
| 242 | 53% at 1 μmol/L | 84% at 1 μmol/L | 2.86 |
| 244 | 1. 60 | 73% at 1 μmol/L | 52.60 |
| 246 | 2.3 | 4.40 | |
| 248 | 1.88 | 1.51 | 5.57 |
| 252 | 1.80 | 53% at 1 μmol/L | 7.40 |
| 253 | 7.38 | 3.81 | |

INDUSTRIAL APPLICABILITY

The compound represented by formula (I) or a pharmaceutically acceptable salt thereof of the present invention has an excellent PDE7 inhibitory effect, and thus is useful for the treatment or prevention of diseases which are improved by inhibiting PDE7.

## Claims

1. A PDE7 inhibitor comprising a compound represented by the formula (I):

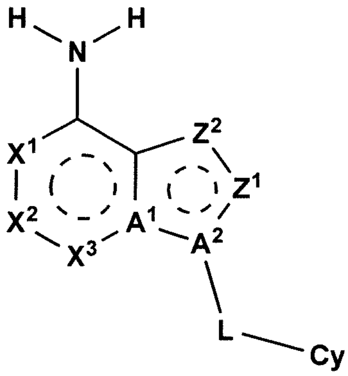

(I)

[wherein:
the partial structure represented by the following formula (1-1):

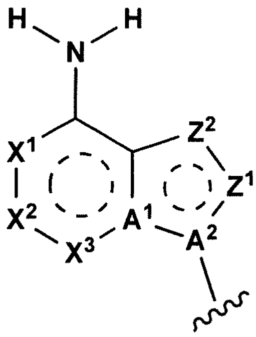

(I-1)

represents a partial structure selected from the group consisting of
the following formula (I-1-A):

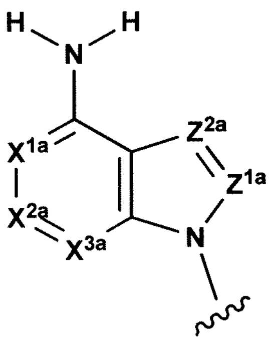

(I-1-A)

(wherein $X^{1a}$ is $CR^{X1a}$ or N; $X^{2a}$ is $CR^{X2a}$ or N; $X^{3a}$ is $CR^{X3a}$ or N; one or two of $X^{1a}$, $X^{2a}$, and $X^{3a}$ is/are N; $Z^{1a}$ is $CR^{Z1a}$ or N; and $Z^{2a}$ is $CR^{Z2a}$ or N);
the following formula (I-1-B):

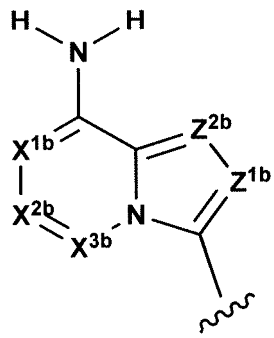

(I-1-B)

(wherein $X^{1b}$ is $CR^{X1b}$ or N; $X^{2b}$ is $CR^{X2b}$ or N; $X^{3b}$ is $CR^{X3b}$ or N; zero, one, or two of $X^{1b}$, $X^{2b}$, and $X^{3b}$ is/are N; $Z^{1b}$ is $CR^{Z1b}$ or N; and $Z^{2b}$ is $CR^{Z2b}$ or N);
the following formula (I-1-C):

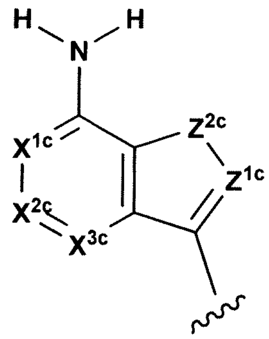

(I-1-C)

(wherein $X^{1c}$ is $CR^{X1c}$ or N; $X^{2c}$ is $CR^{X2c}$ or N; $X^{3c}$ is $CR^{X3c}$ or N; one or two of $X^{1c}$, $X^{2c}$, and $X^{3c}$ is/are N; $Z^{1c}$ is $CR^{Z1c}$ or N; and $Z^{2c}$ is $NR^{Z2c}$ or O), and
the following formula (I-1-D):

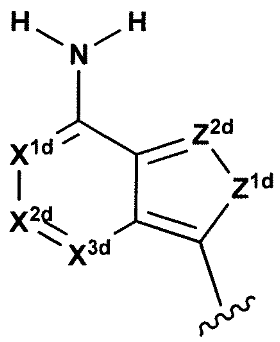

(I-1-D)

(wherein $X^{1d}$ is $CR^{X1d}$ or N; $X^{2d}$ is $CR^{X2d}$ or N; $X^{3d}$ is $CR^{X3d}$ or N; one or two of $X^{1d}$, $X^{2d}$, and $X^{3d}$ is/are N; $Z^{1d}$ is $NR^{Z1d}$ or O; and $Z^{2d}$ is $CR^{Z2d}$ or N);
$R^{X1a}$, $R^{X1b}$, $R^{X1c}$, and $R^{X1d}$ each independently represent a hydrogen atom, an optionally substituted alkyl group, or a halogen atom;
$R^{X2a}$, $R^{X2b}$, $R^{X2c}$, and $R^{X2d}$ each independently represent a hydrogen atom, an optionally substituted alkyl group, an optionally substituted alkoxy group, or an optionally substituted alkylthio group;
$R^{X3a}$, $R^{X3b}$, $R^{X3c}$, and $R^{X3d}$ each independently represent a hydrogen atom, an optionally substituted alkyl group, an optionally substituted cycloalkyl group, a halogen atom, a cyano group, or an optionally substituted aryl group;
$R^{Z1a}$, $R^{Z1b}$, and $R^{Z1c}$ each independently represent a hydrogen atom, a hydroxy group, or an optionally substituted alkyl group;
$R^{Z1d}$ represents a hydrogen atom or an optionally substituted alkyl group;
$R^{Z2a}$, $R^{Z2b}$, and $R^{Z2d}$ each independently represent a hydrogen atom, an optionally substituted alkyl group, an optionally substituted cycloalkyl group, or a halogen atom;

$R^{Z2c}$ represents a hydrogen atom or an optionally substituted alkyl group;
L represents a single bond or $CR^{L1}R^{L2}$;
$R^{L1}$ and $R^{L2}$ each independently represent a hydrogen atom or an optionally substituted alkyl group, or $R^{L1}$ and $R^{L2}$ each independently represent an alkylene group and are combined with each other together with the carbon atom to which they are attached to form an optionally substituted monocyclic saturated hydrocarbon group; and
Cy represents

(i) an aryl group optionally substituted with the same or different 1 to 5 substituent(s) selected from
an optionally substituted alkyl group;
an optionally substituted alkoxy group;
a halogen atom; and
an optionally substituted carboxamide group;
(ii) a heteroaryl group optionally substituted with the same or different 1 to 5 substituent(s) selected from an optionally substituted alkyl group and a halogen atom;
(iii) an alicyclic hydrocarbon group optionally substituted with the same or different 1 to 5 substituent(s) selected from
an optionally substituted alkyl group;
an optionally substituted alkenyl group;
an optionally substituted alkylidene group;
an optionally substituted alkoxy group;
a hydroxy group;
a halogen atom;
an oxo group;
an optionally substituted aryl group; and
an optionally substituted heteroaryl group; or
(iv) a nonaromatic heterocyclic group optionally substituted with the same or different 1 to 5 substituent(s) selected from
an optionally substituted alkyl group;
an optionally substituted cycloalkyl group;
an optionally substituted alkoxy group;
a hydroxy group;
a halogen atom;
an oxo group;
an optionally substituted aryl group;
an optionally substituted heteroaryl group;
an optionally substituted alkylcarbonyl group;
a formyl group;
an optionally substituted alkoxycarbonyl group; and
an optionally substituted arylcarbonyl group]

or a pharmaceutically acceptable salt thereof as an active ingredient.

**2.** The PDE7 inhibitor according to claim 1, wherein
$R^{X1a}$, $R^{X1b}$, $R^{X1c}$, and $R^{X1d}$ each independently represent a hydrogen atom, an alkyl group optionally substituted with the same or different 1 to 7 halogen atom(s), or a halogen atom;
$R^{X2a}$, $R^{X2b}$, $R^{X2c}$, and $R^{X2d}$ each independently represent a hydrogen atom, an alkyl group optionally substituted with the same or different 1 to 7 halogen atom(s), an alkoxy group optionally substituted with the same or different 1 to 7 halogen atom(s), or an alkylthio group optionally substituted with the same or different 1 to 7 halogen atom(s);
$R^{X3a}$, $R^{X3b}$, $R^{X3c}$, and $R^{X3d}$ each independently represent a hydrogen atom, an alkyl group optionally substituted with the same or different 1 to 7 halogen atom(s), a cycloalkyl group optionally substituted with the same or different 1 to 5 halogen atom(s), a halogen atom, a cyano group, or an aryl group optionally substituted with the same or different 1 to 5 halogen atom(s);
$R^{Z1a}$, $R^{Z1b}$, and $R^{Z1c}$ each independently represent a hydrogen atom, a hydroxy group, or an alkyl group optionally substituted with the same or different 1 to 7 halogen atom(s);
$R^{Z1d}$ represents a hydrogen atom or an alkyl group optionally substituted with the same or different 1 to 5 halogen atom(s);
$R^{Z2a}$, $R^{Z2b}$, and $R^{Z2d}$ each independently represent a hydrogen atom, an alkyl group optionally substituted with the same or different 1 to 7 halogen atom(s), a cycloalkyl group optionally substituted with the same or different 1 to 5

halogen atom(s), or a halogen atom;
$R^{Z2c}$ represents a hydrogen atom or an alkyl group optionally substituted with the same or different 1 to 5 halogen atom(s);
L represents a single bond or $CR^{L1}R^{L2}$;
$R^{L1}$ and $R^{L2}$ each independently represent a hydrogen atom or an alkyl group optionally substituted with the same or different 1 to 7 halogen atom(s), or $R^{L1}$ and $R^{L2}$ each independently represent a straight alkylene group and are combined with each other together with the carbon atom to which they are attached to form a monocyclic saturated hydrocarbon group optionally substituted with the same or different 1 to 6 halogen atom(s); and
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s);
(ii) a heteroaryl group optionally substituted with the same or different 1 to 5 substituent(s) selected from an alkyl group optionally substituted with the same or different 1 to 7 halogen atom(s) and a halogen atom;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group optionally substituted with the same or different 1, 2, or 3 substituent(s) selected from an alkyl group optionally substituted with the same or different 1 to 7 halogen atom(s) and a halogen atom;
an alkenyl group optionally substituted with the same or different 1 to 5 halogen atom(s);
an alkylidene group optionally substituted with the same or different 1 to 6 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s); and
a heteroaryl group optionally substituted with the same or different 1, 2, or 3 substituent(s) selected from an alkyl group optionally substituted with the same or different 1 to 7 halogen atom(s) and a halogen atom; or
(iv) a nonaromatic heterocyclic group optionally substituted with the same or different 1 to 5 substituent(s) selected from
an alkyl group optionally substituted with the same or different 1, 2, or 3 substituent(s) selected from an alkoxy group optionally substituted with the same or different 1 to 7 halogen atom(s), a halogen atom, and an aryl group optionally substituted with the same or different 1, 2, or 3 substituent(s) selected from an alkyl group optionally substituted with the same or different 1 to 7 halogen atom(s) and a halogen atom;
a cycloalkyl group optionally substituted with the same or different 1 to 5 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s);
a heteroaryl group optionally substituted with the same or different 1 to 5 halogen atom(s);
an alkylcarbonyl group optionally substituted with the same or different 1, 2, or 3 aryl group(s);
a formyl group;
an alkoxycarbonyl group optionally substituted with the same or different 1 to 7 halogen atom(s); and
an arylcarbonyl group optionally substituted with the same or different 1 to 5 halogen atom(s).

**3.** The PDE7 inhibitor according to claim 2, wherein
$R^{X1a}$, $R^{X1b}$, $R^{X1c}$, and $R^{X1d}$ each represent a hydrogen atom;
$R^{X2a}$, $R^{X2b}$, $R^{X2c}$, and $R^{X2d}$ each independently represent a hydrogen atom, an alkyl group optionally substituted with the same or different 1 to 7 halogen atom(s), an alkoxy group, or an alkylthio group;
$R^{X3a}$, $R^{X3b}$, $R^{X3c}$, and $R^{X3d}$ each independently represent a hydrogen atom, an alkyl group optionally substituted with the same or different 1 to 7 halogen atom(s), a cycloalkyl group, a halogen atom, a cyano group, or an aryl group;

$R^{Z1a}$, $R^{Z1b}$, and $R^{Z1c}$ each independently represent a hydrogen atom, a hydroxy group, or an alkyl group;
$R^{Z1d}$ represents an alkyl group;
$R^{Z2a}$, $R^{Z2b}$, and $R^{Z2d}$ each independently represent a hydrogen atom, an alkyl group optionally substituted with the same or different 1 to 7 halogen atom(s), a cycloalkyl group, or a halogen atom;
$R^{Z2c}$ represents an alkyl group;
L represents a single bond or $CR^{L1}R^{L2}$;
$R^{L1}$ and $R^{L2}$ each independently represent a hydrogen atom or an alkyl group, or $R^{L1}$ and $R^{L2}$ each independently represent a straight alkylene group and are combined with each other together with the carbon atom to which they are attached to form a monocyclic saturated hydrocarbon group; and
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s);
(ii) a heteroaryl group optionally substituted with the same or different 1 to 5 halogen atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group;
an alkenyl group;
an alkylidene group;
an alkoxy group;
a hydroxy group;
a halogen atom;
an oxo group;
an aryl group; and
a heteroaryl group optionally substituted with the same or different 1, 2, or 3 alkyl group(s); or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a cycloalkyl group;
a halogen atom;
an oxo group;
an aryl group;
a heteroaryl group;
an alkylcarbonyl group optionally substituted with the same or different 1, 2, or 3 aryl group(s);
a formyl group; and
an alkoxycarbonyl group.

**4.** The PDE7 inhibitor according to claim 3, wherein
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a heteroaryl group optionally substituted with the same or different 1 to 5 halogen atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;

an alkenyl group;
an alkylidene group;
an alkoxy group;
a hydroxy group;
a halogen atom; and
a heteroaryl group optionally substituted with the same or different 1, 2, or 3 alkyl group(s); or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group.

**5.** The PDE7 inhibitor according to claim 4, wherein
Cy represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a 6 to 11 membered monocyclic or bicyclic aromatic hydrocarbon group;
(ii) a heteroaryl group optionally substituted with the same or different 1 to 5 halogen atom(s), wherein said heteroaryl group is a 5 to 11 membered monocyclic or bicyclic aromatic heterocyclic group comprising 1 to 4 heteroatom(s) selected from an oxygen atom, a sulfur atom, and a nitrogen atom other than carbon atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a $C_3$-$C_8$ cycloalkyl group, a $C_6$-$C_{12}$ bicycloalkyl group, a $C_6$-$C_{12}$ bicycloalkenyl group, a $C_6$-$C_{12}$ spiroalkyl group, or a $C_{10}$-$C_{14}$ tricyclic tricycloalkyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a 4 to 8 membered monocyclic nonaromatic heterocyclic group or a 6 to 12 membered bicyclic nonaromatic heterocyclic group.

**6.** The PDE7 inhibitor according to claim 5, wherein $X^{1a}$, $X^{1b}$, $X^{1c}$, and $X^{1d}$ each represent N.

**7.** The PDE7 inhibitor according to claim 6, wherein
$Z^{1a}$, $Z^{1b}$, and $Z^{1c}$ each represent N; and
$Z^{1d}$ represents $NR^{Z1d}$.

**8.** The PDE7 inhibitor according to claim 7, wherein

$Z^{2a}$, $Z^{2b}$, and $Z^{2d}$ each represent N; and
$Z^{2c}$ represents $NR^{Z2c}$.

**9.** The PDE7 inhibitor according to claim 8, wherein $X^{3a}$, $X^{3b}$, $X^{3c}$, and $X^{3d}$ each represent N.

**10.** A compound represented by the following formula (II):

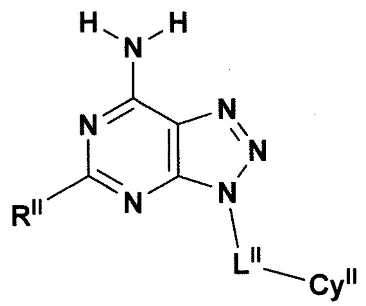

**(II)**

[wherein:

$R^{II}$ represents a hydrogen atom, an alkyl group optionally substituted with the same or different 1 to 7 halogen atom(s), an alkoxy group optionally substituted with the same or different 1 to 7 halogen atom(s), or an alkylthio group optionally substituted with the same or different 1 to 7 halogen atom(s);
$L^{II}$ represents a single bond or $CR^{LII-1}R^{LII-2}$ ;
$R^{LII-1}$ and $R^{LII-2}$ each independently represent a hydrogen atom or an alkyl group optionally substituted with the same or different 1 to 7 halogen atom(s), or $R^{LII-1}$ and $R^{LII-2}$ each independently represent an alkylene group and are combined with each other together with the carbon atom to which they are attached to form a monocyclic saturated hydrocarbon group optionally substituted with the same or different 1 to 6 halogen atom(s); and
$Cy^{II}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s)
(provided that said aryl group is not a phenyl group);
(ii) a heteroaryl group optionally substituted with the same or different 1 to 5 substituent(s) selected from an alkyl group optionally substituted with the same or different 1 to 7 halogen atom(s) and a halogen atom (provided that said heteroaryl group is not a furyl group);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group optionally substituted with the same or different 1, 2, or 3 substituent(s) selected from an alkyl group optionally substituted with the same or different 1 to 7 halogen atom(s) and a halogen atom;
an alkenyl group optionally substituted with the same or different 1 to 5 halogen atom(s);
an alkylidene group optionally substituted with the same or different 1 to 6 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s); and
a heteroaryl group optionally substituted with the same or different 1, 2, or 3 substituent(s) selected from an alkyl group optionally substituted with the same or different 1 to 7 halogen atom(s) and a halogen atom (provided that said alicyclic hydrocarbon group is not a cyclobutyl group, a cyclopentyl group, a cyclopentenyl group, or a 2-cyclohexenyl group); or

(iv) a nonaromatic heterocyclic group optionally substituted with the same or different 1 to 5 substituent(s) selected from
an alkyl group optionally substituted with the same or different 1, 2, or 3 substituent(s) selected from an alkoxy group optionally substituted with the same or different 1 to 7 halogen atom(s), a halogen atom, and an aryl group optionally substituted with the same or different 1, 2, or 3 substituent(s) selected from an alkyl group optionally substituted with the same or different 1 to 7 halogen atom(s) and a halogen atom;
a cycloalkyl group optionally substituted with the same or different 1 to 5 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s);
a heteroaryl group optionally substituted with the same or different 1 to 5 halogen atom(s);
an alkylcarbonyl group optionally substituted with the same or different 1, 2, or 3 aryl group(s);
a formyl group;
an alkoxycarbonyl group optionally substituted with the same or different 1 to 7 halogen atom(s); and
an arylcarbonyl group optionally substituted with the same or different 1 to 5 halogen atom(s)
(provided that said nonaromatic heterocyclic group is not a tetrahydrofuryl group, a dihydrofuran-2-yl group, a tetrahydropyran-2-yl group, a pyrrolidin-3-yl group, a morpholin-2-yl group, or a thiolan-2-yl group) (provided that

(a) $Cy^{II}$ is not a cyclopropyl group or a 2,2-dimethyl-1,3-dioxolanyl group; and
(b) the above compound is not 3-cyclohexyl-3H-[1,2,3]triazolo[4,5-d]pyrimidin-7-amine, 2-[(7-amino-3H-1,2,3-triazolo[4,5-d]pyrimidin-3-yl)methyl]-1-azabicyclo[2.2.2]octan-3-one, 2-(7-amino-3H-1,2,3-triazolo[4,5-d]pyrimidin-3-yl)cyclohexanemethanol, or 4-(7-amino-3H-1,2,3-triazolo[4,5-d]pyrimidin-3-yl)-2-hydroxy-bicyclo[3.1.0]hexane-1-methanol)]

or a pharmaceutically acceptable salt thereof.

**11.** The compound according to claim 10 or a pharmaceutically acceptable salt thereof, wherein
$R^{II}$ represents a hydrogen atom, an alkyl group optionally substituted with the same or different 1 to 7 halogen atom(s), an alkoxy group, or an alkylthio group;
$L^{II}$ represents a single bond or $CR^{LII-1} R^{LII-2}$;
$R^{LII-1}$ and $R^{LII-2}$ each independently represent a hydrogen atom or an alkyl group, or $R^{LII-1}$ and $R^{LII-2}$ each independently represent a straight alkylene group and are combined with each other together with the carbon atom to which they are attached to form a monocyclic saturated hydrocarbon group; and
$Cy^{II}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a 6 to 11 membered monocyclic or bicyclic aromatic hydrocarbon group;
(ii) a heteroaryl group optionally substituted with the same or different 1 to 5 halogen atom(s), wherein said heteroaryl group is a 5 to 11 membered monocyclic or bicyclic aromatic heterocyclic group comprising 1 to 4 heteroatom(s) selected from an oxygen atom, a sulfur atom, and a nitrogen atom other than carbon atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),

wherein said alicyclic hydrocarbon group is a $C_3$-$C_8$ cycloalkyl group, a $C_6$-$C_{12}$ bicycloalkyl group, a $C_6$-$C_{12}$ bicycloalkenyl group, a $C_6$-$C_{12}$ spiroalkyl group, or a $C_{10}$-$C_{14}$ tricyclic tricycloalkyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a 4 to 8 membered monocyclic nonaromatic heterocyclic group or a 6 to 12 membered bicyclic nonaromatic heterocyclic group.

**12.** The compound according to claim 11 or a pharmaceutically acceptable salt thereof, wherein
$L^{II}$ represents a single bond; and
$Cy^{II}$ represents

(i) a naphthyl group or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a piperidinyl group, a piperidino group, a perhydroazepinyl group, a perhydroazocinyl group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

**13.** A compound represented by the following formula (III):

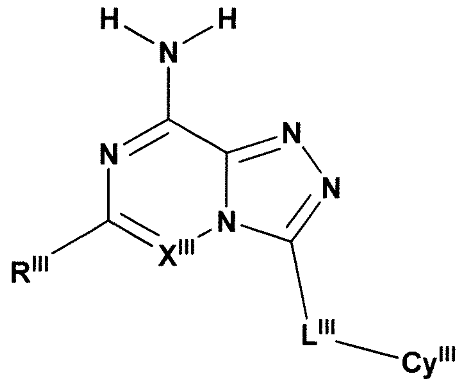

(III)

[wherein:

$X^{III}$ is $CR^{XIII}$ or N;
$R^{III}$ represents a hydrogen atom, an alkyl group optionally substituted with the same or different 1 to 7 halogen atom(s), an alkoxy group optionally substituted with the same or different 1 to 7 halogen atom(s), or an alkylthio group optionally substituted with the same or different 1 to 7 halogen atom(s);
$R^{XIII}$ represents a hydrogen atom, an alkyl group optionally substituted with the same or different 1 to 7 halogen atom(s), a cycloalkyl group optionally substituted with the same or different 1 to 5 halogen atom(s), a halogen atom, a cyano group, or an aryl group optionally substituted with the same or different 1 to 5 halogen atom(s);
$L^{III}$ represents a single bond or $CR^{LIII-1}$ $R^{LIII-2}$;
$R^{LIII-1}$ and $R^{LIII-2}$ each independently represent a hydrogen atom or an alkyl group optionally substituted with the same or different 1 to 7 halogen atom(s), or $R^{LIII-1}$ and $R^{LIII-2}$ each independently represent an alkylene group and are combined with each other together with the carbon atom to which they are attached to form a monocyclic saturated hydrocarbon group optionally substituted with the same or different 1 to 6 halogen atom(s); and
$Cy^{III}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 substituent(s) selected from a halogen atom and an aryl group;
a halogen atom; and
a carboxamide group optionally substituted with the same or different 1 or 2 alkyl group(s) optionally substituted with the same or different 1, 2, or 3 aryl group(s);
(ii) a heteroaryl group optionally substituted with the same or different 1 to 5 substituent(s) selected from an alkyl group optionally substituted with the same or different 1 to 7 halogen atom(s) and a halogen atom;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, an arylalkyloxy group, and an aryl group optionally substituted with the same or different 1, 2, or 3 substituent(s) selected from an alkyl group optionally substituted with the same or different 1 to 7 halogen atom(s) and a halogen atom;
an alkenyl group optionally substituted with the same or different 1 to 5 halogen atom(s);
an alkylidene group optionally substituted with the same or different 1 to 6 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s); and
a heteroaryl group optionally substituted with the same or different 1, 2, or 3 substituent(s) selected from an alkyl group optionally substituted with the same or different 1 to 7 halogen atom(s) and a halogen atom; or
(iv) a nonaromatic heterocyclic group optionally substituted with the same or different 1 to 5 substituent(s) selected from
an alkyl group optionally substituted with the same or different 1, 2, or 3 substituent(s) selected from an

alkoxy group optionally substituted with the same or different 1 to 7 halogen atom(s), a halogen atom, and an aryl group optionally substituted with the same or different 1, 2, or 3 substituent(s) selected from an alkyl group optionally substituted with the same or different 1 to 7 halogen atom(s) and a halogen atom;
a cycloalkyl group optionally substituted with the same or different 1 to 5 halogen atom(s);
an alkoxy group optionally substituted with the same or different 1 to 7 halogen atom(s);
a hydroxy group;
a halogen atom;
an oxo group;
an aryl group optionally substituted with the same or different 1 to 5 halogen atom(s);
a heteroaryl group optionally substituted with the same or different 1 to 5 halogen atom(s);
an alkylcarbonyl group optionally substituted with the same or different 1, 2, or 3 aryl group(s);
a formyl group;
an alkoxycarbonyl group optionally substituted with the same or different 1 to 7 halogen atom(s); and
an arylcarbonyl group optionally substituted with the same or different 1 to 5 halogen atom(s)
(provided that said nonaromatic heterocyclic group is not a tetrahydrofuryl group)
(provided that

(a) when $X^{III}$ is CH, and $Cy^{III}$ is a phenyl group optionally substituted with the same or different 1 or 2 halogen atom(s), then $R^{III}$ is not a hydrogen atom; and
(b) the above compound is not 3-cyclopropyl[1,2,4]triazolo[4,3-a]pyrazin-8-amine)]

or a pharmaceutically acceptable salt thereof.

**14.** The compound according to claim 13 or a pharmaceutically acceptable salt thereof, wherein
$R^{III}$ represents a hydrogen atom, an alkyl group optionally substituted with the same or different 1 to 7 halogen atom(s), an alkoxy group, or an alkylthio group;
$R^{XIII}$ represents a hydrogen atom, an alkyl group optionally substituted with the same or different 1 to 7 halogen atom(s), a cycloalkyl group, a halogen atom, a cyano group, or an aryl group;
$L^{III}$ represents a single bond or $CR^{LIII-1}R^{LIII-2}$; $R^{LIII-1}$ and $R^{LIII-2}$ each independently represent a hydrogen atom or an alkyl group, or $R^{LIII-1}$ and $R^{LIII-2}$ each independently represent a straight alkylene group and are combined with each other together with the carbon atom to which they are attached to form a monocyclic saturated hydrocarbon group; and
$Cy^{III}$ represents

(i) an aryl group optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s),
wherein said aryl group is a 6 to 11 membered monocyclic or bicyclic aromatic hydrocarbon group;
(ii) a heteroaryl group optionally substituted with the same or different 1 to 5 halogen atom(s), wherein said heteroaryl group is a 5 to 11 membered monocyclic or bicyclic aromatic heterocyclic group comprising 1 to 4 heteroatom(s) selected from an oxygen atom, a sulfur atom, and a nitrogen atom other than carbon atom(s);
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a $C_3$-$C_8$ cycloalkyl group, a $C_6$-$C_{12}$ bicycloalkyl group, a $C_6$-$C_{12}$ bicycloalkenyl group, a $C_6$-$C_{12}$ spiroalkyl group, or a $C_{10}$-$C_{14}$ tricyclic tricycloalkyl group; or
(iv) a nonaromatic heterocyclic group optionally substituted with the same or different 1 to 5 substituent(s) selected from

an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a 4 to 8 membered monocyclic nonaromatic heterocyclic group or a 6 to 12 membered bicyclic nonaromatic heterocyclic group.

**15.** The compound according to claim 14 or a pharmaceutically acceptable salt thereof, wherein
$L^{III}$ represents a single bond; and
$Cy^{III}$ represents

(i) a phenyl group, a naphthyl group, or a tetrahydronaphthyl group, each of which is optionally substituted with the same or different 1 to 5 substituent(s) selected from
an alkyl group optionally substituted with the same or different 1 to 7 halogen atom(s);
an alkoxy group optionally substituted with the same or different 1, 2, or 3 aryl group(s);
a halogen atom; and
a carboxamide group optionally substituted with the same or different 1 or 2 alkyl group(s) optionally substituted with the same or different 1, 2, or 3 aryl group(s);
(ii) a tetrahydroindazolyl group;
(iii) an alicyclic hydrocarbon group optionally substituted with the same or different 1 to 5 substituent(s) selected from
an alkyl group optionally substituted with the same or different 1, 2, or 3 substituent(s) selected from a halogen atom, a hydroxy group, an aryloxy group, and an arylalkyloxy group;
an alkenyl group;
an alkylidene group;
an alkoxy group;
a hydroxy group;
a halogen atom; and
a heteroaryl group optionally substituted with the same or different 1, 2, or 3 alkyl group(s),
wherein said alicyclic hydrocarbon group is a cyclobutyl group, a cyclopentyl group, a cyclohexyl group, a cycloheptyl group, a bicyclo[3.1.0]hexyl group, a bicyclo[3.1.0]hexenyl group, a bicyclo[2.2.1]heptyl group, a bicyclo[4.1.0]heptyl group, a spiro[2.3]hexyl group, a spiro[2.5]octyl group, or an adamantyl group; or
(iv) a nonaromatic heterocyclic group optionally substituted with the same or different 1 to 5 substituent(s) selected from
an alkyl group optionally substituted with the same or different 1, 2, or 3 substituent(s) selected from a halogen atom and an aryl group;
a halogen atom;
an aryl group;
a heteroaryl group; and
an alkoxycarbonyl group,
wherein said nonaromatic heterocyclic group is a pyrrolidinyl group, a piperidinyl group, a piperidino group, a perhydroazepinyl group, a perhydroazocinyl group, a morpholinyl group, a morpholino group, a tetrahydropyranyl group, an azabicyclo[3.1.0]hexyl group, an azabicyclo[2.2.1]heptyl group, an azabicyclo[3.2.1]octyl group, an azabicyclo[2.2.2]octyl group, an azaspiro[2.5]octyl group, or an azaspiro[4.5]decyl group.

**16.** The compound according to any one of claims 13 to 15 or a pharmaceutically acceptable salt thereof, wherein $X^{III}$ represents $CR^{XIII}$.

**17.** The compound according to any one of claims 13 to 15 or a pharmaceutically acceptable salt thereof, wherein $X^{III}$ represents N.

**18.** A compound selected from
3-(cis-2-methylcyclohexyl)-3H-[1,2,3]triazolo[4,5-d]pyrimidin-7-amine;
3-(trans-2-methylcyclohexyl)-3H-[1,2,3]triazolo[4,5-d]pyrimidin-7-amine;
3-(cis-2-fluorocyclohexyl)-3H-[1,2,3]triazolo[4,5-d]pyrimidin-7-amine;
3-(2,2-difluorocyclohexyl)-3H-[1,2,3]triazolo[4,5-d]pyrimidin-7-amine;

3-(cis-3-methylcyclohexyl)-3H-[1,2,3]triazolo[4,5-d]pyrimidin-7-amine;
3-(trans-3-methylcyclohexyl)-3H-[1,2,3]triazolo[4,5-d]pyrimidin-7-amine;
3-(3,3-dimethylcyclohexyl)-3H-[1,2,3]triazolo[4,5-d]pyrimidin-7-amine;
3-[cis-3-(trifluoromethyl)cyclohexyl]-3H-[1,2,3]triazolo[4,5-d]pyrimidin-7-amine;
3-(cis-4-methylcyclohexyl)-3H-[1,2,3]triazolo[4,5-d]pyrimidin-7-amine;
3-(trans-4-methylcyclohexyl)-3H-[1,2,3]triazolo[4,5-d]pyrimidin-7-amine;
3-(4,4-dimethylcyclohexyl)-3H-[1,2,3]triazolo[4,5-d]pyrimidin-7-amine;
3-(trans-3,3,5-trimethylcyclohexyl)-3H-[1,2,3]triazolo[4,5-d]pyrimidin-7-amine; and
3-cycloheptyl-3H-[1,2,3]triazolo[4,5-d]pyrimidin-7-amine
or a pharmaceutically acceptable salt thereof.

**19.** A compound selected from
3-cyclohexyl[1,2,4]triazolo[4,3-a]pyrazin-8-amine;
3-(1-fluorocyclohexyl)[1,2,4]triazolo[4,3-a]pyrazin-8-amine;
3-(cis-3-methylcyclohexyl)[1,2,4]triazolo[4,3-a]pyrazin-8-amine;
3-(trans-3-methylcyclohexyl)[1,2,4]triazolo[4,3-a]pyrazin-8-amine;
3-(3,3-dimethylcyclohexyl)[1,2,4]triazolo[4,3-a]pyrazin-8-amine;
3-(spiro[2,5]oct-5-yl)[1,2,4]triazolo[4,3-a]pyrazin-8-amine;
3-[cis-3-(trifluoromethyl)cyclohexyl][1,2,4]triazolo[4,3-a]pyrazin-8-amine;
3-(3,3-difluorocyclohexyl)[1,2,4]triazolo[4,3-a]pyrazin-8-amine;
3-(trans-4-methylcyclohexyl)[1,2,4]triazolo[4,3-a]pyrazin-8-amine;
3-[2-methyl-5-(trifluoromethyl)cyclohexyl][1,2,4]triazolo[4,3-a]pyrazin-8-amine;
3-(cis-5,5-difluoro-2-methylcyclohexyl)[1,2,4]triazolo[4,3-a]pyrazin-8-amine;
3-(trans-3,3-difluoro-5-methylcyclohexyl[1,2,4]triazolo[4,3-a]pyrazin-8-amine;
3-(3,3-difluoro-5,5-dimethylcyclohexyl)[1,2,4]triazolo[4,3-a]pyrazin-8-amine;
3-[cis-2,2-difluoro-5-(trifluoromethyl)cyclohexyl][1,2,4]triazolo[4,3-a]pyrazin-8-amine;
3-(bicyclo[4.1.0]hept-3-yl) [1,2,4]triazolo[4,3-a]pyrazin-8-amine;
3-[(1R,6S,7r)-bicyclo[4.1.0]hept-7-yl][1,2,4]triazolo[4,3-a]pyrazin-8-amine;
3-(2-methylpiperidin-1-yl)[1,2,4]triazolo[4,3-a]pyrazin-8-amine;
3-(2-ethylpiperidin-1-yl)[1,2,4]triazolo[4,3-a]pyrazin-8-amine;
3-(3,3-dimethylpiperidin-1-yl)[1,2,4]triazolo[4,3-a]pyrazin-8-amine;
3-(3,3-dimethylpiperidin-1-yl)-5-methyl[1,2,4]triazolo[4,3-a]pyrazin-8-amine;
3-(3,3-dimethylpiperidin-1-yl)-5-ethyl[1,2,4]triazolo[4,3-a]pyrazin-8-amine;
5-cyclopropyl-3-(3,3-dimethylpiperidin-1-yl)[1,2,4]triazolo[4,3-a]pyrazin-8-amine;
3-(3,3-dimethylpiperidin-1-yl)-5-(trifluoromethyl)[1,2,4]triazolo[4,3-a]pyrazin-8-amine;
5-chloro-3-(3,3-dimethylpiperidin-1-yl) [1,2,4]triazolo[4,3-a]pyrazin-8-amine;
8-amino-3-(3,3-dimethylpiperidin-1-yl)[1,2,4]triazolo[4,3-a]pyrazine-5-carbonitrile;
3-[trans-3,5-dimethylpiperidin-1-yl][1,2,4]triazolo[4,3-a]pyrazin-8-amine;
8-amino-3-(3,5-dimethylpiperidin-1-yl)[1,2,4]triazolo[4,3-a]pyrazine-5-carbonitrile;
3-(3,4-dimethylpiperidin-1-yl)[1,2,4]triazolo[4,3-a]pyrazin-8-amine;
3-(2,3-dimethylpiperidin-1-yl)[1,2,4]triazolo[4,3-a]pyrazin-8-amine;
3-(2,5-dimethylpiperidin-1-yl)[1,2,4]triazolo[4,3-a]pyrazin-8-amine;
8-amino-3-(2,5-dimethylpiperidin-1-yl) [1,2,4]triazolo[4,3-a]pyrazine-5-carbonitrile;
3-(2,4-dimethylpiperidin-1-yl)[1,2,4]triazolo[4,3-a]pyrazin-8-amine;
3-(2,5,5-trimethylpiperidin-1-yl)[1,2,4]triazolo[4,3-a]pyrazin-8-amine;
3-cyclohexyl[1,2,4]triazolo[3,4-f][1,2,4]triazin-8-amine;
3-(cis-2-methylcyclohexyl)[1,2,4]triazolo[3,4-f][1,2,4]triazin-8-amine;
3-(trans-2-methylcyclohexyl)[1,2,4]triazolo[3,4-f][1,2,4]triazin-8-amine;
3-(cis-3-methylcyclohexyl)[1,2,4]triazolo[3,4-f][1,2,4]triazin-8-amine;
3-(trans-3-methylcyclohexyl)[1,2,4]triazolo[3,4-f] [1,2,4]triazin-8-amine;
3-(3,3-dimethylcyclohexyl)[1,2,4]triazolo[3,4-f][1,2,4]triazin-8-amine;
3-[cis-3-(trifluoromethyl)cyclohexyl][1,2,4]triazolo[3,4-f][1,2,4]triazin-8-amine;
3-[trans-3-(trifluoromethyl)cyclohexyl][1,2,4]triazolo[3,4-f][1,2,4]triazin-8-amine;
3-(3,3-difluorocyclohexyl)[1,2,4]triazolo[3,4-f][1,2,4]triazin-8-amine;
3-(cis-5,5-difluoro-2-methylcyclohexyl)[1,2,4]triazolo[3,4-f][1,2,4]triazin-8-amine; and
3-[2-methyl-5-(trifluoromethyl)cyclohexyl][1,2,4]triazolo[3,4-f][1,2,4] triazin-8-amine
or a pharmaceutically acceptable salt thereof.

20. A pharmaceutical composition comprising the compound according to any one of claims 10 to 19 or a pharmaceutically acceptable salt thereof as an active ingredient.

21. Use of the PDE7 inhibitor according to any one of claims 1 to 9 or the compound according to any one of claims 10 to 19 or a pharmaceutically acceptable salt thereof in the manufacture of a medicament for the treatment or prevention of a disease which is improved by inhibiting PDE7.

22. The PDE7 inhibitor according to any one of claims 1 to 9 for the treatment or prevention of a disease which is improved by inhibiting PDE7.

23. The compound according to any one of claims 10 to 19 or a pharmaceutically acceptable salt thereof for the treatment or prevention of a disease which is improved by inhibiting PDE7.

24. A method for treating or preventing a disease which is improved by inhibiting PDE7 comprising administering to a patient an effective amount of the PDE7 inhibitor according to any one of claims 1 to 9 or the compound according to any one of claims 10 to 19 or a pharmaceutically acceptable salt thereof.

## INTERNATIONAL SEARCH REPORT

International application No.
PCT/JP2017/030609

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2002-531567 A (Astra Zeneca AB.), 24 September 2002 (24.09.2002), paragraph [0094] & US 6525060 B1 example 3g) & WO 2000/034283 A1 & EP 1386909 A1 & KR 10-2006-0091056 A & CN 1334816 A | 10,11 |
| X | CHEN, Xiao-Bao et al., Synthesis and Biological Activity of 3-[(6-Chloropyridein-3-yl)methyl]-6-substituted-6,7-dihydro-3H-1,2,3-triazolo[4,5-d]-pyrimidin-7-imines, Journal of Heterocyclic Chemistry, Vol.45, No.5, 2008, p.1493-1497 Compd.4q, Table1 | 10,11,20 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 21 September 2017 (21.09.17) | 03 October 2017 (03.10.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/030609

C (Continuation). DOCUMENTS CONSIDERED TO BE RELEVANT

| Category* | Citation of document, with indication, where appropriate, of the relevant passages | Relevant to claim No. |
|---|---|---|
| A | SAFAVI, Maliheh et al., New methods for the discovery and synthesis of PDE7 inhibitors as new drugs for neurological and inflammatory disorders, Expert Opinion on Drug Discovery, 2013, Vol.8, No.6, p.733-751, entire text | 1-24 |
| A | JP 2008-501618 A (Asubio Pharma Co., Ltd.), 24 January 2008 (24.01.2008), entire text & US 2006/0128728 A1 entire text & WO 2004/111054 A1 & EP 1636235 A1 | 1-24 |
| A | CONVERSO, Antonella et al., Adenosine analogue inhibitors of S-adenosylhomocysteine hydrolase, Bioorganic & Medicinal Chemistry Letters, 2014, Vol.24, No.12, p.2737-2740 entire text, particularly, page 2740, left column, the last paragraph | 1-24 |
| A | NANDHAKUMAR, J. et al., Evaluation of seizure activity after phospho-diesterase and adenylate cyclase inhibition(SQ22536) in animal models of epilepsy, Indian Journal of Science and Technology, 2010, Vol.3, No.7, p.710-717, page 715, right column, the last paragraph | 1-24 |
| P,A | JANKOWSKA, Agnieszka et al., PDE7-Selective and Dual Inhibitors: Advances in Chemical and Biological Research, Current Medicinal Chemistry, 2017.05, Vol.24, No.7, p.673-700, entire text | 1-24 |

Form PCT/ISA/210 (continuation of second sheet) (January 2015)

**INTERNATIONAL SEARCH REPORT**

International application No.
PCT/JP2017/030609

Continuation of A. CLASSIFICATION OF SUBJECT MATTER
(International Patent Classification (IPC))

*A61K31/437*(2006.01)i, *A61K31/4985*(2006.01)i, *A61K31/519*(2006.01)i, *A61K31/52*(2006.01)i, *A61K31/53*(2006.01)i, *A61K31/55*(2006.01)i, *A61P1/04*(2006.01)i, *A61P9/10*(2006.01)i, *A61P11/02*(2006.01)i, *A61P11/06*(2006.01)i, *A61P17/00*(2006.01)i, *A61P17/06*(2006.01)i, *A61P19/00*(2006.01)i, *A61P19/02*(2006.01)i, *A61P21/02*(2006.01)i, *A61P25/00*(2006.01)i, *A61P25/04*(2006.01)i, *A61P25/06*(2006.01)i, *A61P25/08*(2006.01)i, *A61P25/14*(2006.01)i, *A61P25/16*(2006.01)i, *A61P25/20*(2006.01)i, *A61P25/22*(2006.01)i, *A61P25/24*(2006.01)i, *A61P25/28*(2006.01)i, *A61P25/30*(2006.01)i, *A61P25/32*(2006.01)i, *A61P25/34*(2006.01)i, *A61P25/36*(2006.01)i, *A61P29/00*(2006.01)i, *A61P35/00*(2006.01)i, *A61P35/02*(2006.01)i, *A61P37/02*(2006.01)i, *A61P37/08*(2006.01)i, *A61P43/00*(2006.01)i, *C07D487/04*(2006.01)i, *C07D519/00*(2006.01)i

(According to International Patent Classification (IPC) or to both national classification and IPC)

Continuation of B. FIELDS SEARCHED
Minimum documentation searched (International Patent Classification (IPC))

A61K31/437, A61K31/4985, A61K31/519, A61K31/52, A61K31/53, A61K31/55, A61P1/04, A61P9/10, A61P11/02, A61P11/06, A61P17/00, A61P17/06, A61P19/00, A61P19/02, A61P21/02, A61P25/00, A61P25/04, A61P25/06, A61P25/08, A61P25/14, A61P25/16, A61P25/20, A61P25/22, A61P25/24, A61P25/28, A61P25/30, A61P25/32, A61P25/34, A61P25/36, A61P29/00, A61P35/00, A61P35/02, A61P37/02, A61P37/08, A61P43/00, C07D487/04, C07D519/00

Minimum documentation searched (classification system followed by classification symbols)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013176877 A
- JP S52122395 A


### Non-patent literature cited in the description


- *Neuropsychopharmacology,* 2011, vol. 36, 1178-1186
- *Seisan to Gijutu (Manufacturing & Technology),* 2014, vol. 66 (2), 80-83
- *Biochemical and Biophysical Research Communication),* 2000, vol. 271, 575-583
- *Brain Research,* 2010, vol. 1310, 37-45
- *PLOS ONE,* 2014, vol. 9 (9), e0107397
- *British Journal of Pharmacology,* 2008, vol. 155, 308-315
- *Neuropharmacology,* 2014, vol. 77, 257-267
- **T. W. GREENE et al.** Protective Groups in Organic Synthesis. John Wiley & Sons, 2006
- *Tetrahedron,* 2012, vol. 68 (15), 3172-3178
- *Biochemical and Biophysical Research Communication,* 2000, vol. 271, 575-583